(19)

**Europäisches Patentamt**
**European Patent Office**
**Office européen des brevets**

(11) **EP 4 538 971 A2**

(12) **EUROPEAN PATENT APPLICATION**

(43) Date of publication:
16.04.2025 Bulletin 2025/16

(21) Application number: 25153143.0

(22) Date of filing: 18.11.2020

(51) International Patent Classification (IPC):
**G06T 7/194** *(2017.01)*

(52) Cooperative Patent Classification (CPC):
**G06T 7/33; G06T 7/11; G06T 7/136; G06T 7/155; G06T 7/194; G06V 20/695;** G06T 2207/10056; G06T 2207/10064; G06T 2207/30024; G06T 2207/30072; G06T 2207/30204

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 21.11.2019 US 201962938336 P
21.02.2020 US 202062980073 P
20.06.2020 US 202063041825 P

(62) Document number(s) of the earlier application(s) in accordance with Art. 76 EPC:
**20824793.2 / 4 062 373**

(71) Applicant: **10x Genomics, Inc.**
**Pleasanton, CA 94588-3260 (US)**

(72) Inventors:
• **YIN, Yifeng**
**Pleasanton, CA 94588-3260 (US)**
• **BENT, Zachary**
**Pleasanton, CA 94588-3260 (US)**
• **WILLIAMS, Stephen**
**Pleasanton, CA 94588-3260 (US)**
• **FIDDES, Ian**
**Pleasanton, CA 94588-3260 (US)**
• **MELLEN, Jeffrey Clark**
**Pleasanton, CA 94588-3260 (US)**
• **STAAB, Jasper**
**Pleasanton, CA 94588-3260 (US)**
• **WU, Kevin J.**
**Pleasanton, CA 94588-3260 (US)**

• **WEISENFELD, Neil Ira**
**Pleasanton, CA 94588-3260 (US)**
• **BAUMGARTNER, Florian**
**Pleasanton, CA 94588-3260 (US)**
• **CLAYPOOLE, Brynn**
**Pleasanton, CA 94588-3260 (US)**
• **SHAH, Preyas**
**Pleasanton, CA 94588-3260 (US)**
• **DSHKHUNYAN, Narek**
**Pleasanton, CA 94588-3260 (US)**
• **BORGSTROM, Erik Leonard Henrik**
**Pleasanton, CA 94588-3260 (US)**
• **MCCREATH, Benjamin**
**Pleasanton, CA 94588-3260 (US)**

(74) Representative: **Grund, Martin et al**
**Grund Intellectual Property Group**
**Patentanwälte und Solicitor PartG mbB**
**Steinsdorfstraße 2**
**80538 München (DE)**

Remarks:
•The complete document including Reference Table(s) and the Sequence Listing(s) can be downloaded from the EPO website
•This application was filed on 21.01.2025 as a divisional application to the application mentioned under INID code 62.
•Claims filed after the date of filing of the application / after the date of receipt of the divisional application (Rule 68(4) EPC).

(54) **SPATIAL ANALYSIS OF ANALYTES**

(57) Systems and methods for spatial analysis of analytes include placing a sample on a substrate having fiducial markers and capture spots. Then, an image of the sample is acquired and sequence reads are obtained from the capture spots. Each capture probe plurality in a set of capture probe pluralities is (i) at a different capture spot, (ii) directly or indirectly associates with analytes from the sample and (iii) has a unique spatial barcode. The sequencing reads serve to detect the analytes. Sequencing reads include a spatial barcode of the corresponding capture probe plurality. Spatial barcodes localize reads to corresponding capture spots, thereby dividing them into subsets, each subset for a respective capture spot. Fiducial markers facilitate a composite representation comprising (i) the image aligned to the capture spots and (ii) a representation of each subset of sequence reads at respective positions within the image mapping to the corresponding capture spots.

**EP 4 538 971 A2**

1124

1204

Figure 14

## Description

### CROSS REFERENCE TO RELATED APPLICATIONS

This application claims priority to U.S. Provisional Patent Application No. 63/041,825, entitled "Pipeline for Spatial Analysis of Analytes," filed June 20, 2020, United States Provisional Patent Application No. 62/980,073, entitled "Pipeline for Analysis of Analytes," filed February 21, 2020, and United States Provisional Patent Application No. 62/938,336, entitled "Pipeline for Analysis of Analytes," filed November 21, 2019, each of which is hereby incorporated by reference in its entirety.

### TECHNICAL FIELD

This specification describes technologies relating to processing observed analyte data in large, complex datasets, such as spatially arranged next generation sequencing data, and using the data to visualize patterns.

### BACKGROUND

Spatial resolution of analytes in complex tissues provides new insights into the processes underlying biological function and morphology, such as cell fate and development, disease progression and detection, and cellular and tissue-level regulatory networks. *See*, Satija et al., 2015, "Spatial reconstruction of single-cell gene expression data," Nature Biotechnology. 33, 495-502, doi:10.1038.nbt.3192 and Achim et al., 2015, "High-throughput spatial mapping of single-cell RNA-seq data to tissue of origin," Nature Biotechnology 33: 503-509, doi:10.1038/nbt.3209, each of which is hereby incorporated herein by reference in its entirety. An understanding of the spatial patterns or other forms of relationships between analytes can provide information on differential cell behavior. This, in turn, can help to elucidate complex conditions such as complex diseases. For example, the determination that the abundance of an analyte (*e.g.*, a gene) is associated with a tissue subpopulation of a particular tissue class (*e.g.*, disease tissue, healthy tissue, the boundary of disease and healthy tissue, *etc.*) provides inferential evidence of the association of the analyte with a condition such as complex disease. Likewise, the determination that the abundance of an analyte is associated with a particular subpopulation of a heterogeneous cell population in a complex 2-dimensional or 3-dimensional tissue (*e.g.*, a mammalian brain, liver, kidney, heart, a tumor, or a developing embryo of a model organism) provides inferential evidence of the association of the analyte in the particular subpopulation.

Thus, spatial analysis of analytes can provide information for the early detection of disease by identifying at-risk regions in complex tissues and characterizing the analyte profiles present in these regions through spatial reconstruction (*e.g.*, of gene expression, protein expression, DNA methylation, and/or single nucleotide polymorphisms, among others). A high-resolution spatial mapping of analytes to their specific location within a region or subregion reveals spatial expression patterns of analytes, provides relational data, and further implicates analyte network interactions relating to disease or other morphologies or phenotypes of interest, resulting in a holistic understanding of cells in their morphological context. See, 10X, 2019, "Spatially-Resolved Transcriptomics," 10X, 2019, "Inside Visium Spatial Technology," and 10X, 2019, "Visium Spatial Gene Expression Solution," each of which is hereby incorporated herein by reference in its entirety.

Spatial analysis of analytes can be performed by capturing analytes and/or analyte capture agents or analyte binding domains and mapping them to known locations (*e.g.*, using barcoded capture probes attached to a substrate) using a reference image indicating the tissues or regions of interest that correspond to the known locations. For example, in some implementations of spatial analysis, a sample is prepared (*e.g.*, fresh-frozen tissue is sectioned, placed onto a slide, fixed, and/or stained for imaging). The imaging of the sample provides the reference image to be used for spatial analysis. Analyte detection is then performed using, *e.g.*, analyte or analye ligand capture via barcoded capture probes, library construction, and/or sequencing. The resulting barcoded analyte data and the reference image can be combined during data visualization for spatial analysis. See, 10X, 2019, "Inside Visium Spatial Technology."

One difficulty with such analysis is ensuring that a sample or an image of a sample (*e.g.*, a tissue section or an image of a tissue section) is properly aligned with the barcoded capture probes (*e.g.*, using fiducial alignment). Technical limitations in the field are further compounded by the frequent introduction of imperfections in sample quality during conventional wet-lab methods for tissue sample preparation and sectioning. These issues arise either due to the nature of the tissue sample itself (including, *inter alia,* interstitial regions, vacuoles and/or general granularity that is often difficult to interpret after imaging) or from improper handling or sample degradation resulting in gaps or holes in the sample (*e.g.*, tearing samples or obtaining only a partial sample such as from a biopsy). Additionally, wet-lab methods for imaging result in further imperfections, including but not limited to air bubbles, debris, crystalline stain particles deposited on the substrate or tissue, inconsistent or poor-contrast staining, and/or microscopy limitations that produce image blur, over- or under-exposure, and/or poor resolution. See, Uchida, 2013, "Image processing and recognition for biological images," Develop. Growth Differ. 55, 523-549, doi:10.1111/dgd.12054, which is hereby incorporated herein by reference in its entirety. Such imperfections make the alignment more difficult.

Therefore, there is a need in the art for systems and methods that provide improved spatial analyte (e.g., nucleic acid and protein) analysis. Such systems and methods would allow reproducible identification and alignment of tissue samples in images without the need for extensive training and labor costs, and would further improve the accuracy of identification by removing human error due to subjective alignment. Such systems and methods would further provide a cost-effective, user-friendly tool for a practitioner to reliably perform spatial analyte analysis.

## SUMMARY

Technical solutions (e.g., computing systems, methods, and non-transitory computer readable storage mediums) for addressing the above-identified problems are provided in the present disclosure.

The following presents a summary of the present disclosure in order to provide a basic understanding of some of the aspects of the present disclosure. This summary is not an extensive overview of the present disclosure. It is not intended to identify key/critical elements of the present disclosure or to delineate the scope of the present disclosure. Its sole purpose is to present some of the concepts of the present disclosure in a simplified form as a prelude to the more detailed description that is presented later.

One aspect of the present disclosure provides a method of spatial analysis of analytes that comprises A) placing a sample (e.g., a sectioned tissue sample), on a substrate, where the substrate includes a plurality of fiducial markers and a set of capture spots. In some embodiments the set of capture spots comprises at least 1000, 2000, 5000, 10,000, 15,000, 20,000, 25,000, 30,000, 35,000, 40,000, 45,000, 50,000, 55,000, 60,000, 65,000, 70,000, 75,000, 80,000, 85,000, 90,000, 95,000 or 100,000 capture spots. Fiducial markers do not bind to analytes, either directly or indirectly. Rather, fiducial markers serve to provide a reference frame for a substrate. In some embodiments there are more than 5, 10, 15, 20, 25, 30, 35, 40, 45, 50, 55, 60, 65, 70, 75, 80, 85, 90, 95, 100, 200, 500, or 1000 fiducial markers. In some embodiments there are less than 1000 fiducial markers.

One or more images of the biological sample on the substrate is obtained. Each of the one or more images comprises a corresponding plurality of pixels in the form of an array of pixel values. In some embodiments the array of pixel values comprises at least a least 100, 10,000, 100,000, $1 \times 10^6$, $2 \times 10^6$, $3 \times 10^6$, $5 \times 10^6$, $8 \times 10^6$, $10 \times 10^6$, or $15 \times 10^6$ pixel values. In some embodiments, the one or more images are acquired using transmission light microscopy. In some embodiments, the one or more images are acquired using fluorescent microscopy. A plurality of sequence reads, in electronic form, is obtained from the set of capture spots after the A) placing. In some embodiments the plurality of sequenc reads comprises more than 100, 1000, 50,000, 100,000, 500,000, $1 \times 10^6$, $2 \times 10^6$, $3 \times 10^6$, or $5 \times 10^6$ sequence reads. For each given image in the one or more images, each respective capture probe plurality in a set of capture probe pluralities is (i) at a different capture spot in the set of capture spots and (ii) directly or indirectly (e.g., through an analyte capture agent) associates with one or more analytes (e.g., nucleic acids, proteins, and/or metabolites, etc.) from the sectioned biological sample. In some embodiments, each respective capture probe plurality in the set of capture probe pluralities is characterized by at least one unique spatial barcode in a plurality of spatial barcodes.

In some embodiments, a substrate may have two or more capture spots that have the same spatial barcodes. That is, between the two capture spots, neither has a unique spatial barcode. In some such embodiments, these capture spots with duplicate spatial barcodes are considered to be a single capture spot. In other embodiments, capture spots that do not have a unique spatial barcode are not considered to be part of the set of capture spots that is used for localizing respective sequence reads to capture spots of a particular set of capture spots.

In some embodiments at least one percent, at least five percent, at least 10 percent, at least 20 percent, at least 30 percent, or at least 40 percent of the capture spots on a substrate may not have a unique spatial barcode across the capture spots on the substrate. That is, for each respective spatial barcode of each such capture spot, there is at least one other capture spot on the substrate that has the respective spatial barcode. In some such embodiments, these capture spots without a unique spatial barcode are not considered to be part of the set of capture spots that is used for localizing respective sequence reads to capture spots of a particular set of capture spots.

In some embodiments at least ten, at least 100, at least 1000, at least 10,000, at least 100,000, or at least 1,000,000 of the capture spots on a substrate may not have a unique spatial barbcode across the capture spots on the substrate. That is, for each respective spatial barcode of each such capture spot, there is at least one other capture spot on the substrate that has the respective spatial barcode. In some such embodiments, these capture spots without a unique spatial barcode are not considered to be part of the set of capture spots that is used for localizing respective sequence reads to capture spots of a particular set of capture spots.

The plurality of sequence reads comprises sequence reads corresponding to all or portions of the one or more analytes. Each respective sequence read in a respective plurality of sequence reads includes a spatial barcode of the corresponding capture probe plurality in the set of capture probes. The plurality of spatial barcodes is used to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots. For each respective image in the one or more images, the plurality of fiducial markers is used to provide a corresponding composite representation comprising (i) the respective image aligned to the set of capture spots on the substrate and (ii) a representation of each subset of sequence reads at the respective position within the respective image that maps to the corresponding capture spot on the substrate.

In some embodiments, the respective composite representation for an image in the one or more images provides a relative abundance of nucleic acid fragments mapping to each gene in a plurality of genes at each capture spot in the plurality of capture spots.

In some embodiments a respective image is aligned to the set of capture spots on the substrate by a procedure that comprises analyzing the array of pixel values to identify a plurality of derived fiducial spots of the respective image, using a substrate identifier uniquely associated with the substrate to select a first template in a plurality of templates, where each template in the plurality of templates comprises reference positions for a corresponding plurality of reference fiducial spots and a corresponding coordinate system, aligning the plurality of derived fiducial spots of the respective image with the corresponding plurality of reference fiducial spots of the first template using an alignment algorithm to obtain a transformation between the plurality of derived fiducial spots of the respective image and the corresponding plurality of reference fiducial spots of the first template, and using the transformation and the coordinate system of the first template to locate a corresponding position in the respective image of each capture spot in the set of capture spots.

In some embodiments, the alignment algorithm is a local alignment that aligns the respective sequence read to a reference sequence using a scoring system that (i) penalizes a mismatch between a nucleotide in the respective sequence read and a corresponding nucleotide in the reference sequence in accordance with a substitution matrix and (ii) penalizes a gap introduced into an alignment of the sequence read and the reference sequence. In some such embodiments, the local alignment is a Smith-Waterman alignment. In some such embodiments, the reference sequence is all or portion of a reference genome. In some embodiments, the one or more sequence reads that do not overlay any loci in the plurality of loci are removed from the plurality of sequence reads. In some embodiments, the plurality of sequence reads for a given image are RNA-sequence reads and the removing comprises removing one or more sequences reads in the plurality of sequence reads that overlap a splice site in the reference sequence. In some embodiments, the plurality of loci include one or more loci on a first chromosome and one or more loci on a second chromosome other than the first chromosome.

In some embodiments, the transformation and the coordinate system of the first template is used to locate and measure the one or more optical properties of each capture spot in the set of capture spots by assigning each respective pixel in the plurality of pixels to a first class or a second class. The first class indicates the biological sample on the substrate and the second class indicates background. In some embodiments, this is done be a procedure that comprise (i) using the plurality of fiducial markers to define a bounding box within the respective image, (ii) removing respective pixels falling outside the bounding box from the plurality of pixels, (iii) running, after the removing (ii), a plurality of heuristic classifiers on the plurality of pixels (*e.g.*, in grey-scale space), where, for each respective pixel in the plurality of pixels, each respective heuristic classifier in the plurality of heuristic classifiers casts a vote for the respective pixel between the first class and the second class, thereby forming a corresponding aggregated score for each respective pixel in the plurality of pixels, and (iv) applying the aggregated score and intensity of each respective pixel in the plurality of pixels to a segmentation algorithm (e.g., graph cut) to independently assign a probability to each respective pixel in the plurality of pixels of being tissue or background.

In some embodiments, each corresponding aggregated score is a class in a set of classes comprising obvious first class, likely first class, likely second class, and obvious second class.

In some embodiments, the method further comprises, for each respective locus in a plurality of loci, performing a procedure. In such embodiments, the procedure comprises performing an alignment of each respective sequence read in the plurality of sequence reads that maps to the respective locus thereby determining a haplotype identity for the respective sequence read from among a corresponding set of haplotypes for the respective locus. Each respective sequence read in the plurality of sequence reads that maps to the respective locus is categorized by the spatial barcode of the respective sequence read and by the haplotype identity thereby determining the spatial distribution of the one or more haplotypes in the biological sample. The spatial distribution includes, for each capture spot in the set of capture spots on the substrate, an abundance of each haplotype in the set of haplotypes for each loci in the plurality of loci

In some such embodiments, the method further comprises using the spatial distribution to characterize a biological condition of the subject. For instance, in some embodiments, the biological condition is absence or presence of a disease. In some embodiments, the biological condition is a type of a cancer. In some embodiments, the biological condition is a stage of a disease. In some embodiments, the biological condition is a stage of a cancer.

In some embodiments, a tissue mask is overlayed on a respective image. The tissue mask causes each respective pixel in the plurality of pixels of the respective image that has been assigned a greater probability of being tissue to be assigned a first attribute and each respective pixel in the plurality of pixels that has been assigned a greater probability of being background to be assigned a second attribute. In some embodiments, the first attribute is a first color (*e.g.*, one of red and blue) and the second attribute is a second color (*e.g.* the other of red and blue).

In some embodiments, the first attribute is a first level of brightness or opacity and the second attribute is a second level of brightness or opacity.

In some embodiments, each respective representation of a capture spot in the plurality of capture spots in the composite representation is assigned the first attribute or the second attribute based upon the assignment of pixels in the vicinity of the respective representation of the capture spot in the composite representation.

In some embodiments, a capture spot in the set of capture spots comprises a capture domain. In some embodiments, a capture spot in the set of capture spots comprises a cleavage domain. In some embodiments, each capture spot in the set of capture spots is attached directly or attached indirectly to the substrate.

In some embodiments, the one or more analytes comprise five or more analytes, ten or more analytes, fifty or more analytes, one hundred or more analytes, five hundred or more analytes, 1000 or more analytes, 2000 or more analytes, or between 2000 and 100,000 analytes.

In some embodiments, the unique spatial barcode encodes a unique predetermined value selected from the set $\{1, ..., 1024\}$, $\{1, ..., 4096\}$, $\{1, ..., 16384\}$, $\{1, ..., 65536\}$, $\{1, ..., 262144\}$, $\{1, ..., 1048576\}$, $\{1, ..., 4194304\}$, $\{1, ..., 16777216\}$, $\{1, ..., 67108864\}$, or $\{1, ..., 1 \times 10^{12}\}$.

In some embodiments, each respective capture probe plurality in the set of capture probe pluralities includes 1000 or more capture probes, 2000 or more capture probes, 10,000 or more capture probes, 100,000 or more capture probes, $1 \times 10^6$ or more capture probes, $2 \times 10^6$ or more capture probes, or $5 \times 10^6$ or more capture probes.

In some embodiments, each capture probe in the respective capture probe plurality includes a poly-A sequence or a poly-T sequence and the unique spatial barcode that characterizes the respective capture probe plurality. In some embodiments, each capture probe in the respective capture probe plurality includes the same spatial barcode from the plurality of spatial barcodes. In some embodiments, each capture probe in the respective capture probe plurality includes a different spatial barcode from the plurality of spatial barcodes.

In some embodiments, the biological sample is a sectioned tissue sample having depth of 100 microns or less. In some embodiments, each respective section in a plurality of sectioned tissues of the sample is considered a "spatial projection," and multiple co-aligned images are taken of each section.

In some embodiments, the one or more analytes is a plurality of analytes, a respective capture spot in the set of capture spots includes a plurality of capture probes, each capture probe in the plurality of capture probes includes a capture domain that is characterized by a capture domain type in a plurality of capture domain types, and each respective capture domain type in the plurality of capture domain types is configured to bind to a different analyte in the plurality of analytes. In some such embodiments, the plurality of capture domain types comprises between 5 and 15,000 capture domain types and the respective capture probe plurality includes at least five, at least 10, at least 100, or at least 1000 capture probes for each capture domain type in the plurality of capture domain types.

In some embodiments, the one or more analytes is a plurality of analytes, a respective capture spot in the set of capture spots includes a plurality of capture probes, and each capture probe in the plurality of capture probes includes a capture domain that is characterized by a single capture domain type configured to bind to each analyte in the plurality of analytes in an unbiased manner.

In some embodiments, each respective capture spot in the set of capture spots is contained within a 100 micron by 100 micron square on the substrate. In some embodiments, each respective capture spot in the set of capture spots is contained within a 50 micron by 50 micron square on the substrate. In some embodiments, each respective capture spot in the set of capture spots is contained within a 10 micron by 10 micron square on the substrate. In some embodiments, each respective capture spot in the set of capture spots is contained within a 1 micron by 1 micron square on the substrate. In some embodiments, each respective capture spot in the set of capture spots is contained within a 500 nanometer by 500 nanometer square on the substrate. In some embodiments, each respective capture spot in the set of capture spots is contained within a 300 nanometer by 300 nanometer square on the substrate. In some embodiments, each respective capture spot in the set of capture spots is contained within a 200 nanometer by 200 nanometer square on the substrate.

In some embodiments, a distance between a center of each respective capture spot to a neighboring capture spot in the set of capture spots on the substrate is between 40 microns and 300 microns. In some embodiments, a distance between a center of each respective capture spot to a neighboring capture spot in the set of capture spots on the substrate is between 300 nanometers and 5 microns, between 400 nanometers and 4 microns, between 500 nanometers and 3 microns, between 600 nanometers and 2 microns, or between 700 nanometers and 1 micron.

In some embodiments, each capture spot in the set of capture spots has a diameter of 80 microns or less. In some embodiments, each capture spot in the set of capture spots has a diameter of between 25 microns and 65 microns, between 5 microns and 50 microns, between 2 and 7 microns, or between 800 nanometers and 1.5 microns.

In some embodiments, a distance between a center of each respective capture spot to a neighboring capture spot in the set of capture spots on the substrate is between 40 microns and 100 microns, between 300 nanometers and 15 microns, between 400 nanometers and 10 microns, between 500 nanometers and 8 microns, between 600 nanometers and 6 microns, between 700 nanometers and 5 microns, or between 800 nanometers and 4 microns.

In some embodiments, the plurality of heuristic classifiers comprises a first heuristic classifier that identifies a single intensity threshold that divides the plurality of pixels into the first class and the second class, thereby causing the first heuristic classifier to cast a vote for each respective pixel in the plurality of pixels for either the first class or the second class. The single intensity threshold represents a minimization of intra-class intensity variance between the first and second class or a maximization of inter-class variance between the first class and the second class. In some embodiments, the plurality of heuristic classifiers comprises a second heuristic classifier that identifies local neighborhoods of pixels with the same class identified using the first heuristic method and applies a smoothed measure of maximum difference in intensity between pixels in the local neighborhood thereby causing the second heuristic classifier to cast a vote for each respective pixel in the plurality of pixels for either the first class or the second class. In some embodiments, the plurality of heuristic classifiers comprises a third heuristic classifier that performs edge detection on the plurality of pixels to form a plurality of edges in the image, morphologically closes the plurality of edges to form a plurality of morphologically closed regions in the image and assigns pixels in the morphologically closed regions to the first class and pixels outside the morphologically closed regions to the second class, thereby causing the third heuristic classifier to cast a vote for each respective pixel in the plurality of pixels for either the first class or the second class. In some embodiments, the plurality of heuristic classifiers consists of the first, second, and third heuristic classier, each respective pixel assigned by each of the heuristic classifiers in the plurality of classifiers to the second class is labelled as obvious second class, and each respective pixel assigned by each of the plurality of heuristic classifiers as the first class is labelled as obvious first class.

In some embodiments, the segmentation algorithm is a graph cut segmentation algorithm such as GrabCut.

In some embodiments, the cleavage domain comprises a sequence recognized and cleaved by a uracil-DNA glycosylase and/or an endonuclease VIII.

In some embodiments, a capture probe plurality in the set of capture probe pluralities does not comprise a cleavage domain and is not cleaved from the array.

In some embodiments, the one or more analytes comprises DNA or RNA.

In some embodiments, each capture probe plurality in the set of capture probe pluralities is attached directly or attached indirectly to the substrate.

In some embodiments, the obtaining the sequence reads comprises *in-situ* sequencing of the set of capture spots on the substrate. In some embodiments, the obtaining the sequence reads comprises high throughput sequencing of the set of capture spots on the substrate.

In some embodiments, a respective locus in the plurality of loci is biallelic and the corresponding set of haplotypes for the respective locus consists of a first allele and a second allele. In some such embodiments, the respective locus includes a heterozygous single nucleotide polymorphism (SNP), a heterozygous insert, or a heterozygous deletion.

In some embodiments, the plurality of sequence reads comprises 10,000 or more sequence reads, 50,000 or more sequence reads, 100,000 or more sequence reads, or $1 \times 10^6$ or more sequence reads.

In some embodiments, the plurality of loci comprises between two and 100 loci, more than 10 loci, more than 100 loci, or more than 500 loci.

In some embodiments, the unique spatial barcode in the respective sequence read is localized to a contiguous set of oligonucleotides within the respective sequence read. In some such embodiments, the contiguous set of oligonucleotides is an N-mer, where N is an integer selected from the set {4, ..., 20}.

In some embodiments, a respective plurality of sequence reads include 3'-end or 5'-end paired sequence reads.

In some embodiments, the one or more analytes is a plurality of analytes, a respective capture probe plurality in the set of capture probe pluralities includes a plurality of capture probes, and each capture probe in the plurality of capture probes includes a capture domain that is characterized by a single capture domain type configured to bind to each analyte in the plurality of analytes in an unbiased manner.

Another aspect of the present disclosure provides a computer system comprising one or more processors, and memory. One or more programs are stored in the memory and are configured to be executed by the one or more processors. It will be appreciated that this memory can be on a single computer, a network of computers, one or more virtual machines, or in a cloud computing architecture. The one or more programs are for spatial analysis of analytes. The one or more programs include instructions for obtaining one or more images of a biological sample (e.g., a sectioned tissue sample, each section of a sectioned tissue sample, etc.) respectively on the substrate, where each instance of the substrate includes a plurality of fiducial markers and a set of capture spots, and where each respective image comprises a plurality of pixels in the form of an array of pixel values. In some embodiments the array of pixel values comprises at least a least 100, 10,000, 100,000, $1 \times 10^6$, $2 \times 10^6$, $3 \times 10^6$, $5 \times 10^6$, $8 \times 10^6$, $10 \times 10^6$, or $15 \times 10^6$ pixel values. A plurality of sequence reads is obtained, in electronic form, from the set of capture spots after the biological sample is on the substrate. Each respective capture probe plurality in a set of capture probe pluralities is (i) at a different capture spot in the set of capture spots and (ii) associates with one or more analytes from the biological sample. Each respective capture probe plurality in the set of capture probe pluralities is characterized by at least one unique spatial barcode in a plurality of spatial barcodes. The plurality of sequence reads comprises sequence reads corresponding to all or portions of the one or more analytes. Furthermore, each respective sequence read in the plurality of sequence reads includes a spatial barcode of the corresponding capture probe plurality in the set of capture probes. The plurality of spatial barcodes is used to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots. The plurality of fiducial markers is used to provide a composite representation comprising (i) the image aligned to the set of capture spots on the substrate and (ii) a representation of each subset of sequence reads at the respective position within the image that maps to the corresponding capture spot on the substrate.

Still another aspect of the present disclosure provides a computer readable storage medium storing one or more programs. The one or more programs comprise instructions, which when executed by an electronic device with one or more processors and a memory, cause the electronic device to perform spatial analysis of analytes by a method in which an image of a biological sample (e.g., a sectioned tissue sample) on the substrate is obtained. The substrate includes a plurality of fiducial markers and a set of capture spots, and the image comprises a plurality of pixels in the form of an array of pixel values. A plurality of sequence reads is obtained, in electronic form, from the set of capture spots after the biological sample is on the substrate. Each respective capture probe plurality in a set of capture probe pluralities is (i) at a different capture spot in the set of capture spots and (ii) associates with one or more analytes from the biological sample. Each respective capture probe plurality in the set of capture probe pluralities is characterized by at least one unique spatial barcode in a plurality of spatial barcodes. The plurality of sequence reads comprises sequence reads corresponding to all or portions of the one or more analytes. Furthermore, each respective sequence read in the plurality of sequence reads includes a spatial barcode of the corresponding capture probe plurality in the set of capture probes. The plurality of spatial barcodes is used to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots. The plurality of fiducial markers is used to provide a composite representation comprising (i) the image aligned to the set of capture spots on the substrate and (ii) a representation of each subset of sequence reads at the respective position within the image that maps to the corresponding capture spot on the substrate.

Another aspect of the present disclosure provides a computing system including one or more processors and memory storing one or more programs for spatial nucleic analysis. It will be appreciated that this memory can be on a single computer, a network of computers, one or more virtual machines, or in a cloud computing architecture. The one or more programs are configured for execution by the one or more processors. The one or more programs include instructions for performing any of the methods disclosed above.

Still another aspect of the present disclosure provides a computer readable storage medium storing one or more programs to be executed by an electronic device. The one or more programs include instructions for the electronic device to perform spatial nucleic analysis by any of the methods disclosed above. It will be appreciated that the computer readable storage medium can exist as a single computer readable storage medium or any number of component computer readable storage mediums that are physically separated from each other.

Other embodiments are directed to systems, portable consumer devices, and computer readable media associated with methods described herein.

As disclosed herein, any embodiment disclosed herein, when applicable, can be applied to any aspect.

Various embodiments of systems, methods, and devices within the scope of the appended claims each have several aspects, no single one of which is solely responsible for the desirable attributes described herein. Without limiting the scope of the appended claims, some prominent features are described herein. After considering this discussion, and particularly after reading the section entitled "Detailed Description" one will understand how the features of various embodiments are used.

## INCORPORATION BY REFERENCE

All publications, patents, patent applications, and information available on the Internet and mentioned in this specification are herein incorporated by reference to the same extent as if each individual publication, patent, patent application, or item of information was specifically and individually indicated to be incorporated by reference. To the extent publications, patents, patent applications, or item of information available on the Internet incorporated by reference contradict the disclosure contained in the specification, the specification is intended to supersede and/or take precedence over any such contradictory material.

## DESCRIPTION OF DRAWINGS

The following drawings illustrate certain embodiments of the features and advantages of this disclosure. These embodiments are not intended to limit the scope of the appended claims in any manner. Like reference symbols throughout the several views of the patent application indicate like elements.

**FIG. 1** shows an exemplary spatial analysis workflow in accordance with an embodiment of the present disclosure.

**FIG. 2** shows an exemplary spatial analysis workflow in which optional steps are indicated by dashed boxes in accordance with an embodiment of the present disclosure.

**FIGS. 3A** and **3B** show exemplary spatial analysis workflows in which, in **FIG. 3A**, optional steps are indicated by dashed boxes in accordance with embodiments of the present disclosure.

**FIG. 4** shows an exemplary spatial analysis workflow in which optional steps are indicated by dashed boxes in accordance with an embodiment of the present disclosure.

**FIG. 5** shows an exemplary spatial analysis workflow in which optional steps are indicated by dashed boxes in accordance with an embodiment of the present disclosure.

**FIG. 6** is a schematic diagram showing an example of a barcoded capture probe, as described herein in accordance with an embodiment of the present disclosure.

**FIG. 7** is a schematic illustrating a cleavable capture probe in accordance with an embodiment of the present disclosure.

**FIG. 8** is a schematic diagram of an exemplary multiplexed spatially-labelled capture spot in accordance with an embodiment of the present disclosure.

**FIG. 9** illustrates details of a spatial capture spot and capture probe in accordance with an embodiment of the present disclosure.

**FIGS. 10A, 10B, 10C, 10D,** and **10E** illustrate non-limiting methods for spatial nucleic analysis in accordance with some embodiments of the present disclosure, in which optional steps are illustrated by dashed line boxes.

**FIG. 11** is an example block diagram illustrating a computing device in accordance with some embodiments of the present disclosure.

**FIG. 12** is a schematic showing the arrangement of barcoded capture spots within an array in accordance with some embodiments of the present disclosure.

**FIG. 13** is a schematic illustrating a side view of a diffusion-resistant medium, e.g., a lid in accordance with some embodiments of the present disclosure.

**FIG. 14** illustrates a substrate with an image of a biological sample (e.g., tissue sample) on the substrate, in accordance with an embodiment of the present disclosure.

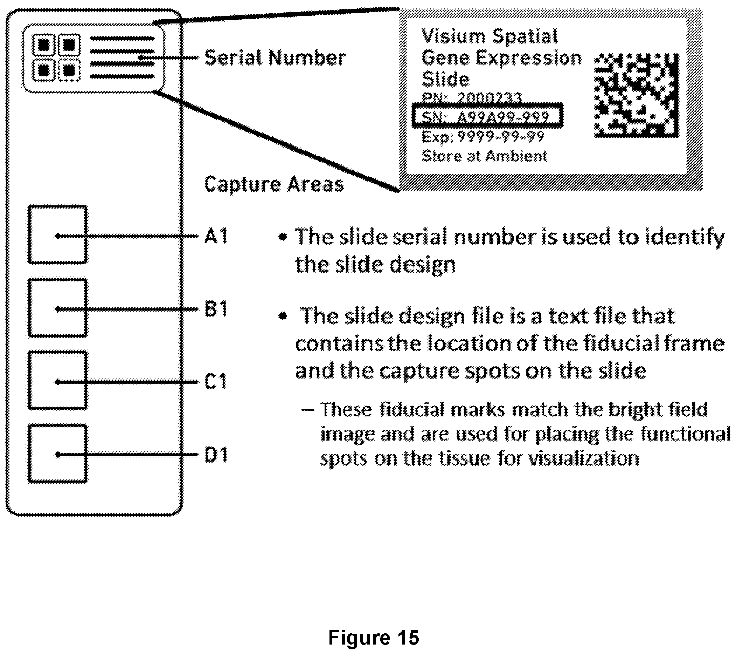

**FIG. 15** illustrates a substrate that has a number of capture areas and a substrate identifier, in accordance with an embodiment of the present disclosure.

**FIG. 16** illustrates a substrate that has a plurality of fiducial markers and a set of capture spots, in accordance with an embodiment of the present disclosure.

**FIG. 17** illustrates an image of a biological sample (e.g., tissue sample) on a substrate, where the biological sample is positioned within a plurality of fiducial markers, in accordance with an embodiment of the present disclosure.

**FIG. 18** illustrates a template that comprises reference positions for a corresponding plurality of reference fiducial spots and a corresponding coordinate system in accordance with an embodiment of the present disclosure.

**FIG. 19** illustrates how the template specifies the locations of the set of capture spots of a substrate in relation to the reference fiducial spots of the substrate using a corresponding coordinate system in accordance with an embodiment of the present disclosure.

**FIG. 20** illustrates the substrate design, including a plurality of fiducial markers and a set of capture spots, to the image, which includes corresponding derived fiducial spots, in accordance with an embodiment of the present disclosure.

**FIG. 21** illustrates the registration of the image with the substrate using a transformation and the coordinate system of the template to register the image to the set of capture spots of the substrate, in accordance with an embodiment of the present disclosure.

**FIG. 22** illustrates the analysis of the image after the registration of the image with the substrate, using a transformation and the coordinate system of the template to register the image to the set of capture spots of the substrate, thereby identifying capture spots on the substrate that have been overlaid by tissue in accordance with an embodiment of the present disclosure.

**FIG. 23** illustrates the capture spots on a substrate that have been overlaid by tissue in accordance with an embodiment of the present disclosure.

**FIG. 24** illustrates extraction of barcodes and UMIs from each sequence read in nucleic acid sequencing data associated with a substrate in accordance with an embodiment of the present disclosure.

**FIG. 25** illustrates alignment of the sequence reads with a reference genome in accordance with an embodiment of the present disclosure.

**FIG. 26** illustrates how sequence reads don't all map to exactly the same place, even if they share a barcode and UMI, due to the random fragmentation that happens during workflow steps in accordance with an embodiment of the present disclosure.

**FIG. 27** illustrates how the barcode of each sequence read is validated against a whitelist of actual barcodes (*e.g.*, in some embodiments the whitelist corresponds to the Chromium Single Cell 3' v3 chemistry gel beads that have about 3.6 million distinct barcodes and thus a whitelist of 3.6 million barcodes) in accordance with an embodiment of the present disclosure.

**FIG. 28** illustrates how the unique molecular identifiers (UMIs) of sequence reads that are 1 mismatch away from a higher count UMI are corrected to that UMI if they share a cell barcode and gene in accordance with some embodiments of the present disclosure.

**FIG. 29** illustrates how using only the confidently mapped reads with valid barcodes and UMIs are used to form UMI counts for a raw feature barcode matrix in accordance with some embodiments of the present disclosure.

**FIG. 30** illustrates how secondary analysis is done on barcodes called as cells (filtered feature barcode matrix), in which principal components analysis on normalized filtered gene-cell matrix is used to reduce G genes to top 10 metagenes, t-SNE is run in PCA space to generate a two-dimensional projection, graph-based (Louvain) and k-means clustering (k=2... 10) is performed in PCA-space to identify clusters of cells, and sSeq (negative-binomial test) algorithm is used to find genes that most uniquely define each cluster, in accordance with an embodiment of the present disclosure.

**FIG. 31** illustrates a pipeline for analyzing an image (e.g., tissue image) in conjunction with nucleic acid sequencing data associated with each capture spot in a plurality of capture spots, thereby performing spatial nucleic acid analysis in accordance with the present disclosure.

**FIG. 32** illustrates how analysis of the tissue image in conjunction with nucleic acid sequencing data can be used to view capture spot clusters in the context of the image in accordance with the present disclosure.

**FIG. 33** illustrates how analysis of the tissue image in conjunction with nucleic acid sequencing data can include zooming into the overlay of capture spot clusters in the context of the image in order to see more detail in accordance with some embodiments of the present disclosure.

**FIG. 34** illustrates how analysis of the tissue image in conjunction with nucleic acid sequencing data can be used to create custom categories and clusters for differential expression analysis in accordance with some embodiments of the present disclosure.

**FIG. 35** illustrates how analysis of the tissue image in conjunction with nucleic acid sequencing data can be used to see expressed genes in the context of the tissue image in accordance with some embodiments of the present disclosure.

**FIGS. 36A, 36B, 36C, 36D, 36E, 36F, 36G, 36H,** and **36I** illustrate the image input **FIG. 36A** of a tissue section on a substrate, the outputs of a variety of heuristic classifiers **FIGS. 36B, 36C, 36D, 36E, 36F,** and **36G,** and the outputs of a segmentation algorithm **FIGS. 36H** and **36I** in accordance with some embodiments of the present disclosure.

**FIG. 37** illustrates a reaction scheme for the preparation of sequence reads for spatial analysis in accordance with some embodiments of the present disclosure.

**FIG. 38A** illustrates an embodiment in which all of the images of a spatial projection are fluorescence images and are all displayed in accordance with an embodiment of the present disclosure.

**FIG. 38B** illustrates the spatial projection of **FIG. 38A** in which only a CD3 channel fluorescence image of the spatial projection is displayed in accordance with an embodiment of the present disclosure.

**FIG. 38C** illustrates the image of **FIG. 38B** in which CD3 is quantified based on measured intensity in accordance with an embodiment of the present disclosure.

**FIG. 39** illustrates an immunofluorescence image, a representation of all or a portion of each subset of sequence reads at each respective position within one or more images that maps to a respective capture spot corresponding to the respective position, as well as composite representations in accordance with some embodiments of the present disclosure.

**FIG. 40** is a schematic diagram of an exemplary analyte capture agent in accordance with some embodiments of the present disclosure.

**FIG. 41A** is a schematic diagram depicting an exemplary interaction between a feature-immobilized capture probe and an analyte capture agent in accordance with some embodiments of the present disclosure.

**FIG. 41B** is an exemplary schematic showing an analyte binding moiety comprising an oligonucleotide having a capture binding domain (indicated by a poly(A) sequence) that is hybridized to a blocking domain (indicated by a poly(T) sequence).

**FIG. 41C** is an exemplary schematic showing an analyte binding moiety that includes an oligonucleotide comprising a hairpin sequence disposed between a blocking domain (indicated by a poly(U) sequence) and a capture binding domain (indicated by a poly(A) sequence). As shown, the blocking domain hybridizes to the capture binding domain.

**FIG. 41D** is an exemplary schematic showing a blocking domain released by RNAse H.

**FIG. 41E** is an exemplary schematic showing an analyte binding moiety that includes an oligonucleotide comprising a capture binding domain that is blocked using caged nucleotides (indicated by pentagons).

**FIG. 42** is an exemplary schematic illustrating a spatially-tagged analyte capture agent where the analyte capture sequence is blocked via a blocking probe, and in which the blocking probe can be removed, for example with an RNAse treatment, in accordance with some embodiments of the present disclosure.

**FIG. 43** is a workflow schematic illustrating exemplary, non-limiting, non-exhaustive steps for spatial analyte identification after antibody staining in a biological sample, where the sample is fixed, stained with fluorescent antibodies and spatially-tagged analyte capture agents, and imaged to detect the spatial location of target analytes within the biological sample, in accordance with some embodiments of the present disclosure.

**FIG. 44** shows exemplary multiplexed imaging results, in which the immunofluorescent image shows immunofluorescent staining for CD29 and CD4 in tissue sections of mouse spleen (far left), while the images in the series of right panels show results of multiplexed, spatially-tagged analyte capture agent workflow, where the spatial location of target proteins, CD29, CD3, CD4, CD8, CD19, B220, F4/80, and CD169 are visualized by sequencing the analyte-corresponding analyte binding moiety barcodes, in accordance with some embodiments of the present disclosure.

**FIG. 45** shows an exemplary workflow for spatial proteomic and genomic analysis in accordance with some embodiments of the present disclosure.

**FIG. 46A** shows a schematic of an analyte capture agent and a spatial gene expression slide.

**FIG. 46B** shows a merged fluorescent image of DAPI staining of a section of human cerebellum tissue.

**FIG. 46C** shows a spatial transcriptomic analysis of the section of human cerebellum from FIG. 46B, overlaid on FIG. 46B.

**FIG. 46D** shows a t-SNE projection of the sequencing data illustrating cell-type clustering of the cerebellum from FIG. 46C.

**FIG. 46E** shows spatial gene expression (top) and protein staining (bottom) of astrocyte marker glutamine synthase (produced by hybridoma clone O91F4), each overlaid on FIG. 46B.

**FIG. 46F** shows spatial gene expression (top) and protein staining (bottom) of oligodendrocyte marker myelin CNPase (produced by hybridoma clone SMI91), each overlaid on FIG. 46B.

**FIG. 46G** shows spatial gene expression (top) and protein staining (bottom) of oligodendrocyte marker myelin basic protein (produced by hybridoma clone P82H9), each overlaid on FIG. 46B.

**FIG. 46H** shows spatial gene expression (top) and protein staining (bottom) of stem cell marker SOX2

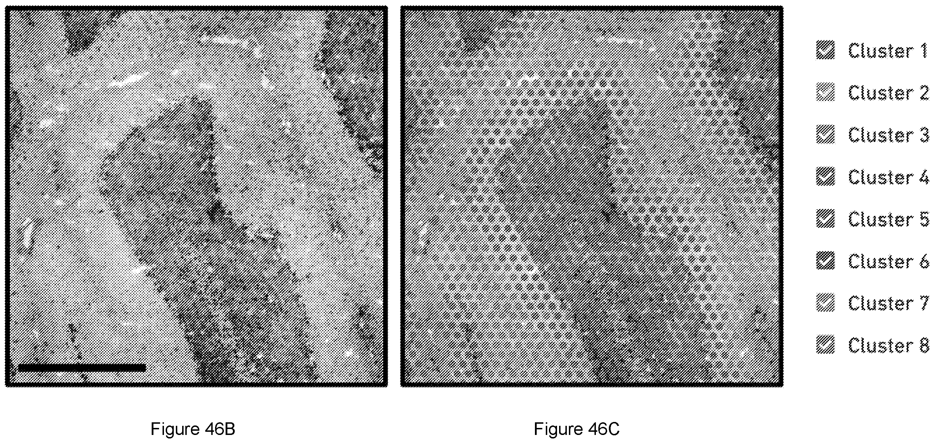

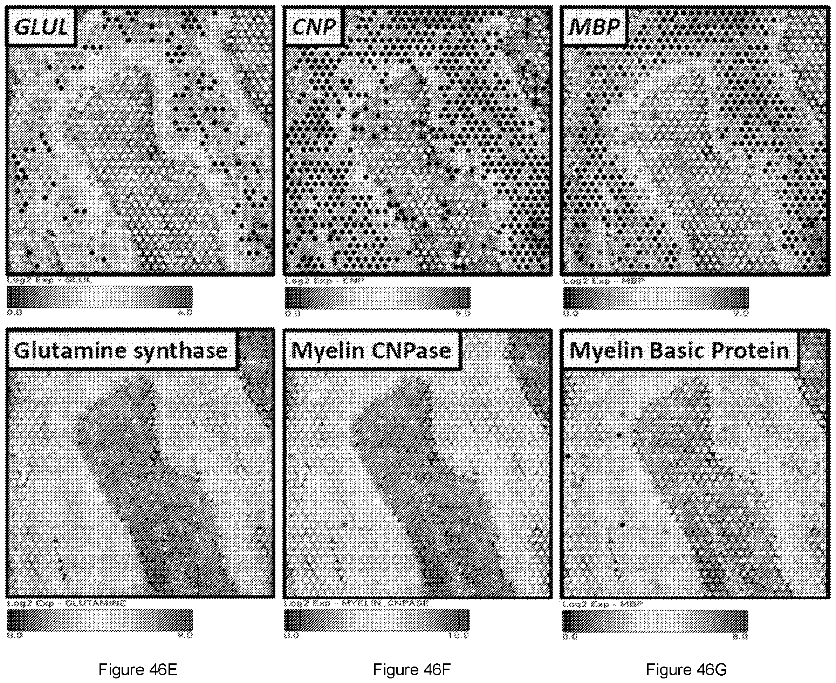

(produced by hybridoma clone 14A6A34), each overlaid on FIG. 46B.

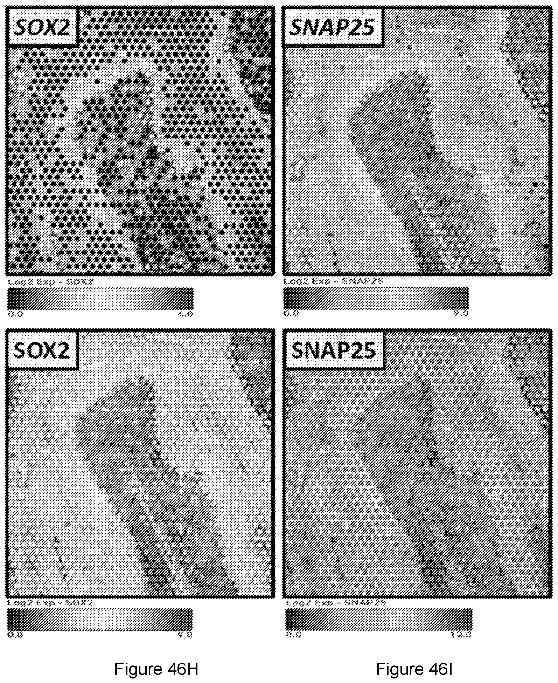

**FIG. 46I** shows spatial gene expression (top) and protein staining (bottom) of neuronal marker SNAP-25 (produced by hybridoma clone SMI81), each overlaid on FIG. 46B.

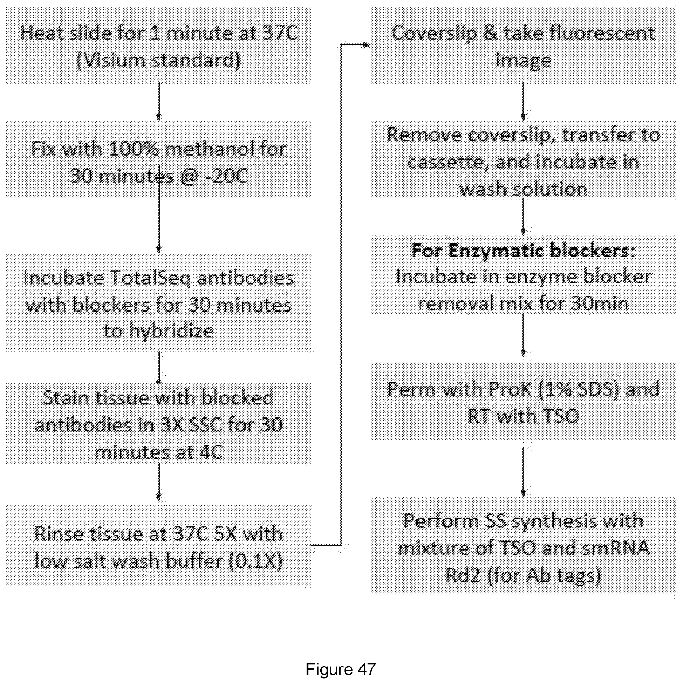

**FIG. 47** is an exemplary workflow for taking a tissue sample and performing analyte capture as described herein.

## DETAILED DESCRIPTION

### I. Introduction

This disclosure describes apparatus, systems, methods, and compositions for spatial analysis of biological samples. This section in particular describes certain general terminology, analytes, sample types, and preparative steps that are referred to in later sections of the disclosure.

### (a) Spatial analysis.

Tissues and cells can be obtained from any source. For example, tissues and cells can be obtained from single-cell or multicellular organisms (*e.g.*, a mammal). Tissues and cells obtained from a mammal (*e.g.*, a human) often have varied analyte levels (*e.g.*, gene and/or protein expression) that can result in differences in cell morphology and/or function. The position of a cell or subset of cells (*e.g.*, neighboring cells and/or non-neighboring cells) within a tissue can affect, for example, the cell's fate, behavior, morphology, signaling and cross-talk with other cells in the tissue. Information regarding the differences in analyte levels (*e.g.*, gene and/or protein expression) within different cells in a tissue of a mammal can also help physicians select or administer a treatment that will be effective and can allow researchers to identify and elucidate differences in cell morphology and/or cell function in single-cell or multicellular organisms (*e.g.* a mammal) based on the detected differences in analyte levels within different cells in the tissue. Differences in analyte levels within different cells in a tissue of a mammal can also provide information on how tissues (*e.g.*, healthy and diseased tissues) function and/or develop. Differences in analyte levels within different cells in a tissue of a mammal can also provide information on different mechanisms of disease pathogenesis in a tissue and mechanism of action of a therapeutic treatment within a tissue. Differences in analyte levels within different cells in a tissue of a mammal can also provide information on the drug resistance mechanisms and the development of the same in a tissue of a mammal. Differences in the presence or absence of analytes within difference cells in a tissue of a multicellular organism (*e.g.*, a mammal) can provide information on drug resistance mechanisms and the development of the same in a tissue of a multicellular organism.

The spatial analysis methodologies herein provide for the detection of differences in an analyte level (*e.g.*, gene and/or protein expression) within different cells in a tissue of a mammal or within a single cell from a mammal. For example, spatial analysis methodologies can be used to detect the differences in analyte levels (*e.g.*, gene and/or protein expression) within different cells in histological slide samples, the data from which can be reassembled to generate a three-dimensional map of analyte levels (*e.g.*, gene and/or protein expression) of a tissue sample (*e.g.*, tissue sample) obtained from a mammal (*e.g.*, with a degree of spatial resolution such as single-cell resolution).

Spatial heterogeneity in developing systems has typically been studied using RNA hybridization, immunohistochemistry, fluorescent reporters, or purification or induction of pre-defined subpopulations and subsequent genomic profiling (*e.g.*, RNA-seq). Such approaches, however, rely on a relatively small set of pre-defined markers, therefore introducing selection bias that limits discovery. These prior approaches also rely on a *priori* knowledge. Spatial RNA assays traditionally relied on staining for a limited number of RNA species. In contrast, single-cell RNA-sequencing allows for deep profiling of cellular gene expression (including non-coding RNA), but the established methods separate cells from their native spatial context.

Spatial analysis methodologies described herein provide a vast amount of analyte level and/or expression data for a variety of multiple analytes within a sample at high spatial resolution, *e.g.*, while retaining the native spatial context. Spatial analysis methods include, for example, the use of a capture probe including a spatial barcode (*e.g.*, a nucleic acid sequence) that provides information as to the position of the capture probe within a cell or a tissue sample (*e.g.*, mammalian cell or a mammalian tissue sample) and a capture domain that is capable of binding to an analyte (*e.g.*, a protein and/or nucleic acid) produced by and/or present in a cell. As described herein, the spatial barcode can be a nucleic acid that has a unique sequence, a unique fluorophore, a unique combination of fluorophores, a unique amino acid sequence, a unique heavy metal or a unique combination of heavy metals, or any other unique detectable agent. The capture domain can be any agent that is capable of binding to an analyte produced by and/or present in a cell (*e.g.*, a nucleic acid that is capable of hybridizing to a nucleic acid from a cell (*e.g.*, an mRNA, genomic DNA, mitochondrial DNA, or miRNA), a substrate including an analyte, a binding partner of an analyte, or an antibody that binds specifically to an analyte). A capture probe can also include a nucleic acid sequence that is complementary to a sequence of a universal forward and/or universal reverse primer. A capture probe can also include a cleavage site (*e.g.*, a cleavage recognition site of a restriction endonuclease), or a photolabile or thermosensitive bond.

The binding of an analyte to a capture probe can be detected using a number of different methods, *e.g.*, nucleic acid sequencing, fluorophore detection, nucleic acid amplification, detection of nucleic acid ligation, and/or detection of nucleic acid cleavage products. In some examples, the detection is used to associate a specific spatial barcode with a specific analyte produced by and/or present in a cell (*e.g.*, a mammalian cell).

Capture probes can be, *e.g.*, attached to a surface, *e.g.*, a solid array, a bead, or a coverslip. In some examples, capture probes are not attached to a surface. In some examples, capture probes are encapsulated within, embedded within, or layered on a surface of a permeable composition (*e.g.*, any of the substrates described herein). For example, capture probes can be encapsulated or disposed within a permeable bead (*e.g.*, a gel bead). In some examples, capture probes are encapsulated within, embedded within, or layered on a surface of a substrate (*e.g.*, any of the exemplary substrates described herein, such as a hydrogel or a porous membrane).

In some examples, a cell or a tissue sample including a cell are contacted with capture probes attached to a substrate (*e.g.*, a surface of a substrate), and the cell or tissue sample is permeabilized to allow analytes to be released from the cell and bind to the capture probes attached to the substrate. In some examples, analytes released from a cell can be actively directed to the capture probes attached to a substrate using a variety of methods, *e.g.*, electrophoresis, chemical gradient, pressure gradient, fluid flow, or magnetic field.

In other examples, a capture probe can be directed to interact with a cell or a tissue sample using a variety of methods, *e.g.*, inclusion of a lipid anchoring agent in the capture probe, inclusion of an agent that binds specifically to, or forms a covalent bond with, a membrane protein in the capture probe, fluid flow, pressure gradient, chemical gradient, or magnetic field.

Non-limiting aspects of spatial analysis methodologies are described in WO 2011/127099, WO 2014/210233, WO 2014/210225, WO 2016/162309, WO 2018/091676, WO 2012/140224, WO 2014/060483, U.S. Patent No. 10,002,316, U.S. Patent No. 9,727,810, U.S. Patent Application Publication No. 2017/0016053, Rodriques et al., Science 363(6434):1463-1467, 2019; WO 2018/045186, Lee et al., Nat. Protoc. 10(3):442-458, 2015; WO 2016/007839, WO 2018/045181, WO 2014/163886, Trejo et al., PLoS ONE 14(2):e0212031, 2019, U.S. Patent Application Publication No. 2018/0245142, Chen et al., Science 348(6233):aaa6090, 2015, Gao et al., BMC Biol. 15:50, 2017, WO 2017/144338, WO 2018/107054, WO 2017/222453, WO 2019/068880, WO 2011/094669, U.S. Patent No. 7,709,198, U.S. Patent No. 8,604,182, U.S. Patent No. 8,951,726, U.S. Patent No. 9,783,841, U.S. Patent No. 10,041,949, WO 2016/057552, WO 2017/147483, WO 2018/022809, WO 2016/166128, WO 2017/027367, WO 2017/027368, WO 2018/136856, WO 2019/075091, U.S. Patent No. 10,059,990, WO 2018/057999, WO 2015/161173, Gupta et al., Nature Biotechnol. 36:1197-1202, 2018, and United States Patent Application No. 16/992,569 entitled "Systems and Methods for Using Spatial Distribution of Haplotypes to Determine a Biological Condition, "filed August 13, 2020, and can be used herein in any combination. Further non-limiting aspects of spatial analysis methodologies are described herein.

(b) General terminology

Specific terminology is used throughout this disclosure to explain various aspects of the apparatus, systems, methods, and compositions that are described. This sub-section includes explanations of certain terms that appear in later sections of the disclosure. To the extent that the descriptions in this section are in apparent conflict with usage in other sections of this disclosure, the definitions in this section will control.

(i) Subject

A "subject" is an animal, such as a mammal (e.g., human or a non-human simian), or avian (e.g., bird), or other organism, such as a plant. Examples of subjects include, but are not limited to, a mammal such as a rodent, mouse, rat, rabbit, guinea pig, ungulate, horse, sheep, pig, goat, cow, cat, dog, primate (e.g. human or non-human primate); a plant such as Arabidopsis thaliana, corn, sorghum, oat, wheat, rice, canola, or soybean; an algae such as Chlamydomonas reinhardtii; a nematode such as Caenorhabditis elegans; an insect such as Drosophila melanogaster, mosquito, fruit fly, honey bee or spider; a fish such as zebrafish; a reptile; an amphibian such as a frog or Xenopus laevis; a Dictyostelium discoideum; a fungi such as Pneumocystis carinii, Takifugu rubripes, yeast, Saccharamoyces cerevisiae or Schizosaccharomyces pombe; or a Plasmodium falciparum.

(ii) Nucleic acid and Nucleotide

The terms "nucleic acid" and "nucleotide" are intended to be consistent with their use in the art and to include naturally-occurring species or functional analogs thereof. Particularly useful functional analogs of nucleic acids are capable of hybridizing to a nucleic acid in a sequence-specific fashion (e.g., capable of hybridizing to two nucleic acids such that ligation can occur between the two hybridized nucleic acids) or are capable of being used as a template for replication of a particular nucleotide sequence. Naturally-occurring nucleic acids generally have a backbone containing phosphodiester bonds. An analog structure can have an alternate backbone linkage including any of a variety of those known in the art. Naturally-occurring nucleic acids generally have a deoxyribose sugar (e.g., found in deoxyribonucleic acid (DNA)) or a ribose sugar (e.g., found in ribonucleic acid (RNA)).

A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine (A), thymine (T), cytosine (C), or guanine (G), and a ribonucleic acid can have one or more bases selected from the group consisting of uracil (U), adenine (A), cytosine (C), or guanine (G). Useful non-native bases that can be included in a nucleic acid or nucleotide are known in the art.

(iii) Probe and Target

A "probe" or a "target," when used in reference to a nucleic acid or sequence of nucleic acids, is intended as a semantic identifier for the nucleic acid or sequence in the context of a method or composition, and does not limit the structure or function of the nucleic acid or sequence beyond what is expressly indicated.

***(iv) Oligonucleotide and Polynucleotide***

The terms "oligonucleotide" and "polynucleotide" are used interchangeably to refer to a single-stranded multimer of nucleotides from about 2 to about 500 nucleotides in length. Oligonucleotides can be synthetic, made enzymatically (*e.g.*, via polymerization), or using a "split-pool" method. Oligonucleotides can include ribonucleotide monomers (*e.g.*, can be oligoribonucleotides) and/or deoxyribonucleotide monomers (*e.g.*, oligodeoxyribonucleotides). In some examples, oligonucleotides include a combination of both deoxyribonucleotide monomers and ribonucleotide monomers in the oligonucleotide (*e.g.*, random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (*e.g.*, covalently or non-covalently) to the multimer structure. For example, an oligonucleotide can include one or more detectable labels (*e.g.*, a radioisotope or fluorophore).

***(v) Barcode***

A "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes.

Barcodes can have a variety of different formats. For example, barcodes can include non-random, semi-random, and/or random nucleic acid and/or amino acid sequences, and synthetic nucleic acid and/or amino acid sequences.

Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

Barcodes can spatially-resolve molecular components found in biological samples, for example, at single-cell resolution (e.g., a barcode can be or can include a "spatial barcode"). In some embodiments, a barcode includes both a UMI and a spatial barcode. In some embodiments, a barcode includes two or more sub-barcodes that together function as a single barcode. In some embodiments, a barcode includes both a UMI and a spatial barcode. In some embodiments, a barcode includes two or more sub-barcodes that together function as a single barcode (e.g., a polynucleotide barcode). For example, a polynucleotide barcode can include two or more polynucleotide sequences (e.g., sub-barcodes) that are separated by one or more non-barcode sequences.

### (vi) Capture Spot

A "capture spot" (alternately, "feature" or "capture probe plurality") is used herein to describe an entity that acts as a support or repository for various molecular entities used in sample analysis. Examples of capture spots include, but are not limited to, a bead, a spot of any two- or three-dimensional geometry (e.g., an ink jet spot, a masked spot, a square on a grid), a well, and a hydrogel pad. In some embodiments, a capture spot is an area on a substrate at which capture probes labelled with spatial barcodes are clustered. Specific non-limiting embodiments of capture spots and substrates are further described below in the present disclosure.

Additional definitions relating generally to spatial analysis of analytes can be found in United States Patent Application Number 16/992,569 entitled "Systems and Methods for Using the Spatial Distribution of Haplotypes to Determine a Biological Condition," filed August 13, 2020, which is hereby incorporated herein by reference.

### (vii) Substrate

As used herein, a "substrate" is any surface onto which capture probes can be affixed (e.g., a chip, solid array, a bead, a coverslip, *etc*).

### (viii) Genome

A "genome" generally refers to genomic information from a subject, which can be, for example, at least a portion of, or the entirety of, the subject's gene-encoded hereditary information. A genome can include coding regions (*e.g.*, that code for proteins) as well as non-coding regions. A genome can include the sequences of some or all of the subject's chromosomes. For example, the human genome ordinarily has a total of 46 chromosomes. The sequences of some or all of these can constitute the genome.

### (ix) Adaptor, Adapter, and Tag

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, *e.g.*, spacer sequences, primer sequences/sites, barcode sequences, unique molecular identifier sequences.

### (x) Antibody

An "antibody" is a polypeptide molecule that recognizes and binds to a complementary target antigen. Antibodies typically have a molecular structure shape that resembles a Y shape, or polymers thereof. Naturally-occurring antibodies, referred to as immunoglobulins, belong to one of the immunoglobulin classes IgG, IgM, IgA, IgD, and IgE. Antibodies can also be produced synthetically. For example, recombinant antibodies, which are monoclonal antibodies, can be synthesized using synthetic genes by recovering the antibody genes from source cells, amplifying into an appropriate vector, and introducing the vector into a host to cause the host to express the recombinant antibody. In general, recombinant antibodies can be cloned from any species of antibody-producing animal using suitable oligonucleotide primers and/or hybridization probes. Recombinant techniques can be used to generate antibodies and antibody fragments, including non-endogenous species.

Synthetic antibodies can be derived from non-immunoglobulin sources. For example, antibodies can be generated from nucleic acids (*e.g.*, aptamers), and from non-immunoglobulin protein scaffolds (such as peptide aptamers) into which hypervariable loops are inserted to form antigen binding sites. Synthetic antibodies based on nucleic acids or peptide structures can be smaller than immunoglobulin-derived antibodies, leading to greater tissue penetration.

Antibodies can also include affimer proteins, which are affinity reagents that typically have a molecular weight of about 12-14 kDa. Affimer proteins generally bind to a target (*e.g.*, a target protein) with both high affinity and specificity. Examples of such targets include, but are not limited to, ubiquitin chains, immunoglobulins, and C-reactive protein. In some embodiments, affimer proteins are derived from cysteine protease inhibitors, and include peptide loops and a variable N-terminal sequence that provides the binding site. Antibodies can also include single domain antibodies (VHH domains and VNAR domains), scFvs, and Fab fragments.

*(c) Analytes*

The apparatus, systems, methods, and compositions described in this disclosure can be used to detect and analyze a wide variety of different analytes. For the purpose of this disclosure, an "analyte" can include any biological substance, structure, moiety, or component to be analyzed. The term "target" can be similarly used to refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is an organelle (*e.g.*, nuclei or mitochondria).

Cell surface features corresponding to analytes can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (*e.g.*, phosphorylation, glycosylation, ubiquitination, nitrosylation, methylation, acetylation or lipidation) state of a cell surface protein, a gap junction, and an adherens junction.

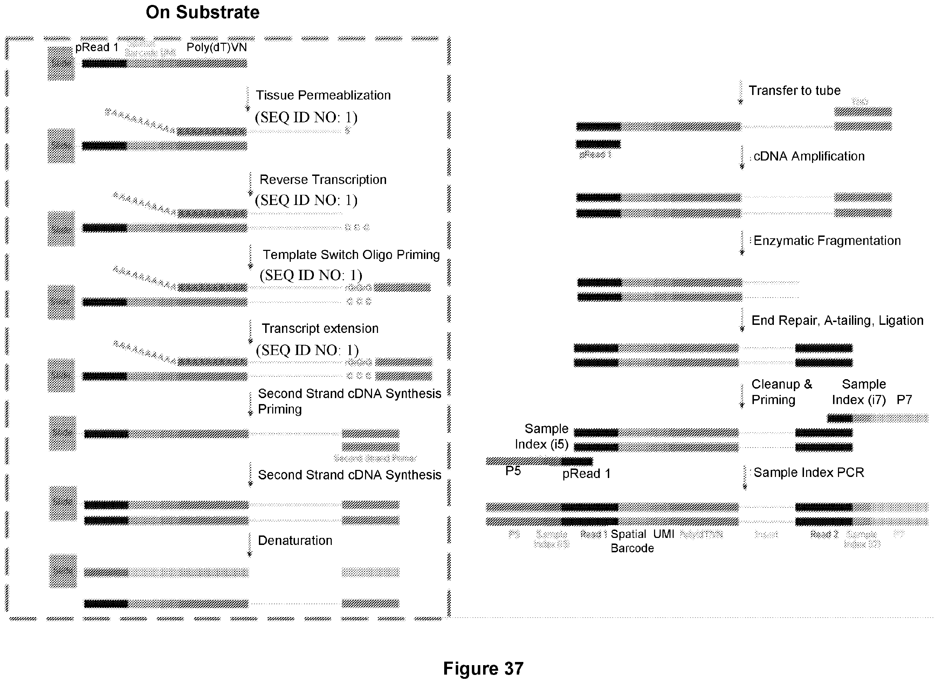

Analytes can be derived from a specific type of cell and/or a specific sub-cellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis. Tissue permeablization is illustrated in **FIG. 37.**

Examples of nucleic acid analytes include DNA analytes such as genomic DNA, methylated DNA, specific methylated DNA sequences, fragmented DNA, mitochondrial DNA, in situ synthesized PCR products, and RNA/DNA hybrids.

Examples of nucleic acid analytes also include RNA analytes such as various types of coding and non-coding RNA. Examples of the different types of RNA analytes include messenger RNA (mRNA), ribosomal RNA (rRNA), transfer RNA (tRNA), microRNA (miRNA), and viral RNA. The RNA can be a transcript (*e.g.*, present in a tissue section). The RNA can be small (*e.g.*, less than 200 nucleic acid bases in length) or large (*e.g.*, RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA (*e.g.*, 16s rRNA or 23s rRNA).

Additional examples of analytes include mRNA and cell surface features (*e.g.*, using the labelling agents described herein), mRNA and intracellular proteins (*e.g.*, transcription factors), mRNA and cell methylation status, mRNA and accessible chromatin (*e.g.*, ATAC-seq, DNase-seq, and/or MNase-seq), mRNA and metabolites (*e.g.*, using the labelling agents described herein), a barcoded labelling agent (*e.g.*, the oligonucleotide tagged antibodies described herein) and a V(D)J sequence of an immune cell receptor (*e.g.*, T-cell receptor), mRNA and a perturbation agent (*e.g.*, a CRISPR crRNA/sgRNA, TALEN, zinc finger nuclease, and/or antisense oligonucleotide as described herein). In some embodiments, a perturbation agent is a small molecule, an antibody, a drug, an aptamer, a miRNA, a physical environmental (*e.g.*, temperature change), or any other known perturbation agents.

Analytes can include a nucleic acid molecule with a nucleic acid sequence encoding at least a portion of a V(D)J sequence of an immune cell receptor (*e.g.*, a TCR or BCR). In some embodiments, the nucleic acid molecule is cDNA first generated from reverse transcription of the corresponding mRNA, using a poly(T) containing primer. The generated cDNA can then be barcoded using a capture probe, featuring a barcode sequence (and optionally, a UMI sequence) that hybridizes with at least a portion of the generated cDNA. In some embodiments, a template switching oligonucleotide hybridizes to a poly(C) tail added to a 3' end of the cDNA by a reverse transcriptase enzyme. The original mRNA template and template switching oligonucleotide can then be denatured from the cDNA and the barcoded capture probe can then hybridize with the cDNA and a complement of the cDNA generated. Additional methods and compositions suitable for barcoding cDNA generated from mRNA transcripts including those encoding V(D)J regions of an immune cell receptor and/or barcoding methods and composition including a template switch oligonucleotide are described in PCT Patent Application PCT/US2017/057269, filed October 18, 2017, and U.S. Patent Application Serial No. 15/825,740, filed November 29, 2017, both of which are incorporated herein by reference in their entireties. V(D)J analysis can also be completed with the use of one or more labelling agents that bind to particular surface features of immune cells and associated with barcode sequences. The one or more labelling agents can include an MHC or MHC multimer.

As described above, the analyte can include a nucleic acid capable of functioning as a component of a gene editing reaction, such as, for example, clustered regularly interspaced short palindromic repeats (CRISPR)-based gene editing. Accordingly, the capture probe can include a nucleic acid sequence that is complementary to the analyte (*e.g.*, a sequence that can hybridize to the CRISPR RNA (crRNA), single guide RNA (sgRNA), or an adapter sequence engineered into a crRNA or sgRNA).

In certain embodiments, an analyte is extracted from a live cell. Processing conditions can be adjusted to ensure that a biological sample remains live during analysis, and analytes are extracted from (or released from) live cells of the sample. Live cell-derived analytes can be obtained only once from the sample, or can be obtained at intervals from a sample that continues to remain in viable condition.

In general, the systems, apparatus, methods, and compositions can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual capture spot of the substrate. Methods for performing multiplexed assays to analyze two or more different analytes will be discussed in a subsequent section of this disclosure.

## (d) Biological samples

### (i) Types of biological samples

A "biological sample" is obtained from the subject for analysis using any of a variety of techniques including, but not limited to, biopsy, surgery, and laser capture microscopy (LCM), and generally includes cells and/or other biological material from the subject. In addition to the subjects described above, a biological sample can also be obtained from non-mammalian organisms (e.g., plants, insects, aracnids, nematodes, fugi, amphibians, and fish. A biological sample can be obtained from a prokaryote such as a bacterium, *e.g., Escherichia coli, Staphylococci* or *Mycoplasma pneumoniae;* archae; a virus such as Hepatitis C virus or human immunodeficiency virus; or a viroid. A biological sample can also be obtained from a eukaryote, such as a patient derived organoid (PDO) or patient derived xenograft (PDX). The biological sample can include organoids, a miniaturized and simplified version of an organ produced in vitro in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (*e.g.*, cancer) or a pre-disposition to a disease, and/or individuals that are in need of therapy or suspected of needing therapy.

The biological sample can include any number of macromolecules, for example, cellular macromolecules and organelles (*e.g.*, mitochondria and nuclei). The biological sample can be a nucleic acid sample and/or protein sample. The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions.

Cell-free biological samples can include extracellular polynucleotides. Extracellular polynucleotides can be isolated from a bodily sample, *e.g.*, blood, plasma, serum, urine, saliva, mucosal excretions, sputum, stool, and tears.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

Biological samples can also include fetal cells. For example, a procedure such as amniocentesis can be performed to obtain a fetal cell sample from maternal circulation. Sequencing of fetal cells can be used to identify any of a number of genetic disorders, including, *e.g.*, aneuploidy such as Down's syndrome, Edwards syndrome, and Patau syndrome. Further, cell surface features of fetal cells can be used to identify any of a number of disorders or diseases.

Biological samples can also include immune cells. Sequence analysis of the immune repertoire of such cells, including genomic, proteomic, and cell surface features, can provide a wealth of information to facilitate an understanding the status and function of the immune system. By way of example, determining the status (*e.g.*, negative or positive) of minimal residue disease (MRD) in a multiple myeloma (MM) patient following autologous stem cell transplantation is considered a predictor of MRD in the MM patient (see, *e.g.*, U.S. Patent Publication No. 2018/0156784, the entire contents of which are incorporated herein by reference).

Examples of immune cells in a biological sample include, but are not limited to, B cells, T cells (*e.g.*, cytotoxic T cells, natural killer T cells, regulatory T cells, and T helper cells), natural killer cells, cytokine induced killer (CIK) cells, myeloid cells, such as granulocytes (basophil granulocytes, eosinophil granulocytes, neutrophil granulocytes/hypersegmented neutrophils), monocytes/macrophages, mast cells, thrombocytes/megakaryocytes, and dendritic cells.

As discussed above, a biological sample can include a single analyte of interest, or more than one analyte of interest. Methods for performing multiplexed assays to analyze two or more different analytes in a single biological sample will be discussed in a subsequent section of this disclosure.

### *(ii) Preparation of biological samples*

A variety of steps can be performed to prepare a biological sample for analysis. Except where indicated otherwise, the preparative steps described below can generally be combined in any manner to appropriately prepare a particular sample for analysis.

### *(1) Tissue sectioning*

A biological sample can be harvested from a subject (e.g., via surgical biopsy, whole subject sectioning, grown in vitro on a growth substrate or culture dish as a population of cells, or prepared for analysis as a tissue slice or tissue section). Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 micrometers thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 10, 15, 2, 3, 4, 5, 6, 7, 8, 9, 10, 11, 12, 13, 14, 15, 20, 30, 40, or 50 micrometers. Thicker sections can also be used if desired or convenient, *e.g.*, at least 70, 80, 90, or 100 micrometers or more. Typically, the thickness of a tissue section is between 1-100 micrometers, 1-50 micrometers, 1-30 micrometers, 1-25 micrometers, 1-20 micrometers, 1-15 micrometers, 1-10 micrometers, 2-8 micrometers, 3-7 micrometers, or 4-6 micrometers, but as mentioned above, sections with thicknesses larger or smaller than these ranges can also be analysed.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

### *(2) Freezing*

In some embodiments, the biological sample (*e.g.*, a tissue section as described above) can be prepared by deep freezing at a temperature suitable to maintain or preserve the integrity (*e.g.*, the physical characteristics) of the tissue structure. Such a temperature can be, *e.g.*, less than -20 °C, or less than -25 °C, -30 °C, -40 °C, -50 °C, - 60 °C, -70 °C, -80 °C, -90°C, -100°C, -110°C, -120°C, -130°C, -140°C, -150°C, -160°C, -170°C, -180°C, -190°C, or - 200°C. The frozen tissue sample can be sectioned, *e.g.*, thinly sliced, onto a substrate surface using any number of suitable methods. For example, a tissue sample can be prepared using a chilled microtome (*e.g.*, a cryostat) set at a temperature suitable to maintain both the structural integrity of the tissue sample and the chemical properties of the nucleic acids in the sample. Such a temperature can be, *e.g.*, less than -15 °C, less than -20 °C, or less than -25 °C. A sample can be snap frozen in isopentane and liquid nitrogen. Frozen samples can be stored in a sealed container prior to embedding.

### *(3) Formalin fixation and paraffin embedding*

In some embodiments, the biological sample can be prepared using formalin-fixation and paraffin-embedding (FFPE), which are established methods. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis, the paraffin-embedding material can be removed from the tissue section (*e.g.*, deparaffinization) by incubating the tissue section in an appropriate solvent (*e.g.*, xylene) followed by a rinse (*e.g.*, 99.5% ethanol for 2 minutes, 96% ethanol for 2 minutes, and 70% ethanol for 2 minutes).

### *(4) Fixation*

As an alternative to formalin fixation described above, a biological sample can be fixed in any of a variety of other fixatives to preserve the biological structure of the sample prior to analysis. For example, a sample can be fixed via immersion in ethanol, methanol, acetone, formaldehyde (*e.g.*, 2% formaldehyde), paraformaldehyde-Triton, glutaraldehyde, or combinations thereof.

In some embodiments, acetone fixation is used with fresh frozen samples, which can include, but are not limited to, cortex tissue, mouse olfactory bulb, human brain tumor, human post-mortem brain, and breast cancer samples. In some embodiments, a compatible fixation method is chosen and/or optimized based on a desired workflow. For example, formaldehyde fixation may be chosen as compatible for workflows using IHC/IF protocols for protein visualization. As another example, methanol fixation may be chosen for workflows emphasizing RNA/DNA library quality. Acetone fixation may be chosen in some applications to permeabilize the tissue. When acetone fixation is performed, pre- permeabilization steps (described below) may not be performed. Alternatively, acetone fixation can be performed in conjunction with permeabilization steps.

### *(5) Embedding*

As an alternative to paraffin embedding described above, a biological sample can be embedded in any of a variety of other embedding materials to provide a substrate to the sample prior to sectioning and other handling steps. In general, the embedding material is removed prior to analysis of tissue sections obtained from the sample. Suitable embedding materials include, but are not limited to, waxes, resins (*e.g.*, methacrylate resins), epoxies, and agar.

### *(6) Staining*

To facilitate visualization, biological samples can be stained using a wide variety of stains and staining techniques. In some embodiments, for example, a sample can be stained using any number of biological stains, including but not limited to, acridine orange, Bismarck brown, carmine, coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, or safranin.

The sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the sample is stained using a detectable label (*e.g.*, radioisotopes, fluorophores, chemiluminescent compounds, bioluminescent compounds, and dyes) as described elsewhere herein. In some embodiments, a biological sample is stained using only one type of stain or one technique. In some embodiments, staining includes biological staining techniques such as H&E staining. In some embodiments, staining includes identifying analytes using fluorescently-conjugated antibodies. In some embodiments, a biological sample is stained using two or more different types of stains, or two or more different staining techniques. For example, a biological sample can be prepared by staining and imaging using one technique (*e.g.*, H&E staining and brightfield imaging), followed by staining and imaging using another technique (*e.g.*, IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destained by washing the sample in HCl, or any other low pH acid (*e.g.*, selenic acid, sulfuric acid, hydroiodic acid, benzoic acid, carbonic acid, malic acid, phosphoric acid, oxalic acid, succinic acid, salicylic acid, tartaric acid, sulfurous acid, trichloroacetic acid, hydrobromic acid, hydrochloric acid, nitric acid, orthophosphoric acid, arsenic acid, selenous acid, chromic acid, citric acid, hydrofluoric acid, nitrous acid, isocyanic acid, formic acid, hydrogen selenide, molybdic acid, lactic acid, acetic acid, carbonic acid, hydrogen sulfide, or combinations thereof). In some embodiments, destaining can include 1, 2, 3, 4, 5, or more washes in a low pH acid (*e.g.*, HCl). In some embodiments, destaining can include adding HCl to a downstream solution (*e.g.*, permeabilization solution). In some embodiments, destaining can include dissolving an enzyme used in the disclosed methods (*e.g.*, pepsin) in a low pH acid (*e.g.*, HCl) solution. In some embodiments, after destaining hematoxylin with a low pH acid, other reagents can be added to the destaining solution to raise the pH for use in other applications. For example, SDS can be added to a low pH acid destaining solution in order to raise the pH as compared to the low pH acid destaining solution alone. As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., 2017, J. Histochem. Cytochem. 65(8): 431-444, Lin et al., 2015, Nat Commun. 6:8390, Pirici et al., 2009, J. Histochem. Cytochem. 57:567-75, and Glass et al., 2009, J. Histochem. Cytochem. 57:899-905, the entire contents of each of which are incorporated herein by reference.

### *(7) Hydrogel embedding*

In some embodiments, hydrogel formation occurs within a biological sample. In some embodiments, a biological sample (*e.g.*, tissue section) is embedded in a hydrogel. In some embodiments, hydrogel subunits are infused into the biological sample, and polymerization of the hydrogel is initiated by an external or internal stimulus. A "hydrogel" as described herein can include a cross-linked 3D network of hydrophilic polymer chains. A "hydrogel subunit" can be a hydrophilic monomer, a molecular precursor, or a polymer that can be polymerized (*e.g.*, cross-linked) to form a three-dimensional (3D) hydrogel network.

A hydrogel can swell in the presence of water. In some embodiments, a hydrogel comprises a natural material. In some embodiments, a hydrogel includes a synthetic material. In some embodiments, a hydrogel includes a hybrid material, *e.g.*, the hydrogel material comprises elements of both synthetic and natural polymers. Any of the materials used in hydrogels or hydrogels comprising a polypeptide-based material described herein can be used. Embedding the sample in this manner typically involves contacting the biological sample with a hydrogel such that the biological sample becomes surrounded by the hydrogel. For example, the sample can be embedded by contacting the sample with a suitable polymer material, and activating the polymer material to form a hydrogel. In some embodiments, the hydrogel is formed such that the hydrogel is internalized within the biological sample.

In some embodiments, the biological sample is immobilized in the hydrogel via cross-linking of the polymer material that forms the hydrogel. Cross-linking can be performed chemically and/or photochemically, or alternatively by any other hydrogel-formation method known in the art. For example, the biological sample can be immobilized in the hydrogel by polyacrylamide crosslinking. Further, analytes of a biological sample can be immobilized in a hydrogel by crosslinking (*e.g.*, polyacrylamide crosslinking).

The composition and application of the hydrogel-matrix to a biological sample typically depends on the nature and preparation of the biological sample (*e.g.*, sectioned, non-sectioned, fresh-frozen, type of fixation). A hydrogel can be any appropriate hydrogel where upon formation of the hydrogel on the biological sample the biological sample becomes anchored to or embedded in the hydrogel. Non-limiting examples of hydrogels are described herein or are known in the art. As one example, where the biological sample is a tissue section, the hydrogel can include a monomer solution and an ammonium persulfate (APS) initiator / tetramethylethylenediamine (TEMED) accelerator solution. As another example, where the biological sample consists of cells (*e.g.*, cultured cells or cells disassociated from a tissue sample), the cells can be incubated with the monomer solution and APS/TEMED solutions. For cells, hydrogel is formed in compartments, including but not limited to devices used to culture, maintain, or transport the cells. For example, hydrogels can be formed with monomer solution plus APS/TEMED added to the compartment to a depth ranging from about 0.1 $\mu$m to about 2 mm.

Additional methods and aspects of hydrogel embedding of biological samples are described for example in Chen et al., 2015, Science 347(6221):543-548, and PCT publication 202020176788A1 entitled "Profiling of biological analytes with spatially barcoded oligonucleotide arrays," the entire contents of each of which are incorporated herein by reference.

### (8) Biological Sample Transfer

In some embodiments, a biological sample immobilized on a substrate (*e.g.*, a biological sample prepared using methanol fixation or formalin-fixation and paraffin-embedding (FFPE)) is transferred to a spatial array using a hydrogel. In some embodiments, a hydrogel is formed on top of a biological sample on a substrate (*e.g.*, glass slide). For example, hydrogel formation can occur in a manner sufficient to anchor (*e.g.*, embed) the biological sample to the hydrogel. After hydrogel formation, the biological sample is anchored to (*e.g.*, embedded in) the hydrogel where separating the hydrogel from the substrate results in the biological sample separating from the substrate along with the hydrogel. The biological sample can then be contacted with a spatial array, thereby allowing spatial profiling of the biological sample. In some embodiments, the hydrogel is removed after contacting the biological sample with the spatial array. For example, methods described herein can include an event-dependent (*e.g.*, light or chemical) depolymerizing hydrogel, where upon application of the event (*e.g.*, external stimuli) the hydrogel depolymerizes. In one example, a biological sample can be anchored to a DTT-sensitive hydrogel, where addition of DTT can cause the hydrogel to depolymerize and release the anchored biological sample. A hydrogel can be any appropriate hydrogel where upon formation of the hydrogel on the biological sample the biological sample becomes anchored to or embedded in the hydrogel. Non-limiting examples of hydrogels are described herein or are known in the art. In some embodiments, a hydrogel includes a linker that allows anchoring of the biological sample to the hydrogel. In some embodiments, a hydrogel includes linkers that allow anchoring of biological analytes to the hydrogel. In such cases, the linker can be added to the hydrogel before, contemporaneously with, or after hydrogel formation. Non-limiting examples of linkers that anchor nucleic acids to the hydrogel can include 6-((Acryloyl)amino) hexanoic acid (Acryloyl-X SE) (available from ThermoFisher, Waltham, MA), Label-IT Amine (available from MirusBio, Madison, WI) and Label X (Chen et al., Nat. Methods 13:679-684, 2016). Any variety of characteristics can determine the transfer conditions required for a given biological sample. Non-limiting examples of characteristics likely to impact transfer conditions include the sample (*e.g.*, thickness, fixation, and cross-linking) and/or the analyte of interest (different conditions to preserve and/or transfer different analytes (*e.g.*, DNA, RNA, and protein)). In some embodiments, hydrogel formation can occur in a manner sufficient to anchor the analytes (*e.g.*, embed) in the biological sample to the hydrogel. In some embodiments, the hydrogel can be imploded (*e.g.*, shrunk) with the anchored analytes (*e.g.*, embedded in the hydrogel) present in the biological sample. In some embodiments, the hydrogel can be expanded (*e.g.*, isometric expansion) with the anchored analytes (*e.g.*, embedded in the hydrogel) present in the biological sample. In some embodiments, the hydrogel can be imploded (*e.g.*, shrunk) and subsequently expanded with anchored analytes (*e.g.*, embedded in the hydrogel) present in the biological sample.

### (9) Isometric expansion

In some embodiments, a biological sample embedded in a hydrogel can be isometrically expanded. Isometric expansion methods that can be used include hydration, a preparative step in expansion microscopy, as described in Chen et al., 2015, Science 347(6221) 543-548, Asano et al., 2018, Current Protocols 80:1, doi:10.1002/cpcb.56; Gao et al., 2017, BMC Biology 15:50, doi:10.1186/s12915-017-0393-3, and Wassie et al, 2018, Expansion microscopy: principles and uses in biological research, Nature Methods 16(1): 33-41, each of which is incorporated by reference in its entirety.

In general, the steps used to perform isometric expansion of the biological sample can depend on the characteristics of the sample (*e.g.*, thickness of tissue section, fixation, cross-linking), and/or the analyte of interest (*e.g.*, different conditions to anchor RNA, DNA, and protein to a gel).

Isometric expansion can be performed by anchoring one or more components of a biological sample to a gel, followed by gel formation, proteolysis, and swelling. Isometric expansion of the biological sample can occur prior to immobilization of the biological sample on a substrate, or after the biological sample is immobilized to a substrate. In some embodiments, the isometrically expanded biological sample can be removed from the substrate prior to contacting expanded biological sample with a spatially barcoded array (e.g., spatially barcoded capture probes on a substrate).

In some embodiments, proteins in the biological sample are anchored to a swellable gel such as a polyelectrolyte gel. An antibody can be directed to the protein before, after, or in conjunction with being anchored to the swellable gel. DNA and/or RNA in a biological sample can also be anchored to the swellable gel via a suitable linker. Examples of such linkers include, but are not limited to, 6-((Acryloyl)amino) hexanoic acid (Acryloyl-X SE) (available from ThermoFisher, Waltham, MA), Label-IT Amine (available from MirusBio, Madison, WI) and Label X (described for example in Chen et al., Nat. Methods 13:679-684, 2016, the entire contents of which are incorporated herein by reference).

Isometric expansion of the sample can increase the spatial resolution of the subsequent analysis of the sample. For example, isometric expansion of the biological sample can result in increased resolution in spatial profiling (e.g., single-cell profiling).The increased resolution in spatial profiling can be determined by comparison of an isometrically expanded sample with a sample that has not been isometrically expanded.

Isometric expansion can enable three-dimensional spatial resolution of the subsequent analysis of the sample. In some embodiments, isometric expansion of the biological sample can occur in the presence of spatial profiling reagents (*e.g.*, analyte capture agents or capture probes). For example, the swellable gel can include analyte capture agents or capture probes anchored to the swellable gel via a suitable linker. In some embodiments, spatial profiling reagents can be delivered to particular locations in an isometrically expanded biological sample.

In some embodiments, a biological sample is isometrically expanded to a volume at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded volume. In some embodiments, the sample is isometrically expanded to at least 2x and less than 20x of its non-expanded volume.

In some embodiments, a biological sample embedded in a hydrogel is isometrically expanded to a volume at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded volume. In some embodiments, the biological sample embedded in a hydrogel is isometrically expanded to at least 2x and less than 20x of its non-expanded volume.

### (10) Substrate attachment

In some embodiments, the biological sample can be attached to a substrate (*e.g.*, a chip). Examples of substrates suitable for this purpose are described in detail below. Attachment of the biological sample can be irreversible or reversible, depending upon the nature of the sample and subsequent steps in the analytical method.

In certain embodiments, the sample can be attached to the substrate reversibly by applying a suitable polymer coating to the substrate, and contacting the sample to the polymer coating. The sample can then be detached from the substrate using an organic solvent that at least partially dissolves the polymer coating. Hydrogels are examples of polymers that are suitable for this purpose.

More generally, in some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

### (11) Unaggregated of cells

In some embodiments, the biological sample corresponds to cells (*e.g.*, derived from a cell culture or a tissue sample). In a cell sample with a plurality of cells, individual cells can be naturally unaggregated. For example, the cells can be derived from a suspension of cells and/or disassociated or disaggregated cells from a tissue or tissue section.

Alternatively, the cells in the sample may be aggregated, and may be disaggregated into individual cells using, for example, enzymatic or mechanical techniques. Examples of enzymes used in enzymatic disaggregation include, but are not limited to, dispase, collagenase, trypsin, or combinations thereof. Mechanical disaggregation can be performed, for example, using a tissue homogenizer.

In some embodiments of unaggregated cells or disaggregated cells, the cells are distributed onto the substrate such that at least one cell occupies a distinct spatial feature on the substrate. The cells can be immobilized on the substrate (*e.g.*, to prevent lateral diffusion of the cells). In some embodiments, a cell immobilization agent can be used to immobilize a non-aggregated or disaggregated sample on a spatially-barcoded array prior to analyte capture. A "cell immobilization agent" can refer to an antibody, attached to a substrate, which can bind to a cell surface marker. In some embodiments, the distribution of the plurality of cells on the substrate follows Poisson statistics.

In some embodiments, cells from a plurality of cells are immobilized on a substrate. In some embodiments, the cells are immobilized to prevent lateral diffusion, for example, by adding a hydrogel and/or by the application of an electric field.

### (12) Suspended and adherent cells

In some embodiments, the biological sample can be derived from a cell culture grown in vitro. Samples derived from a cell culture can include one or more suspension cells which are anchorage-independent within the cell culture. Examples of such cells include, but are not limited to, cell lines derived from hematopoietic cells, and from the following cell lines: Colo205, CCRF-CEM, HL-60, K562, MOLT-4, RPMI-8226, SR, HOP-92, NCI-H322M, and MALME-3M.

Samples derived from a cell culture can include one or more adherent cells that grow on the surface of the vessel that contains the culture medium. Additional non-limiting examples of suspended and adherent cells is found in United States Patent Application No. 16/992,569 entitled "Systems and Methods for Using the Spatial Distributions on Haplotypes to Determine a Biological Condition," filed August 13, 2020, and PCT publication No. 202020176788A1 entitled "Profiling of biological analyes with spatially barcoded oligonucleotide arrays" the entire contents of each of which are incorporated herein by reference.

In some embodiments, a biological sample can be permeabilized to facilitate transfer of analytes out of the sample, and/or to facilitate transfer of species (such as capture probes) into the sample. If a sample is not permeabilized sufficiently, the amount of analyte captured from the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (*e.g.*, acetone, ethanol, and methanol), cross-linking agents (e.g., paraformaldehyde), detergents (*e.g.*, saponin, Triton X-100™, Tween-20™, or sodium dodecyl sulfate (SDS)), and enzymes (*e.g.*, trypsin, proteases (*e.g.*, proteinase K). In some embodiments, the detergent is an anionic detergent (*e.g.*, SDS or N-lauroylsarcosine sodium salt solution). In some embodiments, the biological sample can be permeabilized using any of the methods described herein (e.g., using any of the detergents described herein, *e.g.*, SDS and/or N-lauroylsarcosine sodium salt solution) before or after enzymatic treatment (*e.g.*, treatment with any of the enzymes described herein, e.g., trypin, proteases (*e.g.*, pepsin and/or proteinase K)).

In some embodiments, a biological sample can be permeabilized by exposing the sample to greater than about 10 w/v % (e.g., greater than about 20 w/v %, greater than about 30 w/v %, greater than about 40 w/v%, greater than about 50 w/v %, greater than about 6.0 w/v %, greater than about 7.0 w/v %, greater than about 8.0 w/v %, greater than about 9.0 w/v %, greater than about 10.0 w/v %, greater than about 11.0 w/v %, greater than about 12.0 w/v %, or greater than about 13.0 w/v %) sodium dodecyl sulfate (SDS) and/or N-lauroylsarcosine or N-lauroylsarcosine sodium salt. In some embodiments, a biological sample can be permeabilized by exposing the sample (e.g., for about 5 minutes to about 1 hour, about 5 minutes to about 40 minutes, about 5 minutes to about 30 minutes, about 5 minutes to about 20 minutes, or about 5 minutes to about 10 minutes) to about 10 w/v % to about 14.0 w/v % (e.g., about 20 w/v % to about 14.0 w/v %, about 2.0 w/v % to about 12.0 w/v %, about 2.0 w/v % to about 10.0 w/v %, about 4.0 w/v % to about 14.0 w/v %, about 4.0 w/v % to about 12.0 w/v %, about 4.0 w/v % to about 10.0 w/v %, about 6.0 w/v % to about 14.0 w/v %, about 6.0 w/v % to about 12.0 w/v %, about 6.0 w/v % to about 10.0 w/v %, about 8.0 w/v % to about 14.0 w/v %, about 8.0 w/v % to about 12.0 w/v %, about 8.0 w/v % to about 10.0 w/v %, about 10.0 % w/v % to about 14.0 w/v %, about 10.0 w/v % to about 12.0 w/v %, or about 12.0 w/v % to about 14.0 w/v %) SDS and/or N-lauroylsarcosine salt solution and/or proteinase K (e.g., at a temperature of about 4% to about 35 °C, about 4 ° C to about 25 °C, about 4 ° C to about 20 °C, about 4 °C to about 10 °C, about 10 °C to about 25 °C, about 10 °C to about 20 °C, about 10 °C to about 15 °C, about 35 °C to about 50 °C, about 35 °C to about 45 °C, about 35 °C to about 40 °C, about 40 °C to about 50 °C, about 40 °C to about 45 °C, or about 45 °C to about 50 °C).

In some embodiments, the biological sample can be incubated with a permeabilizing agent to facilitate permeabilization of the sample. Additional methods for sample permeabilization are described, for example, in Jamur et al., 2010, Method Mol. Biol. 588:63-66, 2010, the entire contents of which are incorporated herein by reference.

### Lysis Reagents

In some embodiments, the biological sample can be permeabilized by adding one or more lysis reagents to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes.

Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

In some embodiments, the biological sample can be permeabilized by non-chemical permeabilization methods. Non-chemical permeabilization methods are known in the art. For example, non-chemical permeabilization methods that can be used include, but are not limited to, physical lysis techniques such as electroporation, mechanical permeabilization methods (*e.g.*, bead beating using a homogenizer and grinding balls to mechanically disrupt sample tissue structures), acoustic permeabilization (*e.g.*, sonication), and thermal lysis techniques such as heating to induce thermal permeabilization of the sample.

### Proteases

In some embodiments, a medium, solution, or permeabilization solution may contain one or more proteases. In some embodiments, a biological sample treated with a protease capable of degrading histone proteins can result in the generation of fragmented genomic DNA. The fragmented genomic DNA can be captured using the same capture domain (*e.g.*, capture domain having a poly(T) sequence) used to capture mRNA. In some embodiments, a biological sample is treated with a protease capable of degrading histone proteins and an RNA protectant prior to spatial profiling in order to facilitate the capture of both genomic DNA and mRNA.

In some embodiments, a biological sample is permeabilized by exposing the sample to a protease capable of degrading histone proteins. As used herein, the term "histone protein" typically refers to a linker histone protein (*e.g.,* H1) and/or a core histone protein (*e.g.,* H2A, H2B, H3, and H4). In some embodiments, a protease degrades linker histone proteins, core histone proteins, or linker histone proteins and core histone proteins. Any suitable protease capable of degrading histone proteins in a biological sample can be used. Non-limiting examples of proteases capable of degrading histone proteins include proteases inhibited by leupeptin and TLCK (Tosyl-L-lysyl-chloromethane hydrochloride), a protease encoded by the EUO gene from *Chlamydia trachomatis serovar A,* granzyme A, a serine protease (*e.g.,* trypsin or trypsin-like protease, neutral serine protease, elastase, cathepsin G), an aspartyl protease (*e.g.,* cathepsin D), a peptidase family C1 enzyme (e.g., cathepsin L), pepsin, proteinase K, a protease that is inhibited by the diazomethane inhibitor Z-Phe-Phe-CHN(2) or the epoxide inhibitor E-64, a lysosomal protease, or an azurophilic enzyme (e.g., cathepsin G, elastase, proteinase 3, neutral serine protease). In some embodiments, a serine protease is a trypsin enzyme, trypsin-like enzyme or a functional variant or derivative thereof (*e.g.,* P00761; C0HK48; Q8IYP2; Q8BW11; Q6IE06; P35035; P00760; P06871; Q90627; P16049; P07477; P00762; P35031; P19799; P35036; Q29463; P06872; Q90628; P07478; P07146; P00763; P35032; P70059; P29786; P35037; Q90629; P35030; P08426; P35033; P35038; P12788; P29787; P35039; P35040; Q8NHM4; P35041; P35043; P35044; P54624; P04814; P35045; P32821; P54625; P35004; P35046; P32822; P35047; C0HKA5; C0HKA2; P54627; P35005; C0HKA6; C0HKA3; P52905; P83348; P00765; P35042; P81071; P35049; P51588; P35050; P35034; P35051; P24664; P35048; P00764; P00775; P54628; P42278; P54629; P42279; Q91041; P54630; P42280; C0HKA4) or a combination thereof. In some embodiments, a trypsin enzyme is P00761, P00760, Q29463, or a combination thereof. In some embodiments, a protease capable of degrading one or more histone proteins comprises an amino acid sequence with at least 80% sequence identity to P00761, P00760, or Q29463. In some embodiments, a protease capable of degrading one or more histone proteins comprises an amino acid sequence with at least 85%, 90%, 91%, 92%, 93%, 94%, 95%, 96%, 97%, 98%, or 99% identity to P00761, P00760, or Q29463. A protease may be considered a functional variant if it has at least 50% *e.g.,* at least 60%, 70%, 80%, 90%, 95%, 96%, 97%, 98%, 99%, or 100% of the activity relative to the activity of the protease in condition optimum for the enzyme. In some embodiments, the enzymatic treatment with pepsin enzyme, or pepsin like enzyme, can include: P03954/PEPA1_MACFU; P28712/PEPA1_RABIT; P27677/PEPA2_MACFU; P27821/PEPA2_RABIT; P0DJD8/PEPA3_HUMAN; P27822/PEPA3_RABIT; P0DJD7/PEPA4_HUMAN; P27678/PEPA4_MACFU; P28713/PEPA4_RABIT; P0DJD9/PEPA5_HUMAN; Q9D106/PEPA5_MOUSE; P27823/PEPAF_RABIT; P00792/PEPA_BOVIN; Q9N2D4/PEPA_CALJA; Q9GMY6/PEPA_CANLF; P00793/PEPA_CHICK; P11489/PEPA_MACMU; P00791/PEPA_PIG; Q9GMY7/PEPA_RHIFE; Q9GMY8/PEPA_SORUN; P81497/PEPA_SUNMU; P13636/PEPA_URSTH and functional variants and derivatives thereof, or a combination thereof. In some embodiments, the pepsin enzyme can include: P00791/PEPA_PIG; P00792/PEPA_BOVIN, functional variants, derivatives, or combinations thereof.

Additionally, the protease may be contained in a reaction mixture (solution), which also includes other components (*e.g.,* buffer, salt, chelator (*e.g.,* EDTA), and/or detergent (*e.g.,* SDS, N-Lauroylsarcosine sodium salt solution)). The reaction mixture may be buffered, having a pH of about 6.5-8.5, *e.g.,* about 7.0-8.0. Additionally, the reaction mixture may be used at any suitable temperature, such as about 10-50°C, *e.g.,* about 10-44°C, 11-43°C, 12-42°C, 13-41°C, 14-40°C, 15-39°C, 16-38 °C, 17-37°C, *e.g.,* about 10°C, 12°C, 15°C, 18°C, 20°C, 22°C, 25°C, 28°C, 30°C, 33°C, 35°C or 37 °C, preferably about 35-45°C, *e.g.,* about 37°C.

### *Other Reagents*

In some embodiments, a permeabilization solution can contain additional reagents or a biological sample may be treated with additional reagents in order to optimize biological sample permeabilization. In some embodiments, an additional reagent is an RNA protectant. As used herein, the term "RNA protectant" typically refers to a reagent that protects RNA from RNA nucleases (*e.g.,* RNases). Any appropriate RNA protectant that protects RNA from degradation can be used. A non-limiting example of a RNA protectant includes organic solvents (*e.g.,* at least 60%, 65%, 70%, 75%, 80%, 85%, 90%, or 95% v/v organic solvent), which include, without limitation, ethanol, methanol, propan-2-ol, acetone, trichloroacetic acid, propanol, polyethylene glycol, acetic acid, or a combination thereof. In some embodiments, a RNA protectant includes ethanol, methanol and/or propan-2-ol, or a combination thereof. In some embodiments, a RNA protectant includes RNAlater ICE (ThermoFisher Scientific). In some embodiments, the RNA protectant comprises at least about 60% ethanol. In some embodiments, the RNA protectant comprises about 60-95% ethanol, about 0-35% methanol and about 0-35% propan-2-ol, where the total amount of organic solvent in the medium is not more than about 95%. In some embodiments, the RNA protectant comprises about 60-95% ethanol, about 5-20% methanol and about 5-20% propan-2-ol, where the total amount of organic solvent in the medium is not more than about 95%.

In some embodiments, the RNA protectant includes a salt. The salt may include ammonium sulfate, ammonium bisulfate, ammonium chloride, ammonium acetate, cesium sulfate, cadmium sulfate, cesium iron (II) sulfate, chromium (III) sulfate, cobalt (II) sulfate, copper (II) sulfate, lithium chloride, lithium acetate, lithium sulfate, magnesium sulfate, magnesium chloride, manganese sulfate, manganese chloride, potassium chloride, potassium sulfate, sodium chloride, sodium acetate, sodium sulfate, zinc chloride, zinc acetate and zinc sulfate. In some embodiments, the salt is a sulfate salt, for example, ammonium sulfate, ammonium bisulfate, cesium sulfate, cadmium sulfate, cesium iron (II) sulfate, chromium (III) sulfate, cobalt (II) sulfate, copper (II) sulfate, lithium sulfate, magnesium sulfate, manganese sulfate, potassium sulfate, sodium sulfate, or zinc sulfate. In some embodiments, the salt is ammonium sulfate. The salt may be present at a concentration of about 20 g/100 ml of medium or less, such as about 15g/100 ml, 10g/100 ml, 9g/100 ml, 8g/100 ml, 7g/100 ml, 6g/100 ml, 5g/100 ml or less, *e.g.*, about 4g, 3g, 2g or 1g/100ml.

Additionally, the RNA protectant may be contained in a medium that further includes a chelator (*e.g.*, EDTA), a buffer (*e.g.*, sodium citrate, sodium acetate, potassium citrate, or potassium acetate, preferably sodium acetate), and/or buffered to a pH between about 4-8 (*e.g.*, about 5).

In some embodiments, the biological sample is treated with one or more RNA protectants before, contemporaneously with, or after permeabilization. For example, a biological sample is treated with one or more RNA protectants prior to treatment with one or more permeabilization reagents (*e.g.*, one or more proteases). In another example, a biological sample is treated with a solution including one or more RNA protectants and one or more permeabilization reagents (e.g., one or more proteases). In yet another example, a biological sample is treated with one or more RNA protectants after the biological sample has been treated with one or more permeabilization reagents (*e.g.*, one or more proteases). In some embodiments, a biological sample is treated with one or more RNA protectants prior to fixation.

In some embodiments, identifying the location of the captured analyte in the biological sample includes a nucleic acid extension reaction. In some embodiments where a capture probe captures a fragmented genomic DNA molecule, a nucleic acid extension reaction includes DNA polymerase. For example, a nucleic acid extension reaction includes using a DNA polymerase to extend the capture probe that is hybridized to the captured analyte (*e.g.*, fragmented genomic DNA) using the captured analyte (*e.g.*, fragmented genomic DNA) as a template. The product of the extension reaction includes a spatially-barcoded analyte (*e.g.*, spatially-barcoded fragmented genomic DNA). The spatially-barcoded analyte (*e.g.*, spatially-barcoded fragmented genomic DNA) can be used to identify the spatial location of the analyte in the biological sample. Any DNA polymerase that is capable of extending the capture probe using the captured analyte as a template can be used for the methods described herein. Non-limiting examples of DNA polymerases include T7 DNA polymerase; Bsu DNA polymerase; and E.coli DNA Polymerase pol I.

### *Diffusion-Resistant Media*

In some embodiments, a diffusion-resistant medium, typically used to limit diffusion of analytes, can include at least one permeabilization reagent. For example, the diffusion-resistant medium (*e.g.*, a hydrogel) can include wells (*e.g.*, micro-, nano-, or picowells or pores) containing a permeabilization buffer or reagents. In some embodiments, the diffusion-resistant medium (*e.g.*, a hydrogel) is soaked in permeabilization buffer prior to contacting the hydrogel with a sample. In some embodiments, the hydrogel or other diffusion-resistant medium can contain dried reagents or monomers to deliver permeabilization reagents when the diffusion-resistant medium is applied to a biological sample. In some embodiments, the diffusion-resistant medium, (*e.g.*, hydrogel) is covalently attached to a solid substrate (*e.g.*, an acrylated glass slide).

In some embodiments, the hydrogel can be modified to both deliver permeabilization reagents and contain capture probes. For example, a hydrogel film can be modified to include spatially-barcoded capture probes. The spatially-barcoded hydrogel film is then soaked in permeabilization buffer before contacting the spatially-barcoded hydrogel film to the sample. In another example, a hydrogel can be modified to include spatially-barcoded capture probes and designed to serve as a porous membrane (*e.g.*, a permeable hydrogel) when exposed to permeabilization buffer or any other biological sample preparation reagent. The permeabilization reagent diffuses through the spatially-barcoded permeable hydrogel and permeabilizes the biological sample on the other side of the hydrogel. The analytes then diffuse into the spatially-barcoded hydrogel after exposure to permeabilization reagents. In such cases, the spatially-barcoded hydrogel (*e.g.*, porous membrane) is facilitating the diffusion of the biological analytes in the biological sample into the hydrogel. In some embodiments, biological analytes diffuse into the hydrogel before exposure to permeabilization reagents (*e.g.*, when secreted analytes are present outside of the biological sample or in instances where a biological sample is lysed or permeabilized by other means prior to addition of permeabilization reagents). In some embodiments, the permeabilization reagent is flowed over the hydrogel at a variable flow rate (*e.g.*, any flow rate that facilitates diffusion of the permeabilization reagent across the spatially-barcoded hydrogel). In some embodiments, the permeabilization reagents are flowed through a microfluidic chamber or channel over the spatially-barcoded hydrogel. In some embodiments, after using flow to introduce permeabilization reagents to the biological sample, biological sample preparation reagents can be flowed over the hydrogel to further facilitate diffusion of the biological analytes into the spatially-barcoded hydrogel. The spatially-barcoded hydrogel film thus delivers permeabilization reagents to a sample surface in contact with the spatially-barcoded hydrogel, enhancing analyte migration and capture. In some embodiments, the spatially-barcoded hydrogel is applied to a sample and placed in a permeabilization bulk solution. In some embodiments, the hydrogel film soaked in permeabilization reagents is sandwiched between a sample and a spatially-barcoded array. In some embodiments, target analytes are able to diffuse through the permeabilizing reagent soaked hydrogel and hybridize or bind the capture probes on the other side of the hydrogel. In some embodiments, the thickness of the hydrogel is proportional to the resolution loss. In some embodiments, wells (*e.g.*, micro-, nano-, or picowells) can contain spatially-barcoded capture probes and permeabilization reagents and/or buffer. In some embodiments, spatially-barcoded capture probes and permeabilization reagents are held between spacers. In some embodiments, the sample is punch, cut, or transferred into the well, where a target analyte diffuses through the permeabilization reagent/buffer and to the spatially-barcoded capture probes. In some embodiments, resolution loss may be proportional to gap thickness (*e.g.*, the amount of permeabilization buffer between the sample and the capture probes). In some embodiments, the diffusion-resistant medium (*e.g.*, hydrogel) is between approximately 50-500 micrometers thick including 500, 450, 400, 350, 300, 250, 200, 150, 100, or 50 micrometers thick, or any thickness within 50 and 500 micrometers.

In some embodiments, a biological sample is exposed to a porous membrane (*e.g.*, a permeable hydrogel) to aid in permeabilization and limit diffusive analyte losses, while allowing permeabilization reagents to reach a sample. Membrane chemistry and pore volume can be manipulated to minimize analyte loss. In some embodiments, the porous membrane may be made of glass, silicon, paper, hydrogel, polymer monoliths, or other material. In some embodiments, the material may be naturally porous. In some embodiments, the material may have pores or wells etched into solid material. In some embodiments, the permeabilization reagents are flowed through a microfluidic chamber or channel over the porous membrane. In some embodiments, the flow controls the sample's access to the permeabilization reagents. In some embodiments, the porous membrane is a permeable hydrogel. For example, a hydrogel is permeable when permeabilization reagents and/or biological sample preparation reagents can pass through the hydrogel using diffusion. Any suitable permeabilization reagents and/or biological sample preparation reagents described herein can be used under conditions sufficient to release analytes (*e.g.*, nucleic acid, protein, metabolites, lipids, etc.) from the biological sample. In some embodiments, a hydrogel is exposed to the biological sample on one side and permeabilization reagent on the other side. The permeabilization reagent diffuses through the permeable hydrogel and permeabilizes the biological sample on the other side of the hydrogel. In some embodiments, permeabilization reagents are flowed over the hydrogel at a variable flow rate (*e.g.*, any flow rate that facilitates diffusion of the permeabilization reagent across the hydrogel). In some embodiments, the permeabilization reagents are flowed through a microfluidic chamber or channel over the hydrogel. Flowing permeabilization reagents across the hydrogel enables control of the concentration of reagents. In some embodiments, hydrogel chemistry and pore volume can be tuned to enhance permeabilization and limit diffusive analyte losses.

In some embodiments, a porous membrane is sandwiched between a spatially-barcoded array and the sample, where permeabilization solution is applied over the porous membrane. The permeabilization reagents diffuse through the pores of the membrane and into the biological sample. In some embodiments, the biological sample can be placed on a substrate (*e.g.*, a glass slide). Biological analytes then diffuse through the porous membrane and into to the space containing the capture probes. In some embodiments, the porous membrane is modified to include capture probes. For example, the capture probes can be attached to a surface of the porous membrane using any of the methods described herein. In another example, the capture probes can be embedded in the porous membrane at any depth that allows interaction with a biological analyte. In some embodiments, the porous membrane is placed onto a biological sample in a configuration that allows interaction between the capture probes on the porous membrane and the biological analytes from the biological sample. For example, the capture probes are located on the side of the porous membrane that is proximal to the biological sample. In such cases, permeabilization reagents on the other side of the porous membrane diffuse through the porous membrane into the location containing the biological sample and the capture probes in order to facilitate permeabilization of the biological sample (*e.g.*, also facilitating capture of the biological analytes by the capture probes). In some embodiments, the porous membrane is located between the sample and the capture probes. In some embodiments, the permeabilization reagents are flowed through a microfluidic chamber or channel over the porous membrane.

### *Selective Permeabilization/Selective Lysis*

In some embodiments, biological samples can be processed to selectively release an analyte from a subcellular region of a cell according to established methods. In some embodiments, a method provided herein can include detecting at least one biological analyte present in a subcellular region of a cell in a biological sample. As used herein, a "subcellular region" can refer to any subcellular region. For example, a subcellular region can refer to cytosol, a mitochondria, a nucleus, a nucleolus, an endoplasmic reticulum, a lysosome, a vesicle, a Golgi apparatus, a plastid, a vacuole, a ribosome, cytoskeleton, or combinations thereof. In some embodiments, the subcellular region comprises at least one of cytosol, a nucleus, a mitochondria, and a microsome. In some embodiments, the subcellular region is cytosol. In some embodiments, the subcellular region is a nucleus. In some embodiments, the subcellular region is a mitochondria. In some embodiments, the subcellular region is a microsome.

For example, a biological analyte can be selectively released from a subcellular region of a cell by selective permeabilization or selective lysing. In some embodiments, "selective permeabilization" can refer to a permeabilization method that can permeabilize a membrane of a subcellular region while leaving a different subcellular region substantially intact (*e.g.*, biological analytes are not released from subcellular region due to the applied permeabilization method). Non-limiting examples of selective permeabilization methods include using electrophoresis and/or applying a permeabilization reagent. In some embodiments, "selective lysing" can refer to a lysis method that can lyse a membrane of a subcellular region while leaving a different subcellular region substantially intact (*e.g.*, biological analytes are not released from subcellular region due to the applied lysis method). Several methods for selective permeabilization or lysis are known to one of skill in the art including the methods described in Lu et al. Lab Chip. 2005 Jan;5(1):23-9; Niklas et al., 2011, Anal Biochem 416(2):218-27; Cox and Emili., 2006, Nat Protoc. 1(4):1872-8; Chiang et al., 2000, J Biochem. Biophys. Methods. 20;46(1-2):53-68; and Yamauchi and Herr et al., 2017, Microsyst. Nanoeng. 3. pii: 16079; each of which is incorporated herein by reference in its entirety.

In some embodiments, "selective permeabilization" or "selective lysis" refer to the selective permeabilization or selective lysis of a specific cell type. For example, "selective permeabilization" or "selective lysis" can refer to lysing one cell type while leaving a different cell type substantially intact (*e.g.*, biological analytes are not released from the cell due to the applied permeabilization or lysis method). A cell that is a "different cell type" than another cell can refer to a cell from a different taxonomic kingdom, a prokaryotic cell versus a eukaryotic cell, a cell from a different tissue type, *etc*. Many methods are known to one of skill in the art for selectively permeabilizing or lysing different cell types. Non-limiting examples include applying a permeabilization reagent, electroporation, and/or sonication. See, *e.g.*, International Application No. WO 2012/168003; Han et al., 2019, Microsyst Nanoeng. 5:30*;* Gould et al., 2018 Oncotarget. 20; 9(21): 15606-15615; Oren and Shai, 1997, Biochemistry 36(7), 1826-35; Algayer et al., 2019, Molecules. 24(11). pii: E2079; Hipp et al. 2017, Leukemia 10, 2278; International Application No. WO 2012/168003; and U.S. Patent No. 7,785,869; all of which are incorporated by reference herein in their entireties.

In some embodiments, applying a selective permeabilization or lysis reagent comprises contacting the biological sample with a hydrogel comprising the permeabilization or lysis reagent.

In some embodiments, the biological sample is contacted with two or more arrays (*e.g.*, flexible arrays, as described herein). For example, after a subcellular region is permeabilized and a biological analyte from the subcellular region is captured on a first array, the first array can be removed, and a biological analyte from a different subcellular region can be captured on a second array.

### (13) Selective enrichment of RNA species

In some embodiments, where RNA is the analyte, one or more RNA analyte species of interest can be selectively enriched (*e.g.*, Adiconis et. al., 2013, Comparative analysis of RNA sequencing methods for degraded and low-input samples, Nature 10, 623-632, herein incorporated by reference in its entirety). For example, one or more species of RNA can be selected by addition of one or more oligonucleotides to the sample. In some embodiments, the additional oligonucleotide is a sequence used for priming a reaction by a polymerase. For example, one or more primer sequences with sequence complementarity to one or more RNAs of interest can be used to amplify the one or more RNAs of interest, thereby selectively enriching these RNAs. In some embodiments, an oligonucleotide with sequence complementarity to the complementary strand of captured RNA (*e.g.*, cDNA) can bind to the cDNA. For example, biotinylated oligonucleotides with sequence complementary to one or more cDNAs of interest binds to the cDNA and can be selected using biotinylation-streptavidin affinity using any of a variety of methods known to the field (*e.g.*, streptavidin beads).

Alternatively, one or more species of RNA (*e.g.*, ribosomal and/or mitochondrial RNA) can be down-selected (*e.g.*, removed, depleted) using any of a variety of methods. Non-limiting examples of a hybridization and capture method of ribosomal RNA depletion include RiboMinus™, RiboCop™, and Ribo-Zero™. Another non-limiting RNA depletion method involves hybridization of complementary DNA oligonucleotides to unwanted RNA followed by degradation of the RNA/DNA hybrids using RNase H. Non-limiting examples of a hybridization and degradation method include NEBNext® rRNA depletion, NuGEN AnyDeplete, or RiboZero Plus. Another non-limiting ribosomal RNA depletion method includes ZapR™ digestion, for example SMARTer. In the SMARTer method, random nucleic acid adapters are hybridized to RNA for first-strand synthesis and tailing by reverse transcriptase, followed by template switching and extension by reverse transcriptase. Additionally, first round PCR amplification adds full-length Illumina sequencing adapters (e.g., Illumina indexes). Ribosomal RNA is cleaved by ZapR v2 and R probes v2. A second round of PCR is performed, amplifying non-rRNA molecules (e.g., cDNA). Parts or steps of these ribosomal depletion protocols/kits can be further combined with the methods described herein to optimize protocols for a specific biological sample.

In depletion protocols, probes can be administered to a sample that selectively hybridize to ribosomal RNA (rRNA), thereby reducing the pool and concentration of rRNA in the sample. Probes can be administered to a biological sample that selectively hybridize to mitochondria RNA (mtRNA), thereby reducing the pool and concentration of mtRNA in the sample. In some embodiments, probes complementary to mitochondrial RNA can be added during cDNA synthesis, or probes complementary to both ribosomal and mitochondrial RNA can be added during cDNA synthesis. Subsequent application of capture probes to the sample can result in improved capture of other types of RNA due to a reduction in non-specific RNA (e.g. down-selected RNA) present in the sample. Additionally and alternatively, duplex-specific nuclease (DSN) treatment can remove rRNA (see, *e.g.*, Archer et al, 2014, Selective and flexible depletion of problematic sequences from RNA-seq libraries at the cDNA stage, BMC Genomics 15 401, the entire contents of which are incorporated herein by reference). Furthermore, hydroxyapatite chromatography can remove abundant species (*e.g.*, rRNA) (see, *e.g.*, Vandernoot, 2012, "cDNA normalization by hydroxyapatite chromatography to enrich transcriptome diversity in RNA-seq applications," Biotechniques, 53(6) 373-80, the entire contents of which are incorporated herein by reference).

### (14) Other reagents

Additional reagents can be added to a biological sample to perform various functions prior to analysis of the sample. In some embodiments, nuclease inhibitors such as DNase and RNase inactivating agents or protease inhibitors such as proteinase K, and/or chelating agents such as EDTA, can be added to the sample. In other embodiments nucleases, such as DNase or RNAse, or proteases, such as pepsin or proteinase K, are added to the sample. In some embodiments, additional reagents may be dissolved in a solution or applied as a medium to the sample. In some embodiments, additional reagents (e.g., pepsin) may be dissolved in HCl prior to applying to the sample. For example, hematoxylin, from an H&E stain, can be optionally removed from the biological sample by washing in dilute HCl (0.001M to 0.1M) prior to further processing. In some embodiments, pepsin can be dissolved in dilute HCl (0.001M to 0.1M) prior to further processing. In some embodiments, biological samples can be washed additional times (*e.g.*, 2, 3, 4, 5, or more times) in dilute HCl prior to incubation with a protease (*e.g.*, pepsin), but after proteinase K treatment.

In some embodiments, the sample can be treated with one or more enzymes. For example, one or more endonucleases to fragment DNA, DNA polymerase enzymes, and dNTPs used to amplify nucleic acids can be added. Other enzymes that can also be added to the sample include, but are not limited to, polymerase, transposase, ligase, and DNAse, and RNAse.

In some embodiments, reverse transcriptase enzymes can be added to the sample, including enzymes with terminal transferase activity, primers, and template switch oligonucleotides (TSOs). Template switching can be used to increase the length of a cDNA, *e.g.*, by appending a predefined nucleic acid sequence to the cDNA. Such a step of reverse transcription is illustrated in **FIG. 37.** In some embodiments, the appended nucleic acid sequence comprises one or more ribonucleotides.

In some embodiments, additional reagents can be added to improve the recovery of one or more target molecules (*e.g.*, cDNA molecules, mRNA transcripts). For example, addition of carrier RNA to a RNA sample workflow process can increase the yield of extracted RNA/DNA hybrids from the biological sample. In some embodiments, carrier molecules are useful when the concentration of input or target molecules is low as compared to remaining molecules. Generally, single target molecules cannot form a precipitate, and addition of the carrier molecules can help in forming a precipitate. Some target molecule recovery protocols use carrier RNA to prevent small amounts of target nucleic acids present in the sample from being irretrievably bound. In some embodiments, carrier RNA can be added immediately prior to a second strand synthesis step. In some embodiments, carrier RNA can be added immediately prior to a second strand cDNA synthesis on oligonucleotides released from an array. In some embodiments, carrier RNA can be added immediately prior to a post in vitro transcription clean-up step. In some embodiments, carrier RNA can be added prior to amplified RNA purification and quantification. In some embodiments, carrier RNA can be added before RNA quantification. In some embodiments, carrier RNA can be added immediately prior to both a second strand cDNA synthesis and a post in vitro transcription clean-up step.

### (15) Capture probe interaction

In some embodiments, analytes in a biological sample can be pre-processed prior to interaction with a capture probe. For example, prior to interaction with capture probes, polymerization reactions catalyzed by a polymerase (e.g., DNA polymerase or reverse transcriptase) are performed in the biological sample. In some embodiments, a primer for the polymerization reaction includes a functional group that enhances hybridization with the capture probe. The capture probes can include appropriate capture domains to capture biological analytes of interest (e.g., poly-dT sequence to capture poly(A) mRNA).

In some embodiments, biological analytes are pre-processed for library generation via next generation sequencing. For example, analytes can be pre-processed by addition of a modification (e.g., ligation of sequences that allow interaction with capture probes). In some embodiments, analytes (e.g., DNA or RNA) are fragmented using fragmentation techniques (e.g., using transposases and/or fragmentation buffers).

Fragmentation can be followed by a modification of the analyte. For example, a modification can be the addition through ligation of an adapter sequence that allows hybridization with the capture probe. In some embodiments, where the analyte of interest is RNA, poly(A) tailing is performed. Addition of a poly(A) tail to RNA that does not contain a poly(A) tail can facilitate hybridization with a capture probe that includes a capture domain with a functional amount of poly(dT) sequence.

In some embodiments, prior to interaction with capture probes, ligation reactions catalyzed by a ligase are performed in the biological sample. In some embodiments, ligation can be performed by chemical ligation. In some embodiments, the ligation can be performed using click chemistry as further described below. In some embodiments, the capture domain includes a DNA sequence that has complementarity to a RNA molecule, where the RNA molecule has complementarity to a second DNA sequence, and where the RNA-DNA sequence complementarity is used to ligate the second DNA sequence to the DNA sequence in the capture domain. In these embodiments, direct detection of RNA molecules is possible.

In some embodiments, prior to interaction with capture probes, target-specific reactions are performed in the biological sample. Examples of target specific reactions include, but are not limited to, ligation of target specific adaptors, probes and/or other oligonucleotides, target specific amplification using primers specific to one or more analytes, and target-specific detection using in situ hybridization, DNA microscopy, and/or antibody detection. In some embodiments, a capture probe includes capture domains targeted to target-specific products (e.g., amplification or ligation).

*II. General spatial array-based analytical methodology*

This section of the disclosure describes methods, apparatus, systems, and compositions for spatial array-based analysis of biological samples.

*(a) Spatial analysis methods*

Array-based spatial analysis methods involve the transfer of one or more analytes from a biological sample to an array of capture spots on a substrate, each of which is associated with a unique spatial location on the array. Subsequent analysis of the transferred analytes includes determining the identity of the analytes and the spatial location of each analyte within the sample. The spatial location of each analyte within the sample is determined based on the capture spot to which each analyte is bound in the array, and the capture spot's relative spatial location within the array.

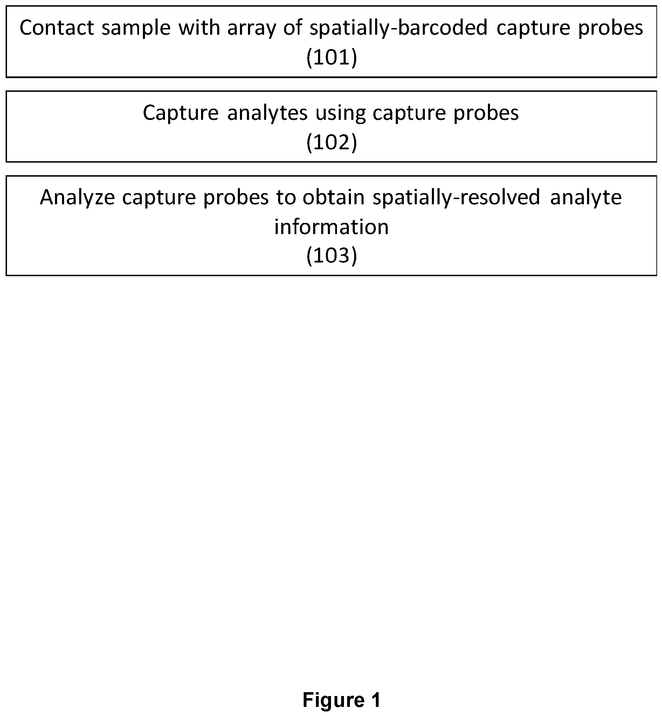

There are at least two general methods to associate a spatial barcode with one or more neighboring cells, such that the spatial barcode identifies the one or more cells, and/or contents of the one or more cells, as associated with a particular spatial location. One general method is to promote analytes out of a cell and towards the spatially-barcoded array. **FIG. 1** depicts an exemplary embodiment of this general method. In **FIG. 1,** the spatially-barcoded array populated with capture probes (as described further herein) is contacted with a sample **101,** and sample is permeabilized **102,** allowing the target analyte to migrate away from the sample and toward the array **102.** The target analyte interacts with a capture probe on the spatially-barcoded array. Once the target analyte hybridizes/is bound to the capture probe, the sample is optionally removed from the array and the capture probes are analyzed in order to obtain spatially-resolved analyte information **103.**

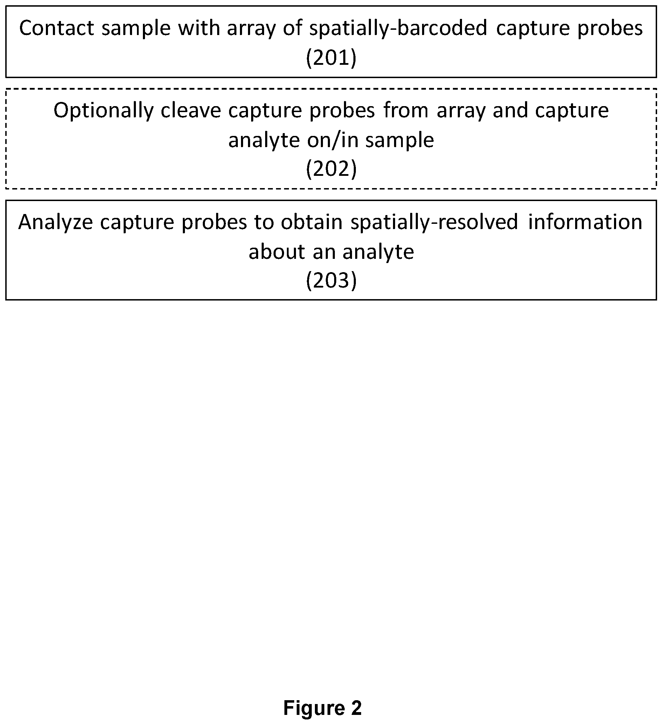

Another general method is to cleave the spatially-barcoded capture probes from an array, and promote the spatially-barcoded capture probes towards and/or into or onto the sample. **FIG. 2** depicts an exemplary embodiment of this general method, the spatially-barcoded array populated with capture probes (as described further herein) can be contacted with a sample **201.** The spatially-barcoded capture probes are cleaved and then interact with cells within the provided sample **202.** The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed to obtain spatially-resolved information about the tagged cell **203.**

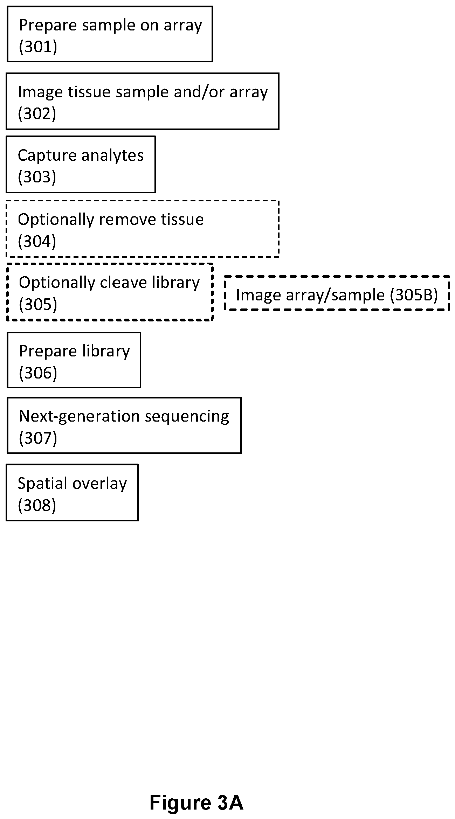

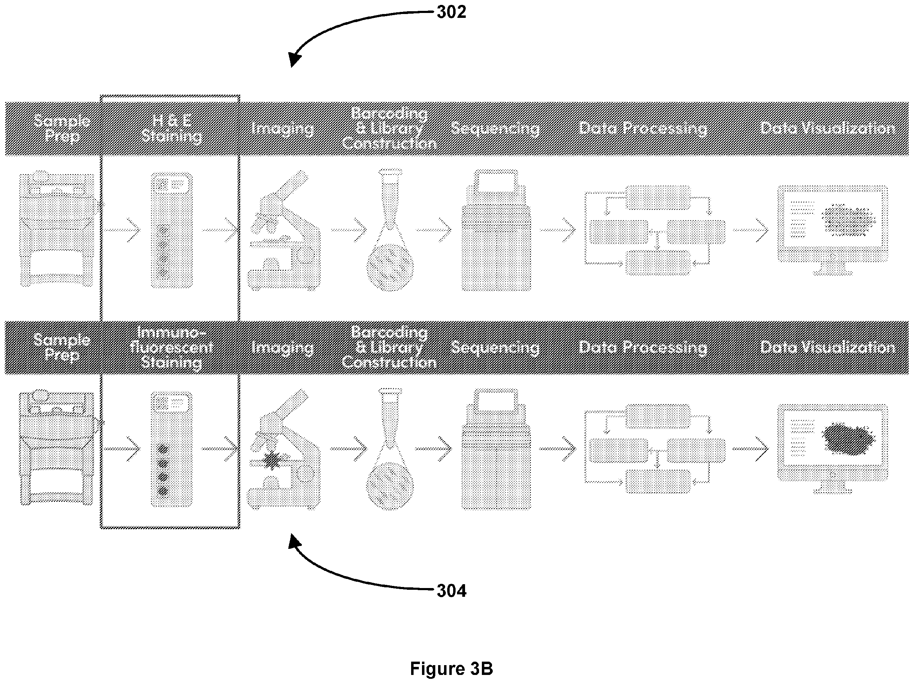

**FIGS. 3A** and **3B** show exemplary workflows that include preparing a sample on a spatially-barcoded array **301.** Sample preparation may include placing the sample on a substrate (*e.g.*, chip, slide, *etc.*), fixing the sample, and/or staining the sample for imaging. The sample (stained or not stained) is then imaged on the array **302** using brightfield (to image the sample, *e.g.*, using a hematoxylin and eosin stain) or fluorescence (to image capture spots) as illustrated in the upper panel **302** of **FIG. 3B)** and/or emission imaging modalities (as illustrated in the lower panel **304** of **FIG. 3B).**

Brightfield images are transmission microscopy images where broad-spectrum, white light is placed on one side of the sample mounted on a substrate and the camera objective is placed on the other side and the sample itself filters the light in order to generate colors or grayscale intensity images **1124,** akin to a stained glass window viewed from inside on a bright day.

In some embodiments, in addition to or instead of brightfield imaging, emission imaging, such as fluorescence imaging is used. In emission imaging approaches, the sample on the substrate is exposed to light of a specific narrow band (first wavelength band) of light and then the light that is re-emitted from the sample at a slightly different wavelength (second wavelength band) is measured. This absorption and re-emission is due to the presence of a fluorophore that is sensitive to the excitation used and can be either a natural property of the sample or an agent the sample has been exposed to in preparation for the imaging. As one example, in an immunofluorescence experiment, an antibody that binds to a certain protein or class of proteins, and that is labeled with a certain fluorophore, is added to the sample. When this is done, the locations on the sample that include the protein or class of proteins will emit the second wavelength band. In fact, multiple antibodies with multiple fluorophores can be used to label multiple proteins in the sample. Each such fluorophore requires excitation with a different wavelength of light and further emits a different unique wavelength of light. In order to spatially resolve each of the different emitted wavelengths of light, the sample is subjected to the different wavelengths of light that will excite the multiple fluorophores on a serial basis and images for each of these light exposures is saved as an image thus generating a plurality of images. For instance, the image is subjected to a first wavelength that excites a first fluorophore to emit at a second wavelength and a first image of the sample is taken while the sample is being exposed to the first wavelength. Then the exposure of the sample to the first wavelength is discontinued and the sample is exposed to a third wavelength (different from the first wavelength) that excites a second fluorophore at a fourth wavelength (different from the second wavelength) and a second image of the sample is taken while the sample is being exposed to the third wavelength. Such a process is repeated for each different fluorophore in the multiple fluorophores (e.g., two or more fluorophores, three or more fluorophores, four or more fluorophores, five or more fluorophores). In this way, a series of images of the tissue, each depicting the spatial arrangement of some different parameter such as a particular protein or protein class, is obtained. In some embodiments, more than one fluorophore is imaged at the same time. In such an approach a combination of excitation wavelengths are used, each for one of the more than one fluorophore, and a single image is collected.

In some embodiments, each of the images collected through emission imaging is gray scaled. To differentiate such grey scaled images, in some embodiments each of the images are assigned a color (shades of red, shades of blue, etc.) and combined into one composite color image for viewing. Such fluorescence imaging allows for the spatial analysis of protein abundance (e.g., spatial proteomics) in the sample. In some embodiments, such spatial abundance is analyzed on its own. In other embodiments such spatial abundance is analyzed together with transcriptomics.

In some embodiments where the sample is analyzed with transcriptomics, along with the brightfield and/or emission imaging (e.g., fluorescence imaging), target analytes are released from the sample and capture probes forming a spatially-barcoded array hybridize or bind the released target analytes 303. The sample can be optionally removed from the array 304 and the capture probes can be optionally cleaved from the array 305. The sample and array are then optionally imaged a second time in both modalities 305B while the analytes are reverse transcribed into cDNA, and an amplicon library is prepared 306 and sequenced 307. The images are then spatially-overlaid in order to correlate spatially-identified sample information 308. When the sample and array are not imaged a second time, 305B, a spot coordinate file is supplied instead. The spot coordinate file replaces the second imaging step 305B. Further, amplicon library preparation 306 can be performed with a unique PCR adapter and sequenced 307.

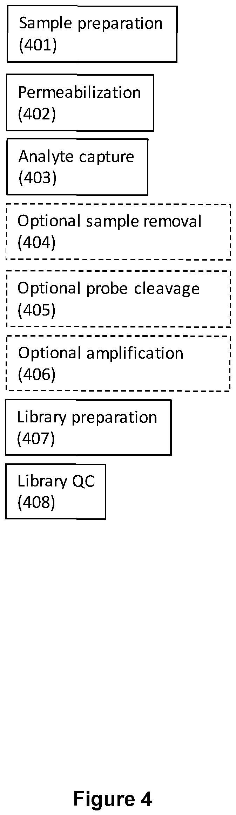

FIG. 4 shows another exemplary workflow that utilizes a spatially-barcoded array on a substrate (e.g., chip), where spatially-barcoded capture probes are clustered at areas called capture spots. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample 401, and the sample is permeabilized through application of permeabilization reagents 402. Permeabilization reagents may be administered by placing the array/sample assembly within a bulk solution. Alternatively, permeabilization reagents may be administered to the sample via a diffusion-resistant medium and/or a physical barrier such as a lid, where the sample is sandwiched between the diffusion-resistant medium and/or barrier and the array-containing substrate. The analytes are migrated toward the spatially-barcoded capture array using any number of techniques disclosed herein. For example, analyte migration can occur using a diffusion-resistant medium lid and passive migration. As another example, analyte migration can be active migration, using an electrophoretic transfer system, for example. Once the analytes are in close proximity to the spatially-barcoded capture probes, the capture probes can hybridize or otherwise bind a target analyte 403. The sample can be optionally removed from the array 404.

The capture probes can be optionally cleaved from the array 405, and the captured analytes can be spatially-barcoded by performing a reverse transcriptase first strand cDNA reaction. A first strand cDNA reaction can be optionally performed using template switching oligonucleotides. For example, a template switching oligonucleotide can hybridize to a poly(C) tail added to a 3' end of the cDNA by a reverse transcriptase enzyme. Template switching is illustrated in FIG. 37. The original mRNA template and template switching oligonucleotide can then be denatured from the cDNA and the spatially-barcoded capture probe can then hybridize with the cDNA and a complement of the cDNA can be generated. The first strand cDNA can then be purified and collected for downstream amplification steps. The first strand cDNA can be optionally amplified using PCR 406, where the forward and reverse primers flank the spatial barcode and target analyte regions of interest, generating a library associated with a particular spatial barcode 407. In some embodiments, the library preparation can be quantified and/or subjected to quality control to verify the success of the library preparation steps 408. In some embodiments, the cDNA comprises a sequencing by synthesis (SBS) primer sequence. The library amplicons are sequenced and analyzed to decode spatial information 407, with an additional library quality control (QC) step 408.

Using the methods, compositions, systems, kits, and devices described herein, RNA transcripts present in biological samples (*e.g.*, tissue samples) can be used for spatial transcriptome analysis. In particular, in some cases, the barcoded oligonucleotides may be configured to prime, replicate, and consequently yield barcoded extension products from an RNA template, or derivatives thereof. For example, in some cases, the barcoded oligonucleotides may include mRNA specific priming sequences, *e.g.*, poly-T primer segments that allow priming and replication of mRNA in a reverse transcription reaction or other targeted priming sequences. Alternatively or additionally, random RNA priming may be carried out using random N-mer primer segments of the barcoded oligonucleotides. Reverse transcriptases (RTs) can use an RNA template and a primer complementary to the 3' end of the RNA template to direct the synthesis of the first strand complementary DNA (cDNA). Many RTs can be used in this reverse transcription reactions, including, for example, avian myeloblastosis virus (AMV) reverse transcriptase, moloney murine leukemia virus (M-MuLV or MMLV), and other variants thereof. Some recombinant M-MuLV reverse transcriptase, such as, for example, PROTOSCRIPT® II reverse transcriptase, can have reduced RNase H activity and increased thermostability when compared to its wild type counterpart, and provide higher specificity, higher yield of cDNA and more full-length cDNA products with up to 12 kilobase (kb) in length. In some embodiments, the reverse transcriptase enzyme is a mutant reverse transcriptase enzyme such as, but not limited to, mutant MMLV reverse transcriptase. In another embodiment, the reverse transcriptase is a mutant MMLV reverse transcriptase such as, but not limited to, one or more variants described in U.S. Patent Publication No. 20180312822 and U.S. Provisional Patent Application No. 62/946,885 filed on December 11, 2019, both of which are incorporated herein by reference in their entireties.

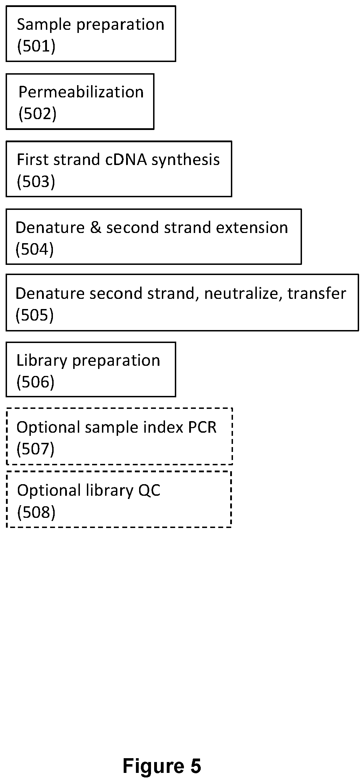

**FIG. 5** depicts an exemplary workflow where the sample is removed from the spatially-barcoded array and the spatially-barcoded capture probes are removed from the array for barcoded analyte amplification and library preparation. Another embodiment includes performing first strand synthesis using template switching oligonucleotides on the spatially-barcoded array without cleaving the capture probes. In this embodiment, sample preparation **501** and permeabilization **502** are performed as described elsewhere herein. Once the capture probes capture the target analyte(s), first strand cDNA created by template switching and reverse transcriptase **503** is then denatured and the second strand is then extended **504.** The second strand cDNA is then denatured from the first strand cDNA, neutralized, and transferred to a tube **505.** cDNA quantification and amplification can be performed using standard techniques discussed herein. The cDNA can then be subjected to library preparation **506** and indexing **507,** including fragmentation, end-repair, and a-tailing, and indexing PCR steps. The library can also be optionally tested for quality control (QC) **508.**

In a non-limiting example of the workflows described above, a biological sample (*e.g.* tissue section), can be fixed with methanol, stained with hematoxylin and eosin, and imaged. Optionally, the sample can be destained prior to permeabilization. The images can be used to map spatial analyte abundance (*e.g.*, gene expression) patterns back to the biological sample. A permeabilization enzyme can be used to permeabilize the biological sample directly on the slide. Analytes (*e.g.*, polyadenylated mRNA) released from the overlying cells of the biological sample can be captured by capture probes within a capture area on a substrate. Reverse transcription (RT) reagents can be added to permeabilized biological samples. Incubation with the RT reagents can produce spatially-barcoded full-length cDNA from the captured analytes (*e.g.*, polyadenylated mRNA). Second strand reagents (*e.g.*, second strand primers, enzymes) can be added to the biological sample on the slide to initiate second strand synthesis. The resulting cDNA can be denatured from the capture probe template and transferred (*e.g.*, to a clean tube) for amplification, and/or library construction. The spatially-barcoded, full-length cDNA can be amplified via PCR prior to library construction. The cDNA can then be enzymatically fragmented and size-selected in order to optimize the cDNA amplicon size. P5, P7, i7, and i5 can be used as sample indexes, and TruSeq Read 2 can be added via End Repair, A-tailing, Adaptor Ligation, and PCR. The cDNA fragments can then be sequenced using paired-end sequencing using TruSeq Read 1 and TruSeq Read 2 as sequencing primer sites. See, Illumina, Indexed Sequencing Overview Guides, February 2018, Document 15057455v04; and Illumina Adapter Sequences, May 2019, Document #1000000002694v11, each of which is hereby incorporated by reference, for information on P5, P7, i7, i5, TruSeq Read 2, indexed sequencing, and other reagents described herein.

In some embodiments, performing correlative analysis of data produced by this workflow, and other workflows described herein, can yield over 95% correlation of genes expressed across two capture areas (e.g. 95% or greater, 96% or greater, 97% or greater, 98% or greater, or 99% or greater). When performing the described workflows using single cell RNA sequencing of nuclei, in some embodiments, correlative analysis of the data can yield over 90% (*e.g.* over 90%, 91%, 92%, 93%, 94%, 95%, 96%, 97%, 98%, or 99%) correlation of genes expressed across two capture areas.

In some embodiments, after cDNA is generated (*e.g.*, by reverse transcription) the cDNA can be amplified directly on the substrate surface. Generating multiple copies of the cDNA (*e.g.*, cDNA synthesized from captured analytes) via amplification directly on the substrate surface can improve final sequencing library complexity. Thus, in some embodiments, cDNA can be amplified directly on the substrate surface by isothermal nucleic acid amplification. In some embodiments, isothermal nucleic acid amplification can amplify RNA or DNA.

In some embodiments, isothermal amplification can be faster than a standard PCR reaction. In some embodiments, isothermal amplification can be linear amplification (*e.g.*, asymmetrical with a single primer), or exponential amplification (*e.g.*, with two primers). In some embodiments, isothermal nucleic acid amplification can be performed by a template-switching oligonucleotide primer. In some embodiments, the template switching oligonucleotide adds a common sequence onto the 5' end of the RNA being reverse transcribed. For example, after a capture probe interacts with an analyte (*e.g.*, mRNA) and reverse transcription is performed such that additional nucleotides are added to the end of the cDNA creating a 3' overhang as described herein. In some embodiments, a template switching oligonucleotide hybridizes to untemplated poly(C) nucleotides added by a reverse transcriptase to continue replication to the 5' end of the template switching oligonucleotide, thereby generating full-length cDNA ready for further amplification. In some embodiments, the template switching oligonucleotide adds a common 5' sequence to full-length cDNA that is used for cDNA amplification (*e.g.*, a reverse complement of the template switching oligonucleotide).

In some embodiments, once a full-length cDNA molecule is generated, the template switching oligonucleotide can serve as a primer in a cDNA amplification reaction (*e.g.*, with a DNA polymerase). In some embodiments, double stranded cDNA (*e.g.*, first strand cDNA and second strand reverse complement cDNA) can be amplified via isothermal amplification with either a helicase or recombinase, followed by a strand displacing DNA polymerase. The strand displacing DNA polymerase can generate a displaced second strand resulting in an amplified product.

In any of isothermal amplification methods described herein, barcode exchange (*e.g.*, spatial barcode) can occur after the first amplification cycle where there are unused capture probes on the substrate surface. In some embodiments, the free 3' OH end of the unused capture probes can be blocked by any suitable 3'OH blocking method. In some embodiments, the 3'OH can be blocked by hairpin ligation.

Isothermal nucleic acid amplification can be used in addition to, or as an alternative to standard PCR reactions (*e.g.*, a PCR reaction that requires heating to about 95°C to denature double stranded DNA). Isothermal nucleic acid amplification generally does not require the use of a thermocycler, however in some embodiments, isothermal amplification can be performed in a thermocycler. In some embodiments, isothermal amplification can be performed from about 35°C to about 75°C. In some embodiments, isothermal amplification can be performed from about 40°C, about 45°C, about 50°C, about 55°C, about 60°C, about 65°C, or about 70°C or anywhere in between depending on the polymerase and auxiliary enzymes used.

Isothermal nucleic acid amplification techniques are known in the art, and can be used alone or in combination with any of the spatial methods described herein. For example, non-limiting examples of suitable isothermal nucleic acid amplification techniques include transcription mediated amplification, nucleic acid sequence-based amplification, signal mediated amplification of RNA technology, strand displacement amplification, rolling circle amplification, loop-mediated isothermal amplification of DNA (LAMP), isothermal multiple displacement amplification, recombinase polymerase amplification, helicase-dependent amplification, single primer isothermal amplification, and circular helicase-dependent amplification (See, *e.g.*, Gill and Ghaemi, Nucleic acid isothermal amplification technologies: a review, Nucleosides, Nucleotides, & Nucleic Acids, 27(3), 224-43, doi: 10.1080/15257770701845204 (2008), which is incorporated herein by reference in its entirety).

In some embodiments, the isothermal nucleic acid amplification is helicase-dependent nucleic acid amplification. Helicase-dependent isothermal nucleic acid amplification is described in Vincent et. al., 2004, Helicase-dependent isothermal DNA amplification, EMBO Rep., 795-800 and U.S. Patent No. 7,282,328, which are both incorporated herein by reference in their entireties. Further, helicase-dependent nucleic acid amplification on a substrate (*e.g.*, on-chip) is described in Andresen et. al., 2009, Helicase-dependent amplification: use in OnChip amplification and potential for point-of-care diagnostics, Expert Rev Mol Diagn. 9, 645-650, doi: 10.1586/erm.09.46, which is incorporated herein by reference in its entirety. In some embodiments, the isothermal nucleic acid amplification is recombinase polymerase nucleic acid amplification. Recombinase polymerase nucleic acid amplification is described in Piepenburg et al., 2006, DNA Detection Using Recombinant Proteins, PLoS Biol. 4, 7 e204 and Li et. al., 2019, Review: a comprehensive summary of a decade development of the recombinase polymerase amplification, Analyst 144, 31-67, doi: 10.1039/C8AN01621F (2019), both of which are incorporated herein by reference in their entireties.

Generally, isothermal amplification techniques use standard PCR reagents (e.g., buffer, dNTPs *etc.*) known in the art. Some isothermal amplification techniques can require additional reagents. For example, helicase dependent nucleic acid amplification uses a single-strand binding protein and an accessory protein. In another example, recombinase polymerase nucleic acid amplification uses recombinase (*e.g.*, T4 UvsX), recombinase loading factor (*e.g.*, TF UvsY), single-strand binding protein (*e.g.*, T4 gp32), crowding agent (*e.g.*, PEG-35K), and ATP.

After isothermal nucleic acid amplification of the full-length cDNA described by any of the methods herein, the isothermally amplified cDNAs (*e.g.*, single-stranded or double-stranded) can be recovered from the substrate, and optionally followed by amplification with typical cDNA PCR in microcentrifuge tubes. The sample can then be used with any of the spatial methods described herein.

*Immunohistochemistry and Immunofluorescence*

In some embodiments, immunofluorescence or immunohistochemistry protocols (direct and indirect staining techniques) is performed as a part of, or in addition to, the exemplary spatial workflows presented herein. For example, tissue sections can be fixed according to methods described herein. The biological sample can be transferred to an array (*e.g.*, capture probe array), where analytes (*e.g.*, proteins) are probed using immunofluorescence protocols. For example, the sample can be rehydrated, blocked, and permeabilized (3XSSC, 2% BSA, 0.1% Triton X, 1 U/μl RNAse inhibitor for 10 min at 4°C) before being stained with fluorescent primary antibodies (1:100 in 3XSSC, 2% BSA, 0.1% Triton X, 1 U/μl RNAse inhibitor for 30 min at 4°C). The biological sample can be washed, coverslipped (in glycerol + 1 U/μl RNAse inhibitor), imaged (*e.g.*, using a confocal microscope or other apparatus capable of fluorescent detection), washed, and processed according to analyte capture or spatial workflows described herein.

As used herein, an "antigen retrieval buffer" can improve antibody capture in IF/IHC protocols. An exemplary protocol for antigen retrieval can be preheating the antigen retrieval buffer (e.g., to 95°C), immersing the biological sample in the heated antigen retrieval buffer for a predetermined time, and then removing the biological sample from the antigen retrieval buffer and washing the biological sample.

In some embodiments, optimizing permeabilization can be useful for identifying intracellular analytes. Permeabilization optimization can include selection of permeabilization agents, concentration of permeabilization agents, and permeabilization duration. Tissue permeabilization is discussed elsewhere herein.

In some embodiments, blocking an array and/or a biological sample in preparation of labeling the biological sample decreases unspecific binding of the antibodies to the array and/or biological sample (decreases background). Some embodiments provide for blocking buffers/blocking solutions that can be applied before and/or during application of the label, where the blocking buffer can include a blocking agent, and optionally a surfactant and/or a salt solution. In some embodiments, a blocking agent can be bovine serum albumin (BSA), serum, gelatin (*e.g.*, fish gelatin), milk (*e.g.*, non-fat dry milk), casein, polyethylene glycol (PEG), polyvinyl alcohol (PVA), or polyvinylpyrrolidone (PVP), biotin blocking reagent, a peroxidase blocking reagent, levamisole, Carnoy's solution, glycine, lysine, sodium borohydride, pontamine sky blue, Sudan Black, trypan blue, FITC blocking agent, and/or acetic acid. The blocking buffer/blocking solution can be applied to the array and/or biological sample prior to and/or during labeling (*e.g.*, application of fluorophore-conjugated antibodies) to the biological sample.

In some embodiments, additional steps or optimizations can be included in performing IF/IHC protocols in conjunction with spatial arrays. Additional steps or optimizations can be included in performing spatially-tagged analyte capture agent workflows discussed herein.

In some embodiments, provided herein are methods for spatially detecting an analyte (*e.g.*, detecting the location of an analyte, *e.g.*, a biological analyte) from a biological sample (*e.g.*, an analyte present in a biological sample, such as a tissue section) that include: (a) providing a biological sample on a substrate; (b) staining the biological sample on the substrate, imaging the stained biological sample, and selecting the biological sample or subsection of the biological sample (*e.g.*, region of interest) to subject to analysis; (c) providing an array comprising one or more pluralities of capture probes on a substrate; (d) contacting the biological sample with the array, thereby allowing a capture probe of the one or more pluralities of capture probes to capture the analyte of interest; and (e) analyzing the captured analyte, thereby spatially detecting the analyte of interest. Any variety of staining and imaging techniques as described herein or known in the art can be used in accordance with methods described herein. In some embodiments, the staining includes optical labels as described herein, including, but not limited to, fluorescent, radioactive, chemiluminescent, calorimetric, or colorimetric detectable labels. In some embodiments, the staining includes a fluorescent antibody directed to a target analyte (*e.g.*, cell surface or intracellular proteins) in the biological sample. In some embodiments, the staining includes an immunohistochemistry stain directed to a target analyte (*e.g.*, cell surface or intracellular proteins) in the biological sample. In some embodiments, the staining includes a chemical stain such as hematoxylin and eosin (H&E) or periodic acid-schiff (PAS). In some embodiments, significant time (*e.g.*, days, months, or years) can elapse between staining and/or imaging the biological sample and performing analysis. In some embodiments, reagents for performing analysis are added to the biological sample before, contemporaneously with, or after the array is contacted to the biological sample. In some embodiments, step (d) includes placing the array onto the biological sample. In some embodiments, the array is a flexible array where the plurality of spatially-barcoded features (*e.g.*, a substrate with capture probes, a bead with capture probes) are attached to a flexible substrate. In some embodiments, measures are taken to slow down a reaction (*e.g.*, cooling the temperature of the biological sample or using enzymes that preferentially perform their primary function at lower or higher temperature as compared to their optimal functional temperature) before the array is contacted with the biological sample. In some embodiments, step (e) is performed without bringing the biological sample out of contact with the array. In some embodiments, step (e) is performed after the biological sample is no longer in contact with the array. In some embodiments, the biological sample is tagged with an analyte capture agent before, contemporaneously with, or after staining and/or imaging of the biological sample. In such cases, significant time (*e.g.*, days, months, or years) can elapse between staining and/or imaging and performing analysis. In some embodiments, the array is adapted to facilitate biological analyte migration from the stained and/or imaged biological sample onto the array (*e.g.*, using any of the materials or methods described herein). In some embodiments, a biological sample is permeabilized before being contacted with an array. In some embodiments, the rate of permeabilization is slowed prior to contacting a biological sample with an array (*e.g.*, to limit diffusion of analytes away from their original locations in the biological sample). In some embodiments, modulating the rate of permeabilization (*e.g.*, modulating the activity of a permeabilization reagent) can occur by modulating a condition that the biological sample is exposed to (*e.g.*, modulating temperature, pH, and/or light). In some embodiments, modulating the rate of permeabilization includes use of external stimuli (*e.g.*, small molecules, enzymes, and/or activating reagents) to modulate the rate of permeabilization. For example, a permeabilization reagent can be provided to a biological sample prior to contact with an array, which permeabilization reagent is inactive until a condition (*e.g.*, temperature, pH, and/or light) is changed or an external stimulus (*e.g.*, a small molecule, an enzyme, and/or an activating reagent) is provided.

In some embodiments, provided herein are methods for spatially detecting an analyte (*e.g.*, detecting the location of an analyte, *e.g.*, a biological analyte) from a biological sample (*e.g.*, present in a biological sample such as a tissue section) that include: (a) providing a biological sample on a substrate; (b) staining the biological sample on the substrate, imaging the stained biological sample, and selecting the biological sample or subsection of the biological sample (*e.g.*, a region of interest) to subject to spatial transcriptomic analysis; (c) providing an array comprising one or more pluralities of capture probes on a substrate; (d) contacting the biological sample with the array, thereby allowing a capture probe of the one or more pluralities of capture probes to capture the biological analyte of interest; and (e) analyzing the captured biological analyte, thereby spatially detecting the biological analyte of interest.

### (b) Capture probes

A "capture probe," also interchangeably referred to herein as a "probe," refers to any molecule capable of capturing (directly or indirectly) and/or labelling an analyte (*e.g.*, an analyte of interest) in a biological sample. In some embodiments, the capture probe is a nucleic acid or a polypeptide. In some embodiments, the capture probe is a conjugate (*e.g.*, an oligonucleotide-antibody conjugate). In some embodiments, the capture probe includes a barcode (*e.g.*, a spatial barcode and/or a unique molecular identifier (UMI)) and a capture domain.

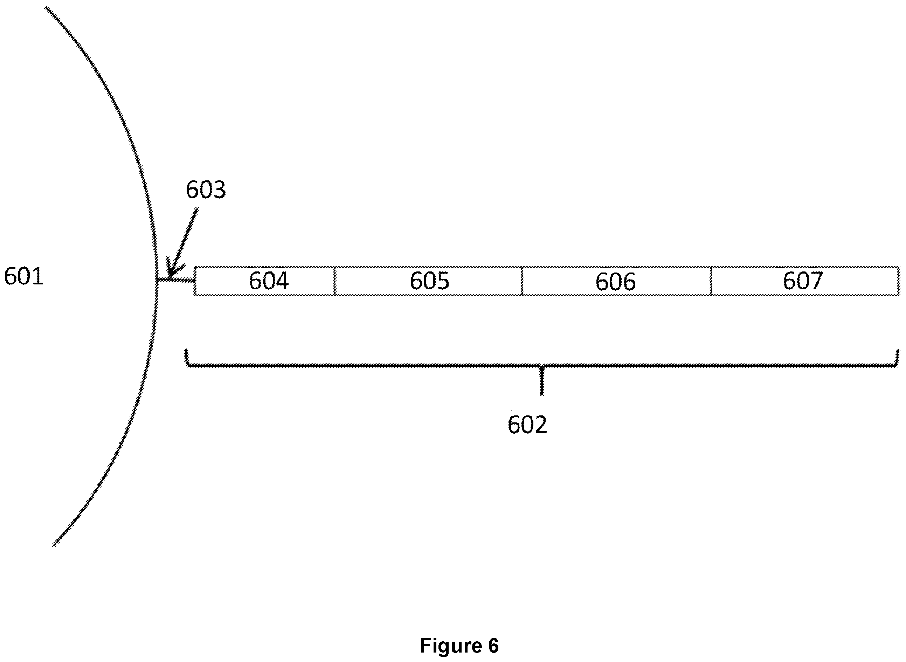

FIG. 6 is a schematic diagram showing an example of a capture probe, as described herein. As shown, the capture probe **602** is optionally coupled to a capture spot **601** by a cleavage domain **603,** such as a disulfide linker.

The capture probe **602** can include functional sequences that are useful for subsequent processing, such as functional sequence **604,** which can include a sequencer specific flow cell attachment sequence, *e.g.*, a P5 sequence, as well as functional sequence **606,** which can include sequencing primer sequences, *e.g.*, an R1 primer binding site, an R2 primer binding site. In some embodiments, sequence **604** is a P7 sequence and sequence **606** is a R2 primer binding site.

A spatial barcode **605** can be included within the capture probe for use in barcoding the target analyte. The functional sequences can be selected for compatibility with a variety of different sequencing systems, *e.g.*, 454 Sequencing, Ion Torrent Proton or PGM, Illumina sequencing instruments, PacBio, Oxford Nanopore, *etc.*, and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **605,** functional sequences **604** (*e.g.*, flow cell attachment sequence) and **606** (*e.g.*, sequencing primer sequences) can be common to all of the probes attached to a given capture spot. The spatial barcode can also include a capture domain **607** to facilitate capture of a target analyte.

### (i) Capture domain.

As discussed above, each capture probe includes at least one capture domain **607.** The "capture domain" is an oligonucleotide, a polypeptide, a small molecule, or any combination thereof, that binds specifically to a desired analyte. In some embodiments, a capture domain can be used to capture or detect a desired analyte.

In some embodiments, the capture domain is a functional nucleic acid sequence configured to interact with one or more analytes, such as one or more different types of nucleic acids (*e.g.*, RNA molecules and DNA molecules). In some embodiments, the functional nucleic acid sequence can include an N-mer sequence (*e.g.*, a random N-mer sequence), which N-mer sequences are configured to interact with a plurality of DNA molecules. In some embodiments, the functional sequence can include a poly(T) sequence, which poly(T) sequences are configured to interact with messenger RNA (mRNA) molecules via the poly(A) tail of an mRNA transcript. In some embodiments, the functional nucleic acid sequence is the binding target of a protein (*e.g.*, a transcription factor, a DNA binding protein, or a RNA binding protein), where the analyte of interest is a protein.

Capture probes can include ribonucleotides and/or deoxyribonucleotides as well as synthetic nucleotide residues that are capable of participating in Watson-Crick type or analogous base pair interactions. In some embodiments, the capture domain is capable of priming a reverse transcription reaction to generate cDNA that is complementary to the captured RNA molecules. In some embodiments, the capture domain of the capture probe can prime a DNA extension (polymerase) reaction to generate DNA that is complementary to the captured DNA molecules. In some embodiments, the capture domain can template a ligation reaction between the captured DNA molecules and a surface probe that is directly or indirectly immobilized on the substrate. In some embodiments, the capture domain can be ligated to one strand of the captured DNA molecules. For example, SplintR ligase along with RNA or DNA sequences (*e.g.*, degenerate RNA) can be used to ligate a single stranded DNA or RNA to the capture domain. In some embodiments, ligases with RNA-templated ligase activity, *e.g.*, SplintR ligase, T4 RNA ligase 2 or KOD ligase, can be used to ligate a single-stranded DNA or RNA to the capture domain. In some embodiments, a capture domain includes a splint oligonucleotide. In some embodiments, a capture domain captures a splint oligonucleotide.

In some embodiments, the capture domain is located at the 3' end of the capture probe and includes a free 3' end that can be extended, *e.g.*, by template dependent polymerization, to form an extended capture probe as described herein. In some embodiments, the capture domain includes a nucleotide sequence that is capable of hybridizing to nucleic acid, *e.g.*, RNA or other analyte, present in the cells of the tissue sample contacted with the array. In some embodiments, the capture domain can be selected or designed to bind selectively or specifically to a target nucleic acid. For example, the capture domain can be selected or designed to capture mRNA by way of hybridization to the mRNA poly(A) tail. Thus, in some embodiments, the capture domain includes a poly(T) DNA oligonucleotide, *e.g.*, a series of consecutive deoxythymidine residues linked by phosphodiester bonds, which is capable of hybridizing to the poly(A) tail of mRNA. In some embodiments, the capture domain can include nucleotides that are functionally or structurally analogous to a poly(T) tail. For example, a poly-U oligonucleotide or an oligonucleotide included of deoxythymidine analogues. In some embodiments, the capture domain includes at least 10, 11, 12, 13, 14, 15, 16, 17, 18, 19, or 20 nucleotides. In some embodiments, the capture domain includes at least 25, 30, or 35 nucleotides.

In some embodiments, a capture probe includes a capture domain having a sequence that is capable of binding to mRNA and/or genomic DNA. For example, the capture probe can include a capture domain that includes a nucleic acid sequence (*e.g.*, a poly(T) sequence) capable of binding to a poly(A) tail of an mRNA and/or to a poly(A) homopolymeric sequence present in genomic DNA. In some embodiments, a homopolymeric sequence is added to an mRNA molecule or a genomic DNA molecule using a terminal transferase enzyme in order to produce an analyte that has a poly(A) or poly(T) sequence. For example, a poly(A) sequence can be added to an analyte (*e.g.*, a fragment of genomic DNA) thereby making the analyte capable of capture by a poly(T) capture domain.

In some embodiments, random sequences, *e.g.*, random hexamers or similar sequences, can be used to form all or a part of the capture domain. For example, random sequences can be used in conjunction with poly(T) (or poly(T) analogue) sequences. Thus, where a capture domain includes a poly(T) (or a "poly(T)-like") oligonucleotide, it can also include a random oligonucleotide sequence (*e.g.*, "poly(T)-random sequence" probe). This can, for example, be located 5' or 3' of the poly(T) sequence, *e.g.*, at the 3' end of the capture domain. The poly(T)-random sequence probe can facilitate the capture of the mRNA poly(A) tail. In some embodiments, the capture domain can be an entirely random sequence. In some embodiments, degenerate capture domains can be used.

In some embodiments, a pool of two or more capture probes form a mixture, where the capture domain of one or more capture probes includes a poly(T) sequence and the capture domain of one or more capture probes includes random sequences. In some embodiments, a pool of two or more capture probes form a mixture where the capture domain of one or more capture probes includes poly(T)-like sequence and the capture domain of one or more capture probes includes random sequences. In some embodiments, a pool of two or more capture probes form a mixture where the capture domain of one or more capture probes includes a poly(T)-random sequences and the capture domain of one or more capture probes includes random sequences. In some embodiments, probes with degenerate capture domains can be added to any of the preceding combinations listed herein. In some embodiments, probes with degenerate capture domains can be substituted for one of the probes in each of the pairs described herein.

The capture domain can be based on a particular gene sequence or particular motif sequence or common/conserved sequence, that it is designed to capture (i.e., a sequence-specific capture domain). Thus, in some embodiments, the capture domain is capable of binding selectively to a desired sub-type or subset of nucleic acid, for example a particular type of RNA, such as mRNA, rRNA, tRNA, SRP RNA, tmRNA, snRNA, snoRNA, SmY RNA, scaRNA, gRNA, RNase P, RNase MRP, TERC, SL RNA, aRNA, cis-NAT, crRNA, lncRNA, miRNA, piRNA, siRNA, shRNA, tasiRNA, rasiRNA, 7SK, eRNA, ncRNA or other types of RNA. In a non-limiting example, the capture domain can be capable of binding selectively to a desired subset of ribonucleic acids, for example, microbiome RNA, such as 16S rRNA.

In some embodiments, a capture domain includes an "anchor" or "anchoring sequence", which is a sequence of nucleotides that is designed to ensure that the capture domain hybridizes to the intended biological analyte. In some embodiments, an anchor sequence includes a sequence of nucleotides, including a 1-mer, 2-mer, 3-mer or longer sequence. In some embodiments, the short sequence is random. For example, a capture domain including a poly(T) sequence can be designed to capture an mRNA. In such embodiments, an anchoring sequence can include a random 3-mer (*e.g.*, GGG) that helps ensure that the poly(T) capture domain hybridizes to an mRNA. In some embodiments, an anchoring sequence can be VN, N, or NN. Alternatively, the sequence can be designed using a specific sequence of nucleotides. In some embodiments, the anchor sequence is at the 3' end of the capture domain. In some embodiments, the anchor sequence is at the 5' end of the capture domain.

In some embodiments, capture domains of capture probes are blocked prior to contacting the biological sample with the array, and blocking probes are used when the nucleic acid in the biological sample is modified prior to its capture on the array. In some embodiments, the blocking probe is used to block or modify the free 3' end of the capture domain. In some embodiments, blocking probes can be hybridized to the capture probes to mask the free 3' end of the capture domain, *e.g.*, hairpin probes, partially double stranded probes, or complementary sequences. In some embodiments, the free 3' end of the capture domain can be blocked by chemical modification, *e.g.*, addition of an azidomethyl group as a chemically reversible capping moiety such that the capture probes do not include a free 3' end. Blocking or modifying the capture probes, particularly at the free 3' end of the capture domain, prior to contacting the biological sample with the array, prevents modification of the capture probes, *e.g.*, prevents the addition of a poly(A) tail to the free 3' end of the capture probes.

Non-limiting examples of 3' modifications include dideoxy C-3' (3'-ddC), 3' inverted dT, 3' C3 spacer, 3'Amino, and 3' phosphorylation. In some embodiments, the nucleic acid in the biological sample can be modified such that it can be captured by the capture domain. For example, an adaptor sequence (including a binding domain capable of binding to the capture domain of the capture probe) can be added to the end of the nucleic acid, *e.g.*, fragmented genomic DNA. In some embodiments, this is achieved by ligation of the adaptor sequence or extension of the nucleic acid. In some embodiments, an enzyme is used to incorporate additional nucleotides at the end of the nucleic acid sequence, *e.g.*, a poly(A) tail. In some embodiments, the capture probes can be reversibly masked or modified such that the capture domain of the capture probe does not include a free 3' end. In some embodiments, the 3' end is removed, modified, or made inaccessible so that the capture domain is not susceptible to the process used to modify the nucleic acid of the biological sample, *e.g.*, ligation or extension.

In some embodiments, the capture domain of the capture probe is modified to allow the removal of any modifications of the capture probe that occur during modification of the nucleic acid molecules of the biological sample. In some embodiments, the capture probes can include an additional sequence downstream of the capture domain, i.e., 3' to the capture domain, namely a blocking domain.

In some embodiments, the capture domain of the capture probe can be a non-nucleic acid domain. Examples of suitable capture domains that are not exclusively nucleic-acid based include, but are not limited to, proteins, peptides, aptamers, antigens, antibodies, and molecular analogs that mimic the functionality of any of the capture domains described herein.

### (ii) Cleavage domain.

Each capture probe can optionally include at least one cleavage domain. The cleavage domain represents the portion of the probe that is used to reversibly attach the probe to an array capture spot, as will be described further below. Further, one or more segments or regions of the capture probe can optionally be released from the array capture spot by cleavage of the cleavage domain. As an example spatial barcodes and/or universal molecular identifiers (UMIs) can be released by cleavage of the cleavage domain.

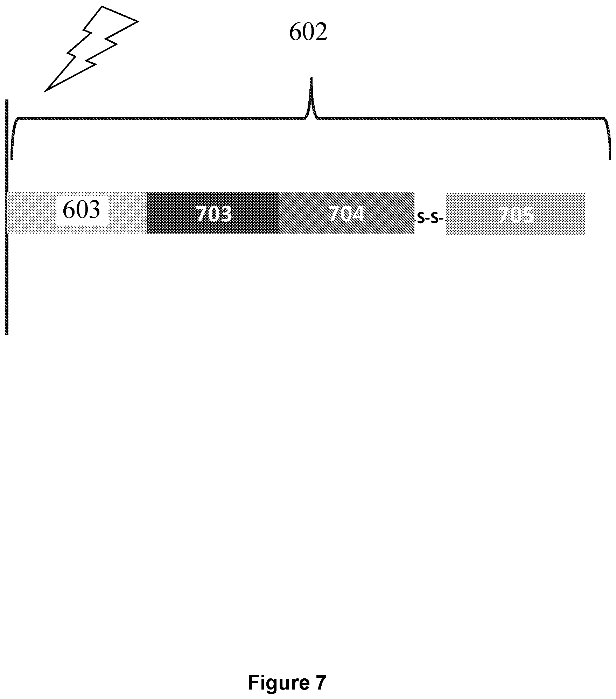

FIG. 7 is a schematic illustrating a cleavable capture probe, where the cleaved capture probe can enter into a non-permeabilized cell and bind to target analytes within the sample. The capture probe **602** contains a cleavage domain **603,** a cell penetrating peptide **703,** a reporter molecule **704,** and a disulfide bond (-S-S-). **705** represents all other parts of a capture probe, for example a spatial barcode and a capture domain.

In some embodiments, the cleavage domain **603** linking the capture probe to a capture spot is a covalent bond capable of cleavage by an enzyme. An enzyme can be added to cleave the cleavage domain, resulting in release of the capture probe from the capture spot. As another example, heating can also result in degradation of the cleavage domain and release of the attached capture probe from the array capture spot. In some embodiments, laser radiation is used to heat and degrade cleavage domains of capture probes at specific locations. In some embodiments, the cleavage domain is a photo-sensitive chemical bond (*e.g.*, a chemical bond that dissociates when exposed to light such as ultraviolet light). In some embodiments, the cleavage domain can be an ultrasonic cleavage domain. For example, ultrasonic cleavage can depend on nucleotide sequence, length, pH, ionic strength, temperature, and the ultrasonic frequency (e.g., 22 kHz, 44 kHz) (Grokhovsky, S.L., Specificity of DNA cleavage by ultrasound, Molecular Biology, 40(2), 276-283 (2006)).

Other examples of cleavage domains **603** include labile chemical bonds such as, but not limited to, ester linkages (*e.g.*, cleavable with an acid, a base, or hydroxylamine), a vicinal diol linkage (*e.g.*, cleavable via sodium periodate), a Diels-Alder linkage (*e.g.*, cleavable via heat), a sulfone linkage (*e.g.*, cleavable via a base), a silyl ether linkage (*e.g.*, cleavable via an acid), a glycosidic linkage (*e.g.*, cleavable via an amylase), a peptide linkage (*e.g.*, cleavable via a protease), or a phosphodiester linkage (*e.g.*, cleavable via a nuclease (*e.g.*, DNAase)).

In some embodiments, the cleavage domain **603** includes a sequence that is recognized by one or more enzymes capable of cleaving a nucleic acid molecule, *e.g.*, capable of breaking the phosphodiester linkage between two or more nucleotides. A bond can be cleavable via other nucleic acid molecule targeting enzymes, such as restriction enzymes (*e.g.*, restriction endonucleases). For example, the cleavage domain can include a restriction endonuclease (restriction enzyme) recognition sequence. Restriction enzymes cut double-stranded or single stranded DNA at specific recognition nucleotide sequences known as restriction sites. In some embodiments, a rare-cutting restriction enzyme, *e.g.*, enzymes with a long recognition site (at least 8 base pairs in length), is used to reduce the possibility of cleaving elsewhere in the capture probe.

Oligonucleotides with photo-sensitive chemical bonds (*e.g.*, photo-cleavable linkers) have various advantages. They can be cleaved efficiently and rapidly (*e.g.*, in nanoseconds and milliseconds). In some cases, photo-masks can be used such that only specific regions of the array are exposed to cleavable stimuli (*e.g.*, exposure to UV light, exposure to light, exposure to heat induced by laser). When a photo-cleavable linker is used, the cleavable reaction is triggered by light, and can be highly selective to the linker and consequently biorthogonal. Typically, wavelength absorption for the photocleavable linker is located in the near-UV range of the spectrum. In some embodiments, Amax of the photocleavable linker is from about 300 nm to about 400 nm, or from about 310 nm to about 365 nm. In some embodiments, λmax of the photocleavable linker is about 300 nm, about 312 nm, about 325 nm, about 330 nm, about 340 nm, about 345 nm, about 355 nm, about 365 nm, or about 400 nm. Non-limiting examples of a photo-sensitive chemical bond that can be used in a cleavage domain are disclosed in PCT publication 202020176788A1 entitled "Profiling of biological analyes with spatially barcoded oligonucleotide arrays" the entire contents of which is incorporated herein by reference.

In some embodiments, the cleavage domain includes a poly-U sequence which can be cleaved by a mixture of Uracil DNA glycosylase (UDG) and the DNA glycosylase-lyase Endonuclease VIII, commercially known as the USER™ enzyme. Releasable capture probes can be available for reaction once released. Thus, for example, an activatable capture probe can be activated by releasing the capture probes from a capture spot.

In some embodiments, where the capture probe is attached indirectly to a substrate, *e.g.*, via a surface probe, the cleavage domain includes one or more mismatch nucleotides, so that the complementary parts of the surface probe and the capture probe are not 100% complementary (for example, the number of mismatched base pairs can one, two, or three base pairs). Such a mismatch is recognized, *e.g.*, by the MutY and T7 endonuclease I enzymes, which results in cleavage of the nucleic acid molecule at the position of the mismatch. As described herein a "surface probe" can be any moiety present on the surface of the substrate capable of attaching to an agent (*e.g.*, a capture probe). In some embodiments, the surface probe is an oligonucleotide. In some embodiments, the surface probe is part of the capture probe.

In some embodiments, where the capture probe is attached to a capture spot indirectly (*e.g.*, immobilized), *e.g.*, via a surface probe, the cleavage domain includes a nickase recognition site or sequence. Nickases are endonucleases which cleave only a single strand of a DNA duplex. Thus, the cleavage domain can include a nickase recognition site close to the 5' end of the surface probe (and/or the 5' end of the capture probe) such that cleavage of the surface probe or capture probe destabilizes the duplex between the surface probe and capture probe thereby releasing the capture probe) from the capture spot.

Nickase enzymes can also be used in some embodiments where the capture probe is attached (*e.g.*, immobilized) to the capture spot directly. For example, the substrate can be contacted with a nucleic acid molecule that hybridizes to the cleavage domain of the capture probe to provide or reconstitute a nickase recognition site, *e.g.*, a cleavage helper probe. Thus, contact with a nickase enzyme will result in cleavage of the cleavage domain thereby releasing the capture probe from the capture spot. Such cleavage helper probes can also be used to provide or reconstitute cleavage recognition sites for other cleavage enzymes, *e.g.*, restriction enzymes.

Some nickases introduce single-stranded nicks only at particular sites on a DNA molecule, by binding to and recognizing a particular nucleotide recognition sequence. A number of naturally-occurring nickases have been discovered, of which at present the sequence recognition properties have been determined for at least four. Nickases are described in U.S. Patent No. 6,867,028, which is incorporated herein by reference in its entirety. In general, any suitable nickase can be used to bind to a complementary nickase recognition site of a cleavage domain. Following use, the nickase enzyme can be removed from the assay or inactivated following release of the capture probes to prevent unwanted cleavage of the capture probes.

In some embodiments, a cleavage domain is absent from the capture probe. Examples of substrates with attached capture probes lacking a cleavage domain are described for example in Macosko et al., (2015) Cell 161, 1202-1214, the entire contents of which are incorporated herein by reference.

Examples of suitable capture domains that are not exclusively nucleic-acid based include, but are not limited to, proteins, peptides, aptamers, antigens, antibodies, and molecular analogs that mimic the functionality of any of the capture domains described herein.

In some embodiments, the region of the capture probe corresponding to the cleavage domain can be used for some other function. For example, an additional region for nucleic acid extension or amplification can be included where the cleavage domain would normally be positioned. In such embodiments, the region can supplement the functional domain or even exist as an additional functional domain. In some embodiments, the cleavage domain is present but its use is optional.

### (iii) Functional domain

Each capture probe can optionally include at least one functional domain. Each functional domain typically includes a functional nucleotide sequence for a downstream analytical step in the overall analysis procedure.

Further details of functional domains that can be used in conjunction with the present disclosure are described in United States Patent Application No. 16/992,569 entitled "Systems and Methods for Using the Spatial Distribution of Haplotypes to Determine a Biological Condition," filed August 13, 2020, as well as PCT publication 202020176788A1 entitled "Profiling of biological analyes with spatially barcoded oligonucleotide arrays" each of which is hereby incorporated herein by reference.

### (iv) Spatial barcode.

As discussed above, the capture probe can include one or more spatial barcodes (*e.g.*, two or more, three or more, four or more, five or more) spatial barcodes. A "spatial barcode" is a contiguous nucleic acid segment or two or more non-contiguous nucleic acid segments that function as a label or identifier that conveys or is capable of conveying spatial information. In some embodiments, a capture probe includes a spatial barcode that possesses a spatial aspect, where the barcode is associated with a particular location within an array or a particular location on a substrate.

A spatial barcode can be part of an analyte, or independent from an analyte (i.e., part of the capture probe). A spatial barcode can be a tag attached to an analyte (*e.g.*, a nucleic acid molecule) or a combination of a tag in addition to an endogenous characteristic of the analyte (*e.g.*, size of the analyte or end sequence(s)). A spatial barcode can be unique. In some embodiments where the spatial barcode is unique, the spatial barcode functions both as a spatial barcode and as a unique molecular identifier (UMI), associated with one particular capture probe.

Spatial barcodes can have a variety of different formats. For example, spatial barcodes can include polynucleotide spatial barcodes; random nucleic acid and/or amino acid sequences; and synthetic nucleic acid and/or amino acid sequences. In some embodiments, a spatial barcode is attached to an analyte in a reversible or irreversible manner. In some embodiments, a spatial barcode is added to, for example, a fragment of a DNA or RNA sample before, during, and/or after sequencing of the sample. In some embodiments, a spatial barcode allows for identification and/or quantification of individual sequencing-reads. In some embodiments, a spatial barcode is a used as a fluorescent barcode for which fluorescently labeled oligonucleotide probes hybridize to the spatial barcode.

In some embodiments, the spatial barcode is a nucleic acid sequence that does not substantially hybridize to analyte nucleic acid molecules in a biological sample. In some embodiments, the spatial barcode has less than 80% sequence identity (*e.g.*, less than 70%, 60%, 50%, or less than 40% sequence identity) to the nucleic acid sequences across a substantial part (*e.g.*, 80% or more) of the nucleic acid molecules in the biological sample.

The spatial barcode sequences can include from about 6 to about 20 or more nucleotides within the sequence of the capture probes. In some embodiments, the length of a spatial barcode sequence can be about 6, 7, 8, 9, 10, 11, 12, 13, 14, 15, 16, 17, 18, 19, 20 nucleotides or longer. In some embodiments, the length of a spatial barcode sequence can be at least about 6, 7, 8, 9, 10, 11, 12, 13, 14, 15, 16, 17, 18, 19, 20 nucleotides or longer. In some embodiments, the length of a spatial barcode sequence is at most about 6, 7, 8, 9, 10, 11, 12, 13, 14, 15, 16, 17, 18, 19, 20 nucleotides or shorter.

These nucleotides can be completely contiguous, *e.g.*, in a single stretch of adjacent nucleotides, or they can be separated into two or more separate subsequences that are separated by 1 or more nucleotides. Separated spatial barcode subsequences can be from about 4 to about 16 nucleotides in length. In some embodiments, the spatial barcode subsequence can be about 4, 5, 6, 7, 8, 9, 10, 11, 12, 13, 14, 15, 16 nucleotides or longer. In some embodiments, the spatial barcode subsequence can be at least about 4, 5, 6, 7, 8, 9, 10, 11, 12, 13, 14, 15, 16 nucleotides or longer. In some embodiments, the spatial barcode subsequence can be at most about 4, 5, 6, 7, 8, 9, 10, 11, 12, 13, 14, 15, 16 nucleotides or shorter.

For multiple capture probes that are attached to a common array capture spot, the one or more spatial barcode sequences of the multiple capture probes can include sequences that are the same for all capture probes coupled to the capture spot, and/or sequences that are different across all capture probes coupled to the capture spot.

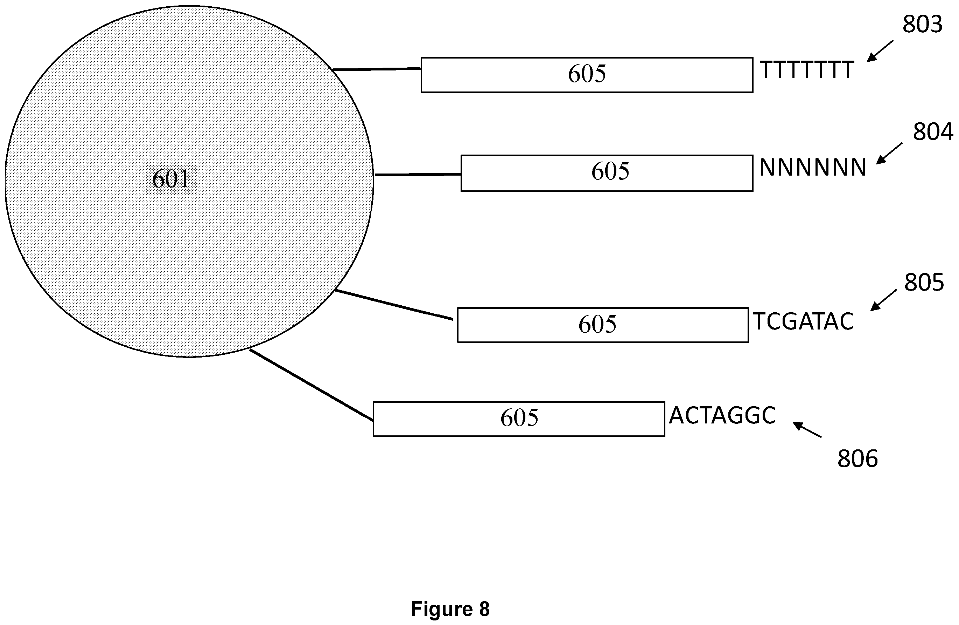

**FIG. 8** is a schematic diagram of an exemplary multiplexed spatially-labelled capture spot. In **FIG 8**, the capture spot **601** can be coupled to spatially-barcoded capture probes, where the spatially-barcoded probes of a particular capture spot can possess the same spatial barcode, but have different capture domains designed to associate the spatial barcode of the capture spot with more than one target analyte. For example, a capture spot may be coupled to four different types of spatially-barcoded capture probes, each type of spatially-barcoded capture probe possessing the spatial barcode **605.** One type of capture probe associated with the capture spot includes the spatial barcode **605** in combination with a poly(T) capture domain **803,** designed to capture mRNA target analytes. A second type of capture probe associated with the capture spot includes the spatial barcode **605** in combination with a random N-mer capture domain **804** for gDNA analysis. A third type of capture probe associated with the capture spot includes the spatial barcode **605** in combination with a capture domain complementary to the capture domain on an analyte capture agent **805.** A fourth type of capture probe associated with the capture spot includes the spatial barcode **605** in combination with a capture probe that can specifically bind a nucleic acid molecule **806** that can function in a CRISPR assay (*e.g.*, CRISPR/Cas9). While only four different capture probe-barcoded constructs are shown in **FIG. 8,** capture-probe barcoded constructs can be tailored for analyses of any given analyte associated with a nucleic acid and capable of binding with such a construct. For example, the schemes shown in **FIG. 8** can also be used for concurrent analysis of other analytes disclosed herein, including, but not limited to: (a) mRNA, a lineage tracing construct, cell surface or intracellular proteins and metabolites, and gDNA; (b) mRNA, accessible chromatin (*e.g.*, ATAC-seq, DNase-seq, and/or MNase-seq) cell surface or intracellular proteins and metabolites, and a perturbation agent (*e.g.*, a CRISPR crRNA/sgRNA, TALEN, zinc finger nuclease, and/or antisense oligonucleotide as described herein); (c) mRNA, cell surface or intracellular proteins and/or metabolites, a barcoded labelling agent (*e.g.*, the MHC multimers described herein), and a V(D)J sequence of an immune cell receptor (*e.g.*, T-cell receptor). In some embodiments, a perturbation agent can be a small molecule, an antibody, a drug, an aptamer, a miRNA, a physical environmental (*e.g.*, temperature change), or any other known perturbation agents.

Capture probes attached to a single array capture spot can include identical (or common) spatial barcode sequences, different spatial barcode sequences, or a combination of both. Capture probes attached to a capture spot can include multiple sets of capture probes. Capture probes of a given set can include identical spatial barcode sequences. The identical spatial barcode sequences can be different from spatial barcode sequences of capture probes of another set.

The plurality of capture probes can include spatial barcode sequences (*e.g.*, nucleic acid barcode sequences) that are associated with specific locations on a spatial array. For example, a first plurality of capture probes can be associated with a first region, based on a spatial barcode sequence common to the capture probes within the first region, and a second plurality of capture probes can be associated with a second region, based on a spatial barcode sequence common to the capture probes within the second region. The second region may or may not be associated with the first region. Additional pluralities of capture probes can be associated with spatial barcode sequences common to the capture probes within other regions. In some embodiments, the spatial barcode sequences can be the same across a plurality of capture probe molecules.

In some embodiments, multiple different spatial barcodes are incorporated into a single arrayed capture probe. For example, a mixed but known set of spatial barcode sequences can provide a stronger address or attribution of the spatial barcodes to a given spot or location, by providing duplicate or independent confirmation of the identity of the location. In some embodiments, the multiple spatial barcodes represent increasing specificity of the location of the particular array point.

### (v) Unique molecular identifier.

The capture probe can include one or more (*e.g.*, two or more, three or more, four or more, five or more) Unique Molecular Identifiers (UMIs). A unique molecular identifier is a contiguous nucleic acid segment or two or more non-contiguous nucleic acid segments that function as a label or identifier for a particular analyte, or for a capture probe that binds a particular analyte (*e.g.*, via the capture domain).

Further details of UMIs that can be used with the systems and methods of the present disclosure are described in United States Patent Application No. 16/992,569 entitled "Systems and Methods for Using the Spatial Distribution of Haplotypes to Determine a Biological Condition," filed August 13, 2020, and PCT publication 202020176788A1 entitled "Profiling of biological analyes with spatially barcoded oligonucleotide arrays," each of which is hereby incorporated herein by reference.

### (vi) Other aspects of capture probes.

For capture probes that are attached to an array capture spot, an individual array capture spot can include one or more capture probes. In some embodiments, an individual array capture spot includes hundreds or thousands of capture probes. In some embodiments, the capture probes are associated with a particular individual capture spot, where the individual capture spot contains a capture probe including a spatial barcode unique to a defined region or location on the array.

In some embodiments, a particular capture spot contains capture probes including more than one spatial barcode (*e.g.*, one capture probe at a particular capture spot can include a spatial barcode that is different than the spatial barcode included in another capture probe at the same particular capture spot, while both capture probes include a second, common spatial barcode), where each spatial barcode corresponds to a particular defined region or location on the array. For example, multiple spatial barcode sequences associated with one particular capture spot on an array can provide a stronger address or attribution to a given location by providing duplicate or independent confirmation of the location. In some embodiments, the multiple spatial barcodes represent increasing specificity of the location of the particular array point. In a non-limiting example, a particular array point can be coded with two different spatial barcodes, where each spatial barcode identifies a particular defined region within the array, and an array point possessing both spatial barcodes identifies the sub-region where two defined regions overlap, *e.g.*, such as the overlapping portion of a Venn diagram.

In another non-limiting example, a particular array point can be coded with three different spatial barcodes, where the first spatial barcode identifies a first region within the array, the second spatial barcode identifies a second region, where the second region is a subregion entirely within the first region, and the third spatial barcode identifies a third region, where the third region is a subregion entirely within the first and second subregions.

In some embodiments, capture probes attached to array capture spots are released from the array capture spots for sequencing. Alternatively, in some embodiments, capture probes remain attached to the array capture spots, and the probes are sequenced while remaining attached to the array capture spots (e.g., via in-situ sequencing). Further aspects of the sequencing of capture probes are described in subsequent sections of this disclosure.

In some embodiments, an array capture spot can include different types of capture probes attached to the capture spot. For example, the array capture spot can include a first type of capture probe with a capture domain designed to bind to one type of analyte, and a second type of capture probe with a capture domain designed to bind to a second type of analyte. In general, array capture spots can include one or more (*e.g.*, two or more, three or more, four or more, five or more, six or more, eight or more, ten or more, 12 or more, 15 or more, 20 or more, 30 or more, 50 or more) different types of capture probes attached to a single array capture spot.

In some embodiments, the capture probe is nucleic acid. In some embodiments, the capture probe is attached to the array capture spot via its 5' end. In some embodiments, the capture probe includes from the 5' to 3' end: one or more barcodes (*e.g.*, a spatial barcode and/or a UMI) and one or more capture domains. In some embodiments, the capture probe includes from the 5' to 3' end: one barcode (*e.g.*, a spatial barcode or a UMI) and one capture domain. In some embodiments, the capture probe includes from the 5' to 3' end: a cleavage domain, a functional domain, one or more barcodes (*e.g.*, a spatial barcode and/or a UMI), and a capture domain. In some embodiments, the capture probe includes from the 5' to 3' end: a cleavage domain, a functional domain, one or more barcodes (*e.g.*, a spatial barcode and/or a UMI), a second functional domain, and a capture domain. In some embodiments, the capture probe includes from the 5' to 3' end: a cleavage domain, a functional domain, a spatial barcode, a UMI, and a capture domain. In some embodiments, the capture probe does not include a spatial barcode. In some embodiments, the capture probe does not include a UMI. In some embodiments, the capture probe includes a sequence for initiating a sequencing reaction.

In some embodiments, the capture probe is immobilized on a capture spot via its 3' end. In some embodiments, the capture probe includes from the 3' to 5' end: one or more barcodes (*e.g.*, a spatial barcode and/or a UMI) and one or more capture domains. In some embodiments, the capture probe includes from the 3' to 5' end: one barcode (*e.g.*, a spatial barcode or a UMI) and one capture domain. In some embodiments, the capture probe includes from the 3' to 5' end: a cleavage domain, a functional domain, one or more barcodes (*e.g.*, a spatial barcode and/or a UMI), and a capture domain. In some embodiments, the capture probe includes from the 3' to 5' end: a cleavage domain, a functional domain, a spatial barcode, a UMI, and a capture domain.

In some embodiments, a capture probe includes an *in situ* synthesized oligonucleotide. The in situ synthesized oligonucleotide can be attached to a substrate, or to a feature on a substrate. In some embodiments, the *in situ* synthesized oligonucleotide includes one or more constant sequences, one or more of which serves as a priming sequence (*e.g.*, a primer for amplifying target nucleic acids). The in situ synthesized oligonucleotide can, for example, include a constant sequence at the 3' end that is attached to a substrate, or attached to a feature on the substrate. Additionally or alternatively, the in situ synthesized oligonucleotide can include a constant sequence at the free 5' end. In some embodiments, the one or more constant sequences can be a cleavable sequence. In some embodiments, the in situ synthesized oligonucleotide includes a barcode sequence, *e.g.*, a variable barcode sequence. The barcode can be any of the barcodes described herein. The length of the barcode can be approximately 8 to 16 nucleotides (*e.g.*, 8, 9, 10, 11, 12, 13, 14, 15, or 16 nucleotides). The length of the in situ synthesized oligonucleotide can be less than 100 nucleotides (*e.g.*, less than 90, 80, 75, 70, 60, 50, 45, 40, 35, 30, 25 or 20 nucleotides). In some instances, the length of the in situ synthesized oligonucleotide is about 20 to about 40 nucleotides. Exemplary in situ synthesized oligonucleotides are produced by Affymetrix. In some embodiments, the *in situ* synthesized oligonucleotide is attached to a capture spot of an array.

Additional oligonucleotides can be ligated to an in situ synthesized oligonucleotide to generate a capture probe. For example, a primer complementary to a portion of the in situ synthesized oligonucleotide (*e.g.*, a constant sequence in the oligonucleotide) can be used to hybridize an additional oligonucleotide and extend (using the in situ synthesized oligonucleotide as a template *e.g.*, a primer extension reaction) to form a double stranded oligonucleotide and to further create a 3' overhang. In some embodiments, the 3' overhang can be created by template-independent ligases (*e.g.*, terminal deoxynucleotidyl transferase (TdT) or poly(A) polymerase). An additional oligonucleotide comprising one or more capture domains can be ligated to the 3' overhang using a suitable enzyme (*e.g.*, a ligase) and a splint oligonucleotide, to generate a capture probe. Thus, in some embodiments, a capture probe is a product of two or more oligonucleotide sequences, (*e.g.*, the *in situ* synthesized oligonucleotide and the additional oligonucleotide) that are ligated together. In some embodiments, one of the oligonucleotide sequences is an in situ synthesized oligonucleotide.

In some embodiments, the capture probe includes a splint oligonucleotide. Two or more oligonucleotides can be ligated together using a splint oligonucleotide and any variety of ligases known in the art or described herein (*e.g.,* SplintR ligase).

In some embodiments, one of the oligonucleotides includes: a constant sequence (*e.g.,* a sequence complementary to a portion of a splint oligonucleotide), a degenerate sequence, and a capture domain (*e.g.,* as described herein). In some embodiments, the capture probe is generated by having an enzyme add polynucleotides at the end of an oligonucleotide sequence. The capture probe can include a degenerate sequence, which can function as a unique molecular identifier.

A capture probe can include a degenerate sequence, which is a sequence in which some positions of a nucleotide sequence contain a number of possible bases. A degenerate sequence can be a degenerate nucleotide sequence including about or at least 5, 6, 7, 8, 9, 10, 11, 12, 13, 14, 15, 16, 17, 18, 19, 20, 25, 30, 35, 40, 45, or 50 nucleotides. In some embodiments, a nucleotide sequence contains 1, 2, 3, 4, 5, 6, 7, 8, 9, 0, 10, 15, 20, 25, or more degenerate positions within the nucleotide sequence. In some embodiments, the degenerate sequence is used as a UMI.

In some embodiments, a capture probe includes a restriction endonuclease recognition sequence or a sequence of nucleotides cleavable by specific enzyme activities. For example, uracil sequences can be enzymatically cleaved from a nucleotide sequence using uracil DNA glycosylase (UDG) or Uracil Specific Excision Reagent (USER). As another example, other modified bases (*e.g.,* modified by methylation) can be recognized and cleaved by specific endonucleases. The capture probes can be subjected to an enzymatic cleavage, which removes the blocking domain and any of the additional nucleotides that are added to the 3' end of the capture probe during the modification process. The removal of the blocking domain reveals and/or restores the free 3' end of the capture domain of the capture probe. In some embodiments, additional nucleotides can be removed to reveal and/or restore the 3' end of the capture domain of the capture probe.

In some embodiments, a blocking domain can be incorporated into the capture probe when it is synthesized, or after its synthesis. The terminal nucleotide of the capture domain is a reversible terminator nucleotide (*e.g.,* 3'-O-blocked reversible terminator and 3'-unblocked reversible terminator), and can be included in the capture probe during or after probe synthesis.

### (vii) Extended Capture Probes

An "extended capture probe" is a capture probe with an enlarged nucleic acid sequence. For example, where the capture probe includes nucleic acid, an "extended 3' end" indicates that further nucleotides were added to the most 3' nucleotide of the capture probe to extend the length of the capture probe, for example, by standard polymerization reactions utilized to extend nucleic acid molecules including templated polymerization catalyzed by a polymerase (e.g., a DNA polymerase or reverse transcriptase).

In some embodiments, extending the capture probe includes generating cDNA from the captured (hybridized) RNA. This process involves synthesis of a complementary strand of the hybridized nucleic acid, e.g., generating cDNA based on the captured RNA template (the RNA hybridized to the capture domain of the capture probe). Thus, in an initial step of extending the capture probe, e.g., the cDNA generation, the captured (hybridized) nucleic acid, e.g., RNA, acts as a template for the extension, e.g., reverse transcription, step.

In some embodiments, the capture probe is extended using reverse transcription. For example, reverse transcription includes synthesizing cDNA (complementary or copy DNA) from RNA, *e.g.,* (messenger RNA), using a reverse transcriptase. In some embodiments, reverse transcription is performed while the tissue is still in place, generating an analyte library, where the analyte library includes the spatial barcodes from the adjacent capture probes. In some embodiments, the capture probe is extended using one or more DNA polymerases.

In some embodiments, the capture domain of the capture probe includes a primer for producing the complementary strand of the nucleic acid hybridized to the capture probe, *e.g.*, a primer for DNA polymerase and/or reverse transcription. The nucleic acid, *e.g.*, DNA and/or cDNA, molecules generated by the extension reaction incorporate the sequence of the capture probe. The extension of the capture probe, *e.g.*, a DNA polymerase and/or reverse transcription reaction, can be performed using a variety of suitable enzymes and protocols.

In some embodiments, a full-length DNA, *e.g.*, cDNA, molecule is generated. In some embodiments, a "full-length" DNA molecule refers to the whole of the captured nucleic acid molecule. However, if the nucleic acid, *e.g.*, RNA, was partially degraded in the tissue sample, then the captured nucleic acid molecules will not be the same length as the initial RNA in the tissue sample. In some embodiments, the 3' end of the extended probes, e.g., first strand cDNA molecules, is modified. For example, a linker or adaptor can be ligated to the 3' end of the extended probes. This can be achieved using single stranded ligation enzymes such as T4 RNA ligase or Circligase™ (available from Lucigen, Middleton, WI). In some embodiments, template switching oligonucleotides are used to extend cDNA in order to generate a full-length cDNA (or as close to a full-length cDNA as possible). In some embodiments, a second strand synthesis helper probe (a partially double stranded DNA molecule capable of hybridizing to the 3' end of the extended capture probe), can be ligated to the 3' end of the extended probe, *e.g.*, first strand cDNA, molecule using a double stranded ligation enzyme such as T4 DNA ligase. Other enzymes appropriate for the ligation step are known in the art and include, e.g., Tth DNA ligase, Taq DNA ligase, *Thermococcus* sp. (strain 9°N) DNA ligase (9°N™ DNA ligase, New England Biolabs), Ampligase™ (available from Lucigen, Middleton, WI), and SplintR (available from New England Biolabs, Ipswich, MA). In some embodiments, a polynucleotide tail, *e.g.*, a poly(A) tail, is incorporated at the 3' end of the extended probe molecules. In some embodiments, the polynucleotide tail is incorporated using a terminal transferase active enzyme.

In some embodiments, double-stranded extended capture probes are treated to remove any unextended capture probes prior to amplification and/or analysis, *e.g.,* sequence analysis. This can be achieved by a variety of methods, *e.g.,* using an enzyme to degrade the unextended probes, such as an exonuclease enzyme, or purification columns.

In some embodiments, extended capture probes are amplified to yield quantities that are sufficient for analysis, *e.g.,* via DNA sequencing. In some embodiments, the first strand of the extended capture probes (*e.g.,* DNA and/or cDNA molecules) acts as a template for the amplification reaction (*e.g.,* a polymerase chain reaction).

In some embodiments, the amplification reaction incorporates an affinity group onto the extended capture probe (*e.g.,* RNA-cDNA hybrid) using a primer including the affinity group. In some embodiments, the primer includes an affinity group and the extended capture probes includes the affinity group. The affinity group can correspond to any of the affinity groups described previously.

In some embodiments, the extended capture probes including the affinity group can be coupled to an array feature specific for the affinity group. In some embodiments, the substrate can include an antibody or antibody fragment. In some embodiments, the array feature includes avidin or streptavidin and the affinity group includes biotin. In some embodiments, the array feature includes maltose and the affinity group includes maltose-binding protein. In some embodiments, the array feature includes maltose-binding protein and the affinity group includes maltose. In some embodiments, amplifying the extended capture probes can function to release the extended probes from the array feature, insofar as copies of the extended probes are not attached to the array feature.

In some embodiments, the extended capture probe or complement or amplicon thereof is released from an array feature. The step of releasing the extended capture probe or complement or amplicon thereof from an array feature can be achieved in a number of ways. In some embodiments, an extended capture probe or a complement thereof is released from the feature by nucleic acid cleavage and/or by denaturation (*e.g.,* by heating to denature a double-stranded molecule).

In some embodiments, the extended capture probe or complement or amplicon thereof is released from the array feature by physical means. For example, methods for inducing physical release include denaturing double stranded nucleic acid molecules. Another method for releasing the extended capture probes is to use a solution that interferes with the hydrogen bonds of the double stranded molecules. In some embodiments, the extended capture probe is released by applying heated water such as water or buffer of at least 85°C, *e.g.,* at least 90, 91, 92, 93, 94, 95, 96, 97, 98, or 99°C. In some embodiments, a solution including salts, surfactants, etc. that can further destabilize the interaction between the nucleic acid molecules is added to release the extended capture probe from the array feature. In some embodiments, a formamide solution can be used to destabilize the interaction between nucleic acid molecules to release the extended capture probe from the array feature.

### (viii) Amplification of Capture Probes

In some embodiments, methods are provided herein for amplifying a capture probe affixed to a spatial array, where amplification of the capture probe increases the number of capture domains and spatial barcodes on the spatial array. In some embodiments where a capture probe is amplified, the amplification is performed by rolling circle amplification. In some embodiments, the capture probe to be amplified includes sequences (*e.g.,* docking sequences, functional sequences, and/or primer sequences) that enable rolling circle amplification. In one example, the capture probe can include a functional sequence that is capable of binding to a primer used for amplification. In another example, the capture probe can include one or more docking sequences (*e.g.,* a first docking sequence and a second docking sequence) that can hybridize to one or more oligonucleotides (*e.g.,* a padlock probe(s)) used for rolling circle amplification. In some embodiments, additional probes are affixed to the substrate, where the additional probes include sequences (*e.g.,* a docking sequence(s), a functional sequence(s), and/or a primer sequence(s)) that enable rolling circle amplification. In some embodiments, the spatial array is contacted with an oligonucleotide (*e.g.,* a padlock probe). As used herein, a "padlock probe" refers to an oligonucleotide that has, at its 5' and 3' ends, sequences that are complementary to adjacent or nearby target sequences (*e.g.,* docking sequences) on a capture probe. Upon hybridization to the target sequences (*e.g.,* docking sequences), the two ends of the padlock probe are either brought into contact or an end is extended until the two ends are brought into contact, allowing circularization of the padlock probe by ligation (*e.g.,* ligation using any of the methods described herein). In some embodiments, after circularization of the oligonucleotide, rolling circle amplification can be used to amplify the ligation product, which includes at least a capture domain and a spatial barcode from the capture probe. In some embodiments, amplification of the capture probe using a padlock oligonucleotide and rolling circle amplification increases the number of capture domains and the number of spatial barcodes on the spatial array.

In some embodiments, a method of increasing capture efficiency of a spatial array includes amplifying all or part of a capture probe affixed to a substrate. For example, amplification of all or part of the capture probes affixed to the substrate can increase the capture efficiency of the spatial array by increasing the number of capture domains and spatial barcodes. In some embodiments, a method of determining a location of an analyte in a biological sample includes using a spatial array having increased capture efficiency (*e.g.,* a spatial array where a capture probe has been amplified as described herein). For example, the capture efficiency of a spatial array can be increased by amplification of all or part of the capture probe prior to contact with a biological sample. The amplification results in an increased number of capture domains that enable capture of more analytes as compared to a spatial array where the capture probe was not amplified prior to contacting the biological sample. In some embodiments, a method of producing a spatial array that has increased capture efficiency includes amplifying all or part of a capture probe. In some embodiments where a spatial array having increased capture efficiency is produced by amplifying all or part of a capture probe, the amplification increases the number of capture domains and the number of spatial barcodes on the spatial array. In some embodiments, a method of determining the location of a capture probe (e.g., a capture probe on a feature) on a spatial array includes amplifying all or part of a capture probe. For example, amplification of the capture probe affixed to the substrate can increase the number of spatial barcodes used for direct decoding (*e.g.,* direct decoding using any of the methods described herein including, without limitation, in situ sequencing) of the location of the capture probe.

### (ix) *Analyte Capture Agents*

This disclosure also provides methods and materials for using analyte capture agents for spatial profiling of biological analytes (*e.g.,* mRNA, genomic DNA, accessible chromatin, and cell surface or intracellular proteins and/or metabolites). As used herein, an "analyte capture agent" (also referred to previously at times as a "cell labelling" agent") refers to an agent that interacts with an analyte (*e.g.,* an analyte in a sample) and with a capture probe (*e.g.,* a capture probe attached to a substrate) to identify the analyte. In some embodiments, the analyte capture agent includes an analyte binding moiety and a capture agent barcode domain.

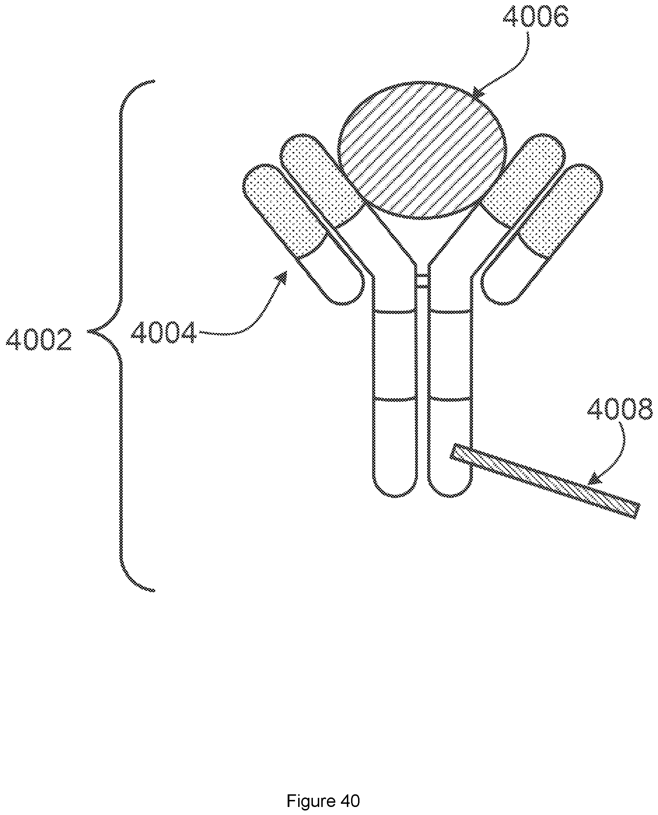

FIG. 40 is a schematic diagram of an exemplary analyte capture agent 4002 for capturing analytes. The analyte capture agent comprises an analyte binding moiety 4004 and a capture agent barcode domain 4008. An analyte binding moiety 4004 is a molecule capable of binding to an analyte 4006 and interacting with a spatially-barcoded capture probe. The analyte binding moiety can bind to the analyte 4006 with high affinity and/or with high specificity. The analyte capture 4002 agent can include a capture agent barcode domain 4008, a nucleotide sequence (*e.g.,* an oligonucleotide), which can hybridize to at least a portion or an entirety of a capture domain of a capture probe. The analyte binding moiety 4004 can include a polypeptide and/or an aptamer (*e.g.,* an oligonucleotide or peptide molecule that binds to a specific target analyte). The analyte binding moiety 4004 can include an antibody or antibody fragment (*e.g.,* an antigen-binding fragment).

As used herein, the term "analyte binding moiety" refers to a molecule or moiety capable of binding to a macromolecular constituent (*e.g.,* an analyte such as a biological analyte). In some embodiments of any of the spatial profiling methods described herein, the analyte binding moiety 4004 of the analyte capture agent 4002 that binds to a biological analyte 4006 can include, but is not limited to, an antibody, or an epitope binding fragment thereof, a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The analyte binding moiety 4004 can bind to the macromolecular constituent (*e.g.,* analyte) with high affinity and/or with high specificity. The analyte binding moiety 4004 can include a nucleotide sequence (*e.g.,* an oligonucleotide), which can correspond to at least a portion or an entirety of the analyte binding moiety. The analyte binding moiety 4004 can include a polypeptide and/or an aptamer (*e.g.,* a polypeptide and/or an aptamer that binds to a specific target molecule, e.g., an analyte). The analyte binding moiety 4004 can include an antibody or antibody fragment (*e.g.,* an antigen-binding fragment) that binds to a specific analyte (*e.g.,* a polypeptide).

In some embodiments, an analyte binding moiety 4004 of an analyte capture agent 4002 includes one or more antibodies or antigen binding fragments thereof. The antibodies or antigen binding fragments including the analyte binding moiety 4004 can specifically bind to a target analyte. In some embodiments, the analyte 4006 is a protein (*e.g.,* a protein on a surface of the biological sample, such as a cell, or an intracellular protein). In some embodiments, a plurality of analyte capture agents comprising a plurality of analyte binding moieties bind a plurality of analytes present in a biological sample. In some embodiments, the plurality of analytes includes a single species of analyte (*e.g.,* a single species of polypeptide). In some embodiments in which the plurality of analytes includes a single species of analyte, the analyte binding moieties of the plurality of analyte capture agents are the same. In some embodiments in which the plurality of analytes includes a single species of analyte, the analyte binding moieties of the plurality of analyte capture agents are the different (*e.g.,* members of the plurality of analyte capture agents can have two or more species of analyte binding moieties, where each of the two or more species of analyte binding moieties binds a single species of analyte, *e.g.,* at different binding sites). In some embodiments, the plurality of analytes includes multiple different species of analyte (*e.g.,* multiple different species of polypeptides).

An analyte capture agent **4002** can include an analyte binding moiety **4004.** The analyte binding moiety **4004** can be an antibody. Exemplary, non-limiting antibodies that can be used as analyte binding moieties **4004 in** an analyte capture agent **4002** or that can be used in the applications disclosed herein include any of the following including variations thereof: A-ACT, A-AT, ACTH, Actin-Muscle-specific, Actin-Smooth Muscle (SMA), AE1, AE1/AE3, AE3, AFP, AKT Phosphate, ALK-1, Amyloid A, Androgen Receptor, Annexin A1, B72.3, BCA-225, BCL-1 (Cyclin D1), BCL-1/CD20, BCL-2, BCL-2/BCL-6, BCL-6, Ber-EP4, Beta-amyloid, Beta-catenin, BG8 (Lewis Y), BOB-1, CA 19.9, CA 125, CAIX, Calcitonin, Caldesmon, Calponin, Calretinin, CAM 5.2, CAM 5.2/AE1, CD1a, CD2, CD3 (M), CD3 (P), CD3/CD20, CD4, CD5, CD7, CD8, CD10, CD14, CD15, CD20, CD21, CD22, CD 23, CD25, CD30, CD31, CD33, CD34, CD35, CD43, CD45 (LCA), CD45RA, CD56, CD57, CD61, CD68, CD71, CD74, CD79a, CD99, CD117 (c-KIT), CD123, CD138, CD163, CDX-2, CDX-2/CK-7, CEA (M), CEA (P), Chromogranin A, Chymotrypsin, CK-5, CK-5/6, CK-7, CK-7/TTF-1, CK-14, CK-17, CK-18, CK-19, CK-20, CK-HMW, CK-LMW, CMV-IH, COLL-IV, COX-2, D2-40, DBA44, Desmin, DOG1, EBER-ISH, EBV (LMP1), E-Cadherin, EGFR, EMA, ER, ERCC1, Factor VIII (vWF), Factor XIIIa, Fascin, FLI-1, FHS, Galectin-3, Gastrin, GCDFP-15, GFAP, Glucagon, Glycophorin A, Glypican-3, Granzyme B, Growth Hormone (GH), GST, HAM 56, HMBE-1, HBP, HCAg, HCG, Hemoglobin A, HEP B CORE (HBcAg), HEP B SURF, (HBsAg), HepPar1, HER2, Herpes I, Herpes II, HHV-8, HLA-DR, HMB 45, HPL, HPV-IHC, HPV (6/11)-ISH, HPV (16/18)-ISH, HPV (31/33)-ISH, HPV WSS-ISH, HPV High-ISH, HPV Low-ISH, HPV High & Low-ISH, IgA, IgD, IgG, IgG4, IgM, Inhibin, Insulin, JC Virus-ISH, Kappa-ISH, KER PAN, Ki-67, Lambda-IHC, Lambda-ISH, LH, Lipase, Lysozyme (MURA), Mammaglobin, MART-1, MBP, M-Cell Tryptase, MEL-5, Melan-A,, Melan-A/Ki-67, Mesothelin, MiTF, MLH-1, MOC-31, MPO, MSH-2, MSH-6, MUC1, MUC2, MUC4, MUC5AC, MUM-1, MYO D1, Myogenin, Myoglobin, Myoin Heavy Chain, Napsin A, NB84a, NEW-N, NF, NK1-C3, NPM, NSE, OCT-2, OCT-3/4, OSCAR, p16, p21, p27/Kip1, p53, p57, p63, p120, P504S, Pan Melanoma, PANC.POLY, Parvovirus B19, PAX-2, PAX-5, PAX-5/CD43, PAX=5/CD5, PAX-8, PC, PD1, Perforin, PGP 9.5, PLAP, PMS-2, PR, Prolactin, PSA, PSAP, PSMA, PTEN, PTH, PTS, RB, RCC, S6, S100, Serotonin, Somatostatin, Surfactant (SP-A), Synaptophysin, Synuclein, TAU, TCL-1, TCR beta, TdT, Thrombomodulin, Thyroglobulin, TIA-1, TOXO, TRAP, TriView™ breast, TriView™ prostate, Trypsin, TS, TSH, TTF-1, Tyrosinase, Ubiqutin, Uroplakin, VEGF, Villin, Vimentin (VIM), VIP, VZV, WT1 (M) N-Terminus, WT1 (P) C-Terminus, and ZAP-70.

Further, exemplary, non-limiting antibodies that can be used as analyte binding moieties **4004** in an analyte capture agent **4002** or that can be used in the applications disclosed herein include any of the following antibodies (and variations thereof) to: cell surface proteins, intracellular proteins, kinases (*e.g.*, AGC kinase family such as AKT1, AKT2, PDK1, Protein Kinase C, ROCK1, ROCK2, SGK3), CAMK kinase family (*e.g.*, AMPK1, AMPK2, CAMK, Chk1, Chk2, Zip), CK1 kinase family, TK kinase family (*e.g.*, Abl2, AXL, CD167, CD246/ALK, c-Met, CSK, c-Src, EGFR, ErbB2 (HER2/neu), ErbB3, ErbB4, FAK, Fyn, LCK, Lyn, PKT7, Syk, Zap70), STE kinase family (*e.g.*, ASK1, MAPK, MEK1, MEK2, MEK3 MEK4, MEK5, PAK1, PAK2, PAK4, PAK6), CMGC kinase family (*e.g.*, Cdk2, Cdk4, Cdk5, Cdk6, Cdk7, Cdk9, Erk1, GSK3, Jnk/MAPK8, Jnk2/MAPK9, JNK3/MAPK10, p38/MAPK), and TKL kinase family (*e.g.*, ALK1, ILK1, IRAK1, IRAK2, IRAK3, IRAK4, LIMK1, LIMK2, M3K11, RAF1, RIP1, RIP3, VEGFR1, VEGFR2, VEGFR3), Aurora A kinase, Aurora B kinase, IKK, Nemo-like kinase, PINK, PLK3, ULK2, WEE1, transcription factors (*e.g.,* FOXP3, ATF3, BACH1, EGR, ELF3, FOXA1, FOXA2, FOX01, GATA), growth factor receptors, and tumor suppressors (*e.g.,* anti-p53, anti-BLM, anti-Cdk2, anti-Chk2, anti-BRCA-1, anti-NBS1, anti-BRCA-2, anti-WRN, anti-PTEN, anti-WT1, anti-p38).

In some embodiments, analyte capture agents **4002** are capable of binding to analytes **4006** present inside a cell. In some embodiments, analyte capture agents are capable of binding to cell surface analytes that can include, without limitation, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (*e.g.*, phosphorylation, glycosylation, ubiquitination, nitrosylation, methylation, acetylation or lipidation) state of a cell surface protein, a gap junction, and an adherens junction. In some embodiments, the analyte capture agents **4002** are capable of binding to cell surface analytes that are posttranslationally modified. In such embodiments, analyte capture agents can be specific for cell surface analytes based on a given state of posttranslational modification (*e.g.*, phosphorylation, glycosylation, ubiquitination, nitrosylation, methylation, acetylation or lipidation), such that a cell surface analyte profile can include posttranslational modification information of one or more analytes.

In some embodiments, the analyte capture agent **4002** includes a capture agent barcode domain **4008** that is conjugated or otherwise attached to the analyte binding moiety. In some embodiments, the capture agent barcode domain **4008** is covalently-linked to the analyte binding moiety **4004.** In some embodiments, a capture agent barcode domain **4008** is a nucleic acid sequence. In some embodiments, a capture agent barcode domain **4008** includes, or is covalently bound to, an analyte binding moiety barcode and an analyte capture sequence **4114.**

As used herein, the term "analyte binding moiety barcode" refers to a barcode that is associated with or otherwise identifies the analyte binding moiety **4004.** In some embodiments, by identifying an analyte binding moiety **4004** and its associated analyte binding moiety barcode, the analyte **4006** to which the analyte binding moiety binds **4004** can also be identified. An analyte binding moiety barcode can be a nucleic acid sequence of a given length and/or sequence that is associated with the analyte binding moiety **4004**. An analyte binding moiety barcode can generally include any of the variety of aspects of barcodes described herein. For example, an analyte capture agent **4002** that is specific to one type of analyte can have coupled thereto a first capture agent barcode domain (*e.g.,* that includes a first analyte binding moiety barcode), while an analyte capture agent that is specific to a different analyte can have a different capture agent barcode domain (*e.g.,* that includes a second barcode analyte binding moiety barcode) coupled thereto. In some aspects, such a capture agent barcode domain can include an analyte binding moiety barcode that permits identification of the analyte binding moiety **4004** to which the capture agent barcode domain is coupled. The selection of the capture agent barcode domain **4008** can allow significant diversity in terms of sequence, while also being readily attachable to most analyte binding moieties (*e.g.,* antibodies or aptamers) as well as being readily detected, (*e.g.,* using sequencing or array technologies).

In some embodiments, the capture agent barcode domain of an analyte capture agent **4002** includes an analyte capture sequence. As used herein, the term "analyte capture sequence" refers to a region or moiety configured to hybridize to, bind to, couple to, or otherwise interact with a capture domain of a capture probe. In some embodiments, an analyte capture sequence includes a nucleic acid sequence that is complementary to or substantially complementary to the capture domain of a capture probe such that the analyte capture sequence hybridizes to the capture domain of the capture probe. In some embodiments, an analyte capture sequence comprises a poly(A) nucleic acid sequence that hybridizes to a capture domain that comprises a poly(T) nucleic acid sequence. In some embodiments, an analyte capture sequence comprises a poly(T) nucleic acid sequence that hybridizes to a capture domain that comprises a poly(A) nucleic acid sequence. In some embodiments, an analyte capture sequence comprises a non-homopolymeric nucleic acid sequence that hybridizes to a capture domain that comprises a non-homopolymeric nucleic acid sequence that is complementary (or substantially complementary) to the non-homopolymeric nucleic acid sequence of the analyte capture region.

In some embodiments of any of the spatial analysis methods described herein that employ an analyte capture agent **4002,** the capture agent barcode domain can be directly coupled to the analyte binding moiety **4004,** or they can be attached to a bead, molecular lattice, *e.g.,* a linear, globular, cross-slinked, or other polymer, or other framework that is attached or otherwise associated with the analyte binding moiety, which allows attachment of multiple capture agent barcode domains to a single analyte binding moiety. Attachment (coupling) of the capture agent barcode domains to the analyte binding moieties **4004** can be achieved through any of a variety of direct or indirect, covalent or non-covalent associations or attachments. For example, in the case of a capture agent barcode domain coupled to an analyte binding moiety **4004** that includes an antibody or antigen-binding fragment, such capture agent barcode domains can be covalently attached to a portion of the antibody or antigen-binding fragment using chemical conjugation techniques (*e.g.,* LIGHTNING-LINK® antibody labelling kits available from Innova Biosciences). In some embodiments, a capture agent barcode domain can be coupled to an antibody or antigen-binding fragment using non-covalent attachment mechanisms (*e.g.,* using biotinylated antibodies and oligonucleotides or beads that include one or more biotinylated linker(s), coupled to oligonucleotides with an avidin or streptavidin linker). Antibody and oligonucleotide biotinylation techniques can be used, and are described for example in Fang et al., 2003, Nucleic Acids Res. 31(2): 708-715, the entire contents of which are incorporated by reference herein. Likewise, protein and peptide biotinylation techniques have been developed and can be used, and are described for example in U.S. Patent No. 6,265,552, the entire contents of which are incorporated by reference herein. Furthermore, click reaction chemistry such as a methyltetrazine-PEG5-NHS ester reaction, a TCO-PEG4-NHS ester reaction, or the like, can be used to couple capture agent barcode domains to analyte binding moieties **4004.** The reactive moiety on the analyte binding moiety can also include amine for targeting aldehydes, amine for targeting maleimide (*e.g.,* free thiols), azide for targeting click chemistry compounds (*e.g.,* alkynes), biotin for targeting streptavidin, phosphates for targeting EDC, which in turn targets active ester (*e.g.,* NH2). The reactive moiety on the analyte binding moiety **4004** can be a chemical compound or group bound to the reactive moiety. Exemplary strategies to conjugate the analyte binding moiety **4004** to the capture agent barcode domain include the use of commercial kits (*e.g.,* Solulink, Thunder link), conjugation of mild reduction of hinge region and maleimide labelling, stain-promoted click chemistry reaction to labeled amides (*e.g.,* copper-free), and conjugation of periodate oxidation of sugar chain and amine conjugation. In the cases where the analyte binding moiety **4004** is an antibody, the antibody can be modified prior to or contemporaneously with conjugation of the oligonucleotide. For example, the antibody can be glycosylated with a chemical substrate-permissive mutant of β-1,4-galactosyltransferase, GalT (Y289L) and azide-bearing uridine diphosphate-N-acetylgalactosamine analog uridine diphosphate -GalNAz. The modified antibody can be conjugated to an oligonucleotide with a dibenzocyclooctyne-PEG4-NHS group. In some embodiments, certain steps (*e.g.,* COOH activation such as EDC) and homobifunctional cross linkers) can be avoided to prevent the analyte binding moieties from conjugating to themselves. In some embodiments of any of the spatial profiling methods described herein, the analyte capture agent (*e.g.,* analyte binding moiety **4004** coupled to an oligonucleotide) can be delivered into the cell, *e.g.,* by transfection (*e.g.,* using transfectamine, cationic polymers, calcium phosphate or electroporation), by transduction (*e.g.,* using a bacteriophage or recombinant viral vector), by mechanical delivery (*e.g.,* magnetic beads), by lipid (*e.g.,* 1,2-dioleoyl-sn-glycero-3-phosphocholine (DOPC)), or by transporter proteins.

An analyte capture agent **4002** can be delivered into a cell using exosomes. For example, a first cell can be generated that releases exosomes comprising an analyte capture agent. An analyte capture agent can be attached to an exosome membrane. An analyte capture agent can be contained within the cytosol of an exosome. Released exosomes can be harvested and provided to a second cell, thereby delivering the analyte capture agent into the second cell. An analyte capture agent can be releasable from an exosome membrane before, during, or after delivery into a cell. In some embodiments, the cell is permeabilized to allow the analyte capture agent **4002** to couple with intracellular constituents (such as, without limitation, intracellular proteins, metabolites, and nuclear membrane proteins). Following intracellular delivery, analyte capture agents **4002** can be used to analyze intracellular constituents as described herein.

In some embodiments of any of the spatial profiling methods described herein, the capture agent barcode domain coupled to an analyte capture agent **4002** can include modifications that render it non-extendable by a polymerase. In some embodiments, when binding to a capture domain of a capture probe or nucleic acid in a sample for a primer extension reaction, the capture agent barcode domain can serve as a template, not a primer. When the capture agent barcode domain also includes a barcode (*e.g.,* an analyte binding moiety barcode), such a design can increase the efficiency of molecular barcoding by increasing the affinity between the capture agent barcode domain and unbarcoded sample nucleic acids, and eliminate the potential formation of adaptor artifacts. In some embodiments, the capture agent barcode domain **4008** can include a random N-mer sequence that is capped with modifications that render it non-extendable by a polymerase. In some cases, the composition of the random N-mer sequence can be designed to maximize the binding efficiency to free, unbarcoded ssDNA molecules. The design can include a random sequence composition with a higher GC content, a partial random sequence with fixed G or C at specific positions, the use of guanosines, the use of locked nucleic acids, or any combination thereof.

A modification for blocking primer extension by a polymerase can be a carbon spacer group of different lengths or a dideoxynucleotide. In some embodiments, the modification can be an abasic site that has an apurine or apyrimidine structure, a base analog, or an analogue of a phosphate backbone, such as a backbone of N-(2-aminoethyl)-glycine linked by amide bonds, tetrahydrofuran, or 1', 2'-Dideoxyribose. The modification can also be a uracil base, 2'OMe modified RNA, C3-18 spacers (*e.g.*, structures with 3-18 consecutive carbon atoms, such as C3 spacer), ethylene glycol multimer spacers (*e.g.*, spacer 18 (hexa-ethyleneglycol spacer)), biotin, di-deoxynucleotide triphosphate, ethylene glycol, amine, or phosphate).

In some embodiments of any of the spatial profiling methods described herein, the capture agent barcode domain **4008** coupled to the analyte binding moiety **4004** includes a cleavable domain. For example, after the analyte capture agent binds to an analyte (*e.g.*, a cell surface analyte), the capture agent barcode domain can be cleaved and collected for downstream analysis according to the methods as described herein. In some embodiments, the cleavable domain of the capture agent barcode domain includes a U-excising element that allows the species to release from the bead. In some embodiments, the U-excising element can include a single-stranded DNA (ssDNA) sequence that contains at least one uracil. The species can be attached to a bead via the ssDNA sequence. The species can be released by a combination of uracil-DNA glycosylase (*e.g.,* to remove the uracil) and an endonuclease (*e.g.,* to induce a ssDNA break). If the endonuclease generates a 5' phosphate group from the cleavage, then additional enzyme treatment can be included in downstream processing to eliminate the phosphate group, *e.g.,* prior to ligation of additional sequencing handle elements, *e.g.,* Illumina full P5 sequence, partial P5 sequence, full R1 sequence, and/or partial R1 sequence.

In some embodiments, multiple different species of analytes (*e.g.,* polypeptides) from the biological sample can be subsequently associated with the one or more physical properties of the biological sample. For example, the multiple different species of analytes can be associated with locations of the analytes in the biological sample. Such information (*e.g.,* proteomic information when the analyte binding moiety(ies) recognizes a polypeptide(s)) can be used in association with other spatial information (*e.g.,* genetic information from the biological sample, such as DNA sequence information, transcriptome information, for example sequences of transcripts, or both). For example, a cell surface protein of a cell can be associated with one or more physical properties of the cell (*e.g.,* a shape, size, activity, or a type of the cell). The one or more physical properties can be characterized by imaging the cell. The cell can be bound by an analyte capture agent comprising an analyte binding moiety that binds to the cell surface protein and an analyte binding moiety barcode that identifies that analyte binding moiety, and the cell can be subjected to spatial analysis (*e.g.,* any of the variety of spatial analysis methods described herein). For example, the analyte capture agent **4002** bound to the cell surface protein can be bound to a capture probe (*e.g.*, a capture probe on an array), which capture probe includes a capture domain that interacts with an analyte capture sequence present on the capture agent barcode domain of the analyte capture agent **902.** All or part of the capture agent barcode domain (including the analyte binding moiety barcode) can be copied with a polymerase using a 3' end of the capture domain as a priming site, generating an extended capture probe that includes the all or part of complementary sequence that corresponds to the capture probe (including a spatial barcode present on the capture probe) and a copy of the analyte binding moiety barcode. In some embodiments, an analyte capture agent with an extended capture agent barcode domain that includes a sequence complementary to a spatial barcode of a capture probe is called a "spatially-tagged analyte capture agent."

In some embodiments, the spatial array with spatially-tagged analyte capture agents can be contacted with a sample, where the analyte capture agent(s) associated with the spatial array capture the target analyte(s). The analyte capture agent(s) containing the extended capture probe(s), which includes a sequence complementary to the spatial barcode(s) of the capture probe(s) and the analyte binding moiety barcode(s), can then be denatured from the capture probe(s) of the spatial array. This allows the spatial array to be reused. The sample can be dissociated into non-aggregated cells (*e.g.,* single cells) and analyzed by the single cell / droplet methods described herein. The spatially-tagged analyte capture agent can be sequenced to obtain the nucleic acid sequence of the spatial barcode of the capture probe and the analyte binding moiety barcode of the analyte capture agent. The nucleic acid sequence of the extended capture probe can thus be associated with an analyte (*e.g.,* cell surface protein), and in turn, with the one or more physical properties of the cell (*e.g.,* a shape or cell type). In some embodiments, the nucleic acid sequence of the extended capture probe can be associated with an intracellular analyte of a nearby cell, where the intracellular analyte was released using any of the cell permeabilization or analyte migration techniques described herein.

In some embodiments of any of the spatial profiling methods described herein, the capture agent barcode domains released from the analyte capture agents can then be subjected to sequence analysis to identify which analyte capture agents were bound to analytes. Based upon the capture agent barcode domains that are associated with a capture spot (*e.g.,* a capture spot at a particular location) on a spatial array and the presence of the analyte binding moiety barcode sequence, an analyte profile can be created for a biological sample. Profiles of individual cells or populations of cells can be compared to profiles from other cells, *e.g.,* 'normal' cells, to identify variations in analytes, which can provide diagnostically relevant information. In some embodiments, these profiles can be useful in the diagnosis of a variety of disorders that are characterized by variations in cell surface receptors, such as cancer and other disorders.

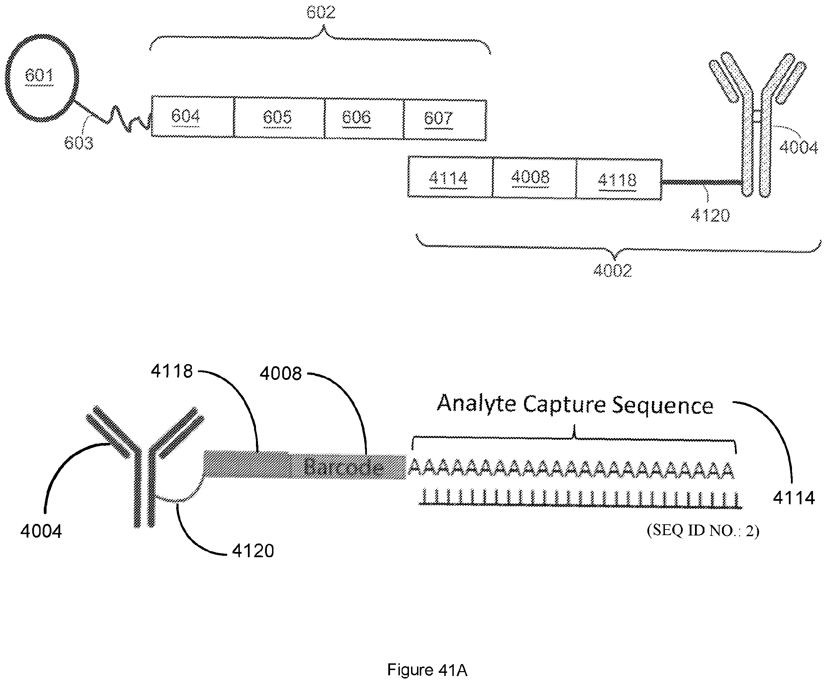

**FIG. 41A,** top panel, is a schematic diagram depicting an exemplary interaction between a feature-immobilized capture probe **602** and an analyte capture agent **4002** (where the terms "feature" and "capture spot" are used interchangeably). The feature-immobilized capture probe **602** can include a spatial barcode **605** as well as one or more functional sequences **604** and **606,** as described elsewhere herein. The capture probe **602** can also include a capture domain **607** that is capable of binding to an analyte capture agent **4002**. In some embodiments, the analyte capture agent **4002** comprises a functional sequence **4118,** capture agent barcode domain **4008,** and an analyte capture sequence **4114.** In some embodiments the analyte capture sequence **4114** is capable of binding to the capture domain **607 of** the capture probe **602.** The analyte capture agent **4002** can also include a linker **4120** that allows the capture agent barcode domain **4008 (4114/4008/4118)** to couple to the analyte binding moiety **4004.**

**FIG. 41A,** bottom panel, further illustrates a spatially-tagged analyte capture agent **4002** in which the analyte capture sequence **4114** (poly-A sequence) of the capture agent barcode domain **4118/4008/4114** can be blocked with a blocking probe (poly-T oligonucleotide).

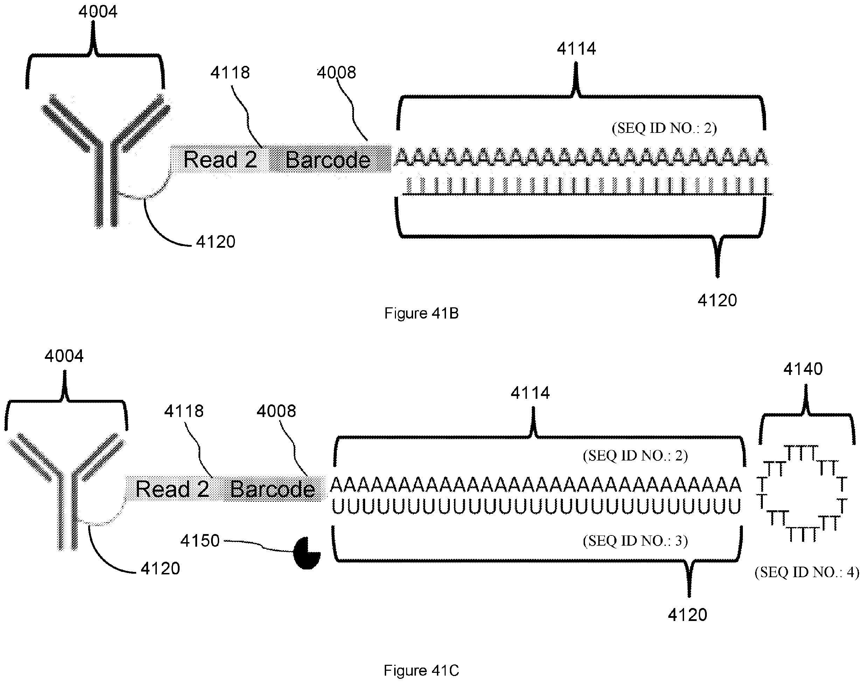

In some embodiments, the capture binding domain can include a sequence that is at least partially complementary to a sequence of a capture domain of a capture probe (e.g., any of the exemplary capture domains described herein). **FIG. 41B** shows an exemplary capture binding domain attached to an analyte-binding moiety used to detect a protein in a biological sample. As show in **FIG. 41B,** an analyte-binding moiety **4004** includes an oligonucleotide that includes a primer (*e.g.,* a read2) sequence **4118,** an analyte-binding-moiety barcode **4008,** a capture binding domain having a first sequence (e.g., a capture binding domain) **4114** (*e.g.,* an exemplary poly A), and a blocking probe or second sequence **4120** (*e.g.,* poly T or poly U), where the blocking sequence blocks the capture binding domain from hybridizing to a capture domain on a capture probe. In some instances, the blocking sequence **4120** is called a blocking probe as disclosed herein. In some instances, the blocking probe is a poly T sequence as exemplified in **FIG. 41B.**

In some instances, as shown in **FIG. 41A,** the blocking probe sequence is not on a contiguous sequence with the capture binding domain. In other words, in some instances, the capture binding domain (also herein called a first sequence) and the blocking sequence are independent polynucleotides. In some instances, it will be apparent to one skilled in the art that the terms "capture binding domain" and "first sequence" are used interchangeably in this disclosure.

In a non-limiting example, the first sequence can be a poly(A) sequence when the capture domain sequence of the capture probe on the substrate is a poly(T) sequence. In some embodiments, the capture binding domain includes a capture binding domain substantially complementary to the capture domain of the capture probe. By substantially complementary, it is meant that the first sequence of the capture binding domain is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a sequence in the capture domain of the capture probe. In another example, the first sequence of the capture binding domain can be a random sequence (*e.g.,* random hexamer) that is at least partially complementary to a capture domain sequence of the capture probe that is also a random sequence. In yet another example, a capture binding domain can be a mixture of a homopolymeric sequence (*e.g.,* a poly(T) sequence) and a random sequence (*e.g.,* random hexamer) when a capture domain sequence of the capture probe is also a sequence that includes a homopolymeric sequence (*e.g.,* a poly(A) sequence) and a random sequence. In some embodiments, the capture binding domain includes ribonucleotides, deoxyribonucleotides, and/or synthetic nucleotides that are capable of participating in Watson-Crick type or analogous base pair interactions. In some embodiments, the first sequence of the capture binding domain sequence includes at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, or at least 24 nucleotides. In some embodiments, the first sequence of the capture binding domain includes at least 25 nucleotides, at least 30 nucleotides, or at least 35 nucleotides.

In some embodiments, the capture binding domain (*e.g.,* the first sequence) and the blocking probe (*e.g.,* the second sequence) of the capture binding domain are located on the same contiguous nucleic acid sequence. Where the capture binding domain and the blocking probe are located on the same contiguous nucleic acid sequence, the second sequence (*e.g.,* a blocking probe) is located 3' of the first sequence. Where the first sequence and the second sequence (*e.g.,* a blocking probe) of the capture binding domain are located on the same contiguous nucleic acid sequence, the second sequence (*e.g.,* the blocking probe) is located 5' of the first sequence. As used herein, the terms second sequence and blocking probe are used interchangeably.

In some instances, the second sequence (*e.g.,* the blocking probe) of the capture binding domain includes a nucleic acid sequence. In some instances, the second sequence is also called a blocking probe or blocking domain, and each term is used interchangeably. In some instances, the blocking domain is a DNA oligonucleotide. In some instances, the blocking domain is an RNA oligonucleotide. In some embodiments, a blocking probe of the capture binding domain includes a sequence that is complementary or substantially complementary to a first sequence of the capture binding domain. In some embodiments, the blocking probe prevents the first sequence of the capture binding domain from binding the capture domain of the capture probe when present. In some embodiments, the blocking probe is removed prior to binding the first sequence of the capture binding domain (*e.g.,* present in a ligated probe) to a capture domain on a capture probe. In some embodiments, a blocking probe of the capture binding domain includes a poly-uridine sequence, a poly-thymidine sequence, or both. In some instances, the blocking probe (or the second sequence) is part of a hairpin structure that specifically binds to a capture binding domain and prevents the capture binding domain from hybridizing to a capture domain of a capture probe. See *e.g.,* **FIG. 41C.**

In some embodiments, the second sequence (*e.g.,* the blocking probe) of the capture binding domain includes a sequence configured to hybridize to the first sequence of the capture binding domain. When the blocking probe is hybridized to the first sequence, the first sequence is blocked from hybridizing with a capture domain of a capture probe. In some embodiments, the blocking probe includes a sequence that is complementary to the first sequence. In some embodiments, the blocking probe includes a sequence that is substantially complementary to the first sequence. In some embodiments, the blocking probe includes a sequence that is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to the first sequence of the capture binding domain.

In some embodiments, the blocking probe of the capture binding domain includes a homopolymeric sequence that is substantially complementary to the first sequence of the capture binding domain. In some embodiments, the blocking probe is configured to hybridize to a poly(A), poly(T), or a poly-rU sequence. In some embodiments, the blocking probe includes a poly(A), poly(T), or a poly(U) sequence. In some embodiments, the first sequence includes a homopolymeric sequence. In some embodiments, the first sequence includes a poly(A), poly(U), or a poly(T) sequence.

In some embodiments, the capture binding domain further includes a hairpin sequence (as shown in **FIG. 41C**). **FIG. 41C** shows an exemplary capture binding domain attached to an analyte-binding moiety used to detect a protein in a biological sample. As shown in **FIG. 41C,** an analyte-binding moiety **4004** includes an oligonucleotide that includes a primer (*e.g.*, a read2) sequence **4118,** an analyte-binding-moiety barcode **4008,** a capture binding domain having a first sequence **4114** (*e.g.*, an exemplary poly A), a blocking probe **4120** and a third sequence **4140,** where the second and/or third sequence can be poly T or poly U or a combination thereof, where the blocking probe creates a hairpin type structure and the third sequence blocks the first sequence from hybridizing to a capture domain on a capture probe. In some instances, the third sequence **4140** is called a blocking sequence. Further, **4150** exemplifies a nuclease capable of digesting the blocking sequencing. In this example, **4150** could be an endonuclease or mixture of nucleases capable of digesting uracils, such as UDG or a uracil specific excision mix such as USER (NEB).

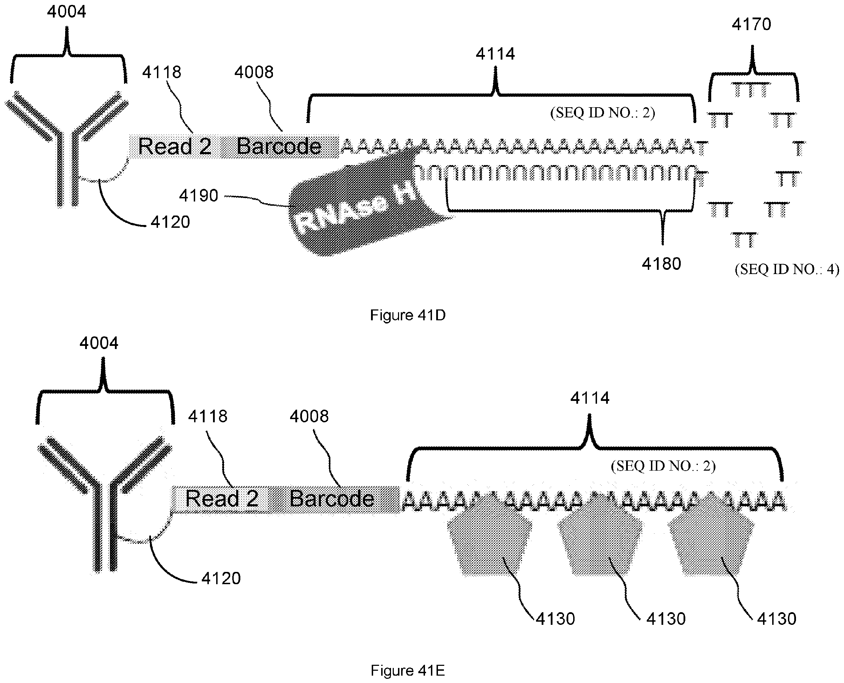

Another embodiment of a hairpin blocker scenario is exemplified in **FIG 41D.** As exemplified in **FIG. 41D,** an analyte-binding moiety **4004** includes an oligonucleotide that includes a primer (*e.g.*, a read2) sequence **4118,** an analyte-binding-moiety barcode **4008,** a capture binding domain having a first sequence (*e.g.*, a capture binding domain) **4114** (*e.g.*, an exemplary poly A), a second hairpin sequence **4170** and a third sequence **4180,** where the third sequence (*e.g.*, a blocking probe) blocks the first sequence from hybridizing to a capture domain on a capture probe. In this example, **4190** exemplifies an RNase H nuclease capable of digesting the uracil blocking sequencing from the DNA:RNA hybrid that is formed by blocking of the first sequence with a uracil containing third sequence.

In some embodiments, the hairpin sequence **4170** is located 5' of the blocking probe in the capture binding domain. In some embodiments, the hairpin sequence **4170** is located 5' of the first sequence in the capture binding domain. In some embodiments, the capture binding domain includes from 5' to 3' a first sequence substantially complementary to the capture domain of a capture probe, a hairpin sequence, and a blocking probe substantially complementary to the first sequence. Alternatively, the capture binding domain includes from 3' to 5' a first sequence substantially complementary to the capture domain of a capture probe, a hairpin sequence, and a blocking probe substantially complementary to the first sequence.

In some embodiments, the hairpin sequence **4170** includes a sequence of about three nucleotides, about four nucleotides, about five nucleotides, about six nucleotides, about seven nucleotides, about eight nucleotides, about nine nucleotides or about 10 or more nucleotides. In some instances, the hairpin is at least about 15 nucleotides, at least about 20 nucleotides, at least about 25 nucleotides, at least about 30 nucleotides, or more nucleotides.

In some embodiments, the hairpin sequence includes DNA, RNA, DNA-RNA hybrid, or includes modified nucleotides. In some instances, the hairpin is a poly(U) sequence. In some instances, the RNA hairpin sequence is digested by USER and/or RNAse H using methods disclosed herein. In some instances, the poly(U) hairpin sequence is digested by USER and/or RNAse H using methods disclosed herein. In some instances, the hairpin is a poly(T) sequence. It is appreciated that the sequence of the hairpin (whether it includes DNA, RNA, DNA-RNA hybrid, or includes modified nucleotides) can be nearly any nucleotide sequence so long as it forms a hairpin, and in some instances, so long as it is digested by USER and/or RNAse H.

In some embodiments, methods provided herein require that the second sequence (*e.g.,* the blocking probe) of the capture binding domain that is hybridized to the first sequence of the capture binding domain is released from the first sequence. In some embodiments, releasing the blocking probe (or second sequence) from the first sequence is performed under conditions where the blocking probe de-hybridizes from the first sequence.

In some embodiments, releasing the blocking probe from the first sequence includes cleaving the hairpin sequence. In some embodiments, the hairpin sequence includes a cleavable linker. For example, the cleavable linker can be a photocleavable linker, UV-cleavable linker, or an enzyme-cleavable linker. In some embodiments, the enzyme that cleaves that enzymatic-cleavable domain is an endonuclease. In some embodiments, the hairpin sequence includes a target sequence for a restriction endonuclease.

In some embodiments, releasing the blocking probe (or the second sequence) of the capture binding domain that is hybridized to the first sequence of the capture binding domain includes contacting the blocking probe with a restriction endonuclease. In some embodiments, releasing the blocking probe from the first sequence includes contacting the blocking probe with an endoribonuclease. In some embodiments, when the blocking probe is an RNA sequence (*e.g.,* a sequence comprising uracils) the endoribonuclease is one or more of RNase H, RNase A, RNase C, or RNase I. In some embodiments, where the endoribonuclease is RNase H. In some embodiments, the RNase H includes RNase H1, RNase H2, or RNase H1 and RNase H2.

In some embodiments, the hairpin sequence includes a homopolymeric sequence. In some embodiments, the hairpin sequence **4170** includes a poly(T) or poly(U) sequence. For example, the hairpin sequence includes a poly(U) sequence. In some embodiments, provided herein are methods for releasing the blocking probe by contacting the hairpin sequence with a Uracil-Specific Excision Reagent (USER) enzyme.

In some embodiments, releasing the blocking probe from the first sequence includes denaturing the blocking probe under conditions where the blocking probe de-hybridizes from the first sequence. In some embodiments, denaturing comprises using chemical denaturation or physical denaturation. For example, where physical denaturation (*e.g.,* temperature) is used to release the blocking probe. In some embodiments, denaturing includes temperature modulation. For example, a first sequence and a blocking probe have predetermined annealing temperatures based on the composition (A, G, C, or T) within the known sequences. In some embodiments, the temperature is modulate up to 5° C, up to 10° C, up to 15° C, up to 20° C, up to 25° C, up to 30° C, or up to 35° C above the predetermined annealing temperature. In some embodiments, the temperature is modulated at 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35° C above the predetermined annealing temperature. In some embodiments, once the temperature is modulated to a temperature above the predetermined annealing temperature, the temperature is cooled down to the predetermined annealing temperature at a ramp rate of about 0.1° C/second to about 1.0° C/second (*e.g.,* about 0.1° C/second to about 0.9° C/second, about 0.1° C/second to about 0.8° C/second, about 0.1° C/second to about 0.7° C/second, about 0.1° C/second to about 0.6° C/second, about 0.1° C/second to about 0.5° C/second, about 0.1° C/second to about 0.4° C/second, about 0.1° C/second to about 0.3° C/second, about 0.1° C/second to about 0.2° C/second, about 0.2° C/second to about 1.0° C/second, about 0.2° C/second to about 0.9° C/second, about 0.2° C/second to about 0.8° C/second, about 0.2° C/second to about 0.7° C/second, about 0.2° C/second to about 0.6° C/second, about 0.2° C/second to about 0.5° C/second, about 0.2° C/second to about 0.4° C/second, about 0.2° C/second to about 0.3° C/second, about 0.3 to about 1.0° C/second, about 0.3° C/second to about 0.9° C/second, about 0.3° C/second to about 0.8° C/second, about 0.3° C/second to about 0.7° C/second, about 0.3° C/second to about 0.6° C/second, about 0.3° C/second to about 0.5° C/second, about 0.3° C/second to about 0.4° C/second, about 0.4° C/second to about 1.0° C/second, about 0.4° C/second to about 0.9° C/second, about 0.4° C/second to about 0.8° C/second, about 0.4° C/second to about 0.7° C/second, about 0.4° C/second to about 0.6° C/second, about 0.4° C/second to about 0.5° C/second, about 0.5° C/second to about 1.0° C/second, about 0.5° C/second to about 0.9° C/second, about 0.5° C/second to about 0.8° C/second, about 0.5° C/second to about 0.7° C/second, about 0.5° C/second to about 0.6° C/second, about 0.6° C/second to about 1.0° C/second, about 0.6° C/second to about 0.9° C/second, about 0.6° C/second to about 0.8° C/second, about 0.6° C/second to about 0.7° C/second, about 0.7° C/second to about 1.0° C/second, about 0.7° C/second to about 0.9° C/second, about 0.7° C/second to about 0.8° C/second, about 0.8° C/second to about 1.0° C/second, about 0.8° C/second to about 0.9° C/second, or about 0.9° C/second to about 1.0° C/second). In some embodiments, denaturing includes temperature cycling. In some embodiments, denaturing includes alternating between denaturing conditions (*e.g.*, a denaturing temperature) and non-denaturing conditions (*e.g.*, annealing temperature).

It is appreciated that, notwithstanding any particular function in an embodiment, the hairpin sequence can be any sequence configuration, so long as a hairpin is formed. Thus, in some instances, it could be, for example, a degenerate sequence, a random sequence, or otherwise (comprising any sequence of polynucleotides).

In some embodiments, the hairpin sequence **4170** further includes a sequence that is capable of binding to a capture domain of a capture probe. For example, releasing the hairpin sequence from the capture binding domain can require that the hairpin sequence is cleaved, where the portion of the hairpin sequence that is left following cleavage includes a sequence that is capable of binding to a capture domain of a capture probe. In some embodiments, all or a portion of the hairpin sequence is substantially complementary to a capture domain of a capture probe. In some embodiments, the sequence that is substantially complementary to a capture domain of a capture probe is located on the free 5' or free 3' end following cleavage of the hairpin sequence. In some embodiments, the cleavage of the hairpin results in a single stranded sequence that is capable of binding to a capture domain of a capture probe on a spatial array. While the release of a hairpin sequence may enable hybridization to a capture domain of a capture probe, it is contemplated that release of the hairpin would not significantly affect the capture of the target analyte by an analyte-binding moiety or a probe oligonucleotide (*e.g.,* a second probe oligonucleotide).

In some instances, the one or more blocking methods disclosed herein include a plurality of caged nucleotides. In some embodiments, provided herein are methods where a capture binding domain includes a plurality of caged nucleotides. The caged nucleotides prevent the capture binding domain from interacting with the capture domain of the capture probe. The caged nucleotides include caged moieties that block Watson-Crick hydrogen bonding, thereby preventing interaction until activation, for example, through photolysis of the caged moiety that releases the caged moiety and restores the caged nucleotides ability to engage in Watson-Crick base pairing with a complement nucleotide.

**FIG. 41E** is demonstrative of blocking a capture binding domain with caged nucleotides. As exemplified in **FIG. 41E,** an analyte-binding moiety **4004** includes an oligonucleotide that includes a primer (*e.g.*, a read2) sequence **4118,** an analyte-binding-moiety barcode **4008** and a capture binding domain having a sequence **4114** (*e.g.*, an exemplary polyA). Caged nucleotides **4130** block the sequence **4114,** thereby blocking the interaction between the capture binding domain and the capture domain of the capture probe. In some embodiments, the capture binding domain includes a plurality of caged nucleotides, where a caged nucleotide of the plurality of caged nucleotides includes a caged moiety that is capable of preventing interaction between the capture binding domain and the capture domain of the capture probe. Non-limiting examples of caged nucleotides, also known as light-sensitive oligonucleotides, are described in Liu et al., 2014, Acc. Chem. Res., 47(1): 45-55 (2014), which is incorporated by reference in its entirety. In some embodiments, the caged nucleotides include a caged moiety selected from the group of 6-nitropiperonyloxymethy (NPOM), 1-(ortho-nitrophenyl)-ethyl (NPE), 2-(ortho-nitrophenyl)propyl (NPP), diethylaminocoumarin (DEACM), and nitrodibenzofuran (NDBF).

In some embodiments, a caged nucleotide includes a non-naturally-occurring nucleotide selected from the group consisting of 6-nitropiperonyloxymethy (NPOM)-caged adenosine, 6-nitropiperonyloxymethy (NPOM)-caged guanosine, 6-nitropiperonyloxymethy (NPOM)-caged uridine, and 6-nitropiperonyloxymethy (NPOM)-caged thymidine. For example, the capture binding domain includes one or more caged nucleotides where the cage nucleotides include one or more 6-nitropiperonyloxymethy (NPOM)-caged guanosine. In another example, the capture binding domain includes one or more caged nucleotides where the cage nucleotides include one or more nitropiperonyloxymethy (NPOM)-caged uridine. In yet another example, the capture binding domain includes one or more caged nucleotides where the caged nucleotide includes one or more 6-nitropiperonyloxymethy (NPOM)-caged thymidine.

In some embodiments, the capture binding domain includes a combination of at least two or more of any of the caged nucleotides described herein. For example, the capture binding domain can include one or more 6-nitropiperonyloxymethy (NPOM)-caged guanosine and one or more nitropiperonyloxymethy (NPOM)-caged uridine. It is appreciated that a capture binding domain can include any combination of any of the caged nucleotides described herein.

In some embodiments, the capture binding domain includes one caged nucleotide, two caged nucleotides, three caged nucleotides, four caged nucleotides, five caged nucleotides, six caged nucleotides, seven caged nucleotides, eight caged nucleotides, nine caged nucleotides, or ten or more caged nucleotides.

In some embodiments, the capture binding domain includes a caged nucleotide at the 3' end. In some embodiments, the capture binding domain includes two caged nucleotides at the 3' end. In some embodiments, the capture binding domain includes at least three caged nucleotides at the 3' end.

In some embodiments, the capture binding domain includes a caged nucleotide at the 5' end. In some embodiments, the capture binding domain includes two caged nucleotides at the 5' end. In some embodiments, the capture binding domain includes at least three caged nucleotides at the 5' end.

In some embodiments, the capture binding domain includes a caged nucleotide at every odd position starting at the 3' end of the capture binding domain. In some embodiments, the capture binding domain includes a caged nucleotide at every odd position starting at the 5' end of the capture binding domain. In some embodiments, the capture binding domain includes a caged nucleotide at every even position starting at the 3' end of the capture binding domain. In some embodiments, the capture binding domain includes a caged nucleotide at every even position starting at the 5' end of the capture binding domain.

In some embodiments, the capture binding domain includes a sequence including at least 10%, at least, 20%, or at least 30% caged nucleotides. In some instances, the percentage of caged nucleotides in the capture binding domain is about 40%, about 50%, about 60%, about 70%, about 80% or higher. In some embodiments, the capture binding domain includes a sequence where every nucleotide is a caged nucleotide. It is understood that the limit of caged nucleotides is based on the sequence of the capture binding domain and on steric limitations of creating caged nucleotides in proximity to one another. Thus, in some instances, particular nucleotides (*e.g.*, guanines) are replaced with caged nucleotides. In some instances, all guanines in a capture binding domain are replaced with caged nucleotides. In some instances, a fraction (*e.g.*, about 10%, about 20%, about 30%, about 40%, about 50%, about 60%, about 70%, about 80%, about 90%, or about 95%) of guanines in a capture binding domain are replaced with caged nucleotides. In some instances, particular nucleotides (*e.g.*, uridines or thymines) are replaced with caged nucleotides. In some instances, all uridines or thymines in a capture binding domain are replaced with caged nucleotides. In some instances, a fraction (*e.g.*, about 10%, about 20%, about 30%, about 40%, about 50%, about 60%, about 70%, about 80%, about 90%, or about 95%) of uridines or thymines in a capture binding domain are replaced with caged nucleotides. Caged nucleotides are disclosed in Govan et al., 2013, Nucleic Acids Research 41; 22, 10518-10528, which is incorporated by reference in its entirety.

In some embodiments, the capture binding domain includes caged nucleotides that are evenly distributed throughout the capture binding domain. For example, a capture binding domain can include a sequence that includes at least 10% caged nucleotides where the caged nucleotides are evenly distributed throughout the capture binding domain. In some embodiments, the capture binding domain includes a sequence that is at least 10% caged nucleotides and where the 10% caged nucleotides are positioned at the 3' of the capture binding domain. In some embodiments, the capture binding domain includes a sequence that is at least 10% caged nucleotides and where the 10% caged nucleotides are positioned at the 5' end of the capture binding domain. In some embodiments, the caged nucleotides are included at every third, at every fourth, at every fifth, at every sixth nucleotide, or a combination thereof, of the capture binding domain sequence.

In some embodiments, provided herein are methods for releasing the caged moiety from the caged nucleotide. In some embodiments, releasing the caged moiety from the caged nucleotide includes activating the caged moiety. In some embodiments, releasing the caged moiety from the caged nucleotide restores the caged nucleotides ability to hybridize to a complementary nucleotide through Watson-Crick hydrogen bonding. For example, restoring the caged nucleotides ability to hybridize with a complementary nucleotide enables/restores the capture binding domain's ability to interact with the capture domain. Upon releasing the caged moiety from the caged nucleotide, the caged nucleotide is no longer "caged" in that the caged moiety is no longer linked (*e.g.,* either covalently or non-covalently) to the caged nucleotide. As used herein, the term "caged nucleotide" can refer to a nucleotide that is linked to a caged moiety or a nucleotide that was linked to a caged moiety but is no longer linked as a result of activation of the caged moiety.

In some embodiments, provided herein are methods for activating the caged moiety thereby releasing the caged moiety from the caged nucleotide. In some embodiments, activating the caged moiety includes photolysis of the caged moiety from the nucleotide. As used herein, "photolysis" can refer to the process of removing or separating a caged moiety from a caged nucleotide using light. In some embodiments, activating (*e.g.,* photolysis) the caged moiety includes exposing the caged moiety to light pulses (*e.g.,* two or more, three or more, four or more, or five or more pulses of light) that in total are sufficient to release the caged moiety from the caged nucleotide. In some embodiments, activating the caged moiety includes exposing the caged moiety to a light pulse (*e.g.,* a single light pulse) that is sufficient to release the caged moiety from the caged nucleotide. In some embodiments, activating the caged moiety includes exposing the caged moiety to a plurality of pulses (*e.g.,* one, or two or more pulses of light) where the light is at a wavelength of about less than about 360nm. In some embodiments, the source of the light that is at a wavelength of about less than 360nm is a UV light. The UV light can originate from a fluorescence microscope, a UV laser or a UV flashlamp, or any source of UV light known in the art.

In some embodiments, once the caged moiety is released from the capture binding domain, the oligonucleotide, probe oligonucleotide, or ligation product that includes the capture binding domain, is able to hybridize to the capture domain of the capture probe. Finally, to identify the location of the analyte or determine the interaction between two or more analyte-binding moieties, all or part of the sequence of the oligonucleotide, probe oligonucleotide, or ligation product, or a complement thereof, can be determined.

For more disclosure on embodiments in which the analyte capture sequence is blocked, see International Patent Application No PCT/US2020/059472 entitled "Enhancing Specificity of Analyte Binding," filed November 6, 2020, which is hereby incorporated by reference.

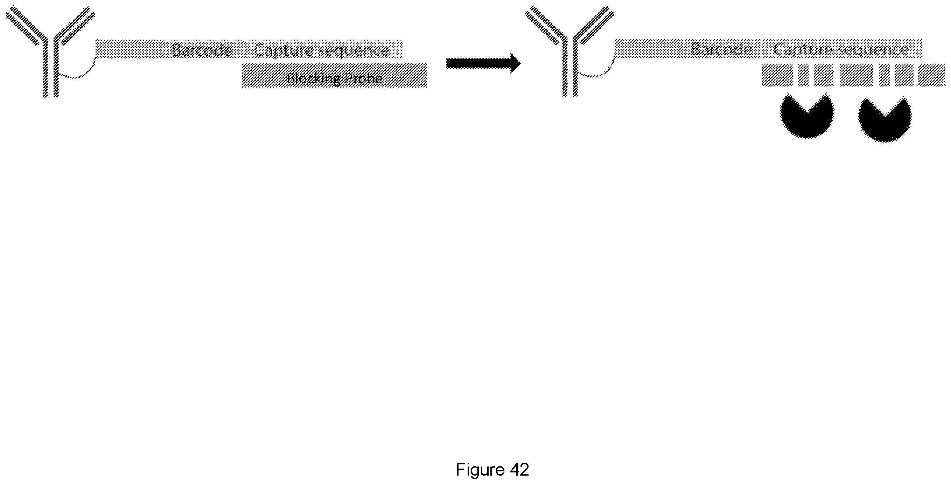

**FIG. 42** illustrates how blocking probes are added to the spatially-tagged analyte capture agent 4002 to prevent non-specific binding to capture domain on the array. In some embodiments, blocking oligonucleotides and antibodies are delivered to tissue where, after binding to tissue target, the blocking oligonucleotides can be subsequently removed (*e.g.*, digested by RNase). In the example illustrated in **FIG. 42**, cleavage of the linker between the oligonucleotide and antibody allows the oligonucleotide to migrate to the capture domain on the array. See Examples 3 and 4 below.

In some embodiments of any of the spatial profiling methods described herein, the methods are used to identify immune cell profiles. Immune cells express various adaptive immunological receptors relating to immune function, such as T cell receptors (TCRs) and B cell receptors (BCRs). T cell receptors and B cell receptors play a part in the immune response by specifically recognizing and binding to antigens and aiding in their destruction. More information on such applications of the disclosed methods is provided in PCT publication 202020176788A1 entitled "Profiling of biological analyes with spatially barcoded oligonucleotide arrays" the entire contents of each of which are incorporated herein by reference.

### (c) Substrate

For the spatial array-based analytical methods described in this section, the substrate (e.g., chip) functions as a support for direct or indirect attachment of capture probes to capture spots of the array. In addition, in some embodiments, a substrate (*e.g.,* the same substrate or a different substrate) is used to provide support to a biological sample, particularly, for example, a thin tissue section. Accordingly, a "substrate" is a support that is insoluble in aqueous liquid and that allows for positioning of biological samples, analytes, capture spots, and/or capture probes on the substrate.

A wide variety of different substrates can be used for the foregoing purposes. In general, a substrate can be any suitable support material. Exemplary substrates include, but are not limited to, glass, modified and/or functionalized glass, hydrogels, films, membranes, plastics (including *e.g.*, acrylics, polystyrene, copolymers of styrene and other materials, polypropylene, polyethylene, polybutylene, polyurethanes, Teflon™, cyclic olefins, polyimides, *etc.*), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

The substrate can also correspond to a flow cell. Flow cells can be formed of any of the foregoing materials, and can include channels that permit reagents, solvents, capture spots, and molecules to pass through the flow cell.

Among the examples of substrate materials discussed above, polystyrene is a hydrophobic material suitable for binding negatively charged macromolecules because it normally contains few hydrophilic groups. For nucleic acids immobilized on glass slides, by increasing the hydrophobicity of the glass surface the nucleic acid immobilization can be increased. Such an enhancement can permit a relatively more densely packed formation (*e.g.,* provide improved specificity and resolution).

In some embodiments, a substrate is coated with a surface treatment such as poly-L-lysine. Additionally or alternatively, the substrate can be treated by silanation, *e.g.,* with epoxy-silane, amino-silane, and/or by a treatment with polyacrylamide.

The substrate can generally have any suitable form or format. For example, the substrate can be flat, curved, *e.g.,* convexly or concavely curved towards the area where the interaction between a biological sample, *e.g.,* tissue sample, and the substrate takes place. In some embodiments, the substrate is a flat, *e.g.,* planar, chip or slide. The substrate can contain one or more patterned surfaces within the substrate (*e.g.,* channels, wells, projections, ridges, divots, etc.).

A substrate can be of any desired shape. For example, a substrate can be typically a thin, flat shape (*e.g.,* a square or a rectangle). In some embodiments, a substrate structure has rounded corners (*e.g.,* for increased safety or robustness). In some embodiments, a substrate structure has one or more cut-off corners (*e.g.,* for use with a slide clamp or cross-table). In some embodiments, where a substrate structure is flat, the substrate structure can be any appropriate type of support having a flat surface (*e.g.,* a chip or a slide such as a microscope slide).

Substrates can optionally include various structures such as, but not limited to, projections, ridges, and channels. A substrate can be micropatterned to limit lateral diffusion (*e.g.,* to prevent overlap of spatial barcodes). A substrate modified with such structures can be modified to allow association of analytes, capture spots (*e.g.,* beads), or probes at individual sites. For example, the sites where a substrate is modified with various structures can be contiguous or non-contiguous with other sites.

In some embodiments, the surface of a substrate can be modified so that discrete sites are formed that can only have or accommodate a single capture spot. In some embodiments, the surface of a substrate can be modified so that capture spots adhere to random sites.

In some embodiments, the surface of a substrate is modified to contain one or more wells, using techniques such as (but not limited to) stamping techniques, microetching techniques, and molding techniques. In some embodiments in which a substrate includes one or more wells, the substrate can be a concavity slide or cavity slide. For example, wells can be formed by one or more shallow depressions on the surface of the substrate. In some embodiments, where a substrate includes one or more wells, the wells can be formed by attaching a cassette (*e.g.,* a cassette containing one or more chambers) to a surface of the substrate structure.

In some embodiments, the structures of a substrate (*e.g.,* wells) can each bear a different capture probe. Different capture probes attached to each structure can be identified according to the locations of the structures in or on the surface of the substrate. Exemplary substrates include arrays in which separate structures are located on the substrate including, for example, those having wells that accommodate capture spots.

In some embodiments, a substrate includes one or more markings on a surface of the substrate, *e.g.,* to provide guidance for correlating spatial information with the characterization of the analyte of interest. For example, a substrate can be marked with a grid of lines (*e.g.,* to allow the size of objects seen under magnification to be easily estimated and/or to provide reference areas for counting objects). In some embodiments, fiducial markers can be included on the substrate. Such markings can be made using techniques including, but not limited to, printing, sandblasting, and depositing on the surface.

In some embodiments where the substrate is modified to contain one or more structures, including but not limited to wells, projections, ridges, or markings, the structures can include physically altered sites. For example, a substrate modified with various structures can include physical properties, including, but not limited to, physical configurations, magnetic or compressive forces, chemically functionalized sites, chemically altered sites, and/or electrostatically altered sites.

In some embodiments where the substrate is modified to contain various structures, including but not limited to wells, projections, ridges, or markings, the structures are applied in a pattern. Alternatively, the structures can be randomly distributed.

In some embodiments, a substrate is treated in order to minimize or reduce non-specific analyte hybridization within or between capture spots. For example, treatment can include coating the substrate with a hydrogel, film, and/or membrane that creates a physical barrier to non-specific hybridization. Any suitable hydrogel can be used. For example, hydrogel matrices prepared according to the methods set forth in U.S. Patent Nos. 6,391,937, 9,512,422, and 9,889,422, and U.S. Patent Application Publication Nos. U.S. 2017/0253918 and U.S. 2018/0052081, can be used. The entire contents of each of the foregoing documents are incorporated herein by reference.

Treatment can include adding a functional group that is reactive or capable of being activated such that it becomes reactive after receiving a stimulus (*e.g.,* photoreactive). Treatment can include treating with polymers having one or more physical properties (*e.g.,* mechanical, electrical, magnetic, and/or thermal) that minimize non-specific binding (*e.g.,* that activate a substrate at certain locations to allow analyte hybridization at those locations).

The substrate (*e.g.,* or a bead or a capture spot on an array) can include tens to hundreds of thousands or millions of individual oligonucleotide molecules (*e.g.,* at least about 10,000, 50,000, 100,000, 500,000, 1,000,000, 10,000,000, 100,000,000, 1,000,000,000 or 10,000,000,000 oligonucleotide molecules).

In some embodiments, the surface of the substrate is coated with a cell permissive coating to allow adherence of live cells. A "cell-permissive coating" is a coating that allows or helps cells to maintain cell viability (*e.g.,* remain viable) on the substrate. For example, a cell-permissive coating can enhance cell attachment, cell growth, and/or cell differentiation, *e.g.,* a cell-permissive coating can provide nutrients to the live cells. A cell-permissive coating can include a biological material and/or a synthetic material. Non-limiting examples of a cell-permissive coating include coatings that feature one or more extracellular matrix (ECM) components (*e.g.*, proteoglycans and fibrous proteins such as collagen, elastin, fibronectin and laminin), poly-lysine, poly-L-ornithine, and/or a biocompatible silicone (*e.g.*, CYTOSOFT®). For example, a cell-permissive coating that includes one or more extracellular matrix components can include collagen Type I, collagen Type II, collagen Type IV, elastin, fibronectin, laminin, and/or vitronectin. In some embodiments, the cell-permissive coating includes a solubilized basement membrane preparation extracted from the Engelbreth-Holm-Swarm (EHS) mouse sarcoma (*e.g.*, MATRIGEL®). In some embodiments, the cell-permissive coating includes collagen.

Where the substrate includes a gel (*e.g.*, a hydrogel or gel matrix), oligonucleotides within the gel can attach to the substrate. The terms "hydrogel" and "hydrogel matrix" are used interchangeably herein to refer to a macromolecular polymer gel including a network. Within the network, some polymer chains can optionally be cross-linked, although cross-linking does not always occur.

Further details and non-limiting embodiments relating to hydrogels and hydrogel subunits that can be used in the present disclosure are described in United States Patent Application No. 16/992,569 entitled "Systems and Methods for Using the Spatial Distribution of Haplotypes to Determine a Biological Condition," filed August 13, 2020, which is hereby incorporated herein by reference.

Further examples of substrates, including for example fiducial markers on such substrates, are disclosed in PCT publication 202020176788A1 entitled "Profiling of biological analyes with spatially barcoded oligonucleotide arrays" which is hereby incorporated by reference.

### (d) Arrays

In many of the methods disclosed herein, capture spots are collectively positioned on a substrate. An "array" is a specific arrangement of a plurality of capture spots (also termed "features") that is either irregular or forms a regular pattern. Individual capture spots in the array differ from one another based on their relative spatial locations. In general, at least two of the plurality of capture spots in the array include a distinct capture probe (*e.g.,* any of the examples of capture probes described herein).

Arrays can be used to measure large numbers of analytes simultaneously. In some embodiments, oligonucleotides are used, at least in part, to create an array. For example, one or more copies of a single species of oligonucleotide (*e.g.,* capture probe) can correspond to or be directly or indirectly attached to a given capture spot in the array. In some embodiments, a given capture spot in the array includes two or more species of oligonucleotides (*e.g.,* capture probes). In some embodiments, the two or more species of oligonucleotides (*e.g.,* capture probes) attached directly or indirectly to a given capture spot on the array include a common (*e.g.,* identical) spatial barcode.

As defined above, a "capture spot" is an entity that acts as a support or repository for various molecular entities used in sample analysis. Examples of capture spots include, but are not limited to, a bead, a spot of any twoor three-dimensional geometry (*e.g.,* an inkjet spot, a masked spot, a square on a grid), a well, and a hydrogel pad. In some embodiments, capture spots are directly or indirectly attached or fixed to a substrate (*e.g.,* of a chip). In some embodiments, the capture spots are not directly or indirectly attached or fixed to a substrate, but instead, for example, are disposed within an enclosed or partially enclosed three dimensional space (*e.g.,* wells or divots).

In some embodiments, capture spots are directly or indirectly attached or fixed to a substrate (*e.g.,* of a chip) that is liquid permeable. In some embodiments, capture spots are directly or indirectly attached or fixed to a substrate that is biocompatible. In some embodiments, capture spots are directly or indirectly attached or fixed to a substrate that is a hydrogel.

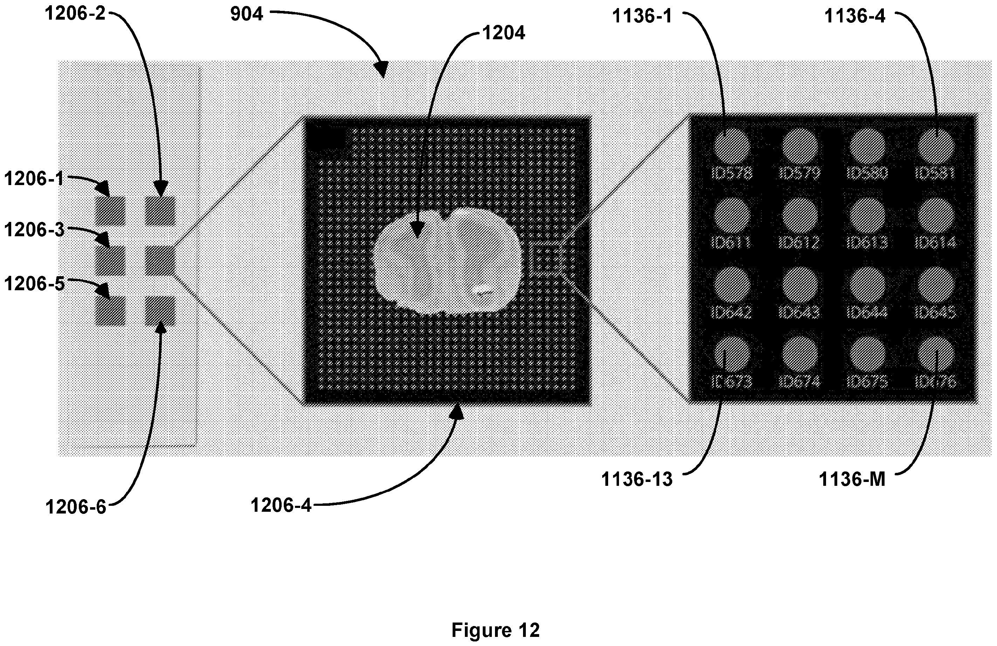

**FIG. 12** depicts an exemplary arrangement of barcoded capture spots within an array. From left to right, **FIG. 12** shows (L) a slide including six spatially-barcoded arrays, (C) An enlarged schematic of one of the six spatially-barcoded arrays, showing a grid of barcoded capture spots in relation to a biological sample, and (R) an enlarged schematic of one section of an array, showing the specific identification of multiple capture spots within the array (labelled as ID578, ID579, ID580, *etc.*).

As used herein, the term "bead array" refers to an array that includes a plurality of beads as the capture spots in the array. In some embodiments, the beads are attached to a substrate (*e.g.,* of a chip). For example, the beads can optionally attach to a substrate such as a microscope slide and in proximity to a biological sample (*e.g.,* a tissue section that includes cells). The beads can also be suspended in a solution and deposited on a surface (*e.g.,* a membrane, a tissue section, or a substrate (*e.g.,* a microscope slide)).

Examples of arrays of beads on or within a substrate include beads located in wells such as the BeadChip array (available from Illumina Inc., San Diego, CA), arrays used in sequencing platforms from 454 LifeSciences (a subsidiary of Roche, Basel, Switzerland), and array used in sequencing platforms from Ion Torrent (a subsidiary of Life Technologies, Carlsbad, CA). Examples of bead arrays are described in, *e.g.,* U.S. Patent Nos. 6,266,459; 6,355,431; 6,770,441; 6,859,570; 6,210,891; 6,258,568; and 6,274,320; U.S. Patent Application Publication Nos. 2009/0026082; 2009/0127589; 2010/0137143; and 2010/0282617; and PCT Patent Application Publication Nos. WO 00/063437 and WO 2016/162309, the entire contents of each of which is incorporated herein by reference.

### (i) Arrays for Analyte Capture

In some embodiments, some or all capture spots in an array include a capture probe. In some embodiments, an array can include a capture probe attached directly or indirectly to the substrate.

The capture probe includes a capture domain (*e.g.,* a nucleotide sequence) that can specifically bind (*e.g.,* hybridize) to a target analyte (*e.g.,* mRNA, DNA, or protein) within a sample. In some embodiments, the binding of the capture probe to the target (*e.g.,* hybridization) is detected and quantified by detection of a visual signal, *e.g.,* a fluorophore, a heavy metal (*e.g.,* silver ion), or chemiluminescent label, which has been incorporated into the target. In some embodiments, the intensity of the visual signal correlates with the relative abundance of each analyte in the biological sample. Since an array can contain thousands or millions of capture probes (or more), an array of capture spots with capture probes can interrogate many analytes in parallel.

In some embodiments, a substrate includes one or more capture probes that are designed to capture analytes from one or more organisms. In a non-limiting example, a substrate can contain one or more capture probes designed to capture mRNA from one organism (*e.g.,* a human) and one or more capture probes designed to capture DNA from a second organism (*e.g.,* a bacterium).

The capture probes can be attached to a substrate or capture spot using a variety of techniques. In some embodiments, the capture probe is directly attached to a capture spot that is fixed on an array. In some embodiments, the capture probes are immobilized to a substrate by chemical immobilization. For example, a chemical immobilization can take place between functional groups on the substrate and corresponding functional elements on the capture probes. Exemplary corresponding functional elements in the capture probes can either be an inherent chemical group of the capture probe, *e.g.,* a hydroxyl group, or a functional element can be introduced on to the capture probe. An example of a functional group on the substrate is an amine group. In some embodiments, the capture probe to be immobilized includes a functional amine group or is chemically modified in order to include a functional amine group. Means and methods for such a chemical modification are well known in the art.

In some embodiments, the capture probe is a nucleic acid. In some embodiments, the capture probe is immobilized on the capture spot or the substrate via its 5' end. In some embodiments, the capture probe is immobilized on a capture spot or a substrate via its 5' end and includes from the 5' to 3' end: one or more barcodes (*e.g.,* a spatial barcode and/or a UMI) and one or more capture domains. In some embodiments, the capture probe is immobilized on a capture spot via its 5' end and includes from the 5' to 3' end: one barcode (*e.g.,* a spatial barcode or a UMI) and one capture domain. In some embodiments, the capture probe is immobilized on a capture spot or a substrate via its 5' end and includes from the 5' to 3' end: a cleavage domain, a functional domain, one or more barcodes (*e.g.,* a spatial barcode and/or a UMI), and a capture domain.

In some embodiments, the capture probe is immobilized on a capture spot or a substrate via its 5' end and includes from the 5' to 3' end: a cleavage domain, a functional domain, one or more barcodes (*e.g.,* a spatial barcode and/or a UMI), a second functional domain, and a capture domain. In some embodiments, the capture probe is immobilized on a capture spot or a substrate via its 5' end and includes from the 5' to 3' end: a cleavage domain, a functional domain, a spatial barcode, a UMI, and a capture domain. In some embodiments, the capture probe is immobilized on a capture spot or a substrate via its 5' end and does not include a spatial barcode. In some embodiments, the capture probe is immobilized on a capture spot or a substrate via its 5' end and does not include a UMI. In some embodiments, the capture probe includes a sequence for initiating a sequencing reaction.

In some embodiments, the capture probe is immobilized on a capture spot or a substrate via its 3' end. In some embodiments, the capture probe is immobilized on a capture spot or a substrate via its 3' end and includes from the 3' to 5' end: one or more barcodes (*e.g.,* a spatial barcode and/or a UMI) and one or more capture domains. In some embodiments, the capture probe is immobilized on a capture spot or a substrate via its 3' end and includes from the 3' to 5' end: one barcode (*e.g.,* a spatial barcode or a UMI) and one capture domain. In some embodiments, the capture probe is immobilized on a capture spot or a substrate via its 3' end and includes from the 3' to 5' end: a cleavage domain, a functional domain, one or more barcodes (*e.g.,* a spatial barcode and/or a UMI), and a capture domain. In some embodiments, the capture probe is immobilized on a capture spot or a substrate via its 3' end and includes from the 3' to 5' end: a cleavage domain, a functional domain, a spatial barcode, a UMI, and a capture domain.

The localization of the functional group within the capture probe to be immobilized can be used to control and shape the binding behavior and/or orientation of the capture probe, *e.g.,* the functional group can be placed at the 5' or 3' end of the capture probe or within the sequence of the capture probe. In some embodiments, a capture probe can further include a support (*e.g.,* a support attached to the capture probe, a support attached to the capture spot, or a support attached to the substrate). A typical support for a capture probe to be immobilized includes moieties which are capable of binding to such capture probes, *e.g.,* to amine-functionalized nucleic acids. Examples of such supports are carboxy, aldehyde, or epoxy supports.

In some embodiments, the substrates on which capture probes can be immobilized can be chemically activated, *e.g.,* by the activation of functional groups, available on the substrate. The term "activated substrate" relates to a material in which interacting or reactive chemical functional groups are established or enabled by chemical modification procedures. For example, a substrate including carboxyl groups can be activated before use. Furthermore, certain substrates contain functional groups that can react with specific moieties already present in the capture probes.

In some embodiments, a covalent linkage is used to directly couple a capture probe to a substrate. In some embodiments a capture probe is indirectly coupled to a substrate through a linker separating the "first" nucleotide of the capture probe from the support, i.e., a chemical linker. In some embodiments, a capture probe does not bind directly to the array, but interacts indirectly, for example by binding to a molecule which itself binds directly or indirectly to the array. In some embodiments, the capture probe is indirectly attached to a substrate (*e.g.,* via a solution including a polymer).

In some embodiments, where the capture probe is immobilized on the capture spot of the array indirectly, *e.g.,* via hybridization to a surface probe capable of binding the capture probe, the capture probe can further include an upstream sequence (5' to the sequence that hybridizes to the nucleic acid, *e.g.,* RNA of the tissue sample) that is capable of hybridizing to 5' end of the surface probe. Alone, the capture domain of the capture probe can be seen as a capture domain oligonucleotide, which can be used in the synthesis of the capture probe in embodiments where the capture probe is immobilized on the array indirectly.

In some embodiments, a substrate is comprised of an inert material or matrix (*e.g.,* glass slides) that has been functionalized by, for example, treatment with a material comprising reactive groups which enable immobilization of capture probes. See, for example, WO 2017/019456, the entire contents of which are herein incorporated by reference. Non-limiting examples include polyacrylamide hydrogels supported on an inert substrate (*e.g.,* glass slide; see WO 2005/065814 and U.S. Patent Application No. 2008/0280773, the entire contents of which are incorporated herein by reference).

In some embodiments, functionalized biomolecules (*e.g.,* capture probes) are immobilized on a functionalized substrate using covalent methods. Methods for covalent attachment include, for example, condensation of amines and activated carboxylic esters (*e.g.,* N-hydroxysuccinimide esters); condensation of amine and aldehydes under reductive amination conditions; and cycloaddition reactions such as the Diels-Alder [4+2] reaction, 1,3-dipolar cycloaddition reactions, and [2+2] cycloaddition reactions. Methods for covalent attachment also include, for example, click chemistry reactions, including [3+2] cycloaddition reactions (*e.g.,* Huisgen 1,3-dipolar cycloaddition reaction and copper(I)-catalyzed azide-alkyne cycloaddition (CuAAC)); thiol-ene reactions; the Diels-Alder reaction and inverse electron demand Diels-Alder reaction; [4+1] cycloaddition of isonitriles and tetrazines; and nucleophilic ring-opening of small carbocycles (*e.g.,* epoxide opening with amino oligonucleotides). Methods for covalent attachment also include, for example, maleimides and thiols; and *para*-nitrophenyl ester-functionalized oligonucleotides and polylysine-functionalized substrate. Methods for covalent attachment also include, for example, disulfide reactions; radical reactions (see, *e.g.*, U.S. Patent No. 5,919,626, the entire contents of which are herein incorporated by reference); and hydrazide-functionalized substrate (*e.g.*, where the hydrazide functional group is directly or indirectly attached to the substrate) and aldehyde-functionalized oligonucleotides (see, *e.g.*, Yershov et al. (1996) Proc. Natl. Acad. Sci. USA 93, 4913-4918, the entire contents of which are herein incorporated by reference).

In some embodiments, functionalized biomolecules (*e.g.*, capture probes) are immobilized on a functionalized substrate using photochemical covalent methods. Methods for photochemical covalent attachment include, for example, immobilization of antraquinone-conjugated oligonucleotides (see, *e.g.*, Koch et al., 2000, Bioconjugate Chem. 11, 474-483, the entire contents of which is herein incorporated by reference).

In some embodiments, functionalized biomolecules (*e.g.*, capture probes are immobilized on a functionalized substrate using non-covalent methods. Methods for non-covalent attachment include, for example, biotin-functionalized oligonucleotides and streptavidin-treated substrates (see, *e.g.*, Holmstrøm et al. (1993) Analytical Biochemistry 209, 278-283 and Gilles et al. (1999) Nature Biotechnology 17, 365-370, the entire contents of which are herein incorporated by reference).

In some embodiments, an oligonucleotide (*e.g.*, a capture probe) can be attached to a substrate or capture spot according to the methods set forth in U.S. Patent Nos. 6,737,236, 7,259,258, 7,375,234, 7,427,678, 5,610,287, 5,807,522, 5,837,860, and 5,472,881; U.S. Patent Application Publication Nos. 2008/0280773 and 2011/0059865; Shalon et al. (1996) Genome Research, 639-645; Rogers et al. (1999) Analytical Biochemistry 266, 23-30; Stimpson et al. (1995) Proc. Natl. Acad. Sci. USA 92, 6379-6383; Beattie et al. (1995) Clin. Chem. 45, 700-706; Lamture et al. (1994) Nucleic Acids Research 22, 2121-2125; Beier et al. (1999) Nucleic Acids Research 27, 1970-1977; Joos et al. (1997) Analytical Biochemistry 247, 96-101; Nikiforov et al. (1995) Analytical Biochemistry 227, 201-209; Timofeev et al. (1996) Nucleic Acids Research 24, 3142-3148; Chrisey et al. (1996) Nucleic Acids Research 24, 3031-3039; Guo et al. (1994) Nucleic Acids Research 22, 5456-5465; Running and Urdea (1990) BioTechniques 8, 276-279; Fahy et al. (1993) Nucleic Acids Research 21, 1819-1826; Zhang *et al.* (1991) 19, 3929-3933; and Rogers et al. (1997) Gene Therapy 4, 1387-1392. The entire contents of each of the foregoing documents are incorporated herein by reference.

In some embodiments, the surface of a substrate is coated with a cell permissive coating to facilitate adherence of live cells. A "cell-permissive coating" is a coating that allows or helps cells to maintain cell viability (*e.g.*, remain viable) on the substrate. For example, a cell-permissive coating can enhance cell attachment, cell growth, and/or cell differentiation, *e.g.*, a cell-permissive coating can provide nutrients to the live cells. A cell-permissive coating can include a biological material and/or a synthetic material. Non-limiting examples of a cell-permissive coating include coatings that feature one or more extracellular matrix (ECM) components (*e.g.*, proteoglycans and fibrous proteins such as collagen, elastin, fibronectin and laminin), poly-lysine, poly-L-ornithine, and/or a biocompatible silicone (*e.g.*, CYTOSOFT®). For example, a cell-permissive coating that includes one or more extracellular matrix components can include collagen Type I, collagen Type II, collagen Type IV, elastin, fibronectin, laminin, and/or vitronectin. In some embodiments, the cell-permissive coating includes a solubilized basement membrane preparation extracted from the Engelbreth-Holm-Swarm (EHS) mouse sarcoma (*e.g.*, MATRIGEL®). In some embodiments, the cell-permissive coating includes collagen.

A "conditionally removable coating" is a coating that can be removed from the surface of a substrate upon application of a releasing agent. In some embodiments, a conditionally removable coating includes a hydrogel as described in further detail in U.S. Patent Application Number 16/992,569 entitled "Systems and Methods for Using the Spatial Distribution of Haplotypes to Determine a Biological Condition," filed August 13, 2020.

### (ii) Generation of Capture Probes in an Array Format

Arrays can be prepared by a variety of methods. In some embodiments, arrays are prepared through the synthesis (e.g., in-situ synthesis) of oligonucleotides on the array, or by jet printing or lithography. For example, light-directed synthesis of high-density DNA oligonucleotides can be achieved by photolithography or solid-phase DNA synthesis. To implement photolithographic synthesis, synthetic linkers modified with photochemical protecting groups can be attached to a substrate and the photochemical protecting groups can be modified using a photolithographic mask (applied to specific areas of the substrate) and light, thereby producing an array having localized photodeprotection. Many of these methods are known in the art, and are described *e.g.*, in Miller et al., 2009, "Basic concepts of microarrays and potential applications in clinical microbiology," Clinical Microbiology Reviews 22.4, 611-633; US201314111482A; US9593365B2; US2019203275; and WO2018091676, which are each incorporated herein by reference in the entirety.

### (1) Spotting or Printing

In some embodiments, the arrays are "spotted" or "printed" with oligonucleotides and these oligonucleotides (*e.g.,* capture probes) are then attached to the substrate. The oligonucleotides can be applied by either noncontact or contact printing. A noncontact printer can use the same method as computer printers (*e.g.,* bubble jet or inkjet) to expel small droplets of probe solution onto the substrate. The specialized inkjet-like printer can expel nanoliter to picoliter volume droplets of oligonucleotide solution, instead of ink, onto the substrate. In contact printing, each print pin directly applies the oligonucleotide solution onto a specific location on the surface. The oligonucleotides can be attached to the substrate surface by the electrostatic interaction of the negative charge of the phosphate backbone of the DNA with a positively charged coating of the substrate surface or by UV-cross-linked covalent bonds between the thymidine bases in the DNA and amine groups on the treated substrate surface. In some embodiments, the substrate is a glass slide. In some embodiments, the oligonucleotides (*e.g.,* capture probes) are attached to the substrate by a covalent bond to a chemical matrix, *e.g.,* epoxy-silane, amino-silane, lysine, polyacrylamide, etc.

### (2) In situ Synthesis

The arrays can also be prepared by *in situ* synthesis. In some embodiments, these arrays can be prepared using photolithography. Photolithography typically relies on UV masking and light-directed combinatorial chemical synthesis on a substrate to selectively synthesize probes directly on the surface of the array, one nucleotide at a time per spot, for many spots simultaneously. In some embodiments, a substrate contains covalent linker molecules that have a protecting group on the free end that can be removed by light. UV light is directed through a photolithographic mask to deprotect and activate selected sites with hydroxyl groups that initiate coupling with incoming protected nucleotides that attach to the activated sites. The mask is designed in such a way that the exposure sites can be selected, and thus specify the coordinates on the array where each nucleotide can be attached. The process can be repeated, a new mask is applied activating different sets of sites and coupling different bases, allowing arbitrary oligonucleotides to be constructed at each site. This process can be used to synthesize hundreds of thousands of different oligonucleotides. In some embodiments, maskless array synthesizer technology can be used. It uses an array of programmable micromirrors to create digital masks that reflect the desired pattern of UV light to deprotect the features.
In some embodiments, the inkjet spotting process can also be used for in-situ oligonucleotide synthesis. The different nucleotide precursors plus catalyst can be printed on the substrate, and are then combined with coupling and deprotection steps. This method relies on printing picoliter volumes of nucleotides on the array surface in repeated rounds of base-by-base printing that extends the length of the oligonucleotide probes on the array.

### (3) Electrical Fields

Arrays can also be prepared by active hybridization via electric fields to control nucleic acid transport. Negatively charged nucleic acids can be transported to specific sites, or capture spots, when a positive current is applied to one or more test sites on the array. The surface of the array can contain a binding molecule, *e.g.,* streptavidin, which allows for the formation of bonds (*e.g.,* streptavidin-biotin bonds) once electronically addressed biotinylated probes reach their targeted location. The positive current is then removed from the active capture spots, and new test sites can be activated by the targeted application of a positive current. The process are repeated until all sites on the array are covered.
An array for spatial analysis can be generated by various methods as described herein. In some embodiments, the array has a plurality of capture probes comprising spatial barcodes. These spatial barcodes and their relationship to the locations on the array can be determined. In some cases, such information is readily available, because the oligonucleotides are spotted, printed, or synthesized on the array with a pre-determined pattern. In some cases, the spatial barcode can be decoded by methods described herein, *e.g.,* by in-situ sequencing, by various labels associated with the spatial barcodes etc. In some embodiments, an array can be used a template to generate a daughter array. Thus, the spatial barcode can be transferred to the daughter array with a known pattern.

### (4) Ligation

In some embodiments, an array comprising barcoded probes can be generated through ligation of a plurality of oligonucleotides. In some instances, an oligonucleotide of the plurality contains a portion of a barcode, and the complete barcode is generated upon ligation of the plurality of oligonucleotides. For example, a first oligonucleotide containing a first portion of a barcode can be attached to a substrate (*e.g.,* using any of the methods of attaching an oligonucleotide to a substrate described herein), and a second oligonucleotide containing a second portion of the barcode can then be ligated onto the first oligonucleotide to generate a complete barcode. Different combinations of the first, second and any additional portions of a barcode can be used to increase the diversity of the barcodes. In instances where the second oligonucleotide is also attached to the substrate prior to ligation, the first and/or the second oligonucleotide can be attached to the substrate via a surface linker which contains a cleavage site. Upon ligation, the ligated oligonucleotide is linearized by cleaving at the cleavage site.

To increase the diversity of the barcodes, a plurality of second oligonucleotides comprising two or more different barcode sequences can be ligated onto a plurality of first oligonucleotides that comprise the same barcode sequence, thereby generating two or more different species of barcodes. To achieve selective ligation, a first oligonucleotide attached to a substrate containing a first portion of a barcode can initially be protected with a protective group (*e.g.,* a photocleavable protective group), and the protective group can be removed prior to ligation between the first and second oligonucleotide. In instances where the barcoded probes on an array are generated through ligation of two or more oligonucleotides, a concentration gradient of the oligonucleotides can be applied to a substrate such that different combinations of the oligonucleotides are incorporated into a barcoded probe depending on its location on the substrate.

Probes can be generated by directly ligating additional oligonucleotides onto existing oligonucleotides via a splint oligonucleotide. In some embodiments, oligonucleotides on an existing array can include a recognition sequence that can hybridize with a splint oligonucleotide. The recognition sequence can be at the free 5' end or the free 3' end of an oligonucleotide on the existing array. Recognition sequences useful for the methods of the present disclosure may not contain restriction enzyme recognition sites or secondary structures (*e.g.,* hairpins), and may include high contents of Guanine and Cytosine nucleotides.

### *(5) Polymerases*

Barcoded probes on an array can also be generated by adding single nucleotides to existing oligonucleotides on an array, for example, using polymerases that function in a template-independent manner. Single nucleotides can be added to existing oligonucleotides in a concentration gradient, thereby generating probes with varying length, depending on the location of the probes on the array.

### *(6) Modification of Existing Capture Probes*

Arrays can also be prepared by modifying existing arrays, for example, by modifying the oligonucleotides attached to the arrays. For instance, probes can be generated on an array that comprises oligonucleotides that are attached to the array at the 3' end and have a free 5' end. The oligonucleotides can be in situ synthesized oligonucleotides, and can include a barcode. The length of the oligonucleotides can be less than 50 nucleotides (nts) (*e.g.,* less than 45, 40, 35, 30, 25, 20, 15, or 10 nts). To generate probes using these oligonucleotides, a primer complementary to a portion of an oligonucleotide (*e.g.,* a constant sequence shared by the oligonucleotides) can be used to hybridize with the oligonucleotide and extend (using the oligonucleotide as a template) to form a duplex and to create a 3' overhang. The 3' overhang thus allows additional nucleotides or oligonucleotides to be added on to the duplex. A capture probe can be generated by, for instance, adding one or more oligonucleotides to the end of the 3' overhang (*e.g.,* via splint oligonucleotide mediated ligation), where the added oligonucleotides can include the sequence or a portion of the sequence of a capture domain

In some embodiments, arrays are prepared according to the methods set forth in WO 2012/140224, WO 2014/060483, WO 2016/162309, WO 2017/019456, WO 2018/091676, and WO 2012/140224, and U.S. Patent Application No. 2018/0245142. The entire contents of the foregoing documents are herein incorporated by reference

In some embodiments, a capture spot on the array includes a bead. In some embodiments, two or more beads are dispersed onto a substrate to create an array, where each bead is a capture spot on the array. Beads can optionally be dispersed into wells on a substrate, *e.g.,* such that only a single bead is accommodated per well.

Further details and non-limiting embodiments relating to beads, bead arrays, bead properties (*e.g.,* structure, materials, construction, cross-linking, degradation, reagents, and/or optical properties), and for covalently and non-covalently bonding beads to substrates are described in United States Patent Application Number 16/992,569, U.S. Patent Publication No. 20110059865A1, United States Provisional Patent Application Number 62/839,346, U.S. Patent No. 9,012,022, and PCT publication 202020176788A1 entitled "Profiling of biological analyes with spatially barcoded oligonucleotide arrays" each of which is incorporated herein by reference in its entirety.

### *(i) Capture spot sizes*

Capture spots on an array can be a variety of sizes. In some embodiments, a capture spot of an array has a diameter or maximum dimension between 1 $\mu$m to 100 $\mu$m. In some embodiments, a capture spot of an array has a diameter or maximum dimension of between 1 μm to 10 μm, 1 μm to 20 μm, 1 μm to 30 μm, 1 μm to 40 μm, 1 μm to 50 μm, 1 μm to 60 μm, 1 μm to 70 μm, 1 μm to 80 μm, 1 μm to 90 μm, 90 μm to 100 μm, 80 μm to 100 μm, 70 μm to 100 μm, 60 μm to 100 μm, 50 μm to 100 μm, 40 μm to 100 μm, 30 μm to 100 μm, 20 μm to 100 μm, or 10 μm to 100 μm. In some embodiments, the capture spot has a diameter or maximum dimension between 30 μm to 100 μm, 40 μm to 90 μm, 50 μm to 80 μm, 60 μm to 70 μm, or any range within the disclosed sub-ranges. In some embodiments, the capture spot has a diameter or maximum dimension no larger than 95 μm, 90 μm, 85 μm, 80 μm, 75 μm, 70 μm, 65 μm, 60 μm, 55 μm, 50 μm, 45 μm, 40 μm, 35 μm, 30 μm, 25 μm, 20 μm, 15 μm, 14 μm, 13 μm, 12 μm, 11 μm, 10 μm, 9 μm, 8 μm, 7 μm, 6 μm, 5 μm, 4 μm, 3 μm, 2 μm, or 1 μm. In some embodiments, the capture spot has a diameter or maximum dimension of approximately 65 μm.

In some embodiments, a plurality of capture spots has a mean diameter or mean maximum dimension between 1 μm to 100 μm. For example, between 1 μm to 10 μm, 1 μm to 20 μm, 1 μm to 30 μm, 1 μm to 40 μm, 1 μm to 50 μm, 1 μm to 60 μm, 1 μm to 70 μm, 1 μm to 80 μm, 1 μm to 90 μm, 90 μm to 100 μm, 80 μm to 100 μm, 70 μm to 100 μm, 60 μm to 100 μm, 50 μm to 100 μm, 40 μm to 100 μm, 30 μm to 100 μm, 20 μm to 100 μm, or 10 μm to 100 μm. In some embodiments, the plurality of capture spots has a mean diameter or mean maximum dimension between 30 μm to 100 μm, 40 μm to 90 μm, 50 μm to 80 μm, 60 μm to 70 μm, or any range within the disclosed sub-ranges. In some embodiments, the plurality of capture spots has a mean diameter or a mean maximum dimension no larger than 95 μm, 90 μm, 85 μm, 80 μm, 75 μm, 70 μm, 65 μm, 60 μm, 55 μm, 50 μm, 45 μm, 40 μm, 35 μm, 30 μm, 25 μm, 20 μm, 15 μm, 14 μm, 13 μm, 12 μm, 11 μm, 10 μm, 9 μm, 8 μm, 7 μm, 6 μm, 5 μm, 4 μm, 3 μm, 2 μm, or 1 μm. In some embodiments, the plurality of capture spots has a mean average diameter or a mean maximum dimension of approximately 65 μm.

In some embodiments, where the capture spot is a bead, the bead can have a diameter or maximum dimension no larger than 100 μm (*e.g.,* no larger than 95 μm, 90 μm, 85 μm, 80 μm, 75 μm, 70 μm, 65 μm, 60 μm, 55 μm, 50 μm, 45 μm, 40 μm, 35 μm, 30 μm, 25 μm, 20 μm, 15 μm, 14 μm, 13 μm, 12 μm, 11 μm, 10 μm, 9 μm, 8 μm, 7 μm, 6 μm, 5 μm, 4 μm, 3 μm, 2 μm, or 1 μm).

In some embodiments, a plurality of beads has an average diameter no larger than 100 μm. In some embodiments, a plurality of beads has an average diameter or maximum dimension no larger than 95 μm, 90 μm, 85 μm, 80 μm, 75 μm, 70 μm, 65 μm, 60 μm, 55 μm, 50 μm, 45 μm, 40 μm, 35 μm, 30 μm, 25 μm, 20 μm, 15 μm, 14 μm, 13 μm, 12 μm, 11 μm, 10 μm, 9 μm, 8 μm, 7 μm, 6 μm, 5 μm, 4 μm, 3 μm, 2 μm, or 1 μm.

In some embodiments, the volume of the bead can be at least about 1 $\mu m^3$, e.g., at least 1 $\mu m^3$, 2 $\mu m^3$, 3 $\mu m^3$, 4 $\mu m^3$, 5 $\mu m^3$, 6 $\mu m^3$, 7 $\mu m^3$, 8 $\mu m^3$, 9 $\mu m^3$, 10 $\mu m^3$, 12 $\mu m^3$, 14 $\mu m^3$, 16 $\mu m^3$, 18 $\mu m^3$, 20 $\mu m^3$, 25 $\mu m^3$, 30 $\mu m^3$, 35 $\mu m^3$, 40 $\mu m^3$, 45 $\mu m^3$, 50 $\mu m^3$, 55 $\mu m^3$, 60 $\mu m^3$, 65 $\mu m^3$, 70 $\mu m^3$, 75 $\mu m^3$, 80 $\mu m^3$, 85 $\mu m^3$, 90 $\mu m^3$, 95 $\mu m^3$, 100 $\mu m^3$, 125 $\mu m^3$, 150 $\mu m^3$, 175 $\mu m^3$, 200 $\mu m^3$, 250 $\mu m^3$, 300 $\mu m^3$, 350 $\mu m^3$, 400 $\mu m^3$, 450 $\mu m^3$, $\mu m^3$, 500 $\mu m^3$, 550 $\mu m^3$, 600 $\mu m^3$, 650 $\mu m^3$, 700 $\mu m^3$, 750 $\mu m^3$, 800 $\mu m^3$, 850 $\mu m^3$, 900 $\mu m^3$, 950 $\mu m^3$, 1000 $\mu m^3$, 1200 $\mu m^3$, 1400 $\mu m^3$, 1600 $\mu m^3$, 1800 $\mu m^3$, 2000 $\mu m^3$, 2200 $\mu m^3$, 2400 $\mu m^3$, 2600 $\mu m^3$, 2800 $\mu m^3$, 3000 $\mu m^3$, or greater.

In some embodiments, the bead can have a volume of between about 1 $\mu m^3$ and 100 $\mu m^3$, such as between about 1 $\mu m^3$ and 10 $\mu m^3$, between about 10 $\mu m^3$ and 50 $\mu m^3$, or between about 50 $\mu m^3$ and 100 $\mu m^3$. In some embodiments, the bead can include a volume of between about 100 $\mu m^3$ and 1000 $\mu m^3$, such as between about 100 $\mu m^3$ and 500 $\mu m^3$ or between about 500 $\mu m^3$ and 1000 $\mu m^3$. In some embodiments, the bead can include a volume between about 1000 $\mu m^3$ and 3000 $\mu m^3$, such as between about 1000 $\mu m^3$ and 2000 $\mu m^3$ or between about 2000 $\mu m^3$ and 3000 $\mu m^3$. In some embodiments, the bead can include a volume between about 1 $\mu m^3$ and 3000 $\mu m^3$, such as between about 1 $\mu m^3$ and 2000 $\mu m^3$, between about 1 $\mu m^3$ and 1000 $\mu m^3$, between about 1 $\mu m^3$ and 500 $\mu m^3$, or between about 1 $\mu m^3$ and 250 $\mu m^3$.

The capture spot can include one or more cross-sections that can be the same or different. In some embodiments, the capture spot can have a first cross-section that is different from a second cross-section. The capture spot can have a first cross-section that is at least about 0.0001 micrometer, 0.001 micrometer, 0.01 micrometer, 0.1 micrometer, or 1 micrometer. In some embodiments, the capture spot can include a cross-section (e.g., a first cross-section) of at least about 1 micrometer (μm), 2 μm, 3 μm, 4 μm, 5 μm, 6 μm, 7 μm, 8 μm, 9 μm, 10 μm, 11 μm, 12 μm, 13 μm, 14 μm, 15 μm, 16 μm, 17 μm, 18 μm, 19 μm, 20 μm, 25 μm, 30 μm, 35 μm, 40 μm, 45 μm, 50 μm, 55 μm, 60 μm, 65 μm, 70 μm, 75 μm, 80 μm, 85 μm, 90 μm, 100 μm, 120 μm, 140 μm, 160 μm, 180 μm, 200 μm, 250 μm, 300 μm, 350 μm, 400 μm, 450 μm, 500 μm, 550 μm, 600 μm, 650 μm, 700 μm, 750 μm, 800 μm, 850 μm, 900 μm, 950 μm, 1 millimeter (mm), or greater. In some embodiments, the capture spot can include a cross-section (e.g., a first cross-section) of between about 1 μm and 500 μm, such as between about 1 μm and 100 μm, between about 100 μm and 200 μm, between about 200 μm and 300 μm, between about 300 μm and 400 μm, or between about 400 μm and 500 μm. For example, the capture spot can include a cross-section (*e.g.,* a first cross-section) of between about 1 μm and 100 μm. In some embodiments, the capture spot can have a second cross-section that is at least about 1 μm. For example, the capture spot can include a second cross-section of at least about 1 micrometer (μm), 2 μm, 3 μm, 4 μm, 5 μm, 6 μm, 7 μm, 8 μm, 9 μm, 10 μm, 11 μm, 12 μm, 13 μm, 14 μm, 15 μm, 16 μm, 17 μm, 18 μm, 19 μm, 20 μm, 25 μm, 30 μm, 35 μm, 40 μm, 45 μm, 50 μm, 55 μm, 60 μm, 65 μm, 70 μm, 75 μm, 80 μm, 85 μm, 90 μm, 100 μm, 120 μm, 140 μm, 160 μm, 180 μm, 200 μm, 250 μm, 300 μm, 350 μm, 400 μm, 450 μm, 500 μm, 550 μm, 600 μm, 650 μm, 700 μm, 750 μm, 800 μm, 850 μm, 900 μm, 950 μm, 1 millimeter (mm), or greater.

In some embodiments, the capture spot can include a second cross-section of between about 1 μm and 500 μm, such as between about 1 μm and 100 μm, between about 100 μm and 200 μm, between about 200 μm and 300 μm, between about 300 μm and 400 μm, or between about 400 μm and 500 μm. For example, the capture spot can include a second cross-section of between about 1 μm and 100 μm.

In some embodiments, capture spots can be of a nanometer scale (*e.g.*, capture spots can have a diameter or maximum cross-sectional dimension of about 100 nanometers (nm) to about 900 nanometers (nm) (*e.g.*, 850 nm or less, 800 nm or less, 750 nm or less, 700 nm or less, 650 nm or less, 600 nm or less, 550 nm or less, 500 nm or less, 450 nm or less, 400 nm or less, 350 nm or less, 300 nm or less, 250 nm or less, 200 nm or less, 150 nm or less). A plurality of capture spots can have an average diameter or average maximum cross-sectional dimension of about 100 nanometers (nm) to about 900 nanometers (nm) (*e.g.*, 850 nm or less, 800 nm or less, 750 nm or less, 700 nm or less, 650 nm or less, 600 nm or less, 550 nm or less, 500 nm or less, 450 nm or less, 400 nm or less, 350 nm or less, 300 nm or less, 250 nm or less, 200 nm or less, 150 nm or less). In some embodiments, a capture spot has a diameter or size that is about the size of a single cell (*e.g.,* a single cell under evaluation).

Capture spots can be of uniform size or heterogeneous size. "Polydispersity" generally refers to heterogeneity of sizes of molecules or particles. The polydispersity (PDI) can be calculated using the equation PDI = Mw/Mn, where Mw is the weight-average molar mass and Mn is the number-average molar mass. In certain embodiments, capture spots can be provided as a population or plurality of capture spots having a relatively monodisperse size distribution. Where it can be desirable to provide relatively consistent amounts of reagents, maintaining relatively consistent capture spot characteristics, such as size, can contribute to the overall consistency.

In some embodiments, the beads provided herein can have size distributions that have a coefficient of variation in their cross-sectional dimensions of less than 50%, less than 40%, less than 30%, less than 20%, less than 15%, less than 10%, less than 5%, or lower. In some embodiments, a plurality of beads provided herein has a polydispersity index of less than 50%, less than 45%, less than 40%, less than 35%, less than 30%, less than 25%, less than 20%, less than 15%, less than 10%, less than 5%, or lower.

### (ii) Capture spot density

In some embodiments, an array (*e.g.*, two-dimensional array) comprises a plurality number of capture spots. In some embodiments, an array includes between 4000 and 10,000 capture spots, or any range within 4000 to 6000 capture spots. For example, an array includes between 4,000 to 4,400 capture spots, 4,000 to 4,800 capture spots, 4,000 to 5,200 capture spots, 4,000 to 5,600 capture spots, 5,600 to 6,000 capture spots, 5,200 to 6,000 capture spots, 4,800 to 6,000 capture spots, or 4,400 to 6,000 capture spots. In some embodiments, the array includes between 4,100 and 5,900 capture spots, between 4,200 and 5,800 capture spots, between 4,300 and 5,700 capture spots, between 4,400 and 5,600 capture spots, between 4,500 and 5,500 capture spots, between 4,600 and 5,400 capture spots, between 4,700 and 5,300 capture spots, between 4,800 and 5,200 capture spots, between 4,900 and 5,100 capture spots, or any range within the disclosed sub-ranges. For example, the array can include about 4,000 capture spots, about 4,200 capture spot, about 4,400 capture spots, about 4,800 capture spots, about 5,000 capture spots, about 5,200 capture spots, about 5,400 capture spots, about 5,600 capture spots, or about 6,000 capture spots. In some embodiments, the array comprises at least 4,000 capture spots. In some embodiments, the array includes approximately 5,000 capture spots.

In some embodiments, the capture spots of the array can be arranged in a pattern. In some embodiments, the center of a capture spot of an array is between 1 μm and 100 μm from the center of another capture spot of the array. For example, the center of a capture spot is between 20 μm to 40 μm, 20 μm to 60 μm, 20 μm to 80 μm, 80 μm to 100 μm, 60 μm to 100 μm, or 40 μm to 100 μm from the center of another capture spot of the array. In some embodiments, the center of a capture spot of an array is between 30 μm and 100 μm, 40 μm and 90 μm, 50 μm and 80 μm, 60 μm and 70 μm, or any range within the disclosed sub-ranges from the center of another capture spot of the array. In some embodiments, the center of a capture spot of an array is approximately 65 μm from the center of another capture spot of the array. In some embodiments, the center of a capture spot of an array is between 80 μm to 120 μm from the center of another capture spot of the array.

In some embodiments, a plurality of capture spots of an array are uniformly positioned. In some embodiments, a plurality of capture spots of an array are not uniformly positioned. In some embodiments, the positions of a plurality of capture spots of an array are predetermined. In some embodiments, the positioned of a plurality of capture spots of an array are not predetermined.

In some embodiments, the size and/or shape of a plurality of capture spots of an array are approximately uniform. In some embodiments, the size and/or shape of a plurality of capture spots of an array is substantially not uniform.

In some embodiments, an array is approximately 8 mm by 8 mm. In some embodiments, an array is smaller than 8 mm by 8 mm.

In some embodiments, the array can be a high density array. In some embodiments, the high density array can be arranged in a pattern. In some embodiments, the high-density pattern of the array is produced by compacting or compressing capture spots together in one or more dimensions. In some embodiments, a high-density pattern may be created by spot printing or other techniques described herein. In some embodiments, the center of a capture spots of the array is between 80 $\mu$m and 120 $\mu$m from the center of another capture spot of the array. In some embodiments, the center of a capture spot of the array is between 85 $\mu$m and 115 $\mu$m, between 90 $\mu$m and 110 $\mu$m, 95 $\mu$m and 105 $\mu$m, or any range within the disclosed sub-ranges from the center of another capture spot of the array. In some embodiments, the center of a capture spot of the array is approximately 100 $\mu$m from the center of another capture spot of the array.

### (iii) Array resolution

As used herein, a "low resolution" array (*e.g.,* a low resolution spatial array) refers to an array with capture spots having an average diameter of about 20 microns or greater. In some embodiments, substantially all (*e.g.,* 80% or more) of the capture probes within a single capture spot include the same barcode (*e.g.,* spatial barcode) such that upon deconvolution, resulting sequencing data from the detection of one or more analytes can be correlated with the spatial barcode of the capture spot, thereby identifying the location of the capture spot on the array, and thus determining the location of the one or more analytes in the biological sample.

A "high-resolution" array refers to an array with capture spots having an average diameter of about 1 micron to about 10 microns. This range in average diameter of capture spots corresponds to the approximate diameter of a single mammalian cell. Thus, a high-resolution spatial array is capable of detecting analytes at, or below, mammalian single-cell scale.

In some embodiments, resolution of an array can be improved by constructing an array with smaller capture spots. In some embodiments, resolution of an array can be improved by increasing the number of capture spots in the array. In some embodiments, the resolution of an array can be improved by packing capture spots closer together. For example, arrays including 5,000 capture spots were determined to provide higher resolution as compared to arrays including 1,000 capture spots (data not shown).

In some embodiments, the capture spots of the array may be arranged in a pattern, and in some cases, high-density pattern. In some embodiments, the high-density pattern of the array is produced by compacting or compressing capture spots together in one or more dimensions. In some embodiments, a high-density pattern may be created by spot printing or other techniques described herein. The number of median genes captures per cell and the median UMI counts per cell were higher when an array including 5,000 capture spots was used as compared to array including 1,000 capture spots (data not shown).

In some embodiments, an array includes a capture spot, where the capture spot incudes one or more capture probes (*e.g*., any of the capture probes described herein).

### (e) Analyte capture

In this section, general aspects of systems and methods for capturing analytes are described. Individual method steps and system features can be present in combination in many different embodiments; the specific combinations described herein do not in any way limit other combinations of steps and features.

Generally, analytes can be captured when contacting a biological sample with, *e.g.,* a substrate comprising capture probes (*e.g.,* substrate with capture probes embedded, spotted, printed on the substrate or a substrate with capture spots (*e.g.,* beads, wells) comprising capture probes).

As used herein, "contact," "contacted," and/ or "contacting," a biological sample with a substrate comprising capture spots refers to any contact (*e.g.,* direct or indirect) such that capture probes can interact (*e.g.,* capture) with analytes from the biological sample. For example, the substrate may be near or adjacent to the biological sample without direct physical contact, yet capable of capturing analytes from the biological sample. In some embodiments the biological sample is in direct physical contact with the substrate. In some embodiments, the biological sample is in indirect physical contact with the substrate. For example, a liquid layer may be between the biological sample and the substrate. In some embodiments, the analytes diffuse through the liquid layer. In some embodiments the capture probes diffuse through the liquid layer. In some embodiments reagents may be delivered via the liquid layer between the biological sample and the substrate. In some embodiments, indirect physical contact may be the presence of a second substrate (*e.g.,* a hydrogel, a film, a porous membrane) between the biological sample and the first substrate comprising capture spots with capture probes. In some embodiments, reagents are delivered by the second substrate to the biological sample.

### (i) Diffusion-resistant media / lids

To increase efficiency by encouraging analyte diffusion toward the spatially-labelled capture probes, a diffusion-resistant medium can be used. In general, molecular diffusion of biological analytes occurs in all directions, including toward the capture probes (i.e. toward the spatially-barcoded array), and away from the capture probes (i.e. into the bulk solution). Increasing diffusion toward the spatially-barcoded array reduces analyte diffusion away from the spatially-barcoded array and increases the capturing efficiency of the capture probes.

In some embodiments, a biological sample is placed on the top of a spatially-barcoded substrate and a diffusion-resistant medium is placed on top of the biological sample. For example, the diffusion-resistant medium can be placed onto an array that has been placed in contact with a biological sample. In some embodiments, the diffusion-resistant medium and spatially-labelled array are the same component. For example, the diffusion-resistant medium can contain spatially-labelled capture probes within or on the diffusion-resistant medium (*e.g.,* coverslip, slide, hydrogel, or membrane). In some embodiments, a sample is placed on a support and a diffusion-resistant medium is placed on top of the biological sample. Additionally, a spatially-barcoded capture probe array can be placed in close proximity over the diffusion-resistant medium. For example, a diffusion-resistant medium may be sandwiched between a spatially-labelled array and a sample on a support. In some embodiments, the diffusion-resistant medium is disposed or spotted onto the sample. In other embodiments, the diffusion-resistant medium is placed in close proximity to the sample.

In general, the diffusion-resistant medium can be any material known to limit diffusivity of biological analytes. For example, the diffusion-resistant medium can be a solid lid (*e.g.,* coverslip or glass slide). In some embodiments, the diffusion-resistant medium may be made of glass, silicon, paper, hydrogel polymer monoliths, or other material. In some embodiments, the glass side can be an acrylated glass slide. In some embodiments, the diffusion-resistant medium is a porous membrane. In some embodiments, the material may be naturally porous. In some embodiments, the material may have pores or wells etched into solid material. In some embodiments, the pore size can be manipulated to minimize loss of target analytes. In some embodiments, the membrane chemistry can be manipulated to minimize loss of target analytes. In some embodiments, the diffusion-resistant medium (i.e. hydrogel) is covalently attached to a solid support (i.e. glass slide). In some embodiments, the diffusion-resistant medium can be any material known to limit diffusivity of polyA transcripts. In some embodiments, the diffusion-resistant medium can be any material known to limit the diffusivity of proteins. In some embodiments, the diffusion-resistant medium can be any material know to limit the diffusivity of macromolecular constituents.

In some embodiments, a diffusion-resistant medium includes one or more diffusion-resistant media. For example, one or more diffusion-resistant media can be combined in a variety of ways prior to placing the media in contact with a biological sample including, without limitation, coating, layering, or spotting. As another example, a hydrogel can be placed onto a biological sample followed by placement of a lid (*e.g.,* glass slide) on top of the hydrogel.

In some embodiments, a force (*e.g.,* hydrodynamic pressure, ultrasonic vibration, solute contrasts, microwave radiation, vascular circulation, or other electrical, mechanical, magnetic, centrifugal, and/or thermal forces) is applied to control diffusion and enhance analyte capture. In some embodiments, one or more forces and one or more diffusion-resistant media are used to control diffusion and enhance capture. For example, a centrifugal force and a glass slide can used contemporaneously. Any of a variety of combinations of a force and a diffusion-resistant medium can be used to control or mitigate diffusion and enhance analyte capture.

In some embodiments, the diffusion-resistant medium, along with the spatially-barcoded array and sample, is submerged in a bulk solution In some embodiments, the bulk solution includes permeabilization reagents. In some embodiments, the diffusion-resistant medium includes at least one permeabilization reagent. In some embodiments, the diffusion-resistant medium (i.e. hydrogel) is soaked in permeabilization reagents before contacting the diffusion-resistant medium to the sample. In some embodiments, the diffusion-resistant medium can include wells (*e.g.,* micro-, nano-, or picowells) containing a permeabilization buffer or reagents. In some embodiments, the diffusion-resistant medium can include permeabilization reagents. In some embodiments, the diffusion-resistant medium can contain dried reagents or monomers to deliver permeabilization reagents when the diffusion-resistant medium is applied to a biological sample. In some embodiments, the diffusion-resistant medium is added to the spatially-barcoded array and sample assembly before the assembly is submerged in a bulk solution In some embodiments, the diffusion-resistant medium is added to the spatially-barcoded array and sample assembly after the sample has been exposed to permeabilization reagents. In some embodiments, the permeabilization reagents are flowed through a microfluidic chamber or channel over the diffusion-resistant medium. In some embodiments, the flow controls the sample's access to the permeabilization reagents. In some embodiments, the target analytes diffuse out of the sample and toward a bulk solution and get embedded in a spatially-labelled capture probeembedded diffusion-resistant medium.

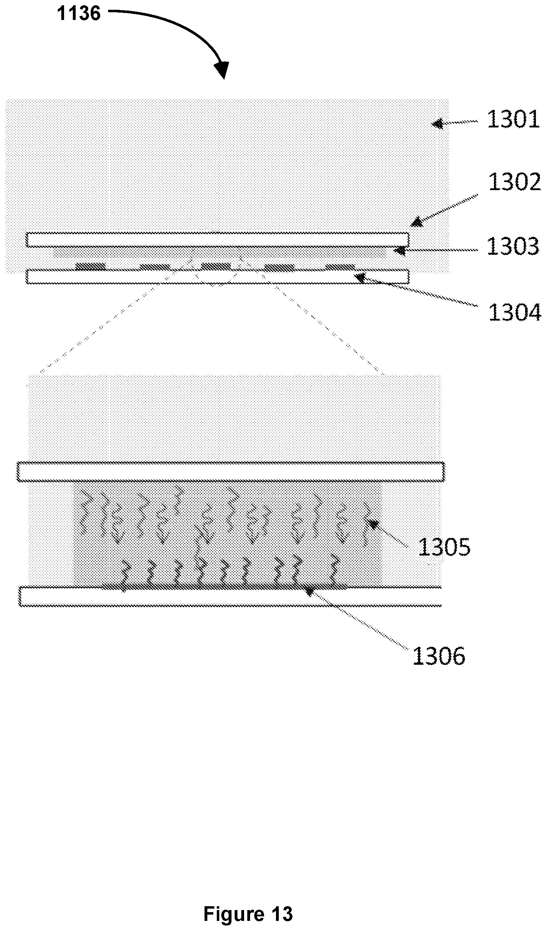

**FIG. 13** is an illustration of an exemplary use of a diffusion-resistant medium. A diffusion-resistant medium **1302** can be contacted with a sample **1303.** In **FIG 13,** a glass slide **1304** is populated with spatially-barcoded capture probes **1306,** and the sample **1303, 1305** is contacted with the array **1304, 1306.** A diffusion-resistant medium **1302** can be applied to the sample **1303,** where the sample **1303** is sandwiched between a diffusion-resistant medium **1302** and a capture probe coated slide **1304.** When a permeabilization solution **1301** is applied to the sample, using the diffusion-resistant medium/lid **1302** directs migration of the analytes **1305** toward the capture probes **1306** by reducing diffusion of the analytes out into the medium. Alternatively, the lid may contain permeabilization reagents.

### (ii) Conditions for capture

Capture probes on the substrate (or on a capture spot on the substrate) interact with released analytes through a capture domain, described elsewhere, to capture analytes. In some embodiments, certain steps are performed to enhance the transfer or capture of analytes by the capture probes of the array. Examples of such modifications include, but are not limited to, adjusting conditions for contacting the substrate with a biological sample (*e.g.,* time, temperature, orientation, pH levels, pre-treating of biological samples, etc.), using force to transport analytes (*e.g.,* electrophoretic, centrifugal, mechanical, etc.), performing amplification reactions to increase the amount of biological analytes *(e.g.,* PCR amplification, in situ amplification, clonal amplification), and/or using labeled probes for detecting of amplicons and barcodes.

In some embodiments, capture of analytes is facilitated by treating the biological sample with permeabilization reagents. If a biological sample is not permeabilized sufficiently, the amount of analyte captured on the substrate can be too low to enable adequate analysis. Conversely, if the biological sample is too permeable, the analyte can diffuse away from its origin in the biological sample, such that the relative spatial relationship of the analytes within the biological sample is lost. Hence, a balance between permeabilizing the biological sample enough to obtain good signal intensity while still maintaining the spatial resolution of the analyte distribution in the biological sample is desired. Methods of preparing biological samples to facilitation are known in the art and can be modified depending on the biological sample and how the biological sample is prepared (*e.g.,* fresh frozen, FFPE, etc).

### (iii) Passive capture methods

In some embodiments, analytes are migrated from a sample to a substrate. Methods for facilitating migration can be passive (*e.g.,* diffusion) and/or active (*e.g.,* electrophoretic migration of nucleic acids). Non-limiting examples of passive migration can include simple diffusion and osmotic pressure created by the rehydration of dehydrated objects.

Passive migration by diffusion uses concentration gradients. Diffusion is movement of untethered objects toward equilibrium. Therefore, when there is a region of high object concentration and a region of low object concentration, the object (capture probe, the analyte, etc.) moves to an area of lower concentration. In some embodiments, untethered analytes move down a concentration gradient.

In some embodiments, different reagents are added to the biological sample, such that the biological sample is rehydrated while improving capture of analytes. In some embodiments, the biological sample is rehydrated with permeabilization reagents. In some embodiments, the biological sample is rehydrated with a staining solution (*e.g.,* hematoxylin and eosin stain).

### (iv) Active capture methods

In some examples of any of the methods described herein, an analyte in a cell or a biological sample can be transported (*e.g.,* passively or actively) to a capture probe (*e.g.*, a capture probe affixed to a solid surface).

For example, analytes in a cell or a biological sample can be transported to a capture probe (*e.g.,* an immobilized capture probe) using an electric field (*e.g.,* using electrophoresis), a pressure gradient, fluid flow, a chemical concentration gradient, a temperature gradient, and/or a magnetic field. For example, analytes can be transported through, *e.g.,* a gel (*e.g.,* hydrogel matrix), a fluid, or a permeabilized cell, to a capture probe (*e.g.,* an immobilized capture probe).

In some examples, an electrophoretic field can be applied to analytes to facilitate migration of the analytes towards a capture probe. In some examples, a sample contacts a substrate and capture probes fixed on a substrate (*e.g.,* a slide, cover slip, or bead), and an electric current is applied to promote the directional migration of charged analytes towards the capture probes fixed on the substrate. An electrophoresis assembly, where a cell or a biological sample is in contact with a cathode and capture probes (*e.g.,* capture probes fixed on a substrate), and where the capture probes (*e.g.,* capture probes fixed on a substrate) is in contact with the cell or biological sample and an anode, can be used to apply the current.

Electrophoretic transfer of analytes can be performed while retaining the relative spatial alignment of the analytes in the sample. As such, an analyte captured by the capture probes (*e.g.,* capture probes fixed on a substrate) retains the spatial information of the cell or the biological sample.

In some examples, a spatially-addressable microelectrode array is used for spatially-constrained capture of at least one charged analyte of interest by a capture probe. The microelectrode array can be configured to include a high density of discrete sites having a small area for applying an electric field to promote the migration of charged analyte(s) of interest. For example, electrophoretic capture can be performed on a region of interest using a spatially-addressable microelectrode array.

### (v) Region of interest

A biological sample can have regions that show morphological feature(s) that may indicate the presence of disease or the development of a disease phenotype. For example, morphological features at a specific site within a tumor biopsy sample can indicate the aggressiveness, therapeutic resistance, metastatic potential, migration, stage, diagnosis, and/or prognosis of cancer in a subject. A change in the morphological features at a specific site within a tumor biopsy sample often correlate with a change in the level or expression of an analyte in a cell within the specific site, which can, in turn, be used to provide information regarding the aggressiveness, therapeutic resistance, metastatic potential, migration, stage, diagnosis, and/or prognosis of cancer in a subject. A region or area within a biological sample that is selected for specific analysis (*e.g.,* a region in a biological sample that has morphological features of interest) is often described as "a region of interest."

A region of interest in a biological sample can be used to analyze a specific area of interest within a biological sample, and thereby, focus experimentation and data gathering to a specific region of a biological sample (rather than an entire biological sample). This results in increased time efficiency of the analysis of a biological sample.

A region of interest can be identified in a biological sample using a variety of different techniques, *e.g.,* expansion microscopy, bright field microscopy, dark field microscopy, phase contrast microscopy, electron microscopy, fluorescence microscopy, reflection microscopy, interference microscopy, and confocal microscopy, and combinations thereof. For example, the staining and imaging of a biological sample can be performed to identify a region of interest. In some examples, the region of interest can correspond to a specific structure of cytoarchitecture. In some embodiments, a biological sample can be stained prior to visualization to provide contrast between the different regions of the biological sample. The type of stain can be chosen depending on the type of biological sample and the region of the cells to be stained. In some embodiments, more than one stain can be used to visualize different aspects of the biological sample, *e.g.,* different regions of the sample, specific cell structures (*e.g.,* organelles), or different cell types. In other embodiments, the biological sample can be visualized or imaged without staining the biological sample.

In some embodiments, imaging can be performed using one or more fiducial markers, i.e., objects placed in the field of view of an imaging system that appear in the image produced. Fiducial markers are typically used as a point of reference or measurement scale. Fiducial markers can include, but are not limited to, detectable labels such as fluorescent, radioactive, chemiluminescent, calorimetric, and colorimetric labels. The use of fiducial markers to stabilize and orient biological samples is described, for example, in Carter et al., Applied Optics 46:421-427, 2007), the entire contents of which are incorporated herein by reference.

In some embodiments, a fiducial marker can be present on a substrate to provide orientation of the biological sample. In some embodiments, a microsphere can be coupled to a substrate to aid in orientation of the biological sample. In some examples, a microsphere coupled to a substrate can produce an optical signal (*e.g.,* fluorescence). In another example, a microsphere can be attached to a portion (*e.g.,* corner) of an array in a specific pattern or design (*e.g.,* hexagonal design) to aid in orientation of a biological sample on an array of capture spots on the substrate. In some embodiments, a fiducial marker can be an immobilized molecule with which a detectable signal molecule can interact to generate a signal. For example, a marker nucleic acid can be linked or coupled to a chemical moiety capable of fluorescing when subjected to light of a specific wavelength (or range of wavelengths). Such a marker nucleic acid molecule can be contacted with an array before, contemporaneously with, or after the tissue sample is stained to visualize or image the tissue section. Although not required, it can be advantageous to use a marker that can be detected using the same conditions (*e.g.,* imaging conditions) used to detect a labelled cDNA.

In some embodiments, fiducial markers are included to facilitate the orientation of a tissue sample or an image thereof in relation to an immobilized capture probes on a substrate. Any number of methods for marking an array can be used such that a marker is detectable only when a tissue section is imaged. For instance, a molecule, *e.g.,* a fluorescent molecule that generates a signal, can be immobilized directly or indirectly on the surface of a substrate. Markers can be provided on a substrate in a pattern (*e.g.,* an edge, one or more rows, one or more lines, etc.).

In some embodiments, a fiducial marker can be randomly placed in the field of view. For example, an oligonucleotide containing a fluorophore can be randomly printed, stamped, synthesized, or attached to a substrate (*e.g.,* a glass slide) at a random position on the substrate. A tissue section can be contacted with the substrate such that the oligonucleotide containing the fluorophore contacts, or is in proximity to, a cell from the tissue section or a component of the cell (*e.g.,* an mRNA or DNA molecule). An image of the substrate and the tissue section can be obtained, and the position of the fluorophore within the tissue section image can be determined (*e.g.,* by reviewing an optical image of the tissue section overlaid with the fluorophore detection). In some embodiments, fiducial markers can be precisely placed in the field of view (*e.g.,* at known locations on a substrate). In this instance, a fiducial marker can be stamped, attached, or synthesized on the substrate and contacted with a biological sample. Typically, an image of the sample and the fiducial marker is taken, and the position of the fiducial marker on the substrate can be confirmed by viewing the image.

In some examples, fiducial markers can surround the array. In some embodiments the fiducial markers allow for detection of, *e.g.,* mirroring. In some embodiments, the fiducial markers may completely surround the array. In some embodiments, the fiducial markers may not completely surround the array. In some embodiments, the fiducial markers identify the corners of the array. In some embodiments, one or more fiducial markers identify the center of the array. In some embodiments, the fiducial markers comprise patterned spots, where the diameter of one or more patterned spot fiducial markers is approximately 100 micrometers. The diameter of the fiducial markers can be any useful diameter including, but not limited to, 50 micrometers to 500 micrometers in diameter. The fiducial markers may be arranged in such a way that the center of one fiducial marker is between 100 micrometers and 200 micrometers from the center of one or more other fiducial markers surrounding the array. In some embodiments, the array with the surrounding fiducial markers is approximately 8 mm by 8 mm. In some embodiments, the array without the surrounding fiducial markers is smaller than 8 mm by 8 mm.

In some embodiments, staining and imaging a biological sample prior to contacting the biological sample with a spatial array is performed to select samples for spatial analysis. In some embodiments, the staining includes applying a fiducial marker as described above, including fluorescent, radioactive, chemiluminescent, calorimetric, or colorimetric detectable markers. In some embodiments, the staining and imaging of biological samples allows the user to identify the specific sample (or region of interest) the user wishes to assess.

In some embodiments, a lookup table (LUT) can be used to associate one property with another property of a capture spot. These properties include, *e.g.,* locations, barcodes (*e.g.,* nucleic acid barcode molecules), spatial barcodes, optical labels, molecular tags, and other properties.

In some embodiments, a lookup table can associate a nucleic acid barcode molecule with a capture spot. In some embodiments, an optical label of a capture spot can permit associating the capture spot with a biological particle (*e.g.,* cell or nuclei). The association of a capture spot with a biological particle can further permit associating a nucleic acid sequence of a nucleic acid molecule of the biological particle to one or more physical properties of the biological particle (*e.g.,* a type of a cell or a location of the cell). For example, based on the relationship between the barcode and the optical label, the optical label can be used to determine the location of a capture spot, thus associating the location of the capture spot with the barcode sequence of the capture spot. Subsequent analysis (*e.g.,* sequencing) can associate the barcode sequence and the analyte from the sample. Accordingly, based on the relationship between the location and the barcode sequence, the location of the biological analyte can be determined (*e.g.,* in a specific type of cell or in a cell at a specific location of the biological sample).

In some embodiments, a capture spot can have a plurality of nucleic acid barcode molecules attached thereto. The plurality of nucleic acid barcode molecules can include barcode sequences. The plurality of nucleic acid molecules attached to a given capture spot can have the same barcode sequences, or two or more different barcode sequences. Different barcode sequences can be used to provide improved spatial location accuracy.

In some embodiments, a substrate is treated in order to minimize or reduce non-specific analyte hybridization within or between capture spots. For example, treatment can include coating the substrate with a hydrogel, film, and/or membrane that creates a physical barrier to non-specific hybridization. Any suitable hydrogel can be used. For example, hydrogel matrices prepared according to the methods set forth in U.S. Patent Nos. 6,391,937, 9,512,422, and 9,889,422, and U.S. Patent Publication Nos. U.S. 2017/0253918 and U.S. 2018/0052081, can be used. The entire contents of each of the foregoing documents are incorporated herein by reference.

Treatment can include adding a functional group that is reactive or capable of being activated such that it becomes reactive after receiving a stimulus (*e.g.,* photoreactive). Treatment can include treating with polymers having one or more physical properties (*e.g.,* mechanical, electrical, magnetic, and/or thermal) that minimize non-specific binding (*e.g.,* that activate a substrate at certain locations to allow analyte hybridization at those locations).

In some examples, an array (*e.g.,* any of the exemplary arrays described herein) can be contained with only a portion of a biological sample (*e.g.,* a cell, a feature, or a region of interest). In some examples, a biological sample is contacted with only a portion of an array (*e.g.,* any of the exemplary arrays described herein). In some examples, a portion of the array can be deactivated such that it does not interact with the analytes in the biological sample (*e.g.,* optical deactivation, chemical deactivation, heat deactivation, or blocking of the capture probes in the array (*e.g.,* using blocking probes)). In some examples, a region of interest can be removed from a biological sample and then the region of interest can be contacted to the array (*e.g.,* any of the arrays described herein). A region of interest can be removed from a biological sample using microsurgery, laser capture microdissection, chunking, a microtome, dicing, trypsinization, labelling, and/or fluorescence-assisted cell sorting.

### (f) Analysis of captured analytes

In some embodiments, after contacting a biological sample with a substrate that includes capture probes, a removal step can optionally be performed to remove all or a portion of the biological sample from the substrate. In some embodiments, the removal step includes enzymatic and/or chemical degradation of cells of the biological sample. For example, the removal step can include treating the biological sample with an enzyme (*e.g.*, a proteinase, *e.g.*, proteinase K) to remove at least a portion of the biological sample from the substrate. In some embodiments, the removal step can include ablation of the tissue (*e.g.*, laser ablation).

In some embodiments, a method for spatially detecting an analyte (*e.g.*, detecting the location of an analyte, *e.g.*, a biological analyte) from a biological sample (*e.g.*, present in a biological sample) comprises: (a) optionally staining and/or imaging a biological sample on a substrate; (b) permeabilizing (*e.g.*, providing a solution comprising a permeabilization reagent to) the biological sample on the substrate; (c) contacting the biological sample with an array comprising a plurality of capture probes, where a capture probe of the plurality captures the biological analyte; and (d) analyzing the captured biological analyte, thereby spatially detecting the biological analyte; where the biological sample is fully or partially removed from the substrate.

In some embodiments, a biological sample is not removed from the substrate. For example, the biological sample is not removed from the substrate prior to releasing a capture probe (*e.g.*, a capture probe bound to an analyte) from the substrate. In some embodiments, such releasing comprises cleavage of the capture probe from the substrate (*e.g.*, via a cleavage domain). In some embodiments, such releasing does not comprise releasing the capture probe from the substrate (*e.g.*, a copy of the capture probe bound to an analyte can be made and the copy can be released from the substrate, *e.g.*, via denaturation). In some embodiments, the biological sample is not removed from the substrate prior to analysis of an analyte bound to a capture probe after it is released from the substrate. In some embodiments, the biological sample remains on the substrate during removal of a capture probe from the substrate and/or analysis of an analyte bound to the capture probe after it is released from the substrate. In some embodiments, analysis of an analyte bound to capture probe from the substrate can be performed without subjecting the biological sample to enzymatic and/or chemical degradation of the cells (*e.g.*, permeabilized cells) or ablation of the tissue (*e.g.*, laser ablation).

In some embodiments, at least a portion of the biological sample is not removed from the substrate. For example, a portion of the biological sample can remain on the substrate prior to releasing a capture probe (*e.g.*, a capture prove bound to an analyte) from the substrate and/or analyzing an analyte bound to a capture probe released from the substrate. In some embodiments, at least a portion of the biological sample is not subjected to enzymatic and/or chemical degradation of the cells (*e.g.*, permeabilized cells) or ablation of the tissue (*e.g.*, laser ablation) prior to analysis of an analyte bound to a capture probe from the support.

In some embodiments, a method for spatially detecting an analyte (*e.g.*, detecting the location of an analyte, *e.g.*, a biological analyte) from a biological sample (*e.g.*, present in a biological sample) comprises: (a) optionally staining and/or imaging a biological sample on a substrate; (b) permeabilizing (*e.g.*, providing a solution comprising a permeabilization reagent to) the biological sample on the substrate; (c) contacting the biological sample with an array comprising a plurality of capture probes, where a capture probe of the plurality captures the biological analyte; and (d) analyzing the captured biological analyte, thereby spatially detecting the biological analyte; where the biological sample is not removed from the substrate.

In some embodiments, a method for spatially detecting a biological analyte of interest from a biological sample comprises: (a) staining and imaging a biological sample on a support; (b) providing a solution comprising a permeabilization reagent to the biological sample on the support; (c) contacting the biological sample with an array on a substrate, where the array comprises one or more capture probe pluralities thereby allowing the one or more pluralities of capture probes to capture the biological analyte of interest; and (d) analyzing the captured biological analyte, thereby spatially detecting the biological analyte of interest; where the biological sample is not removed from the support.

In some embodiments, the method further includes selecting a region of interest in the biological sample to subject to spatial transcriptomic analysis. In some embodiments, one or more of the one or more capture probes include a capture domain. In some embodiments, one or more of the one or more capture probe pluralities comprise a unique molecular identifier (UMI). In some embodiments, one or more of the one or more capture probe pluralities comprise a cleavage domain. In some embodiments, the cleavage domain comprises a sequence recognized and cleaved by a uracil-DNA glycosylase, apurinic/apyrimidinic (AP) endonuclease (APE1), U uracil-specific excision reagent (USER), and/or an endonuclease VIII. In some embodiments, one or more capture probes do not comprise a cleavage domain and is not cleaved from the array.

After analytes from the sample have hybridized or otherwise been associated with capture probes, analyte capture agents, or other barcoded oligonucleotide sequences according to any of the methods described above in connection with the general spatial cell-based analytical methodology, the barcoded constructs that result from hybridization/association are analyzed via sequencing to identify the analytes.

In some embodiments, the methods described herein can be used to assess analyte levels and/or expression in a cell or a biological sample over time (*e.g.*, before or after treatment with an agent or different stages of differentiation). In some examples, the methods described herein can be performed on multiple similar biological samples or cells obtained from the subject at a different time points (*e.g.*, before or after treatment with an agent, different stages of differentiation, different stages of disease progression, different ages of the subject, or before or after development of resistance to an agent).

Further details and non-limiting embodiments relating to removal of sample from the array, release and amplification of analytes, analysis of captured analytes (*e.g.* by sequencing and/or multiplexing), and spatial resolution of analyte information (*e.g.,* using lookup tables) are described in United States Patent Application Number 16/992,569 entitled "Systems and Methods for Using the Spatial Distribution of Haplotypes to Determine a Biological Condition," filed August 13, 2019, which is hereby incorporated herein by reference.

### III. Specific embodiments

This disclosure also provides methods and systems for spatial nucleic acid and/or protein analysis. Provided below are detailed descriptions and explanations of various embodiments of the present disclosure. These embodiments are non-limiting and do not preclude any alternatives, variations, changes, and substitutions that can occur to those skilled in the art from the scope of this disclosure.

### (a) Systems for spatial analyte analyses

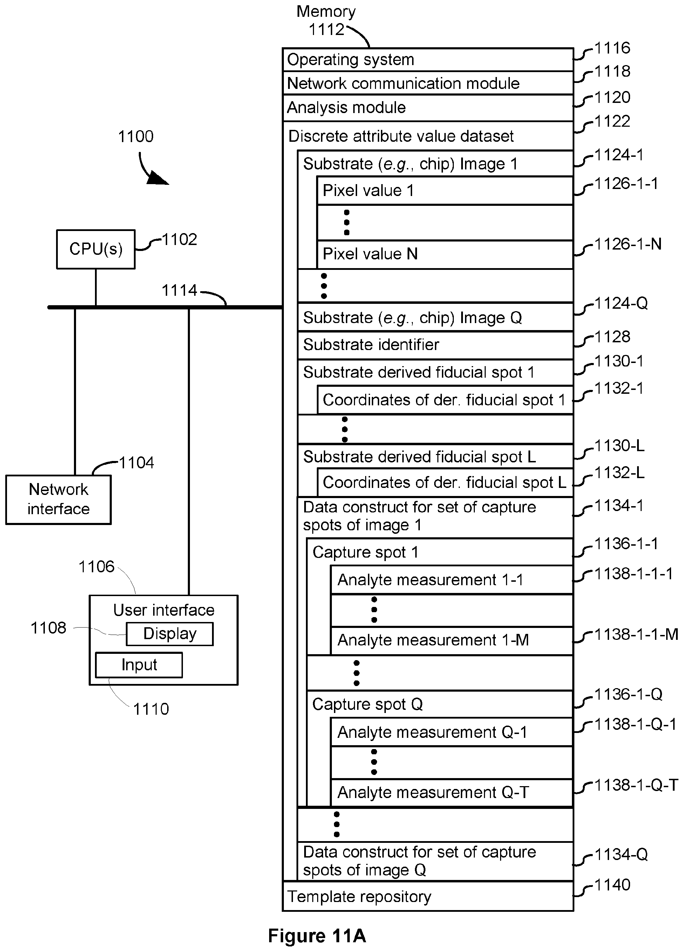

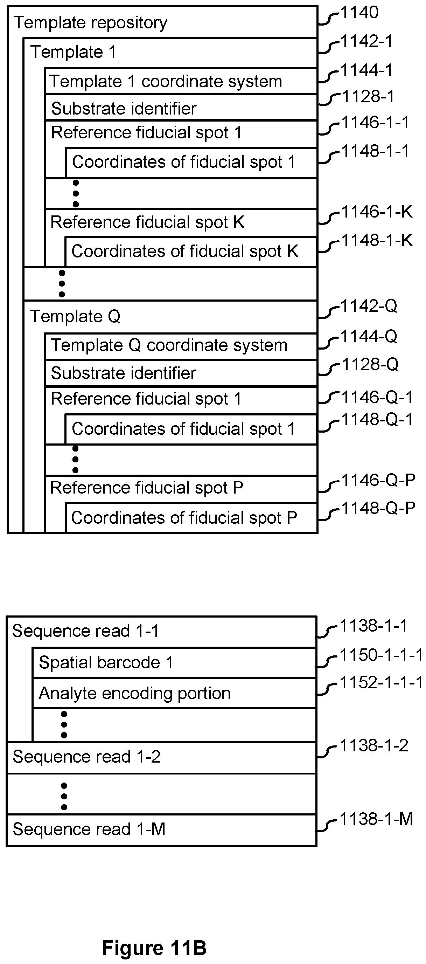

**FIG. 11** is a block diagram illustrating an exemplary, non-limiting system for spatial analysis in accordance with some implementations. The system **1100** in some implementations includes one or more processing units CPU(s) **1102** (also referred to as processors), one or more network interfaces **1104,** a user interface 1106, a memory **1112,** and one or more communication buses **1114** for interconnecting these components. The communication buses **1114** optionally include circuitry (sometimes called a chipset) that interconnects and controls communications between system components. The memory 1112 typically includes high-speed random access memory, such as DRAM, SRAM, DDR RAM, ROM, EEPROM, flash memory, CD-ROM, digital versatile disks (DVD) or other optical storage, magnetic cassettes, magnetic tape, magnetic disk storage or other magnetic storage devices, other random access solid state memory devices, or any other medium which can be used to store desired information; and optionally includes non-volatile memory, such as one or more magnetic disk storage devices, optical disk storage devices, flash memory devices, or other non-volatile solid state storage devices. The memory **1112** optionally includes one or more storage devices remotely located from the CPU(s) **1102.** The memory **1112,** or alternatively the non-volatile memory device(s) within the memory **1112,** comprises a non-transitory computer readable storage medium. It will be appreciated that this memory 1112 can be distributed across one or more computers. In some implementations, the memory **1112** or alternatively the non-transitory computer readable storage medium stores the following programs, modules and data structures, or a subset thereof:

an optional operating system **1116,** which includes procedures for handling various basic system services and for performing hardware dependent tasks;

an optional network communication module (or instructions) **1118** for connecting the device **1100** with other devices, or a communication network;

an analysis module **1120** for spatial analyte (e.g., nucleic acid) analysis;

a discrete attribute dataset **1122** comprising (i) one or more subsrate images **1124,** each respective substrate image comprising a plurality of pixel values **1126** (*e.g.*, **1126-1-1,** ..., **1126-1-N,** where N is a positive integer) and (ii) a substrate identifier **1128;**

a plurality of derived fiducial spots **1130** (*e.g.,* **1130-1,** ..., **1130-L,** where L is a positive integer), and corresponding coordinates **1132** (*e.g.* **1132-1,** ..., **1132-L)** identified in the substrate image **1124;**

a respective data construct **1134** for each respective substrate image **1124,** for a set of capture spots in the substrate, the respective data construct comprising, for each capture spot **1136** (*e.g.*, **1136-1-1,** ..., **1136-1-Q),** analyte measurements **1138,** such as sequence read data (*e.g.* **1138-1-1-1,** ..., **1138-1-1-M,** ... **1138-1-Q-1,** ..., **1138-1-Q-T,** where Q and T are independent positive integers), where in the case in which the analyte measurements are sequence reads, they further include unique spatial barcodes **1150** (*e.g.,* **1150-1-1-1)** and analyte encoding portions **1152** (*e.g.*, **1152-1-1-1); and**

a template repository **1140** comprising a plurality of templates **1142-1,** ... **1142-Q,** respectively comprising corresponding coordinates systems **1144-1,** ..., **1144-Q,** reference fiducial spots **1146-1-1,** ..., **1146-1-K, 1146-Q-1,** ..., **1146-Q-P,** and corresponding coordinates **1148-1-1,** ..., **1148-1-K, 1148-Q-1,** ..., **1146-Q-P.**

In some implementations, the user interface **1106** includes an input device (*e.g.*, a keyboard, a mouse, a touchpad, a track pad, and/or a touch screen) **1110** for a user to interact with the system **1100** and a display **1108.**
In some implementations, one or more of the above identified elements are stored in one or more of the previously mentioned memory devices, and correspond to a set of instructions for performing a function described above. The above identified modules or programs (*e.g.*, sets of instructions) need not be implemented as separate software programs, procedures or modules, and thus various subsets of these modules may be combined or otherwise re-arranged in various implementations. In some implementations, the memory **1112** optionally stores a subset of the modules and data structures identified above. Furthermore, in some embodiments, the memory stores additional modules and data structures not described above. In some embodiments, one or more of the above identified elements is stored in a computer system, other than that of system **1100,** that is addressable by system **1100** so that system **1100** may retrieve all or a portion of such data when needed.

Although **FIG. 11** shows an exemplary system **1100,** the figure is intended more as functional description of the various features that may be present in computer systems than as a structural schematic of the implementations described herein. In practice, and as recognized by those of ordinary skill in the art, items shown separately could be combined and some items could be separated.

### (b) Methods for spatial analysis of analytes

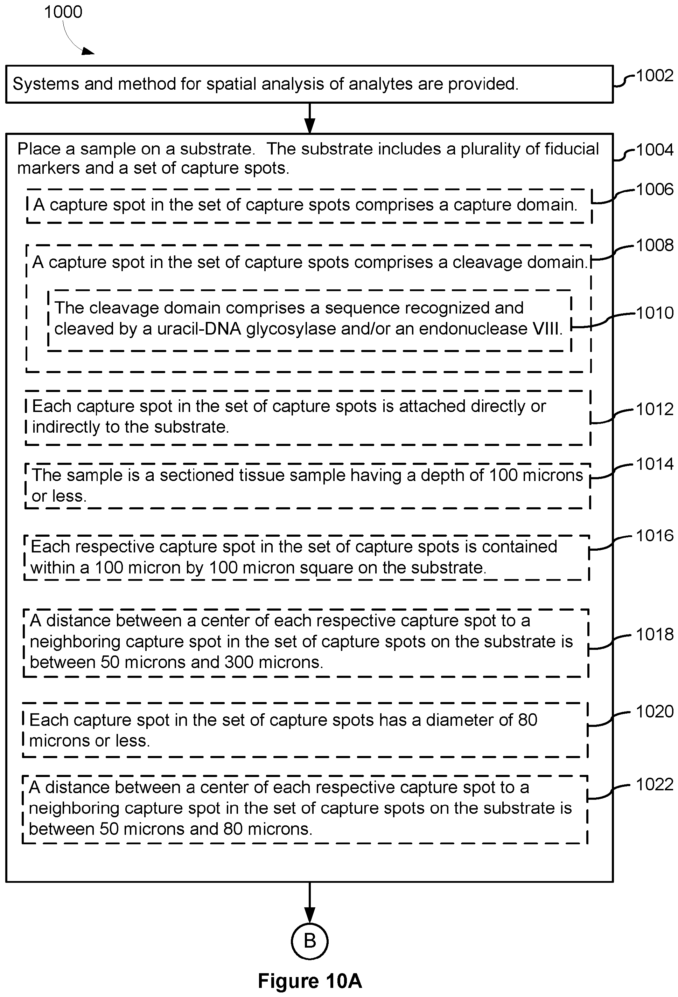

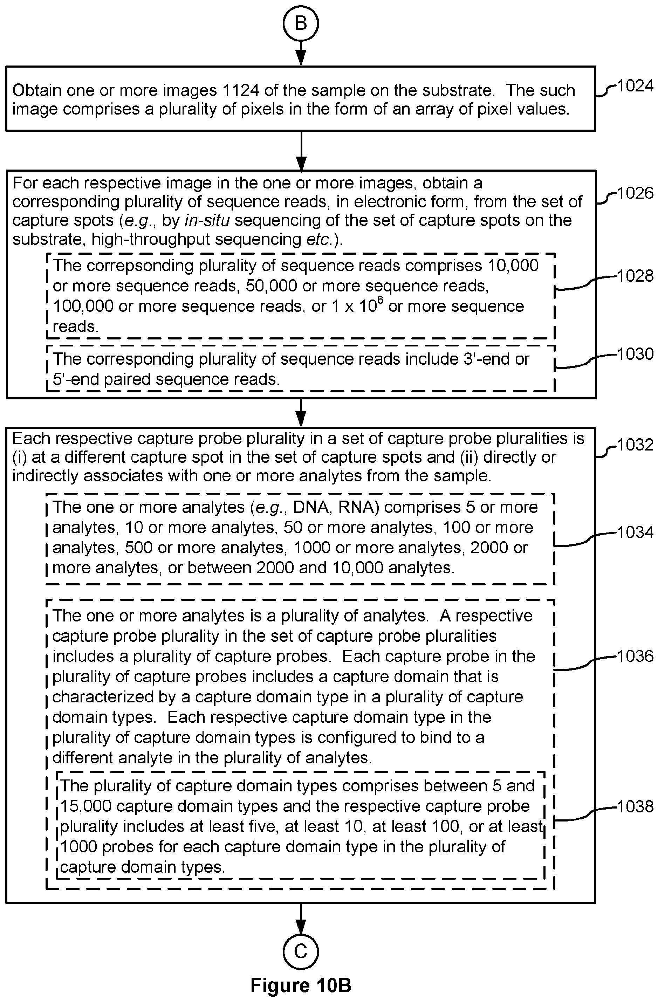

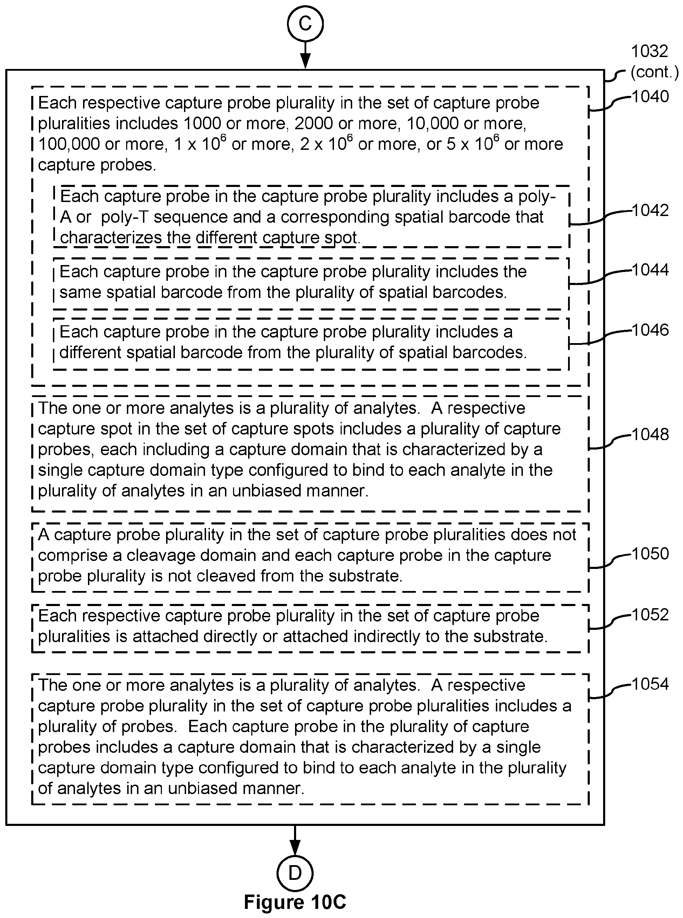

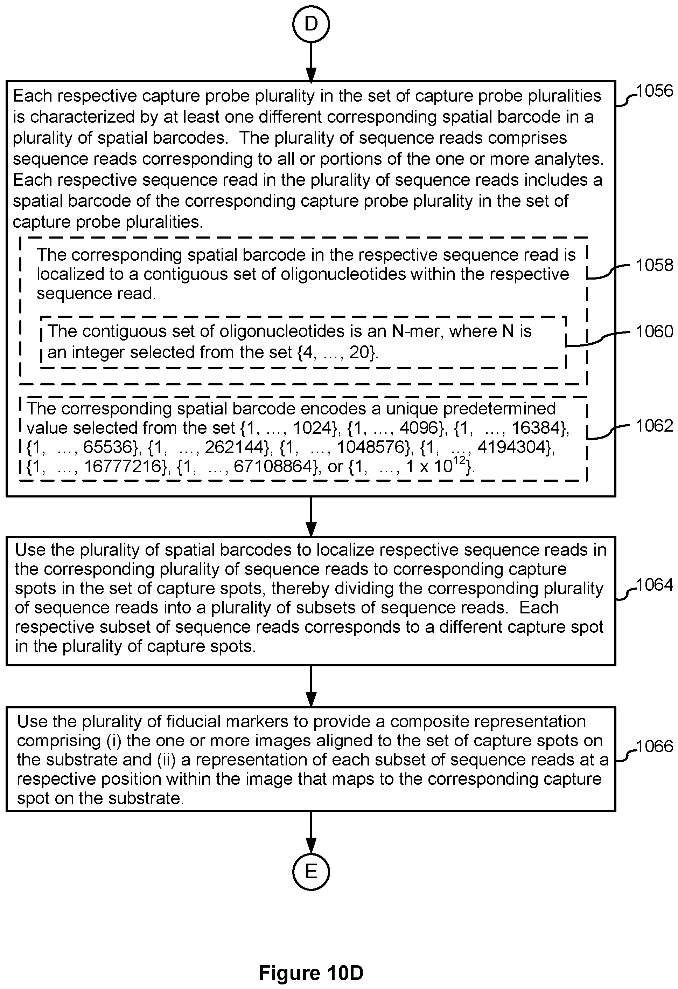

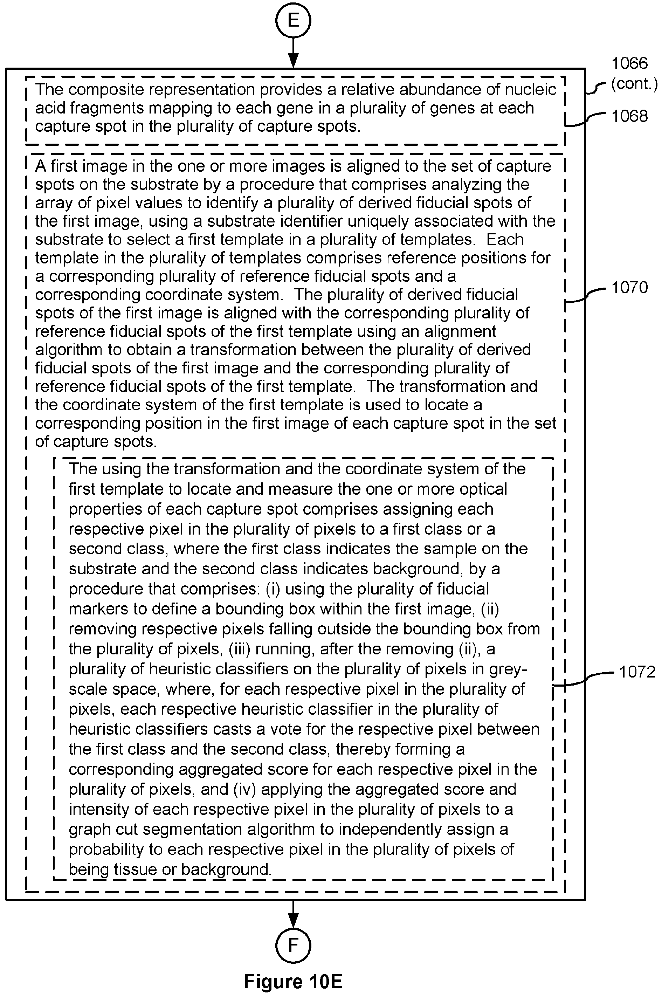

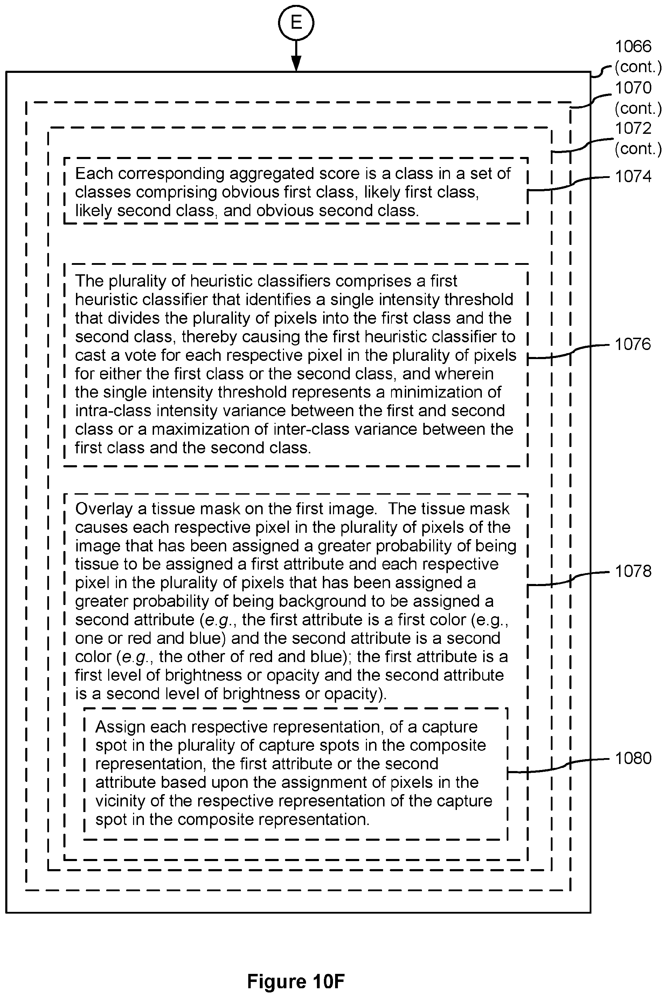

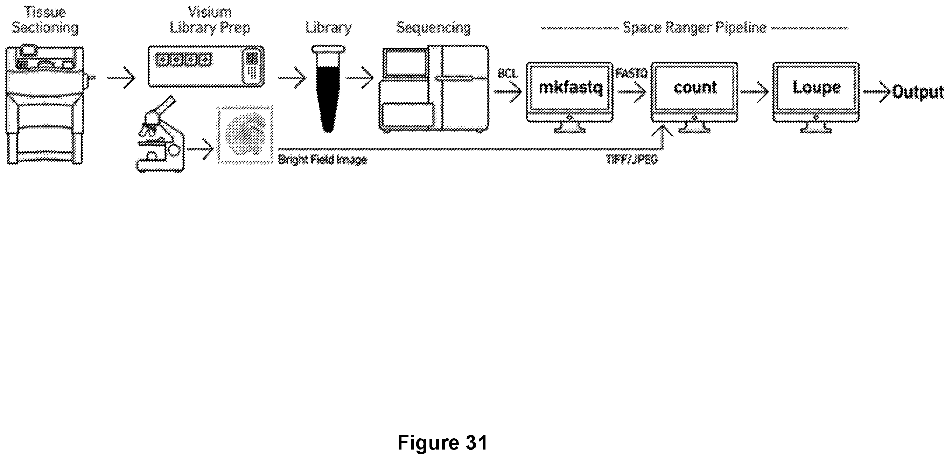

**FIG. 10** is a flow chart **1000** illustrating a method for spatial analysis of analytes **1002.** In some embodiments, the method takes place at a computer system **1100** having one or more processors **1102,** and memory **1112** storing one or more programs for execution by the one or more processors **1102.** It will be appreciated that the memory can be on a single computer, distributed across several computers, in one or more virtual machines and/or in a cloud computing architecture. **FIG. 31** provides an example overview of flow chart **1000,** including where each of the below descriptions, referenced as **FIG. 10** blocks, are found in the example overview.

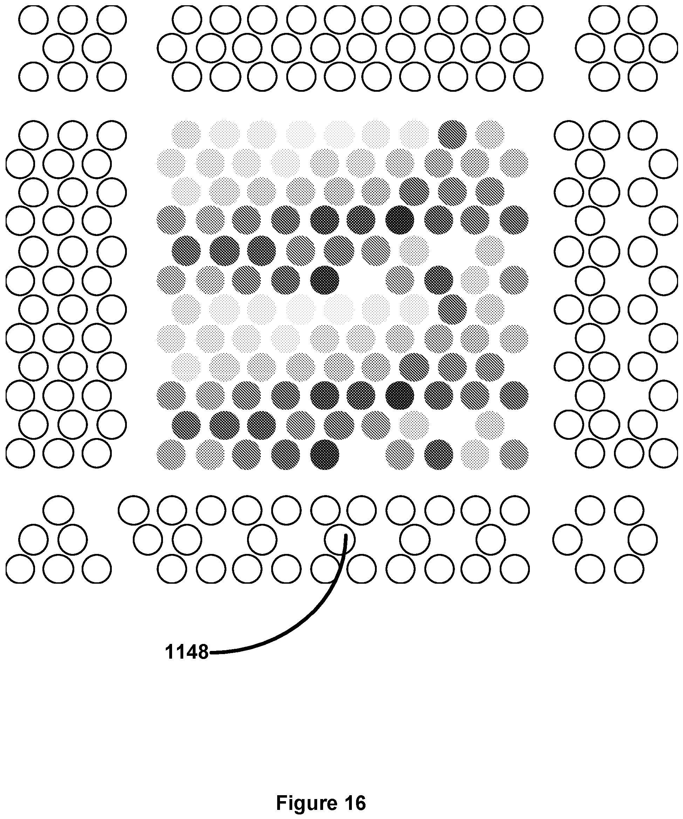

Referring to block **1004,** a sample (*e.g.*, sectioned tissue sample **1204 of FIG. 12)** is placed on a substrate. In some embodiments the sample is a biological sample. Example suitable types of biological samples are disclosed above in I. Introduction; (d) Biological samples. Example suitable types of substrates are disclosed above in II. General Spatial Array-Based Methodology; (c) Substrate. The substrate includes a plurality of fiducial markers and a set of capture spots. **FIG. 16** illustrates a substrate (*e.g.*, chip) that has a plurality of fiducial markers **1148** and a set of capture spots **1136,** in accordance with an embodiment of the present disclosure.

Referring to block **1006 of FIG. 10A,** in some embodiments, a respective capture spot **1136** in the set of capture spots includes a plurality of capture probes. Example suitable capture probes are discussed above in II. General spatial array-based methodology; (b) Capture probes. In such embodiments, each capture probe in the plurality of capture probes includes a capture domain that is characterized by a capture domain type in a plurality of capture domain types. Example capture domains are discussed above, for example, in I. Introduction; (a) Spatial analysis; and II. General spatial array-based methodology; (b) Capture probes; (i) Capture domains. Each respective capture domain type in the plurality of capture domain types is configured to bind directly or indirectly to a different analyte in the plurality of analytes. Example analytes are discussed above, for example, in I. Introduction; (c) Analytes. Thus, in some such embodiments, each capture domain type corresponds to a specific analyte (*e.g.*, a specific oligonucleotide or binding moiety for a specific gene). In some embodiments, each capture domain type in the plurality of capture domain types is configured to bind to the same analyte (*e.g.*, specific binding complementarity to mRNA for a single gene) or to different analytes (*e.g.*, specific binding complementarity to mRNA for a plurality of genes).

In some embodiments, a respective capture probe, and thus a capture probe plurality indirectly binds to an analyte through any of the capture agents **4002** of the present disclosure. Examples of capture agents are illustrated in FIGS. **40** and **41.** Moreover, **FIG. 41A,** upper panel, illustrates the indirect association of a capture probe **602** with an analyte capture agent **4002.** As illustrated in **FIG. 40,** in some embodiments the analyte capture agent **4002** specifically interacts with (binds with) a particular analyte **4006.** Thus, referring back to **FIG. 41A,** upper panel, when an analyte capture agent **4002** is bound to an analyte **4006,** and the analyte capture agent **4002** is associated with the capture probe **602** (*e.g.*, through the interaction of the capture domain **607** of the capture probes **602** to the analyte capture sequence **4114** of the analyte capture agent **4002** as illustrated in **FIG. 41A,** upper panel, the capture probe **602** is indirectly associated with the analyte **4006.**

Referring to block **1008,** in some embodiments, a capture spot in the set of capture spots comprises a cleavage domain. Example cleavage domains are disclosed in II. General Spatial Array-Based Methodology; (b) Capture probes; (ii) cleavage domain. Referring to block **1010,** in some embodiments, the cleavage domain comprises a sequence recognized and cleaved by a uracil-DNA glycosylase and/or an endonuclease VIII. Referring to block **1012,** in some embodiments, each capture spot (*e.g.,* the capture probes of the capture spots) in the set of capture spots is attached directly or attached indirectly to the substrate. More information on cleavage domains and how the capture probes are attached directly or indirectly to a substrate is discussed above in, for example, II. General spatial array-based methodology; (b) Capture probes; (ii) Cleavage domain.

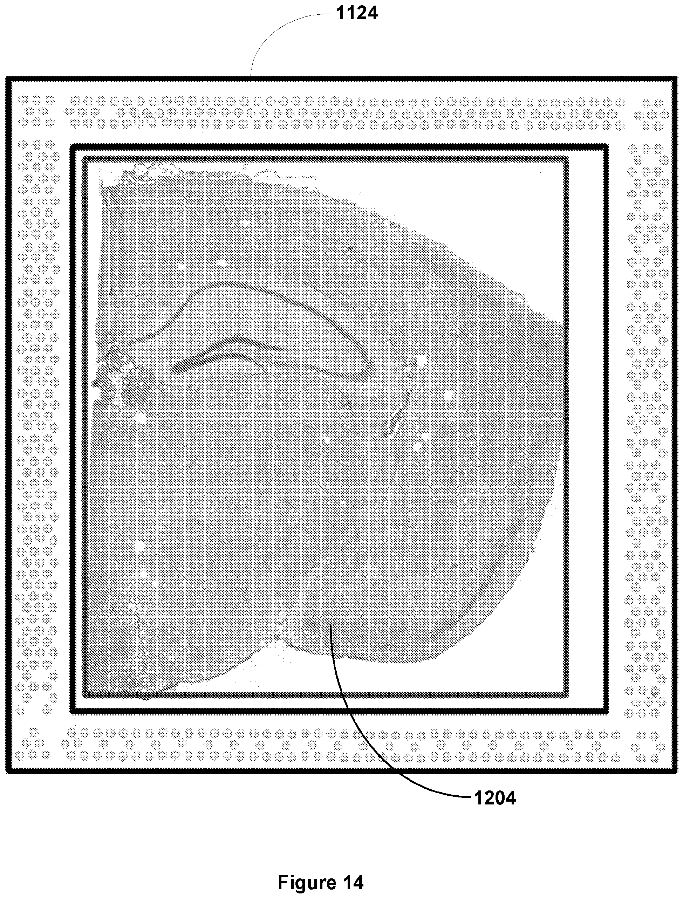

Referring to block **1014,** in some embodiments, the biological sample is a sectioned tissue sample having a depth of 100 microns or less. In some embodiments, the sectioned tissue sample has a depth of 80 microns or less, 70 microns or less, 60 microns or less, 50 microns or less, 40 microns or less, 25 microns or less, or 20 microns or less. In some embodiments, the sectioned tissue sample has a depth of between 10 microns and 20 microns. See, 10X, 2019, "Visium Spatial Gene Expression Solution." In some embodiments, the sectioned tissue sample has a depth of between 1 and 10 microns. Further embodiments of sectioned tissue samples are provided above in the Detailed Description (*e.g.*, under I. Introduction; (d) Biological samples; (ii) Preparation of biological samples; (1) Tissue sectioning). In some embodiments, a tissue section is a similar size and shape to the underlying substrate. In some embodiments, a tissue section is a different size and shape from the underlying substrate. In some embodiments, a tissue section is on all or a portion of the substrate. For example, **FIG. 14** illustrates a tissue section with dimensions roughly comparable to the substrate, such that a large proportion of the substrate is in contact with the tissue section. In some embodiments, several biological specimens from a subject are concurrently analyzed. For instance, in some embodiments several different sections of a tissue are concurrently analyzed. In some embodiments, 1, 2, 3, 4, 5, 6, 7, 8, 9, 10, 11, 12, 13, 14, 15, 16, 17, 18, 19, or 20 different biological samples from a subject are concurrently analyzed. For example, in some embodiments 1, 2, 3, 4, 5, 6, 7, 8, 9, 10, 11, 12, 13, 14, 15, 16, 17, 18, 19, or 20 different tissue sections from a single biological sample from a single subject are concurrently analyzed. In such embodiments, each such tissue section is considered an independent spatial projection (of the biological sample) and in such embodiments one or more images are acquired of each such tissue section. More generally, each different biological sample is considered an independent spatial projection (of the biological sample) and one or more images are acquired of each such biological sample.

In some embodiments, a tissue section on a substrate is a single uniform section. In some alternative embodiments, multiple tissue sections are on a substrate. In some such embodiments, a single capture area **1206** on a substrate can contain multiple tissue sections, where each tissue section is obtained from either the same biological sample and/or subject or from different biological samples and/or subjects. In some embodiments, a tissue section is a single tissue section that comprises one or more regions where no cells are present (*e.g.*, holes, tears, or gaps in the tissue). Thus, in some embodiments, such as the above, an image of a tissue section on a substrate can contain regions where tissue is present and regions where tissue is not present.

Referring to block **1016,** in some embodiments, each respective capture spot in a set of capture spots is contained within a 100 micron by 100 micron square on the substrate (*e.g.*, on the substrate of a chip). In some embodiments, each respective capture spot in a set of capture spots is contained within a 90 micron by 90 micron square on the substrate (*e.g.*, on the substrate of a chip). In some embodiments, each respective capture spot in a set of capture spots is contained within a 80 micron by 80 micron square on the substrate (*e.g.*, on the substrate of a chip). In some embodiments, each respective capture spot in a set of capture spots is contained within a 70 micron by 70 micron square on the substrate (*e.g.*, on the substrate of a chip). In some embodiments, at least 10 percent, 20 percent, 30 percent, 40 percent, 50 percent, 60 percent, 70 percent, 80 percent or 90 percent of the capture spots in a set of capture spots are contained within a 70 micron by 70 micron square on the substrate (*e.g.*, on the substrate of a chip). In some embodiments, all or at least some of the respective capture spots in a set of capture spots are contained within a 60 micron by 60 micron square on the substrate (*e.g.*, on the substrate of a chip). In some embodiments, all or at least some of the respective capture spots in a set of capture spots are contained within a 50 micron by 50 micron square on the substrate (*e.g.*, on the substrate of a chip). In some embodiments, all or at least some of the respective capture spots in a set of capture spots are contained within a 40 micron by 40 micron square on the substrate (*e.g.,* on the substrate of a chip). In some embodiments, all or at least some of the respective capture spots in a set of capture spots are contained within a 30 micron by 30 micron square on the substrate (*e.g.,* on the substrate of a chip). In some embodiments, all or at least some of the respective capture spots in a set of capture spots are contained within a 20 micron by 20 micron square on the substrate (*e.g.,* on the substrate of a chip). In some embodiments, all or at least some of the respective capture spots in a set of capture spots are contained within a 10 micron by 10 micron square on the substrate (*e.g.,* on the substrate of a chip). In some embodiments, all or at least some of the respective capture spots in a set of capture spots are contained within a 5 micron by 5 micron square on the substrate (*e.g.,* on the substrate of a chip). In some embodiments, at least 30 percent, at least forty percent, at least fifty percent, at least sixty percent, at least seventy percent, at least eighty percent, or at least ninety percent of the capture spots in the set of capture spots are each contained within a respective 4 micron by 4 micron square on the substrate (*e.g.,* on the substrate of a chip). In some embodiments, at least 30 percent, at least forty percent, at least fifty percent, at least sixty percent, at least seventy percent, at least eighty percent, or at least ninety percent of the capture spots in the set of capture spots are each contained within a respective 3 micron by 3 micron square on the substrate (*e.g.,* on the substrate of a chip). In some embodiments, at least 30 percent, at least forty percent, at least fifty percent, at least sixty percent, at least seventy percent, at least eighty percent, or at least ninety percent of the capture spots in the set of capture spots are each contained within a respective 2 micron by 2 micron square on the substrate (*e.g.,* on the substrate of a chip).

Referring to block **1018,** in some embodiments, a distance between a center of each respective capture spot to a neighboring capture spot in the set of capture spots on the substrate (*e.g.,* chip) is between 50 microns and 300 microns. In some embodiments, a distance between a center of each respective capture spot to a neighboring capture spot in the set of capture spots is between 100 microns and 200 microns. In some embodiments, a distance between a center of a respective capture spot to a neighboring capture spot in the set of capture spots is between 2 microns and 10 microns. More information on capture spot size, density and resolution is found above in II. General spatial array-based methodology; (d) Arrays.

In some embodiments, a shape of each capture spot in the set of capture spots on the substrate is a closed-form shape. In some embodiments, the closed-form shape is circular, elliptical, or an N-gon, where N is a value between 1 and 1000. In some embodiments, the closed-form shape is hexagonal.

In some such embodiments, the closed-form shape is circular and at least 30 percent, at least forty percent, at least fifty percent, at least sixty percent, at least seventy percent, at least eighty percent, or at least ninety percent of the capture spots in the set of capture spots has a diameter of between 25 microns and 65 microns. In some embodiments, the closed-form shape is circular or hexagonal, and at least 30 percent, at least forty percent, at least fifty percent, at least sixty percent, at least seventy percent, at least eighty percent, or at least ninety percent of the capture spots in the set of capture spots has a diameter of between 30 and 200 microns, and/or a diameter of 100 microns or less. In some embodiments, the closed-form shape is circular and at least 30 percent, at least forty percent, at least fifty percent, at least sixty percent, at least seventy percent, at least eighty percent, or at least ninety percent of the capture spots in the set of capture spots has a diameter of between 25 microns and 200 microns. In some embodiments, the closed-form shape is circular or hexagonal and at least 30 percent, at least forty percent, at least fifty percent, at least sixty percent, at least seventy percent, at least eighty percent, or at least ninety percent of the capture spots in the set of capture spots has a diameter of about 60 microns. In some embodiments, the closed-form shape is circular or hexagonal and at least 30 percent, at least forty percent, at least fifty percent, at least sixty percent, at least seventy percent, at least eighty percent, or at least ninety percent of the capture spot in the set of capture spots has a diameter of between 2 microns and 7 microns.

Referring to block **1020,** in some embodiments at least 30 percent, at least forty percent, at least fifty percent, at least sixty percent, at least seventy percent, at least eighty percent, at least ninety percent of the capture spots in a set of capture spots has a diameter of less than 80 microns. More information on capture spot size, density and resolution is found above in II. General spatial array-based methodology; (d) Arrays.

Referring to block **1022,** in some embodiments, a distance between a center of each respective capture spot to a neighboring capture spot in a set of capture spots on the substrate is between 50 microns and 80 microns. More information on capture spot size, density and resolution is found above in II. General spatial array-based methodology; (d) Arrays.

In some embodiments, the positions of a plurality of capture spots on substrates are arranged in a predetermined array type format. In some embodiments, the positions of the plurality of capture spots on a substrate are not predetermined. In some embodiments, a substrate comprises fiducial markers, and the position of the fiducial markers is predetermined such that they can be mapped to a spatial location. In some embodiments, a substrate comprises a number of capture spots that is between 500 and 1000, 1000 to 5000, 5000 to 10,000, 10,000 to 15,000, 15,000 to 20,000, or more than 20,000. In some embodiments, a substrate comprises between 1000 and 5000 capture spots, where capture spots are arranged on the substrate hexagonally or in a grid.

In some embodiments, each respective capture spot includes 1000 or more capture probes, 2000 or more capture probes, 10,000 or more capture probes, 100,000 or more capture probes, $1 \times 10^6$ or more capture probes, $2 \times 10^6$ or more capture probes, or $5 \times 10^6$ or more capture probes. In some embodiments, each capture probe in the respective capture spot includes a poly-A sequence or a poly-T sequence and the unique spatial barcode that characterizes the respective capture spot. In some embodiments, each capture probe in the respective capture spot includes the same spatial barcode or a different spatial barcode from the plurality of spatial barcodes.

Numerous alternative combinations of capture domain types, capture spot sizes, arrays, probes, spatial barcodes analytes, and/or other features of capture spots including but not limited to dimensions, designs, and modifications are also possible, and are discussed in detail at length above (*e.g.*, in Section (II) General spatial array-based analytical methodology; Subsections (b) Capture probes, (c) Substrate, and (d) Arrays).

Referring to block **1024** of **FIG. 10B,** in some embodiments one or more images **1124** of the biological sample, on the substrate, are obtained. Each such image comprises a plurality of pixels in the form of an array of pixel values. In some embodiments the array of pixel values comprises at least a least 100, 10,000, 100,000, $1 \times 10^6$, $2 \times 10^6$, $3 \times 10^6$, $5 \times 10^6$, $8 \times 10^6$, $10 \times 10^6$, or $15 \times 10^6$ pixel values. In some embodiments, an image is acquired using transmission light microscopy (*e.g.*, bright field transmission light microscopy, dark field transmission light microscopy, oblique illumination transmission light microscopy, dispersion staining transmission light microscopy, phase contrast transmission light microscopy, differential interference contrast transmission light microscopy, emission imaging, *etc.*). See, *for example,* Methods in Molecular Biology, 2018, Light Microscopy Method and Protocols, Markaki and Harz eds., Humana Press, New York, New York, ISBN-13: 978-1493983056, which is hereby incorporated by reference. As an illustration, **FIG. 14** shows an example of an image **1124** of a biological sample on a substrate in accordance with some embodiments.

In some embodiments, an image **1124** is a bright-field microscopy image in which the imaged sample appears dark on a bright background. In some such embodiments, the sample has been stained. For instance, in some embodiments, the sample has been stained with Haemotoxylin and Eosin and the image **1124** is a bright-field microscopy image. In some embodiments the sample has been stained with a Periodic acid-Schiff reaction stain (stains carbohydrates and carbohydrate rich macromolecules a deep red color) and the image is a bright-field microscopy image. In some embodiments the sample has been stained with a Masson's trichrome stain (nuclei and other basophilic structures are stained blue, cytoplasm, muscle, erythrocytes and keratin are stained bright-red, collagen is stained green or blue, depending on which variant of the technique is used) and the image is a bright-field microscopy image. In some embodiments, the sample has been stained with an Alcian blue stain (a mucin stain that stains certain types of mucin blue, and stains cartilage blue and can be used with H&E, and with van Gieson stains) and the image is a bright-field microscopy image. In some embodiments the sample has been stained with a van Gieson stain (stains collagen red, nuclei blue, and erythrocytes and cytoplasm yellow, and can be combined with an elastin stain that stains elastin blue/black) and the image is a bright-field microscopy image. In some embodiments the sample has been stained with a reticulin stain, an Azan stain, a Giemsa stain, a Toluidine blue stain, an isamin blue/eosin stain, a Nissl and methylene blue stain, and/or a sudan black and osmium stain and the image is a brightfield microscopy image.

In some embodiments, rather than being a bright-field microscopy image of a sample, an image **1124** is an immunohistochemistry (IHC) image. IHC imaging relies upon a staining technique using antibody labels. One form of immunohistochemistry (IHC) imaging is immunofluorescence (IF) imaging. In an example of IF imaging, primary antibodies are used that specifically label a protein in the biological sample, and then a fluorescently labelled secondary antibody or other form of probe is used to bind to the primary antibody, to show up where the first (primary) antibody has bound. A light microscope, equipped with fluorescence, is used to visualize the staining. The fluorescent label is excited at one wavelength of light, and emits light at a different wavelength. Using the right combination of filters, the staining pattern produced by the emitted fluorescent light is observed. In some embodiments, a biological sample is exposed to several different primary antibodies (or other forms of probes) in order to quantify several different proteins in a biological sample. In some such embodiments, each such respective different primary antibody (or probe) is then visualized with a different fluorescence label (different channel) that fluoresces at a unique wavelength or wavelength range (relative to the other fluorescence labels used). In this way, several different proteins in the biological sample can be visualized.

More generally, in some embodiments of the present disclosure, in addition to brightfield imaging or instead of brightfield imaging, fluorescence imaging is used to acquire one or more spatial images of the sample. As used herein the term "fluorescence imaging" refers to imaging that relies on the excitation and re-emission of light by fluorophores, regardless of whether they're added experimentally to the sample and bound to antibodies (or other compounds) or simply natural features of the sample. The above-described IHC imaging, and in particular IF imaging, is just one form of fluorescence imaging. Accordingly, in some embodiments, each respective image **1124** in a single spatial projection (*e.g.,* of a biological sample) represents a different channel in a plurality of channels, where each such channel in the plurality of channels represent an independent (*e.g.,* different) wavelength or a different wavelength range (*e.g.,* corresponding to a different emission wavelength). In some embodiments, the images 1124 of a single spatial projection will have been taken of a tissue (*e.g.,* the same tissue section) by a microscope at multiple wavelengths, where each such wavelength corresponds to the excitation frequency of a different kind of substance (containing a fluorophore) within or spatially associated with the sample. This substance can be a natural feature of the sample (*e.g.,* a type of molecule that is naturally within the sample), or one that has been added to the sample. One manner in which such substances are added to the sample is in the form of probes that excite at specific wavelengths. Such probes can be directly added to the sample, or they can be conjugated to antibodies that are specific for some sort of antigen occurring within the sample, such as one that is exhibited by a particular protein. In this way, a user can use the spatial projection, comprising a plurality of such images **1124** to be able to see capture spot data on top of fluorescence image data, and to look at the relation between gene (or antibody) expression against another cellular marker, such as the spatial abundance of a particular protein that exhibits a particular antigen. In typical embodiments, each of the images **1124** of a given spatial projection will have the same dimensions and position relative to a single set of capture spot locations associated with the spatial projection. Each respective spatial projection in a discrete attribute value dataset **1122** will have its own set of capture spot locations associated with the respective spatial projection. Thus, for example, even though a first and second spatial projection in a given discrete attribute dataset **1122** make use of the same probe set, they will both have their own set of capture spot locations for this probe set. This is because, for example, each spatial projection represents images that are taken from an independent target (*e.g.,* different tissue slices, *etc.*).

In some embodiments, both a bright-field microscopy image and a set of fluorescence images (*e.g.,* immunohistochemistry images) are taken of a biological sample and are in the same spatial projection for the biological sample.

In some embodiments, substrates in the form of slides or chips are used to provide support to a biological sample, particularly, for example, a thin tissue section. In some embodiments, a substrate is a support that allows for positioning of biological samples, analytes, capture spots, and/or capture probes on the substrate. More information on substrates is found above in II. General spatial array-based methodology; (c) Substrate.

In some embodiments, the biological sample is subjected to immunohistochemistry prior to image acquisition and fluorescence imaging is used to acquire the image. In some embodiments, the biological sample is subjected to fluorescence imaging to acquire images without application of immunohistochemistry to the sample.

In some embodiments in which fluorescence imaging is conducted, the image is acquired using Epiillumination mode, where both the illumination and detection are performed from one side of the sample.

In some such embodiments, the image is acquired using confocal microscopy, two-photon imaging, wide-field multiphoton microscopy, single plane illumination microscopy or light sheet fluorescence microscopy. See, for example, Adaptive Optics for Biological Imaging, 2013, Kubby ed., CRC Press, Boca Raton, Florida; and Confocal and Two-Photon Microscopy: Foundations, Applications and Advances, 2002, Diaspro ed., Wiley Liss, New York, New York; and Handbook of Biological Confocal Microscopy, 2002, Pawley ed., Springer Science+Business Media, LLC, New York, New York each of which is hereby incorporated by reference.

In some embodiments, the set of images (of a projection) are images created using fluorescence imaging, for example, by making use of various immunohistochemistry (IHC) probes that excite at various different wavelengths. See, *for example,* Day and Davidson, 2014, "The Fluorescent Protein Revolution (In Cellular and Clinical Imaging)," CRC Press, Taylor & Francis Group, Boca Raton, Florida; "Quantitative Imaging in Cell Biology" Methods in Cell Biology 123, 2014, Wilson and Tran, eds.; Advanced Fluorescence Reporters in Chemistry and Biology II: Molecular Constructions, Polymers and Nanoparticles (Springer Series on Fluorescence), 2010, Demchenko, ed., Springer-Verlag, Berlin, Germany; Fluorescence Spectroscopy and Microscopy: Methods and Protocols (Methods in Molecular Biology) 2014th Edition, 2014, Engelborghs and Visser, eds., HumanPress, each of which is hereby incorporated by reference for their disclosure on fluorescence imaging.

An image can be obtained in any electronic image file format, including but not limited to JPEG/JFIF, TIFF, Exif, PDF, EPS, GIF, BMP, PNG, PPM, PGM, PBM, PNM, WebP, HDR raster formats, HEIF, BAT, BPG, DEEP, DRW, ECW, FITS, FLIF, ICO, ILBM, IMG, PAM, PCX, PGF, JPEG XR, Layered Image File Format, PLBM, SGI, SID, CD5, CPT, PSD, PSP, XCF, PDN, CGM, SVG, PostScript, PCT, WMF, EMF, SWF, XAML, and/or RAW.

In some embodiments, an image is obtained in any electronic color mode, including but not limited to grayscale, bitmap, indexed, RGB, CMYK, HSV, lab color, duotone, and/or multichannel. In some embodiments, the image is manipulated (*e.g.,* stitched, compressed and/or flattened). In some embodiments, an image size is between 1 KB and 1 MB, between 1 MB and 0.5 GB, between 0.5 GB and 5 GB, between 5 GB and 10 GB, or greater than 10 GB. In some embodiments, the image includes between 1 million and 25 million pixels. In some embodiments, each capture spot is represented by five or more, ten or more, 100 or more, 1000 or more contiguous pixels in an image. In some embodiments, each capture spot is represented by between 1000 and 250,000 contiguous pixels in a native image 125.

In some embodiments, an image is represented as an array (*e.g.,* matrix) comprising a plurality of pixels, such that the location of each respective pixel in the plurality of pixels in the array (*e.g.,* matrix) corresponds to its original location in the image. In some embodiments, an image is represented as a vector comprising a plurality of pixels, such that each respective pixel in the plurality of pixels in the vector comprises spatial information corresponding to its original location in the image.

In some embodiments, an image **1124** is acquired using a Nikon Eclipse Ti2 with brightfield and fluorescence capacity (TRITC) or an ImageXpress Nano Automated Cell Imaging System or equivalent. In some embodiments an image **1124** is acquired with a microscope having a 4X (Plan APO λ; NA 0.20), 10X (Plan APO λ; NA 0.45), or 20X (Plan APO λ; NA 0.75) objective lens or equivalent.

In some embodiments, an image **1124** is a color image (*e.g.,* $3 \times 8$ bit, $2424 \times 2424$ pixel resolution). In some embodiments, an image **1124** is a monochrome image (*e.g.,* 14 bit, $2424 \times 2424$ pixel resolution).

In some embodiments, an image is acquired using transmission light microscopy. In some embodiments, the biological sample is stained prior to imaging using, *e.g.,* fluorescent, radioactive, chemiluminescent, calorimetric, or colorimetric detectable markers. In some embodiments, the biological sample is stained using live/dead stain (*e.g.,* trypan blue). In some embodiments, the biological sample is stained with Haemotoxylin and Eosin, a Periodic acid-Schiff reaction stain (stains carbohydrates and carbohydrate rich macromolecules a deep red color), a Masson's trichrome stain (nuclei and other basophilic structures are stained blue, cytoplasm, muscle, erythrocytes and keratin are stained bright-red, collagen is stained green or blue, depending on which variant of the technique is used), an Alcian blue stain (a mucin stain that stains certain types of mucin blue, and stains cartilage blue and can be used with H&E, and with van Gieson stains), a van Gieson stain (stains collagen red, nuclei blue, and erythrocytes and cytoplasm yellow, and can be combined with an elastin stain that stains elastin blue/black), a reticulin stain, an Azan stain, a Giemsa stain, a Toluidine blue stain, an isamin blue/eosin stain, a Nissl and methylene blue stain, and/or a sudan black and osmium stain. In some embodiments, biological samples are stained as described in I. Introduction; (d) Biological samples; (ii) Preparation of biological samples; (6) staining. In some embodiments, the image is acquired using optical microscopy (*e.g.,* bright field, dark field, dispersion staining, phase contrast, differential interference contrast, interference reflection, fluorescence, confocal, single plane illumination, wide-field multiphoton, deconvolution, transmission electron microscopy, and/or scanning electron microscopy). In some embodiments, the image is acquired after staining the tissue section but prior to analyte capture.

In some embodiments, the exposure time for the image **1124** is between 2 and 10 milliseconds. In some embodiments, the biological sample is exposed to a light source (or equivalent) with a wavelength range of 380-680 nm is during the acquisition of the image. In some embodiments, the minimum capture resolution is 2.18 μm/pixel.

In some embodiments, a substrate (*e.g.,* chip) can comprise any suitable support material, including, but not limited to, glass, modified and/or functionalized glass, hydrogels, films, membranes, plastics (including *e.g.,* acrylics, polystyrene, copolymers of styrene and other materials, polypropylene, polyethylene, polybutylene, polyurethanes, TEFLON™, cyclic olefins, polyimides, *etc.*), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate. In some embodiments, a chip can be printed, patterned, or otherwise modified to comprise capture spots that allow association with analytes upon contacting a biological sample (*e.g.,* a tissue section). Further detailed embodiments of substrate properties, structure, and/or modifications are described above in II. General spatial array-based analytical methodology; (c) Substrate.

Referring to **FIG. 12,** in some embodiments, the substrate can comprises a capture area **1206,** where the capture area comprises a plurality of barcoded capture spots **1136** for one or more reactions and/or assays, and where a reaction comprises one or more tissue types for spatial analysis. In some embodiments, the substrate comprises 2, 3, 4, 5, 6, 7, 8, 9, 10, 11, 12, 13, 14, 15, 16, 17, 18, 19, 20, more than 20, more than 30, more than 40, or more than 50 capture areas **1206** for a plurality of reactions and/or assays. For example, in some embodiments, the substrate is a spatial gene expression slide (*e.g.,* Visium) comprising four capture areas **1206,** each capture area having the dimensions 6.5 mm × 6.5 mm, such that the substrate comprises a capacity for four reactions and up to four tissue types. In some such embodiments, each capture area comprises 5,000 barcoded capture spots **1136,** where each capture spot is 55 μm in diameter and the distance between the centers of two respective capture spots is 100 μm. See, 10X, 2019, "Visium Spatial Gene Expression Solution," which is hereby incorporated herein by reference. Further specific embodiments of capture spots are detailed below in the present disclosure as well as in II. General spatial array-based methodology; (d) Arrays. *See also,* United States Patent Application No. 16/992,569 entitled "Systems and Methods for Using the Spatial Distribution of Haplotypes to Determine a Biological Condition," filed August 13, 2020, and United States Provisional Patent Application No. 62/839,346 entitled "Spatial Transcriptomics of Biological Analytes in Tissue Samples," filed April 26, 2019, each of which is hereby incorporated by reference.

Referring again to block **1004,** the biological sample is obtained from a subject. As defined above, in some embodiments, a subject is a mammal such as a rodent, mouse, rat, rabbit, guinea pig, ungulate, horse, sheep, pig, goat, cow, cat, dog, primate (*e.g.,* human or non-human primate); a plant such as *Arabidopsis thaliana,* corn, sorghum, oat, wheat, rice, canola, or soybean; an algae such as *Chlamydomonas reinhardtii;* a nematode such as *Caenorhabditis elegans;* an insect such as *Drosophila melanogaster,* mosquito, fruit fly, honey bee or spider; a fish such as zebrafish; a reptile; an amphibian such as a frog or *Xenopus laevis; a Dictyostelium discoideum*; a fungi such as *Pneumocystis carinii, Takifugu rubripes*, yeast, *Saccharamoyces cerevisiae* or *Schizosaccharomyces pombe*; or a *Plasmodium falciparum.* These examples are non-limiting and do not preclude substitution of any alternative subjects that will occur to one skilled in the art.

In some embodiments, the biological sample is a tissue sample, and the tissue sample is obtained from any tissue and/or organ derived from any subject, including but not limited to those subjects listed above. In some embodiments, a tissue sample is obtained from, *e.g.,* heart, kidney, ovary, breast, lymph node, adipose, brain, small intestine, stomach, liver, quadriceps, lung, testes, thyroid, eyes, tongue, large intestine, spleen, and/or mammary gland, skin, muscle, diaphragm, pancreas, bladder, prostate, among others. Tissue samples can be obtained from healthy or unhealthy tissue (*e.g.,* inflamed, tumor, carcinoma, or other). Additional examples of tissue samples are shown in Table 1 and catalogued, for example, in 10X, 2019, "Visium Spatial Gene Expression Solution," which is hereby incorporated herein by reference.

*Table 1: Examples of tissue samples*

| Organism | Tissue | Healthy/Diseased |
|---|---|---|
| Human | Brain | Cerebrum Glioblastoma Multiforme |
| Human | Breast | Healthy |
| Human | Breast | Invasive Ductal Carcinoma |
| Human | Breast | Invasive Lobular Carcinoma |
| Human | Heart | Healthy |
| Human | Kidney | Healthy |
| Human | Kidney | Nephritis |
| Human | Large Intestine | Colorectal Cancer |
| Human | Lung | Papillary Carcinoma |

(continued)

| Organism | Tissue | Healthy/Diseased |
|---|---|---|
| Human | Lymph Node | Healthy |
| Human | Lymph Node | Inflamed |
| Human | Ovaries | Tumor |
| Human | Spleen | Inflamed |
| Mouse | Brain | Healthy |
| Mouse | Eyes | Healthy |
| Mouse | Heart | Healthy |
| Mouse | Kidney | Healthy |
| Mouse | Large Intestine | Healthy |
| Mouse | Liver | Healthy |
| Mouse | Lungs | Healthy |
| Mouse | Ovary | Healthy |
| Mouse | Quadriceps | Healthy |
| Mouse | Small Intestine | Healthy |
| Mouse | Spleen | Healthy |
| Mouse | Stomach | Healthy |
| Mouse | Testes | Healthy |
| Mouse | Thyroid | Healthy |
| Mouse | Tongue | Healthy |
| Rat | Brain | Healthy |
| Rat | Heart | Healthy |
| Rat | Kidney | Healthy |
| Mouse | Tongue | Healthy |
| Rat | Brain | Healthy |
| Rat | Heart | Healthy |
| Rat | Kidney | Healthy |

In some embodiments, the sectioned tissue is prepared by tissue sectioning, as described above in I. Introduction; (d) Biological samples; (ii) Preparation of biological samples; (1) Tissue sectioning. Briefly, in some embodiments, thin sections of tissue are prepared from a biological sample (*e.g.,* using a mechanical cutting apparatus such as a vibrating blade microtome, or by applying a touch imprint of a biological sample to a suitable substrate material). In some embodiments, a biological sample is frozen, fixed and/or cross-linked, or encased in a matrix (*e.g.,* a resin or paraffin block) prior to sectioning to preserve the integrity of the biological sample during sectioning. Further implementations of biological sample preparation are provided above in I. Introduction; (d) Biological samples; (ii) Preparation of biological samples; (2) Freezing, (3) Formalin fixation and paraffin embedding, (4) Fixation, and (5) Embedding. As an example, referring to **FIG. 3**, preparation of a biological sample using tissue sectioning comprises a first step 301 of an exemplary workflow for spatial analysis.

Referring to block **1026,** a plurality of sequence reads is obtained, in electronic form, from the set of capture spots (*e.g.,* by in-situ sequencing of the set of capture spots on the substrate, high-throughput sequencing etc.). Referring to block **1032** and as illustrated for example in **FIG. 12**, in some embodiments, each respective capture spot **1136** in the set of capture spots is (i) at a different position in a two-dimensional array and (ii) directly or indirectly associates with one or more analytes from the tissue. Further, in such embodiments, each respective capture spot in the set of capture spots is characterized by at least one unique spatial barcode in a plurality of spatial barcodes. Example suitable methods for obtaining sequence reads are disclosed in United States Patent Application No. 16/992,569, entitled "Systems and Methods for Using the Spatial Distribution of Haplotypes to Determine a Biological Condition," filed August 13, 2020, and United States Provisional Patent Application No. 62/839,346 entitled "Spatial Transcriptomics of Biological Analytes in Tissue Samples," filed April 26, 2019, each of which is hereby incorporated by reference.

In accordance with block **1024,** in some embodiments, after analytes from the sample have hybridized or otherwise been associated with capture probes, analyte capture agents, or other barcoded oligonucleotide sequences of the capture spots **1136** according to any of the methods described above in connection with the general spatial cell-based analytical methodology, the barcoded constructs that result from hybridization/association are analyzed via sequencing to identify the analytes. In some such embodiments, one hundred thousand or more, one million or more, ten million or more, or one hundred million or more sequence reads collected from a single tissue sample associated with an image in a projection are used to determine the unique UMI count (discrete attribute value) on a locus by locus and capture spot by capture spot basis in the resulting discrete attribute value dataset.

In some embodiments, where a sample is barcoded directly via hybridization with capture probes or analyte capture agents hybridized, bound, or associated with either the cell surface, or introduced into the cell, as described above, sequencing can be performed on the intact sample. Alternatively, if the barcoded sample has been separated into fragments, cell groups, or individual cells, as described above, sequencing can be performed on individual fragments, cell groups, or cells. For analytes that have been spatially barcoded via partitioning with beads, as described above, individual analytes (*e.g.,* cells, or cellular contents following lysis of cells) can be extracted from the partitions by breaking the partitions, and then analyzed by sequencing to identify the analytes.

A wide variety of different sequencing methods can be used to analyze spatially barcoded analyte constructs. In general, sequenced polynucleotides can be, for example, nucleic acid molecules such as deoxyribonucleic acid (DNA) or ribonucleic acid (RNA), including variants or derivatives thereof (*e.g.,* single stranded DNA or DNA/RNA hybrids, and nucleic acid molecules with a nucleotide analog).

Sequencing of spatially barcoded polynucleotides can be performed by various commercial systems. More generally, sequencing can be performed using nucleic acid amplification, polymerase chain reaction (PCR) (*e.g.,* digital PCR and droplet digital PCR (ddPCR), quantitative PCR, real time PCR, multiplex PCR, PCR-based singleplex methods, emulsion PCR), and/or isothermal amplification.

Other examples of methods for sequencing spatially barcoded genetic material include, but are not limited to, DNA hybridization methods (*e.g.,* Southern blotting), restriction enzyme digestion methods, Sanger sequencing methods, next-generation sequencing methods (*e.g.,* single-molecule real-time sequencing, nanopore sequencing, and Polony sequencing), ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, shortread sequencing, single-molecule sequencing, sequencing-by-synthesis, real-time sequencing, reverse-terminator sequencing, nanopore sequencing, 454 sequencing, Solexa Genome Analyzer sequencing, SOLiD™ sequencing, MS-PET sequencing, and any combinations thereof.

Sequence analysis of the nucleic acid molecules (including barcoded nucleic acid molecules or derivatives thereof) can be direct or indirect. Thus, the sequence analysis substrate (which can be viewed as the molecule which is subjected to the sequence analysis step or process) can directly be the barcoded nucleic acid molecule or it can be a molecule which is derived therefrom (*e.g.,* a complement thereof). Thus, for example, in the sequence analysis step of a sequencing reaction, the sequencing template can be the barcoded nucleic acid molecule or it can be a molecule derived therefrom. For example, a first and/or second strand DNA molecule can be directly subjected to sequence analysis (*e.g.* sequencing), *e.g.,* can directly take part in the sequence analysis reaction or process (*e.g.* the sequencing reaction or sequencing process, or be the molecule that is sequenced or otherwise identified). Alternatively, the spatially barcoded nucleic acid molecule can be subjected to a step of second strand synthesis or amplification before sequence analysis (*e.g.,* sequencing or identification by another technique). The sequence analysis substrate (*e.g.,* template) can thus be an amplicon or a second strand of a barcoded nucleic acid molecule.

In some embodiments, both strands of a double stranded molecule can be subjected to sequence analysis (*e.g.,* sequenced). In some embodiments, single stranded molecules (*e.g.* barcoded nucleic acid molecules) can be analyzed (*e.g.* sequenced). To perform single molecule sequencing, the nucleic acid strand can be modified at the 3' end.

Massively parallel sequencing techniques can be used for sequencing nucleic acids, as described above. In one embodiment, a massively parallel sequencing technique can be based on reversible dye-terminators. As an example, DNA molecules are first attached to primers on, *e.g.,* a glass or silicon substrate, and amplified so that local clonal colonies are formed (bridge amplification). Four types of ddNTPs are added, and non-incorporated nucleotides are washed away. Unlike pyrosequencing, the DNA is only extended one nucleotide at a time due to a blocking group (*e.g.,* 3' blocking group present on the sugar moiety of the ddNTP). A detector acquires images of the fluorescently labelled nucleotides, and then the dye along with the terminal 3' blocking group is chemically removed from the DNA, as a precursor to a subsequent cycle. This process can be repeated until the required sequence data is obtained.

As another example, massively parallel pyrosequencing techniques can also be used for sequencing nucleic acids. In pyrosequencing, the nucleic acid is amplified inside water droplets in an oil solution (emulsion PCR), with each droplet containing a single nucleic acid template attached to a single primer-coated bead that then forms a clonal colony. The sequencing system contains many picolitre-volume wells each containing a single bead and sequencing enzymes. Pyrosequencing uses luciferase to generate light for detection of the individual nucleotides added to the nascent nucleic acid and the combined data are used to generate sequence reads.

As another example application of pyrosequencing, released PPi can be detected by being immediately converted to adenosine triphosphate (ATP) by ATP sulfurylase, and the level of ATP generated can be detected via luciferase-produced photons, such as described in Ronaghi et al., 1996, Anal. Biochem. 242(1), 84-9; Ronaghi, 2001, Genome Res. 11(1), 3-11; Ronaghi et al., 1998, Science 281 (5375), 363; and U.S. Pat. Nos. 6,210,891, 6,258,568, and 6,274,320, the entire contents of each of which are incorporated herein by reference.

In some embodiments, sequencing is performed by detection of hydrogen ions that are released during the polymerisation of DNA. A microwell containing a template DNA strand to be sequenced can be flooded with a single type of nucleotide. If the introduced nucleotide is complementary to the leading template nucleotide, it is incorporated into the growing complementary strand. This causes the release of a hydrogen ion that triggers a hypersensitive ion sensor, which indicates that a reaction has occurred. If homopolymer repeats are present in the template sequence, multiple nucleotides will be incorporated in a single cycle. This leads to a corresponding number of released hydrogen ions and a proportionally higher electronic signal.

In some embodiments, sequencing is performed *in-situ. In-situ* sequencing methods are particularly useful, for example, when the biological sample remains intact after analytes on the sample surface (*e.g.,* cell surface analytes) or within the sample (*e.g.,* intracellular analytes) have been barcoded. In-situ sequencing typically involves incorporation of a labeled nucleotide (*e.g.,* fluorescently labeled mononucleotides or dinucleotides) in a sequential, template-dependent manner or hybridization of a labeled primer (*e.g.,* a labeled random hexamer) to a nucleic acid template such that the identities (*e.g.,* nucleotide sequence) of the incorporated nucleotides or labeled primer extension products can be determined, and consequently, the nucleotide sequence of the corresponding template nucleic acid. Aspects of *in-situ* sequencing are described, for example, in Mitra et al., 2003, Anal. Biochem. 320, 55-65, and Lee et al., 2014, Science 343(6177), 1360-1363, the entire contents of each of which are incorporated herein by reference.

In addition, examples of methods and systems for performing in-situ sequencing are described in PCT Patent Application Publication Nos. WO2014/163886, WO2018/045181, WO2018/045186, and in U.S. Patent Nos. 10,138,509 and 10,179,932, the entire contents of each of which are incorporated herein by reference. Example techniques for in-situ sequencing include, but are not limited to, STARmap (described for example in Wang et al., 2018, Science 361(6499), 5691, MERFISH (described for example in Moffitt, 2016, Methods in Enzymology 572, 1-49), and FISSEQ (described for example in U.S. Patent Application Publication No. 2019/0032121) each of which is hereby incorporated herein by reference.

For analytes that have been barcoded via partitioning, barcoded nucleic acid molecules or derivatives thereof (*e.g.,* barcoded nucleic acid molecules to which one or more functional sequences have been added, or from which one or more features have been removed) can be pooled and processed together for subsequent analysis such as sequencing on high throughput sequencers. Processing with pooling can be implemented using barcode sequences. For example, barcoded nucleic acid molecules of a given partition can have the same barcode, which is different from barcodes of other spatial partitions. Alternatively, barcoded nucleic acid molecules of different partitions can be processed separately for subsequent analysis (*e.g.,* sequencing).

In some embodiments, where capture probes do not contain a spatial barcode, the spatial barcode can be added after the capture probe captures analytes from a biological sample and before analysis of the analytes. When a spatial barcode is added after an analyte is captured, the barcode can be added after amplification of the analyte (*e.g.,* reverse transcription and polymerase amplification of RNA). In some embodiments, analyte analysis uses direct sequencing of one or more captured analytes, such as direct sequencing of hybridized RNA. In some embodiments, direct sequencing is performed after reverse transcription of hybridized RNA. In some embodiments direct sequencing is performed after amplification of reverse transcription of hybridized RNA.

In some embodiments, direct sequencing of captured RNA is performed by sequencing-by-synthesis (SBS). In some embodiments, a sequencing primer is complementary to a sequence in one or more of the domains of a capture probe (*e.g.,* functional domain). In such embodiments, sequencing-by-synthesis can include reverse transcription and/or amplification in order to generate a template sequence (*e.g.,* functional domain) from which a primer sequence can bind.

SBS can involve hybridizing an appropriate primer, sometimes referred to as a sequencing primer, with the nucleic acid template to be sequenced, extending the primer, and detecting the nucleotides used to extend the primer. Preferably, the nucleic acid used to extend the primer is detected before a further nucleotide is added to the growing nucleic acid chain, thus allowing base-by-base in situ nucleic acid sequencing. The detection of incorporated nucleotides is facilitated by including one or more labelled nucleotides in the primer extension reaction. To allow the hybridization of an appropriate sequencing primer to the nucleic acid template to be sequenced, the nucleic acid template should normally be in a single stranded form. If the nucleic acid templates making up the nucleic acid spots are present in a double stranded form these can be processed to provide single stranded nucleic acid templates using methods well known in the art, for example by denaturation, cleavage etc. The sequencing primers which are hybridized to the nucleic acid template and used for primer extension are preferably short oligonucleotides, for example, 15 to 25 nucleotides in length. The sequencing primers can be provided in solution or in an immobilized form. Once the sequencing primer has been annealed to the nucleic acid template to be sequenced by subjecting the nucleic acid template and sequencing primer to appropriate conditions, primer extension is carried out, for example using a nucleic acid polymerase and a supply of nucleotides, at least some of which are provided in a labelled form, and conditions suitable for primer extension if a suitable nucleotide is provided.

Preferably after each primer extension step, a washing step is included in order to remove unincorporated nucleotides which can interfere with subsequent steps. Once the primer extension step has been carried out, the nucleic acid colony is monitored to determine whether a labelled nucleotide has been incorporated into an extended primer. The primer extension step can then be repeated to determine the next and subsequent nucleotides incorporated into an extended primer. If the sequence being determined is unknown, the nucleotides applied to a given colony are usually applied in a chosen order which is then repeated throughout the analysis, for example dATP, dTTP, dCTP, dGTP.

SBS techniques which can be used are described for example, but not limited to, those in U.S. Patent Pub. No. 2007/0166705, U.S. Patent 7,566,537, U.S. Patent 7,057,026, U.S. Patent Pub. No. 2006/0240439, U.S. Patent Pub. No. 2006/0281109, PCT Pub. No. WO 05/065814, U.S. Patent Pub. No. 2005/0100900, PCT Pub. No. WO 06/064199, PCT Pub. No. WO07/010,251, U.S. Patent 8,951,781B2, U.S. Patent 9,193,996, and U.S. Patent 9,453,258B2, the entire contents of each of which are incorporated herein by reference.

In some embodiments, direct sequencing of captured RNA is performed by sequential fluorescence hybridization (*e.g.,* sequencing by hybridization). In some embodiments, a hybridization reaction where RNA is hybridized to a capture probe is performed in situ. In some embodiments, captured RNA is not amplified prior to hybridization with a sequencing probe. In some embodiments, RNA is amplified prior to hybridization with sequencing probes (e.g., reverse transcription to cDNA and amplification of cDNA). In some embodiments, amplification is performed using single-molecule hybridization chain reaction. In some embodiments, amplification is performed using rolling chain amplification.

Sequential fluorescence hybridization can involve sequential hybridization of probes including degenerate primer sequences and a detectable label. A degenerate primer sequence is a short oligonucleotide sequence capable of hybridizing to any nucleic acid fragment independent of the sequence of said nucleic acid fragment. For example, such a method could include the steps of: (a) providing a mixture including four probes, each of which includes either A, C, G, or T at the 5'-terminus, further including degenerate nucleotide sequence of 5 to 11 nucleotides in length, and further including a functional domain (e.g., fluorescent molecule) that is distinct for probes with A, C, G, or T at the 5'-terminus; (b) associating the probes of step (a) to the target polynucleotide sequences, whose sequence needs will be determined by this method; (c) measuring the activities of the four functional domains and recording the relative spatial location of the activities; (d) removing the reagents from steps (a)-(b) from the target polynucleotide sequences; and repeating steps (a)-(d) for n cycles, until the nucleotide sequence of the spatial domain for each bead is determined, with modification that the oligonucleotides used in step (a) are complementary to part of the target polynucleotide sequences and the positions 1 through n flanking the part of the sequences. Because the barcode sequences are different, in some embodiments, these additional flanking sequences are degenerate sequences. The fluorescent signal from each spot on the array for cycles 1 through n can be used to determine the sequence of the target polynucleotide sequences.

In some embodiments, direct sequencing of captured RNA using sequential fluorescence hybridization is performed in vitro. In some embodiments, captured RNA is amplified prior to hybridization with a sequencing probe (*e.g.,* reverse transcription to cDNA and amplification of cDNA). In some embodiments, a capture probe containing captured RNA is exposed to the sequencing probe targeting coding regions of RNA. In some embodiments, one or more sequencing probes are targeted to each coding region. In some embodiments, the sequencing probe is designed to hybridize with sequencing reagents (e.g., a dye-labeled readout oligonucleotides). A sequencing probe can then hybridize with sequencing reagents. In some embodiments, output from the sequencing reaction is imaged. In some embodiments, a specific sequence of cDNA is resolved from an image of a sequencing reaction. In some embodiments, reverse transcription of captured RNA is performed prior to hybridization to the sequencing probe. In some embodiments, the sequencing probe is designed to target complementary sequences of the coding regions of RNA (e.g., targeting cDNA).

In some embodiments, a captured RNA is directly sequenced using a nanopore-based method. In some embodiments, direct sequencing is performed using nanopore direct RNA sequencing in which captured RNA is translocated through a nanopore. A nanopore current can be recorded and converted into a base sequence. In some embodiments, captured RNA remains attached to a substrate during nanopore sequencing. In some embodiments, captured RNA is released from the substrate prior to nanopore sequencing. In some embodiments, where the analyte of interest is a protein, direct sequencing of the protein can be performed using nanopore-based methods. Examples of nanopore-based sequencing methods that can be used are described in Deamer et al., 200, Trends Biotechnol. 18, 14 7-151; Deamer et al., 2002, Acc. Chem. Res. 35:817-825; Li et al., 2003, Nat. Mater. 2:611-615; Soni et al., 2007, Clin. Chem. 53, 1996-2001; Healy et al., 2007 Nanomed. 2, 459-481; Cockroft et al., 2008, J. Am. Chem. Soc. 130, 818-820; and in U.S. Patent 7,001,792, each of which hereby is incorporated by reference herein.

In some embodiments, direct sequencing of captured RNA is performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al., 2005, Science 309, 1728-1732, and in U.S. Patent Nos. 5,599,675; 5,750,341; 6,969,488; 6,172,218; and 6,306,597, each of which is hereby incorporated by reference herein.

In some embodiments, nucleic acid hybridization is used for sequencing. These methods utilize labeled nucleic acid decoder probes that are complementary to at least a portion of a barcode sequence. Multiplex decoding can be performed with pools of many different probes with distinguishable labels. Non-limiting examples of nucleic acid hybridization sequencing are described for example in U.S. Pat. No. 8,460,865, and in Gunderson et al., 2004, Genome Research 14:870-877, the entire contents of each of which are incorporated herein by reference.

In some embodiments, commercial high-throughput digital sequencing techniques is used to analyze barcode sequences, in which DNA templates are prepared for sequencing not one at a time, but in a bulk process, and where many sequences are read out preferably in parallel, or alternatively using an ultra-high throughput serial process that itself may be parallelized. Examples of such techniques include Illumina® sequencing (e.g., flow cell-based sequencing techniques), sequencing by synthesis using modified nucleotides (such as commercialized in TruSeq™ and HiSeq™ technology by Illumina, Inc., San Diego, CA), HeliScope™ by Helicos Biosciences Corporation, Cambridge, MA, and PacBio RS by Pacific Biosciences of California, Inc., Menlo Park, CA), sequencing by ion detection technologies (Ion Torrent, Inc., South San Francisco, CA), and sequencing of DNA nanoballs (Complete Genomics, Inc., Mountain View, CA).

In some embodiments, detection of a proton released upon incorporation of a nucleotide into an extension product is used in the methods described herein. For example, the sequencing methods and systems described in U.S. Patent Application Publication Nos. 2009/0026082, 2009/0127589, 2010/0137143, and 2010/0282617, each of which is hereby incorporated by reference, can be used to directly sequence barcodes.

In some embodiments, real-time monitoring of DNA polymerase activity is used during sequencing. For example, nucleotide incorporations can be detected through fluorescence resonance energy transfer (FRET), as described for example in Levene et al., 2003, Science 299, 682-686, Lundquist et al., 2008, Opt. Lett. 33, 1026-1028, and Korlach et al., 2008, Proc. Natl. Acad. Sci. USA 105, 1176-1181. The entire contents of each of the foregoing references are incorporated herein by reference herein.

Referring to block **1028** of **FIG. 10B,** in some embodiments, a plurality of sequence reads for a respective image **1124** comprises 10,000 or more sequence reads, 50,000 or more sequence reads, 100,000 or more sequence reads, or 1 x $10^6$ or more sequence reads.

Referring to block **1030** of **FIG. 10B,** in some embodiments, a plurality of sequence reads for a respective image **1124** include 3'-end or 5'-end paired sequence reads.

Referring to block **1034** of **FIG. 10B,** in some embodiments the one or more analytes (e.g., DNA, RNA) comprises 5 or more analytes, 10 or more analytes, 50 or more analytes, 100 or more analytes, 500 or more analytes, 1000 or more analytes, 2000 or more analytes, or between 2000 and 100,000 analytes. Example analytes are disclosed above in the section entitled I. Introduction (c) Analytes.

Referring to block **1036** of **FIG. 10B,** in some embodiments the one or more analytes is a plurality of analytes. A respective capture probe plurality in the set of capture probe pluralities includes a plurality of capture probes. Each capture probe in the plurality of capture probes includes a capture domain that is characterized by a capture domain type in a plurality of capture domain types. Each respective capture domain type in the plurality of capture domain types is configured to bind to a different analyte in the plurality of analytes. Information on capture domain types is found in above in II. General Spatial Array-Based Analytical Methodology; (b) Capture probes; (ii) Capture domain.

Referring to block **1038** of **FIG. 10B,** in some embodiments, the plurality of capture domain types comprises between 5 and 15,000 capture domain types and the respective capture probe plurality includes at least five, at least 10, at least 100, or at least 1000 capture probes for each capture domain type in the plurality of capture domain types.

Referring to block **1040** of **FIG. 10C,** in some embodiments, each respective capture probe plurality in the set of capture probe pluralities includes 1000 or more, 2000 or more, 10,000 or more, 100,000 or more, 1 x $10^6$ or more, $2 \times 10^6$ or more, or $5 \times 10^6$ or more capture probes.

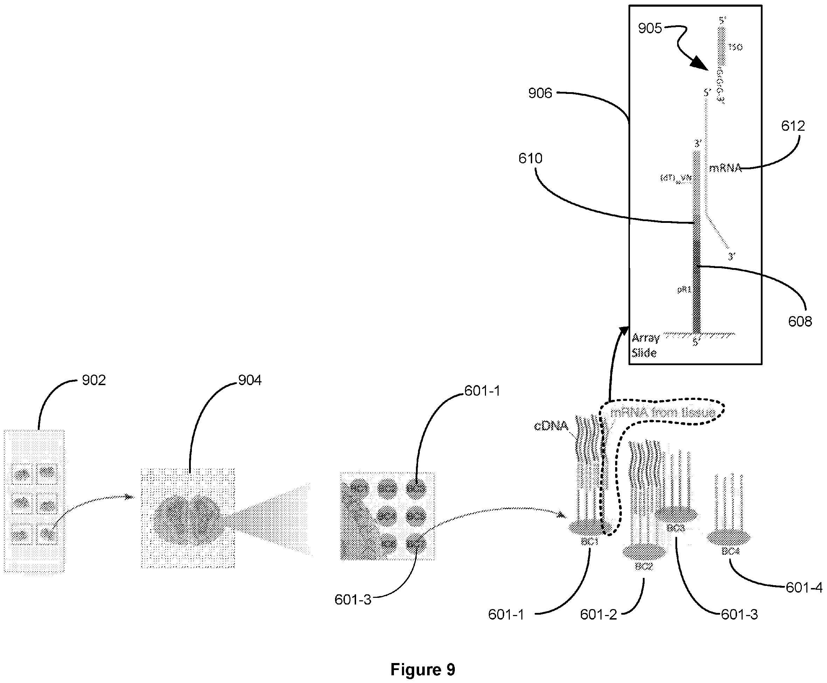

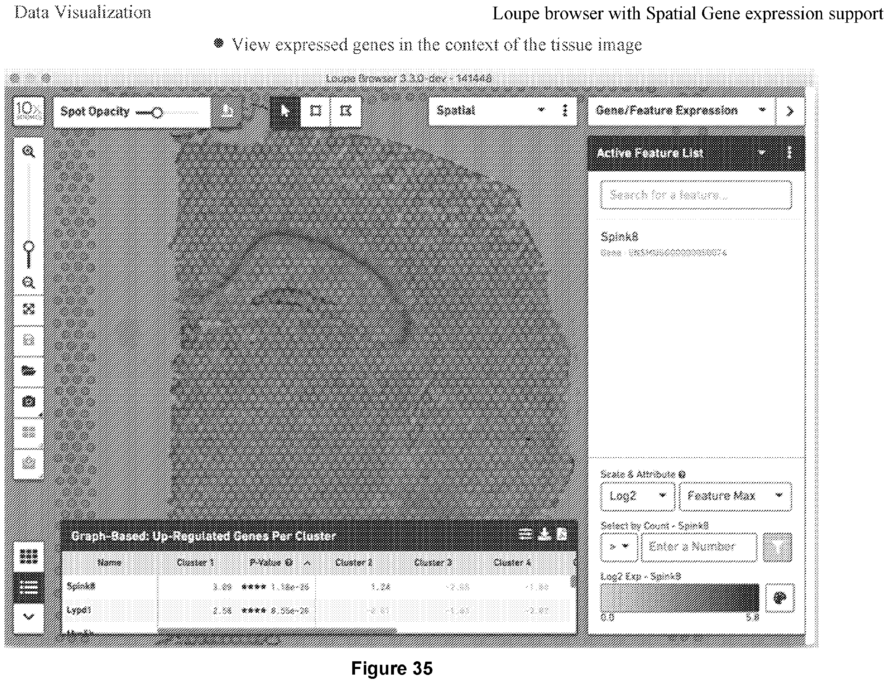

Referring to block **1042** of **FIG. 10C,** in some embodiments each capture probe in the capture probe plurality includes a poly-A or poly-T sequence and a unique spatial barcode that characterizes the different capture spot. Referring to block **1044** of **FIG. 10C,** in some embodiments each capture probe in the capture probe plurality includes the same spatial barcode from the plurality of spatial barcodes. Referring to block **1046** of **FIG. 10C,** in some embodiments each capture probe in the capture probe plurality includes a different spatial barcode from the plurality of spatial barcodes. For instance, as illustrated in **FIG. 9,** a substrate (microscopic slide **902)** containing marked capture areas (*e.g.*, 6.5 × 6.5 mm) **904** are used where thin tissue sections of a biological sample are placed and imaged to form images. Each capture area **904** contains a number (*e.g.*, 5000 printed regions) of barcoded mRNA capture probes, each such region referred to herein as a capture spot **601** with dimensions of 100 $\mu$m or less (*e.g.*, 55 $\mu$m in diameter and a center-to-center distance of 200 $\mu$m or less (e.g., 100 $\mu$m). Tissue is permeabilized and mRNAs are hybridized to the barcoded capture probes **905** directly underneath. As shown in more detail in panel **906,** for a particular capture probe **605,** cDNA synthesis connects the spatial barcode **608** and the captured mRNA **608,** and UMI counts from analysis of sequence reads, are later overlaid with the tissue image as illustrated in **FIG. 35.** In **FIG. 35,** for each respective capture spot, the corresponding UMI counts, in $\log_2$ space, mapping onto the gene Spink8 are overlaid on the image. Returning to **FIG. 9,** for each respective capture spot 601, there are thousands of capture probes **605,** with each respective capture probe **605** containing the spatial barcode **608** corresponding to the respective capture spot **601,** and a unique UMI identifier **610.** The mRNA **612** from the tissue sample binds to the capture probe **605** and the mRNA sequence, along with the UMI **610** and spatial barcode **608** are copied in cDNA copies thereby ensuring that the spatial location of the mRNA within the tissue is captured at the level of capture spot **601** resolution. More details on capture probes, including spatial barcodes and unique molecular identifiers, is disclosed in United States Provisional Patent Application No. 62/980,073, entitled "Pipeline for Analysis of Analytes," filed February 21, 2020, attorney docket number 104371-5033-PR01, which is hereby incorporated by reference.

Referring to block **1048** of **FIG. 10C,** in some embodiments the one or more analytes is a plurality of analytes. A respective capture spot in the set of capture spots includes a plurality of capture probes **601,** each including a capture domain **905** that is characterized by a single capture domain type configured to bind to each analyte in the plurality of analytes in an unbiased manner. Thus, in some such embodiments, the capture domain comprises a non-specific capture moiety (*e.g.*, an oligo-dT binding moiety).

Referring to block **1050** of **FIG. 10C,** in some embodiments a capture probe plurality in the set of capture probe pluralities does not comprise a cleavage domain and each capture probe in the capture probe plurality is not cleaved from the substrate.

Referring to block **1052** of **FIG. 10C,** in some embodiments each respective capture probe plurality in the set of capture probe pluralities is attached directly or attached indirectly to the substrate. Examples of how a capture probe **905** can be attached to a substrate are disclosed II. General spatial array-based methodology.

Referring to block **1054** of **FIG. 10C,** in some embodiments, the one or more analytes is a plurality of analytes. A respective capture probe plurality in the set of capture probe pluralities includes a plurality of probes. Each capture probe in the plurality of capture probes includes a capture domain that is characterized by a single capture domain type configured to bind to each analyte in the plurality of analytes in an unbiased manner.

Referring to block **1056** of **FIG. 10D,** in some embodiments, each respective capture probe plurality in the set of capture probe pluralities is characterized by at least one unique spatial barcode in a plurality of spatial barcodes. The plurality of sequence reads comprises sequence reads that correspond to all or portions of the one or more analytes. Each respective sequence read in the plurality of sequence reads includes a spatial barcode of the corresponding capture probe plurality in the set of capture probe pluralities. For instance, in some embodiments, the analytes are proteins and a sequence read can correspond to a tag as a proxy for the analyte. In other embodiments, the analytes are nucleic acids and the sequence reads comprise all or portions of such nucleic acids.

Referring to block **1058** of **FIG. 10D,** in some embodiments, the unique spatial barcode in the respective sequence read is localized to a contiguous set of oligonucleotides within the respective sequence read. For instance referring to block **1060** of **FIG. 10D,** in some embodiments, the contiguous set of oligonucleotides is an N-mer, where N is an integer selected from the set {4, ..., 20}.

Referring to block **1062** of **FIG. 10D,** in some embodiments, the unique spatial barcode encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x $10^{12}$}. Examples of spatial barcodes are disclosed in II. General spatial array-based methodology; (b) Capture probes; (iv) Spatial barcodes.

In some embodiments, the plurality of spatial barcodes is used to localize respective sequence reads in the plurality of sequence reads to corresponding capture spots in the set of capture spots, thereby dividing a plurality of sequence reads of a respective image **1124** into a plurality of subsets of sequence reads. Each respective subset of sequence reads corresponds to a different capture spot in the plurality of capture spots. Examples on how spatial barcodes can be used to localize sequence reads to specific capture probes is disclosed in II. General spatial array-based methodology; (b) Capture probes; (iv) spatial barcode. See *also,* United States Provisional Patent Application No. 62/839,346 entitled "Spatial Transcriptomics of Biological Analytes in Tissue Samples," filed April 26, 2019, which is hereby incorporated by reference.

Referring to block **1066** of **FIG. 10D,** the plurality of fiducial markers is used to provide a composite representation comprising (i) one or more images **1124** aligned to the set of capture spots on the substrate and (ii) a representation of each subset of sequence reads at a respective position within each of the one or more images that maps to the corresponding capture spot on the substrate.

Referring to block **1068 of FIG. 10E,** in some embodiments, the composite representation provides a relative abundance of nucleic acid fragments (number of unique UMI from a capture spot) mapping to each gene in a plurality of genes at each capture spot in the plurality of capture spots. For example, **FIG. 35** illustrates a composite representation of the relative abundance (*e.g.*, expression) of a particular gene in the context of the capture spots. See *also,* United States Provisional Application No. 62/909,071, entitled "Systems and Methods for Visualizing a Pattern in a Dataset," filed October 1, 2019, which is hereby incorporated by reference, for additional illustrations of composite representations of the relative abundance of nucleic acid fragments mapping to each gene in a plurality of genes at each capture spot in the plurality of capture spots.

Referring to block **1070 of FIG. 10E,** in some embodiments, an image **1124** is aligned to the set of capture spots **1136** on a substrate by a procedure that comprises analyzing the array of pixel values **1126** to identify a plurality of derived fiducial spots **1130** of the respective image, using a substrate identifier **1128** (*e.g.*, a serial number, hologram, tracking code, image, color, graphic) uniquely associated with the substrate to select a template **1142** in a plurality of templates, where each template in the plurality of templates comprises reference positions **1148** for a corresponding plurality of reference fiducial spots **1146** and a corresponding coordinate system **1144.** The plurality of derived fiducial spots **1130** of the respective image **1124** are aligned with the corresponding plurality of reference fiducial spots **1146** of the selected template **1142** using an alignment algorithm to obtain a transformation between the plurality of derived fiducial spots **1130** of the respective image **1124** and the corresponding plurality of reference fiducial spots **1146** of the selected template **1142.** The transformation and the coordinate system of the selected template **1142** is then used to locate a corresponding position in the respective image of each capture spot in the set of capture spots.

With reference to the procedure of **block 1070,** the substrate that is imaged includes a plurality of fiducial markers. Fiducial markers are described in further detail in II. General spatial array-based analytical methodology; (c) Substrate and (e) Analyte capture; (v) Region of interest. Briefly, in some embodiments, fiducial markers are included on the substrate as one or more markings on the surface of the substrate of the chip. In some embodiments, fiducial markers serve as guides for correlating spatial information with the characterization of the analytes of interest. In some embodiments, fiducial markers are prepared on the substrate using any one of the following non-limiting techniques: chrome-deposition on glass, gold nanoparticles, laser-etching, tubewriter-ink, microspheres, Epson 802, HP 65 Black XL, permanent marker, fluorescent oligos, amine iron oxide nanoparticles, amine thulium doped upconversion nanophosphors, and/or amine Cd-based quantum dots. Other techniques for fiducial marker preparation include sand-blasting, printing, depositing, or physical modification of the substrate surface.

In some embodiments, the fiducial markers are non-transiently attached to the outer boundary of the substrate (*e.g.*, the outerboundry of the capture area **1206** illustrated in **FIG. 12)** and the biological sample is within the boundary of the fiducial markers. In some embodiments, the fiducial markers are transiently attached to the outer boundary of the substrate (*e.g.*, by attachment of an adaptor, a slide holder, and/or a cover slip). In some embodiments, the fiducial markers are transiently attached to the outer boundary of the substrate before or after the biological sample is on the substrate. In some embodiments, the fiducial markers are transiently or non-transiently attached to the substrate after the sample is on the substrate but prior to obtaining the image.

**FIG. 12** illustrates an image of a tissue **1204** on a substrate, where the image includes a plurality of fiducial markers, in accordance with some embodiments. The fiducial markers are arranged along the external border of the substrate, surrounding the capture spot array and the tissue. In some such embodiments, the fiducial markers comprise patterned spots, and the patterned spots indicate the edges and corners of the capture spot array. In some such embodiments, a different pattern of fiducial markers is provided at each corner, allowing the image to be correlated with spatial information using any orientation (*e.g.*, rotated and/or mirror image).

The array of pixel values are analyzed to identify a plurality of derived fiducial spots **1130** of the image. In some embodiments, this is performed by identifying a plurality of candidate derived fiducial spots within the image by thresholding the array of pixel values within the image with a plurality of different threshold values thereby achieving a plurality of threshold images and identifying, within the plurality of threshold images, groups of pixels having white values. In one such embodiment, for one such threshold value T, each respective $pixel_{i,j}$ in the image is replaced with a black pixel if the respective $pixel_{i,j}$ intensity is less than the threshold value ($Ii,j < T$), or a white pixel if the respective $pixel_{i,j}$ intensity is greater than the threshold value ($Ii,j > T$). In some embodiments, the value for the threshold is selected automatically using the image. See for example, Sezgin and Sankur, 2004, "Survey over image thresholding techniques and quantitative performance evaluation," Journal of Electronic Imaging 13(1), 146-165 for disclosure on methods for thresholding, including selecting suitable thresholding values, and types of thresholding including histogram shape-based methods. As disclosed in Sezgin and Sankur, *Id.,* suitable thresholding methods include, but are not limited to, histogram shape-base thresholding methods where, for example, the peaks, valleys and curvatures of the smoothed histogram are analyzed. Suitable thresholding methods also include clustering-based methods where gray-level samples are clustered in two parts as background and foreground (object), or alternately are modeled as a mixture of two Gaussians.

Suitable thresholding methods also include entropy-based methods that use the entropy of the foreground and background regions, the cross-entropy between the original and binarized image, *etc*. *See*, for example, Zhang, 2011, "Optimal multi-level Thresholding based on Maximum Tsallis Entropy via an Artificial Bee Colony Approach," Entropy 13(4): pp. 841-859, which is hereby incorporated by reference. Suitable thresholding methods further include object attribute-based thresholding methods that search for a measure of similarity between the gray-level and the binarized images, such as fuzzy shape similarity, edge coincidence, *etc*. Suitable thresholding methods further include spatial methods that use higher-order probability distribution and/or correlation between pixels.

Suitable thresholding methods further include local methods that adapt the threshold value on each pixel to the local image characteristics. In such local thresholding methods, a different T is selected for each pixel in the image.

Thus as the above disclosed, in some embodiments several different values of T are used to threshold an image whereas in other embodiments a single T is used to threshold an image. The net result of the thresholding is the identification of plurality of candidate derived fiducial spots. Under classical thresholding, these candidate derived fiducial spots are groups of white pixels. However, the present disclosure is not so limited and one of skill in the art will fully appreciate that white and black can be reversed, such that the candidate derived fiducial spots are groups of black pixels. However, for the ease of describing the workflow, the candidate derived fiducial spots will be considered groups of white pixels identified by the thresholding.

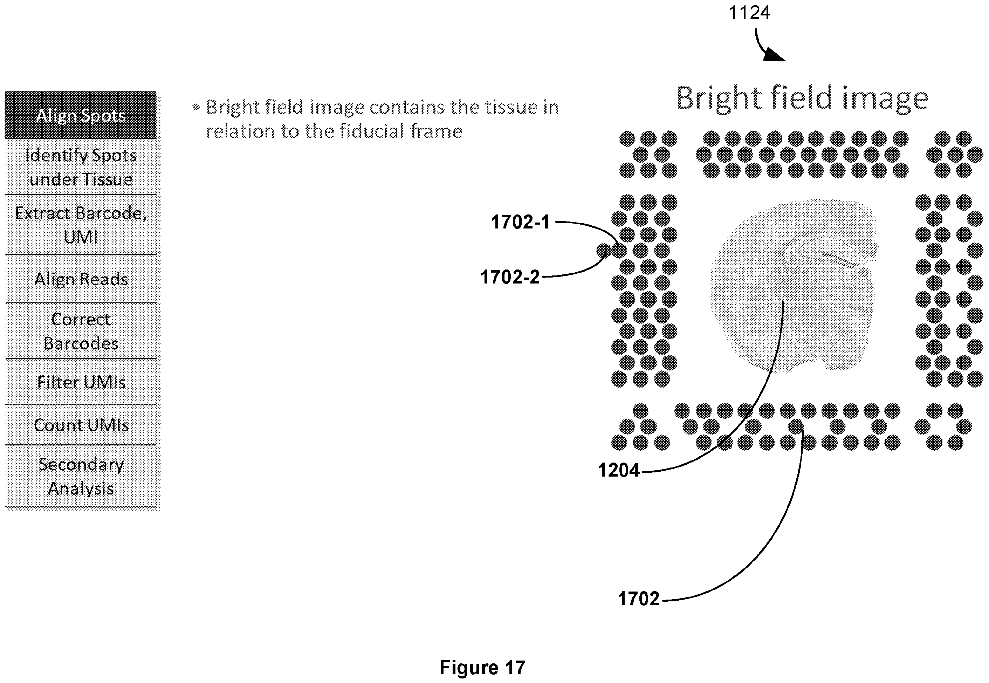

**FIG 17** illustrates an image **1124** that includes the biological sample **1204** and a plurality of candidate derived fiducial spots **1702** on the perimeter of the image. In some embodiments, there are between 5 and 1000 candidate derived fiducial spots **1702,** between 5 and 500 candidate derived fiducial spots **1702,** or between 5 and 300 candidate derived fiducial spots **1702.**

The plurality of candidate derived fiducial spots are clustered based on spot size, thereby distributing the plurality of candidate derived fiducial spots into a plurality of subsets of candidate derived fiducial spots.

Clustering is described at pages 211-256 of Duda and Hart, Pattern Classification and Scene Analysis, 1973, John Wiley & Sons, Inc., New York, (hereinafter "Duda 1973") which is hereby incorporated by reference in its entirety. As described in Section 6.7 of Duda 1973, the clustering problem is one of finding natural groupings in a dataset. To identify natural groupings, two issues are addressed. First, a way to measure similarity (or dissimilarity) between two samples is determined. This metric (*e.g.*, similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar." An example of a nonmetric similarity function s(x, x') is provided on page 218 of Duda 1973. Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering that may be used in accordance with block **1046** of **FIG. 10C** in detail. More information on suitable clustering techniques is found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, New Jersey, each of which is hereby incorporated by reference. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering where no preconceived notion of what clusters should form when the training set is clustered are imposed.

In some embodiments, the plurality of candidate derived fiducial spots are clustered into two, three, four, five, six, seven, eight, nine, ten, eleven, twelve, thirteen, fourteen, fifteen, sixteen, seventeen, eighteen, nineteen, or twenty subsets. In some embodiments, the candidate derived fiducial spots are clustered into between two and 100 subsets. Each respective subset of candidate derived fiducial spots in the plurality of subsets of candidate derived fiducial spots has a characteristic size. For instance, in some embodiments, the characteristic size is the average number of pixels in each candidate derived fiducial spot in the respective subset. The subset of candidate derived fiducial spots in the plurality of subsets of candidate derived fiducial spots that has the largest characteristic size is selected as the plurality of derived fiducial spots of the image. For instance, consider the case where the plurality of candidate derived fiducial spots are clustered into two subsets, subset A and subset B, and the average size of the candidate derived fiducial spots in subset A is 49 pixels and the average size of the candidate derived fiducial spots in subset B is 58 pixels. In this instance, the candidate derived fiducial spots in subset B would be chosen as the derived fiducial spots of the image and the candidate derived fiducial spots in subset A would be discarded as noise.

With further reference to **FIG. 17,** in some embodiments, respective pairs of candidate derived fiducial spots that are within a threshold distance of each other are merged. In some embodiments, this threshold distance is a threshold number of pixels, such as one pixel, two pixels, three pixels, four pixels, five pixels, six pixels, seven pixels, eight pixels, nine pixels, ten pixels, twenty pixels, etc. In some embodiments, this threshold distance is a threshold distance between spot centers. For instance, in some embodiments, a respective pair of candidate derived fiducial spots whose centers that are within 1 $\mu$M, within 2 $\mu$M, within 3 $\mu$M, within 4 $\mu$M, within 5 $\mu$M, within 10 $\mu$M or within 20 $\mu$M of each other are merged. In some embodiments, the resultant merged candidate derived fiducial spot is taken midway between the original pair of candidate derived fiducial spots that is merged. In **Fig. 17,** the respective pair of candidate derived fiducial spots 1702-1 / 1702-2 is merged because they fail a distance threshold. In some embodiments, the threshold distance filter is applied to candidate derived fiducial spots. In alternative embodiments, the threshold distance filter is not applied to candidate derived fiducial spots but rather is applied to derived fiducial spots after completion of block **1046.**

In some embodiments respective candidate derived fiducial spots that fail to satisfy a maximum or minimum size criterion are filtered out. In some embodiments, this size filter is applied to candidate derived fiducial spots. In alternative embodiments, this size filter is not applied to candidate derived fiducial spots but rather is applied to derived fiducial spots after completion of block **1046.** In some embodiments, application of this size filter causes respective candidate derived fiducial spots having less than 200 pixels, 150 pixels, 100 pixels, 50 pixels, 40 pixels, 35 pixels, 30 pixels, 25 pixels, 20 pixels, 18 pixels, 16 pixels, 14 pixels, 12 pixels, 10 pixels, 9 pixels, 8 pixels, 7 pixels, 6 pixels, 5 pixels, or 4 pixels or less to be discarded. In some embodiments, application of this size filter causes respective candidate derived fiducial spots having more than 200 pixels, 150 pixels, 100 pixels, 50 pixels, 40 pixels, 35 pixels, 30 pixels, 25 pixels, 20 pixels, 18 pixels, 16 pixels, 14 pixels, 12 pixels, or 10 pixels to be discarded.

In some embodiments respective candidate derived fiducial spots that fail to satisfy a circularity criterion are filtered out. In some embodiments, this circularity filter is applied to candidate derived fiducial spots. In alternative embodiments, this circularity is not applied to candidate derived fiducial spots but rather is applied to derived fiducial spots after completion of block **1046.** In some such embodiments, the circularity of a respective derived fiducial spot is defined by:

$$circularity = \frac{4\pi Area}{(perimeter)^2}$$

where, "Area" is the area of the respective derived fiducial spot, and "perimeter" is the perimeter of the respective derived fiducial spot. Thus, in such embodiments, when this circularity criterion falls outside a suitable range, the respective candidate derived fiducial spot is deemed to not be circular, and thus not possibly representative of a true fiducial spot on the substrate, which in some embodiments are printed such that they are circular. In some embodiments, the circularity of each respective candidate derived fiducial spot is determined using a single-trace method for roundness determination. In some embodiments, the circularity of each respective candidate derived fiducial spot is determined using a multiple-trace method for roundness determination.

In some embodiments, the circularity of each respective candidate derived fiducial spot is determined using a least squares reference circle (LSCI) approach in which reference circle is fitted to the respective candidate derived fiducial spot such that the sum of the squares of the departure of the respective candidate derived fiducial spot from that reference circle is a minimum. Out-of-roundness is then expressed in terms of the maximum departure of the profile from the LSCI, i.e. the highest peak to the lowest valley. In such embodiments, when the out-of-roundness exceeds an acceptable threshold value, the respective candidate derived fiducial spot is discarded. In other embodiments, roundness is measured using a minimum circumcised circle method, minimum zone circle method. See, for example, Petrick et al., 2009, Measurement 2009, Proceedings of the 7th International Conference, Smolenice, Slovakia, pp. 352-355 which is hereby incorporated by reference. The exact threshold used to discard respective candidate derived fiducial spots (or candidate derived fiducial spots) using any of the disclosed methods for calculating circularity, or any method for calculating eccentricity known in the art, is application dependent and, in many instances, is dynamically optimized for a given dataset.

Referring to block **1054 of FIG. 10D,** in some embodiments, respective candidate derived fiducial spots that fail to satisfy a convexity criterion are discarded. In some embodiments, this convexity filter is applied to candidate derived fiducial spots. In alternative embodiments, this convexity filter is not applied to candidate derived fiducial spots but rather is applied to derived fiducial spots after completion of block **1046.** In some embodiments, the convexity filter requires that each respective candidate derived fiducial spot fall into a range between a minimum convexity (less than or equal to one) and a maximum convexity. In some embodiments, the convexity of a respective candidate derived fiducial spot is calculated by the formula:

$$\text{convexity} = \frac{Area}{Area\ of\ Convex\ Hull}$$

where, *"Area"* is the area of the respective candidate derived fiducial spot, and *"Area* of *Convex Hull"* is the area of the convex hull of the respective derived fiducial spot. See Andrew, 1979, "Another efficient algorithm for convex hulls in two dimensions," Information Processing Letters 9 (5), pp. 216-219; and Brown, 1979, "Voronoi diagrams from convex hulls," Information Processing Letters 9(5), pp. 223-228 for calculation of convex hulls. For more information on calculating convexity generally, see Emerging Technology in Modeling and Graphics: Processing of IEM Graph 2018, Jyotsna Kumar Mandal, Debika ed., which is hereby incorporated by reference. In some embodiments, the convexity filter requires that each respective candidate derived fiducial spot fall into a range between a minimum convexity of 0.10, 0.15, 0.20, 0.25, 0.30, 0.35, or 0.45 and a maximum convexity of 0.95, 0.90, 0.85, 0.80, 0.75, 0.70, 0.65, or 0.60.

In some embodiments, respective candidate derived fiducial spots that fail to satisfy an inertia ratio criterion are discarded. In some embodiments, this inertia ratio filter is applied to candidate derived fiducial spots. In alternative embodiments, this inertia ratio filter is not applied to candidate derived fiducial spots but rather is applied to derived fiducial spots. In some embodiments, the inertia ratio filter requires that each respective candidate derived fiducial spot fall into a range between a minimum inertia (less than or equal to one) and a maximum inertia. For more information on calculating inertia generally, see Emerging Technology in Modeling and Graphics: Processing of IEM Graph 2018, Springer Singapore, Jyotsna Kumar Mandal, Debika eds., which is hereby incorporated by reference. In some embodiments, the inertia filter requires that each respective candidate derived fiducial spot fall into a range between a minimum inertia of 0.40, 0.45, 0.50, 0.55, 0.60, 0.65, or 0.70 and a maximum inertia of 1 (full circle).

In some embodiments the substrate identifier **1128** of the substrate is used to select a template 1142 in a plurality of templates (*e.g.*, from a remote computer system, from among the plurality of templates, responsive to sending the substrate identifier to the remote computer system). In other words, the substrate identifier **1128** of the substrate that is presently being analyzed is used to identify a template that has a matching substrate identifier. For instance, referring to **FIG. 11B,** in some embodiments, the plurality of templates is found in a template repository **1140.** Each template **1142** in the plurality of templates includes at least one substrate identifier **1128** that it can be used for and comprises reference positions **1148** (coordinates) for a corresponding plurality of reference fiducial spots **1146** and a corresponding coordinate system **1144.** In some embodiments, the coordinate system is inferred from the coordinates **1148.** In some embodiments, the coordinate system **1144** comprises the location (coordinates) of capture spots **1136** on the substrate that has a substrate identifier **1128** that matches the substrate identifier of the template **1142.**

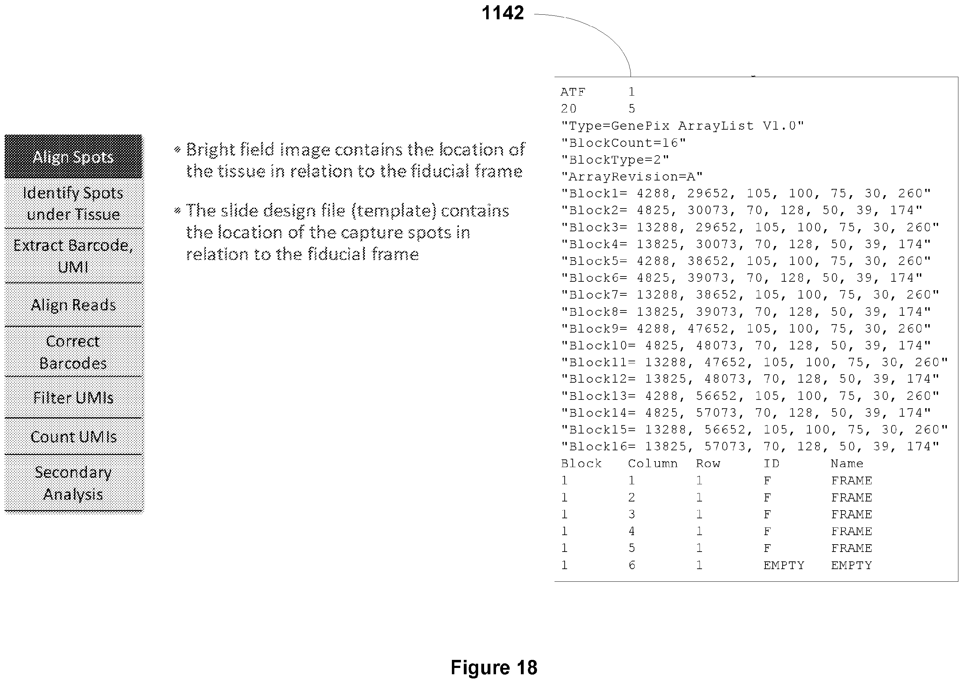

In some embodiments, a template **1142** is formed from a substrate printing instruction file (*e.g.*, a GenePix Array List (GAL) file) that specifies how to print the array capture spots **1136** on the substrate. In some such embodiments, the substrate printing instruction file is analyzed to create a template **1142** for each substrate and this template is provided when the matching substrate identifier **1128** is provided. For information on example substrate printing instruction files, see Zhai, 2001, "Making GenePix Array List (GAL) Files," GenePix Application Note, Molecular Devices, pp. 1-9, which is hereby incorporated by reference. **FIG. 18** illustrates an example of the formation of a template **1142** from a GAL file.

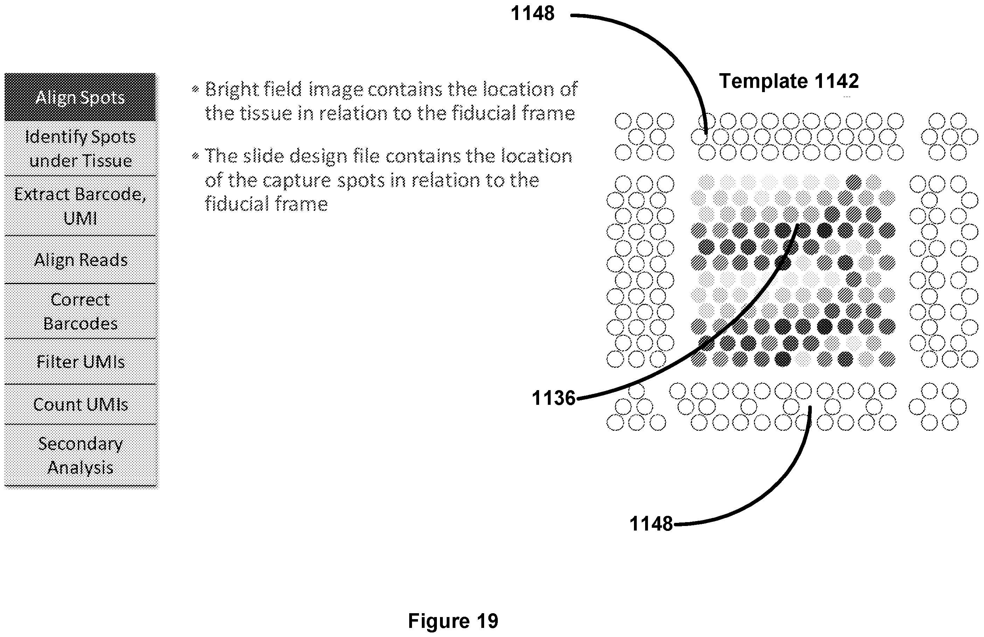

In some embodiments, the corresponding plurality of reference fiducial spots **1146** of the selected template **1142** consists of between 100 fiducial spots and 1000 fiducial spots, between 200 fiducial spots and 800 fiducial spots, between 300 fiducial spots and 700 fiducial spots or between 500 and 600 fiducial spots. That is, the template **1142** has between 100 fiducial spots and 1000 fiducial spots because that is how many fiducial spots are on the substrate that corresponds to the template. In some embodiments, the template **1142** and the corresponding subsrate have less than 100 fiducial spots, less than 50 fiducial spots or less than 25 fiducial spots. In some embodiments, the template **1142** and the corresponding substrate have more than 1000 fiducial spots, more than 1500 fiducial spots or more than 3000 fiducial spots. **FIG. 19** illustrates the positions of fiducial spots at the perimeter of the substrate. As further illustrated in **FIG. 19,** the substrate also includes capture spots **1136** and the coordinate system **1144** of the template **1142** specifies the location of these capture spots on the substrate and, in some embodiments, precisely which capture probes have been printed at each capture spot. In some embodiments, each capture spot has been printed with the same capture probes. In other embodiments, each capture spot is printed with an independent set of capture probes and the template **1142** tracks not only the position on the substrate of each respective capture spot, but also the independent set of capture probes that have been printed on the respective capture spot. In some embodiments, the coordinate system **1144** provides an explicit location of each capture spot **1136** on the substrate. In some embodiments, the coordinate system **1144** provides an orientation of the substrate relative to the fiducial spots and the orientation is used to reference a list of capture spot locations in a data source that is external to the template **1142.** One of skill in the art will appreciate that there are a number of ways to implement the template coordinate system **1144** based on the present disclosure (*e.g.*, as an explicit list of capture spot locations, as an orientation derived from the fiducial spots coupled with an external list of capture spot locations, *etc.*) and all such methods are encompassed by the present disclosure.

In accordance with block **1070 of FIG. 10E,** the plurality of derived fiducial spots **1130** of the image **1124** is aligned with the corresponding plurality of reference fiducial spots **1146** of the first template **1142** using an alignment algorithm to obtain a transformation between the plurality of derived fiducial spots **1130** of the image **1124** and the corresponding plurality of reference fiducial spots **1146** of the first template **1142.** This is a point set registration problem, the goal of which is to assign correspondences between two sets of points (the plurality of derived fiducial spots **1130** of the image **1124** and the plurality of reference fiducial spots **1146** of the template **1142)** and/or to recover the transformation that maps one point set to the other. In some embodiments, in order to determine which of the eight possible orientations a substrate is in (four 90 degree rotations plus reflection), all eight orientations are concurrently run and the orientation with the lowest residual error is chosen, as long as the second lowest residual error is significantly higher.

In some embodiments, the transformation between the plurality of derived fiducial spots **1130** of the image **1124** and the corresponding plurality of reference fiducial spots **1146** of the template **1142** is a rigid transform. A rigid transformation allows only for translation and rotation. Thus, when a rigid transformation is used, the plurality of derived fiducial spots **1130** of the image **1124** are rotated and/or translated to minimize a residual error between the plurality of derived fiducial spots **1130** and the corresponding plurality of reference fiducial spots **1146.**

In some embodiments, the transformation between the plurality of derived fiducial spots **1130** of the image **1124** and the corresponding plurality of reference fiducial spots **1146** of the template **1142** is a similarity transform. A similarity transformation allows for translation, rotation and isotropic (equal-along-each-axis) scaling. Thus, when a similarity transform is used, the plurality of derived fiducial spots **1130** of the image **1124** are rotated, translated, and/or isotropically scaled to minimize a residual error between the plurality of derived fiducial spots **1130** and the corresponding plurality of reference fiducial spots **1146.**

In some embodiments, the transformation is a non-rigid transform that comprises anisotropic scaling and skewing of the plurality of derived fiducial spots **1130** of the image **1124** to minimize a residual error between the plurality of derived fiducial spots **1130** and the corresponding plurality of reference fiducial spots **1146.** In some embodiments the non-rigid transform is an affline transformation. In some embodiments the alignment algorithm is a coherent point drift algorithm. See Myronenko et al., 2007, "Non-rigid point set registration: Coherent Point Drift," NIPS, 1009-1016; and Myronenko and Song, "Point Set Registration: Coherent Point Drift," arXiv:0905.2635v1, 15 May 2009, each of which is hereby incorporated by reference, for disclosure on the coherent point drift algorithm. In some embodiments, the coherent point drift algorithm that is used is an implementation in Python called pycpd." See, the Internet at github.com/siavashk/-pycpd, which is hereby incorporated by reference.

In some embodiments the alignment algorithm is an iterative closest point algorithm. See, *for example,* Chetverikov et al., 2002, "the Trimmed Iterative Closest Point Algorithm," Object recognition supported by user interaction for service robots, Quebec City, Quebec, Canada, ISSN: 1051-4651; and Chetverikov et al., 2005, "Robust Euclidean alignment of 3D point sets; the trimmed iterative closest point algorithm," Image and Vision Computing 23(3), pp. 299-309, each of which is hereby incorporated by reference.

In some embodiments the alignment algorithm is a robust point matching algorithm (See, *for example,* Chui and Rangarajanb, 2003, "A new point matching algorithm for non-rigid registration," Computer Vision and Image Understanding 89(2-3), pp. 114-141, which is hereby incorporated by reference) or a thin-plate-spline robust point matching algorithm (See, *for example,* Yang, 2011, "The thin plate spline robust point matching (TPS-RPM) algorithm: A revisit," Pattern Recognition Letters 32(7), pp. 910-918, which is hereby incorporated by reference.)

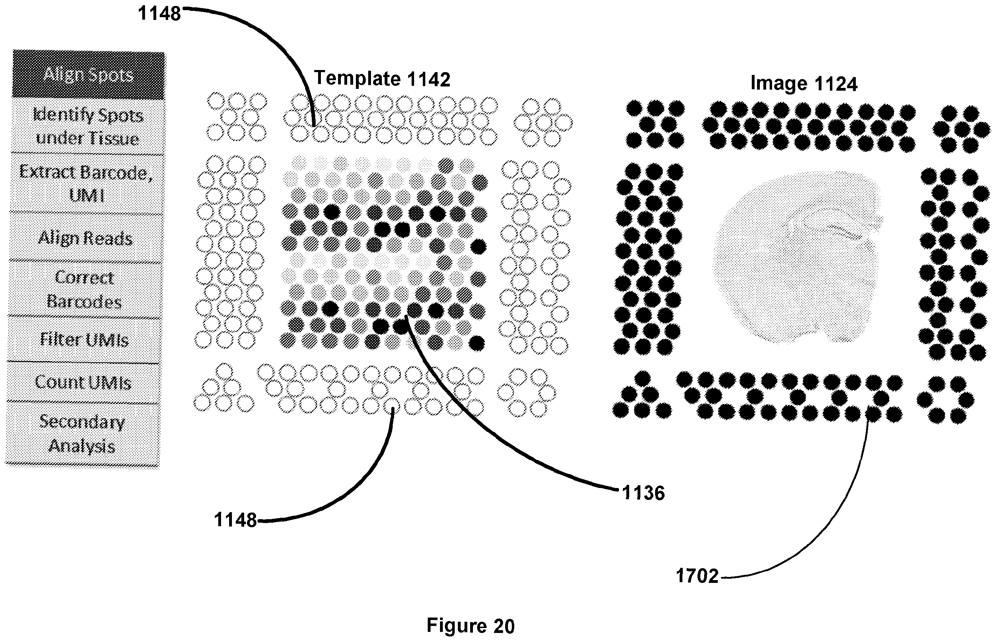

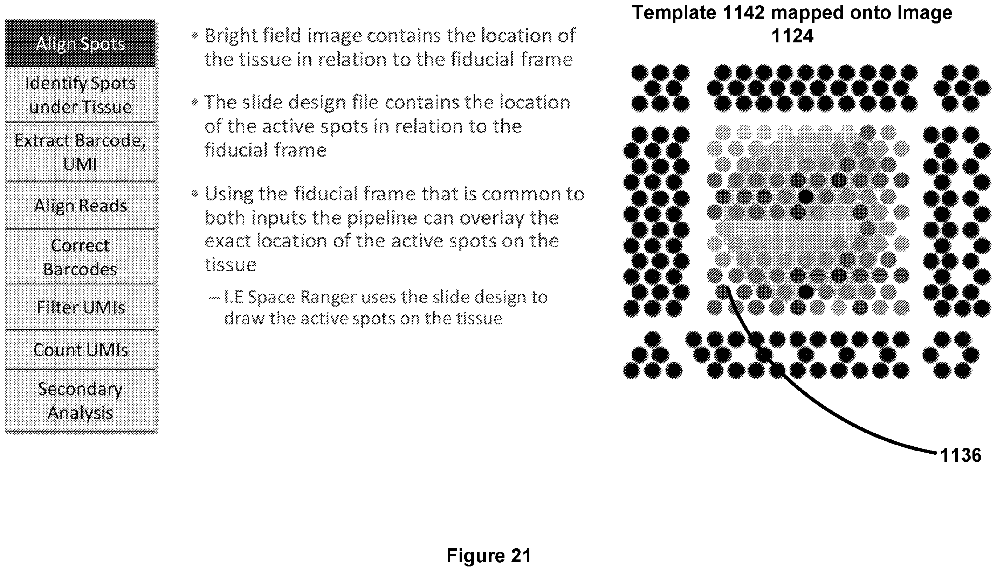

In accordance with block **1070** of **FIG. 10E,** the transformation and the coordinate system **1144 of** the corresponding template **1142** is used to register the image **1124** to the set of capture spots **1136. FIGS. 20** and **21** illustrate. In **FIG. 20,** the alignment causes the transformation that maps the substrate derived fiducial spots **1130** of the image onto the fiducial spots **1148** of the template **1142.** Upon such a mapping, as illustrated in **FIG. 21**, it is now possible to determine the location of each capture spot **1136** in the image **1124.** In other words, the transformation and the coordinate system of the first template can now be used to locate a corresponding position in the image of each capture spot in the set of capture spots.

Referring to block **1072** of **FIG. 10E,** in some embodiments the using the transformation and the coordinate system of the first template to locate and measure the one or more optical properties of each capture spot in the set of capture spots comprises assigning each respective pixel in the plurality of pixels to a first class or a second class, where the first class indicates the biological sample on the substrate and the second class indicates background, by a procedure that comprises: (i) using the plurality of fiducial markers to define a bounding box within the image, (ii) removing respective pixels falling outside the bounding box from the plurality of pixels, (iii) running, after the removing (ii), a plurality of heuristic classifiers on the plurality of pixels (*e.g.*, in color space or grey-scale space), where, for each respective pixel in the plurality of pixels, each respective heuristic classifier in the plurality of heuristic classifiers casts a vote for the respective pixel between the first class and the second class, thereby forming a corresponding aggregated score for each respective pixel in the plurality of pixels, and (iv) applying the aggregated score and intensity of each respective pixel in the plurality of pixels to a segmentation algorithm, such as graph cut, to independently assign a probability to each respective pixel in the plurality of pixels of being tissue or background.

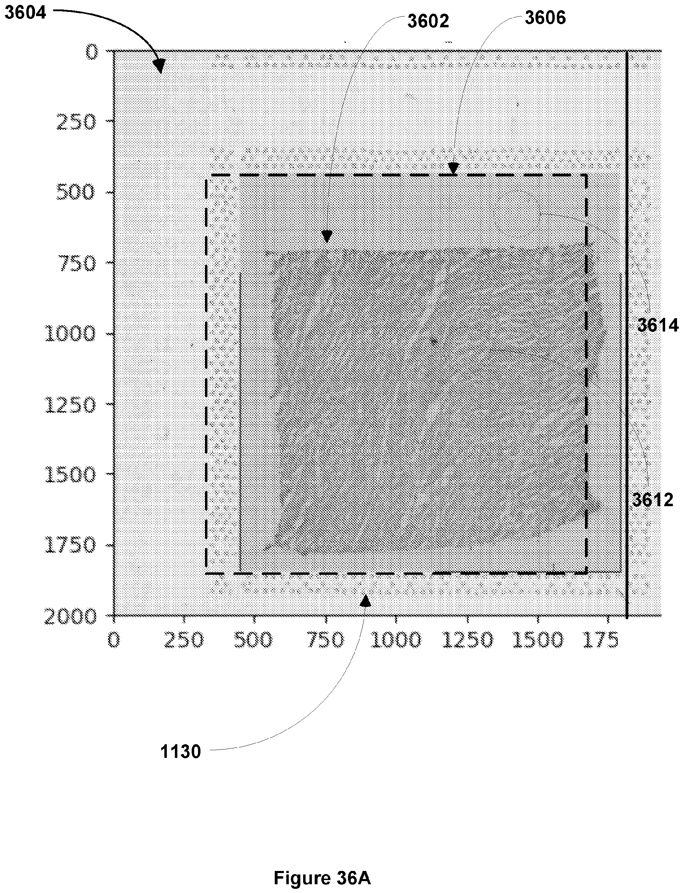

In accordance with block **1072** of **FIG. 10E** and with further reference to **FIG. 36A,** in some embodiments, each respective pixel in the plurality of pixels of the image is assigned to a first class or a second class. The first class indicates the tissue sample **3602** on the substrate **3604** and the second class indicates background (meaning no tissue sample **3602** on the substrate). Thus, for instance, in **FIG. 36A,** most of the pixels within example region **3612** should be assigned the first class and the pixels in example region **3614** should be assigned the second class. In some embodiments, the assigning of each respective pixel as tissue (first class) or background (second class) provides information as to the regions of interest, such that any subsequent spatial analysis of the image (*e.g.*, in accordance with block 1070 above) can be accurately performed using capture spots and/or analytes that correspond to tissue rather than to background. For example, in some instances, obtained images include imaging artifacts including but not limited to debris, background staining, holes or gaps in the tissue section, and/or air bubbles (*e.g.*, under a cover slip and/or under the tissue section preventing the tissue section from contacting the capture array). Then, in some such instances, the ability to distinguish pixels corresponding to tissue from pixels corresponding to background in the obtained image improves the resolution of spatial analysis, *e.g.*, by removing background signals that can impact or obscure downstream analysis, thus limiting the analysis of the plurality of capture probes and/or analytes to a subset of capture probes and/or analytes that correspond to a region of interest (*e.g.*, tissue). See, Uchida, 2013, "Image processing and recognition for biological images," Develop. Growth Differ. 55, 523-549, doi:10.1111/dgd.12054, which is hereby incorporated herein by reference in its entirety, for further embodiments of applications for biological image processing.

In some embodiments, a region of an image that is not classified as tissue is classified as a hole or an object (*e.g.*, debris, hair, crystalline stain particles, and/or air bubbles). In some such embodiments, small holes and/or objects in an image are defined using a threshold size. In some embodiments, the threshold size is the maximum length (*e.g.,* longest side length) of the image divided by two (*e.g.,* in pixels, inches, centimeters, millimeters, and/or arbitrary units), under which any enclosed shape is considered a hole or an object. In some embodiments, the threshold size is the maximum length of the image divided by $N$, where $N$ is any positive value greater than or equal to 1. In some embodiments, small holes and objects are removed from the image (*e.g.,* "filled in") during the assigning of pixels in the image to the first class or the second class, such that an overall region of the image that corresponds to tissue is represented as a contiguous region, and an overall region of the image that corresponds to background is represented as a contiguous region. In some embodiments, small holes and objects are retained in the image during the assigning of pixels in the image to the first class or the second class, such that the region or regions of the image that correspond to tissue do not include small holes and objects, and the region or regions of the image that correspond to background include small holes and objects.

In some embodiments, the assigning of each respective pixel as tissue or background is performed using an algorithm (*e.g.,* using a programming language including but not limited to Python, R, C, C++, Java, and/or Perl), for instance an algorithm implemented by classification module **1120.**

*Defining bounding boxes using fiducial markers.* With further reference to **FIG. 36A,** the assignment of each respective pixel **1126** in the plurality of pixels to a first class or a second class comprises using the plurality of fiducial markers **1130** to define a bounding box within the image. In some embodiments, the bounding box **3606** has a thickness of more than 10, more than 20, more than 30, more than 40, or more than 50 pixels. In some embodiments, the bounding box **3606** has a shape that is the same shape or a different shape as the original image (*e.g.,* a rectangle, square, circle, oblong shape, or $N$-gon, where $N$ is a value between 1 and 20). In some embodiments, the bounding box **3606** has a color or is monochromatic (*e.g.*, white, black, gray). In some embodiments, the bounding box **3606** is blue.

In some embodiments, the bounding box **3606** is defined in the same location as (*e.g.*, on top of) the plurality of fiducial markers (*e.g.*, the fiducial frame). In some embodiments, the bounding **3606** box is defined within or inside the boundary of the fiducial frame. In some such embodiments, the bounding box **906** is defined as a threshold distance inside of the boundary of the fiducial frame (*e.g.*, 1, 2, 3, 4, 5, 6, 7, 8, 9 or 10 pixels, or more than 10, more than 20, more than 30, more than 40, more than 50, or more than 100 pixels inside the fiducial frame). In some embodiments, the bounding box **3606** is defined via user input (*e.g.*, a drawn box around the area of interest). In some embodiments, the bounding box **3606** is defined using two, three, or four fiducial markers located on at least two opposing corners of the fiducial frame.

In some embodiments, the bounding box **3606** is defined using fiducial markers present on the substrate **3604** prior to obtaining the image. In some embodiments, the bounding box **3606** is defined using fiducial markers added to the image after obtaining the image (*e.g.*, via user input or by one or more heuristic functions). In some embodiments, fiducial alignment is performed to align the obtained image with a pre-defined spatial template **1142** using the plurality of fiducial markers as a guide. In some such embodiments, the plurality of fiducial markers **1130** in the obtained image are aligned to a corresponding plurality of fiducial markers **1146** in the spatial template (e.g., as disclosed above with reference to block **1070).** In some embodiments, the spatial template **1142** comprises additional elements with known locations in the spatial template (*e.g.,* capture spots with known locations relative to the fiducial markers). In some embodiments, the fiducial alignment (*e.g.*, in accordance with block 1170) is performed prior to defining the bounding box (*e.g.*, prior to the assigning of each pixel to the first class or the second class). In some embodiments, fiducial alignment is not performed prior to the defining of the bounding box.

In some embodiments, the bounding box **3606** is defined by the edges of the obtained image *(e.g.,* the

EP 4 538 971 A2

dimensions of the image) and/or by the field of view (*e.g.,* scope) of the microscope used for obtaining the image. In some embodiments, the bounding box **3606** is defined as the adjacent edges at the boundary of the obtained image. In some embodiments, the bounding box **3606** is defined as a threshold distance inside the boundary of the obtained image (e.g., 1, 2, 3, 4, 5, 6, 7, 8, 9 or 10 pixels, or more than 10, more than 20, more than 30, more than 40, more than 50, or more than 100 pixels inside the boundary of the image). In some embodiments, the bounding box **3606** is defined as a set of coordinates (*e.g.*, x-y coordinates) corresponding to each of four corners of the bounding box *(e.g.,* [0 + set distance, 0 + set distance], [$W_{image}$ - set distance, 0 + set distance], [0 + set distance, $H_{image}$ - set distance], [$W_{image}$ - set distance, $H_{image}$ - set distance], where $W_{image}$ and $H_{image}$ are the width and height dimensions of the obtained image, respectively, and set distance is a threshold distance inside the boundary of the obtained image). In some embodiments, the threshold distance is pre-defined (*e.g.*, via default and/or user input) or determined heuristically.

In some embodiments, the bounding box **3606** is axis-aligned. In some embodiments, the bounding box **3606** is centered on the center of the obtained image and/or centered on the center of the region enclosed by the fiducial markers. In some embodiments, the bounding box **3606** is not axis-aligned and/or is not centered on either the center of the obtained image or the region enclosed by the fiducial markers. In some embodiments, the threshold distance between each edge of the bounding box **3606** and the respective edges of the obtained image and/or the fiducial frame is the same for each respective edge. In some embodiments, the distance between each edge of the bounding box **3606** and the respective edges of the obtained image and/or the fiducial frame is different for one or more edges. In some embodiments, the bounding box **3606** is rotated on the obtained image to achieve a different alignment of the bounding box **3606** against the obtained image.

In some embodiments, no bounding box is defined and the assigning of each respective pixel in the plurality of pixels to a first class or a second class occurs using the obtained image in its entirety. In some such embodiments, a bounding box is defined as "none."

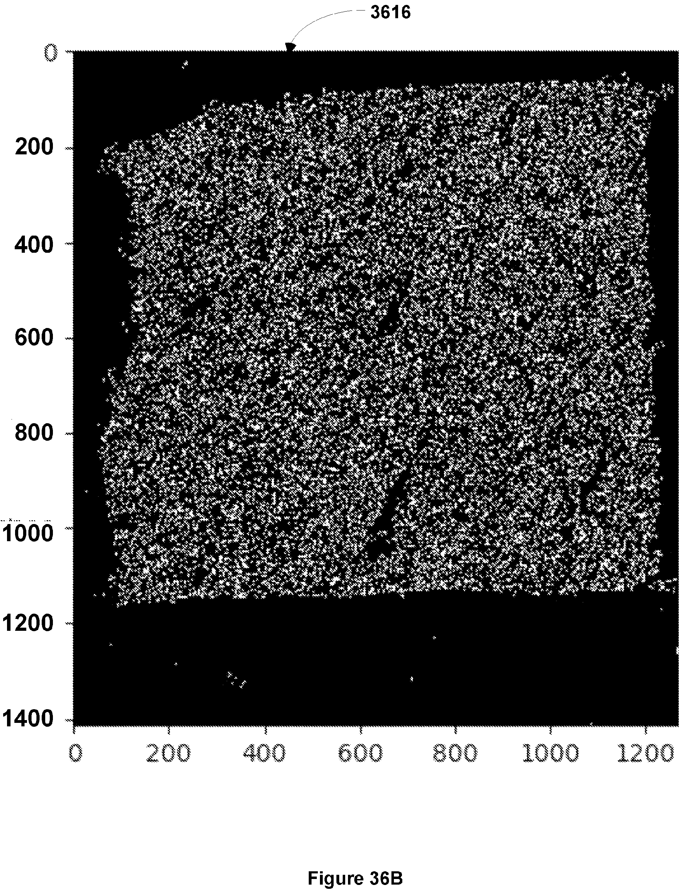

In some embodiments, the assignment of each respective pixel in the plurality of pixels to a first class or a second class further comprises removing respective pixels falling outside the bounding box **3606** from the plurality of pixels. Thus, in some embodiments, the method for tissue classification only considers pixels inside the bounding box **3606.** In some embodiments, the removing of pixels falling outside the bounding box **3606** is performed by creating a new image from the obtained image, comprising only the respective pixels from the obtained image that fall within the bounding box. In some embodiments, the bounding box is defined as being inside the fiducial frame and the removing of the pixels from the plurality of pixels (*e.g.*, to form image **3616** depicted in **FIG. 36B)** includes removing the fiducial markers from the obtained image. In some embodiments, no bounding box is defined and no pixels are removed from the plurality of pixels.

*Application of heuristic classifiers to* a *tissue section image.* In some embodiments, the assignment of each respective pixel in the plurality of pixels to a first class or a second class further comprises running a plurality of heuristic classifiers on the plurality of pixels in grey-scale space. For each respective pixel in the plurality of pixels, each respective heuristic classifier in the plurality of heuristic classifiers casts a vote for the respective pixel between the first class and the second class. Because of this, each pixel has a series of votes, one from each heuristic classifier. By summing the votes made for a given pixel, an aggregated score is formed for the given pixel. Thus, a corresponding aggregated score is formed for each respective pixel in the plurality of pixels from the individual heuristic classifier votes. In some embodiments, the corresponding aggregated score for each respective pixel is used to convert the aggregated score into a class in a set of classes. Referring to block **1074** of **FIG. 10F,** in some embodiments, this set of classes comprises obvious first class, likely first class, likely second class, and obvious second class.

In some embodiments, a pixel comprises one or more pixel values (*e.g.*, intensity value **1126**). In some embodiments, each respective pixel in the plurality of pixels comprises one pixel intensity value **1126,** such that the plurality of pixels represents a single-channel image comprising a one-dimensional integer vector comprising the respective pixel values for each respective pixel. For example, an 8-bit single-channel image (*e.g.*, grey-scale) can comprise $2^8$ or 256 different pixel values (*e.g.*, 0-255). In some embodiments, each respective pixel in the plurality of pixels of an image comprises a plurality of pixel values, such that the plurality of pixels represents a multi-channel image comprising a multi-dimensional integer vector, where each vector element represents a plurality of pixel values for each respective pixel. For example, a 24-bit 3-channel image (*e.g.*, RGB color) can comprise $2^{24}$ (*e.g.*, $2^{8 \times 3}$) different pixel values, where each vector element comprises 3 components, each between 0-255. In some embodiments, an *n*-bit image **1124** comprises up to $2^n$ different pixel values, where *n* is any positive integer. See, Uchida, 2013, "Image processing and recognition for biological images," Develop. Growth Differ. 55, 523-549, doi:10.1111/dgd.12054, which is hereby incorporated herein by reference in its entirety.

In some embodiments, the plurality of pixels is in, or is converted to, grey-scale space by obtaining the image **1124** in grey-scale (*e.g.,* a single-channel image), or by obtaining the image in color (*e.g.,* a multi-channel image) and converting the image to grey-scale after the obtaining and prior to the running of the heuristic classifiers. In some embodiments, each respective pixel in the plurality of pixels in grey-scale space has an integer value between 0 and 255 (*e.g.,* 8-bit unsigned integer value or "uint8"). In some embodiments, the integer value for each respective pixel in the plurality of pixels of the image **1124** in grey-scale space is transformed using *e.g.,* addition, subtraction, multiplication, or division by a value N, where N is any real number. For example, in some embodiments, each respective pixel in the plurality of pixels in grey-scale space has an integer value between 0 and 255, and each integer value for each respective pixel is divided by 255, thus providing integer values between 0 and 1. In some embodiments, the plurality of pixels of the image is in grey-scale space and is transformed using contrast enhancement or tone curve alignment. In some embodiments, the running of the plurality of heuristic classifiers on the plurality of pixels comprises rotating, transforming, resizing, or cropping the obtained image in grey-scale space.

In some embodiments, the plurality of heuristic classifiers comprises a core tissue detection function, and the plurality of heuristic classifiers comprises 1 or more, 2 or more, 3 or more, 4 or more, 5 or more, 6 or more, 7 or more, 8 or more, 9 or more, or 10 or more heuristic classifiers. In some embodiments, the core tissue detection function makes initial predictions about the placement of the tissue on the substrate.

Referring to block **1076** of **FIG. 10F,** in some embodiments, the plurality of heuristic classifiers comprises a first heuristic classifier that identifies a single intensity threshold that divides the plurality of pixels into the first class and the second class. The first heuristic classifier then casts a vote for each respective pixel in the plurality of pixels for either the first class or the second class. The single intensity threshold represents a minimization of intra-class intensity variance between the first and second class or a maximization of inter-class variance between the first class and the second class.

In some embodiments, the single intensity threshold is determined using Otsu's method, where the first heuristic classifier identifies a threshold that minimizes intra-class variance or equivalently maximizes inter-class variance. In some such embodiments, Otsu's method uses a discriminative analysis that determines an intensity threshold such that binned subsets of pixels in the plurality of pixels are as clearly separated as possible. Each respective pixel in the plurality of pixels is binned or grouped into different classes depending on whether the respective intensity value of the respective pixel falls over or under the intensity threshold. For example, in some embodiments, bins are represented as a histogram, and the intensity threshold is identified such that the histogram can be assumed to have a bimodal distribution (*e.g.,* two peaks) and a clear distinction between peaks (*e.g.,* valley).

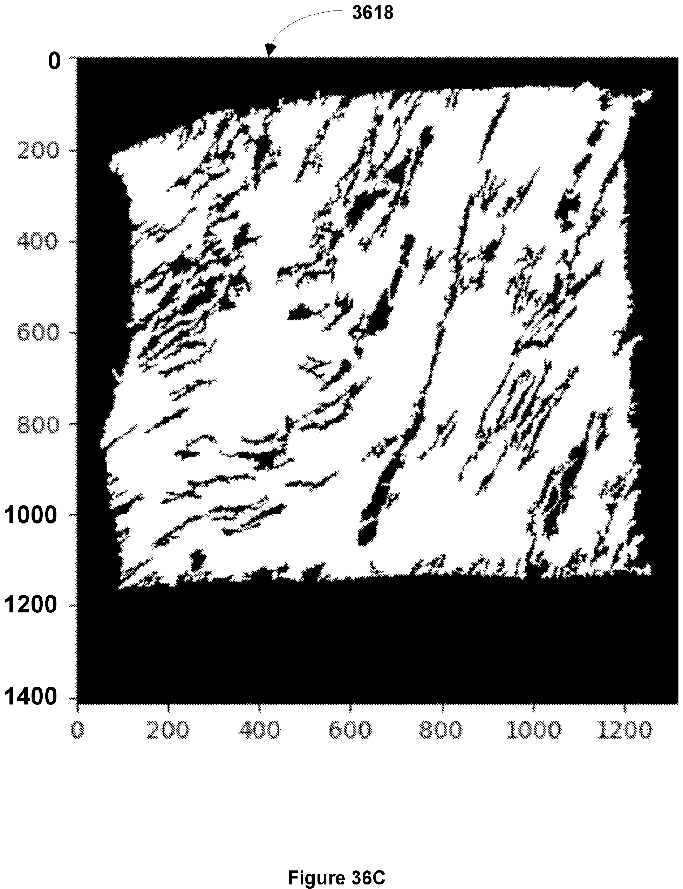

In some such embodiments, the plurality of pixels in the obtained image is filtered such that pixels comprising a pixel intensity above the intensity threshold are considered to be foreground and are converted to white (*e.g.,* uint8 value of 1), while pixels comprising a pixel intensity below the intensity threshold are considered to be background and are converted to black (*e.g.,* uint8 value of 0). An example of an outcome of a heuristic classifier using Otsu's method is illustrated in **FIG. 36C,** which depicts a thresholded image **3618** (*e.g.,* a mask or a layer) after conversion of the acquired image, where each pixel in the plurality of pixels is represented as either a white or a black pixel. Here, Otsu's method is an example of a binarization method using global thresholding. In some embodiments, Otsu's method is robust when the variances of the two classes (*e.g.,* foreground and background) are smaller than the mean variance over the obtained image as a whole.

In some embodiments, the first heuristic classifier uses Otsu's method of global thresholding, and the running of the first heuristic classifier is followed by removal of small holes and objects from the thresholded image (*e.g.,* mask). In some such embodiments, the first heuristic classifier provides a more uniform, binary outcome without small perturbations in the mask. In some embodiments, small holes and objects are not removed from the mask such that small holes and objects can be distinguished from tissue.

In some embodiments, the first heuristic classifier is a binarization method other than Otsu's method. In some such embodiments, the first heuristic classifier is a global thresholding method other than Otsu's method or an optimization-based binarization method. In some such embodiments, a global thresholding method is performed by determining the intensity threshold value manually (*e.g.,* via default or user input). For example, an intensity threshold can be determined at the middle value of the grey-scale range (*e.g.,* 128 between 0-255).

In some embodiments, the intensity threshold value is determined automatically using a histogram of grey-scale pixel values (*e.g.,* using the mode method and/or P-tile method). For example, using the mode method, a histogram of grey-scale pixel values can include a plurality of bins (*e.g.,* up to 256 bins for each possible grey-scale pixel value 0-255), and each respective bin is populated with each respective pixel having the respective grey-scale pixel value. In some embodiments, the plurality of bins has a bimodal distribution and the intensity threshold value is the grey-scale pixel value at which the histogram reaches a minimum (*e.g.,* at the bottom of the valley). Using the P-tile method, each respective bin in a histogram of grey-scale pixel values is populated with each respective pixel having the respective grey-scale pixel value, and a cumulative tally of pixels is calculated for each bin from the highest grey-scale pixel value to the lowest grey-scale pixel value. Given a pre-defined number of pixels P above the intensity threshold value, the threshold value is determined at the bin value at which the cumulative sum of pixels exceed *P*.

In some embodiments, an intensity threshold value is determined by estimating the level of background noise (*e.g.,* in imaging devices including but not limited to fluorescence microscopy). Background noise can be determined using control samples and/or unstained samples during normalization and pre-processing.

In some embodiments, such as when using optimization-based binarization, the assignment of a respective pixel to one of two classes (*e.g.,* conversion to either black or white) is determined by calculating the relative closeness of the converted pixel value to the original pixel value, as well as the relative closeness of the converted pixel value of the respective pixel to the converted pixel values of neighboring pixels (*e.g.,* using a Markov random field). Optimization-based methods thus comprise a smoothing filter that reduces the appearance of small punctate regions of black and/or white and ensures that local neighborhoods exhibit relatively congruent results after binarization. *See,* Uchida, 2013, "Image processing and recognition for biological images," Develop. Growth Differ. 55, 523-549, doi:10.1111/dgd.12054, which is hereby incorporated herein by reference in its entirety.

In some embodiments, the plurality of heuristic classifiers comprises a second heuristic classifier that identifies local neighborhoods of pixels with the same class identified using the first heuristic method. The second heuristic classifier applies a smoothed measure of maximum difference in intensity between pixels in the local neighborhood. The second heuristic classifier thus casts a vote for each respective pixel in the plurality of pixels for either the first class or the second class.

In some embodiments, the local neighborhood of pixels is represented by a disk comprising a radius of fixed length (*e.g.*, 1, 2, 3, 4, 5, 6, 7, 8, 9, or 10 pixels). In some embodiments, the disk has a radius of between 10 and 50 pixels, between 50 and 100 pixels, between 100 and 200 pixels, or more than 200 pixels. In some embodiments, the disk is used to determine the local intensity gradient, where the local intensity gradient is determined by subtracting the local minimum pixel intensity value (*e.g.,* from the subset of pixels within the disk) from the local maximum pixel intensity value (*e.g.,* from the subset of pixels within the disk), giving a value for each pixel in the subset of pixels within the disk that is a difference of pixel intensities within the local neighborhood. In some such embodiments, a high local intensity gradient indicates tissue, while a low local intensity gradient indicates background.

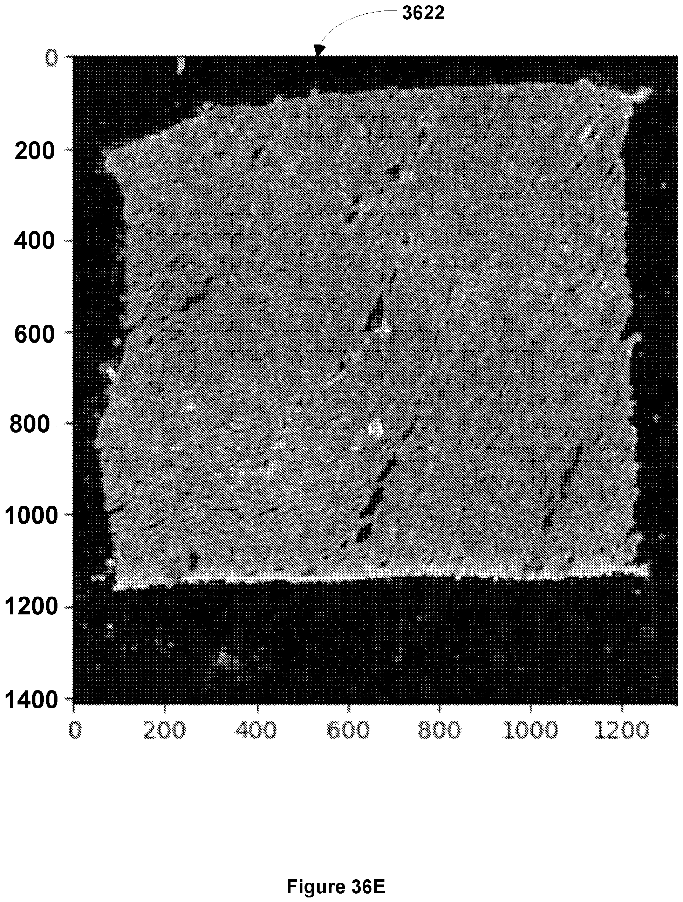

FIG. 36E illustrates a mask 3622 of an obtained image where each pixel in the plurality of pixels in the obtained image is converted to a grey-scale value that is a difference in local intensity values. Unlike the global thresholding methods (*e.g.,* Otsu's method) described above, local intensity gradients are a measure of granularity rather than intensity. For example, whereas global thresholding methods distinguish subsets of pixels that are relatively "light" from subsets of pixels that are relatively "dark," local intensity gradients distinguish regions with patterns of alternating lightness and darkness (*e.g.,* texture) from regions with relatively constant intensities (e.g., smoothness). Local intensity gradient methods are therefore robust in some instances where images comprise textured tissue and moderate resolution, and/or where global thresholding techniques fail to distinguish between classes due to various limitations. These include, in some embodiments, small foreground size compared to background size, small mean difference between foreground and background intensities, high intra-class variance (*e.g.,* inconsistent exposure or high contrast within foreground and/or background regions), and/or background noise (*e.g.,* due to punctate staining, punctate fluorescence, or other intensely pigmented areas resulting from overstaining, overexposure, dye residue and/or debris).

In some embodiments, the first or second heuristic classifier comprises a smoothing method to minimize or reduce noise between respective pixels in a local neighborhood by filtering for differences in pixel intensity values. In some embodiments, smoothing is performed in a plurality of pixels in grey-scale space. In some embodiments, applicable smoothing methods include, but are not limited to, blurring filters, median filters, and/or bilateral filters. For example, in some embodiments, a blurring filter minimizes differences within a local neighborhood by replacing the pixel intensity values 1126 at each respective pixel with the average intensity values of the local neighborhood around the respective pixel. In some embodiments, a median filter utilizes a similar method, but replaces the pixel intensity values at each respective pixel with the median pixel values of the local neighborhood around the respective pixel. Whereas, in some embodiments, blurring filters and median filters cause image masks to exhibit "fuzzy" edges, in some alternative embodiments, a bilateral filter preserves edges by determining the difference in intensity between pixels in a local neighborhood and reducing the smoothing effect in regions where a large difference is observed (*e.g.*, at an edge). See, Uchida, 2013, "Image processing and recognition for biological images," Develop. Growth Differ. 55, 523-549, doi:10.1111/dgd.12054, which is hereby incorporated herein by reference in its entirety.

Thus, in some embodiments, a second heuristic classifier comprises a local intensity gradient filter for a disk with a fixed-length radius also functions as a smoothing filter for the plurality of pixels in the obtained image 1124. The size of the local area defines the smoothing, such that increasing the radius of the disk would increasing the smoothing effect, while decreasing the radius of the disk would increase the resolution of the classifier.

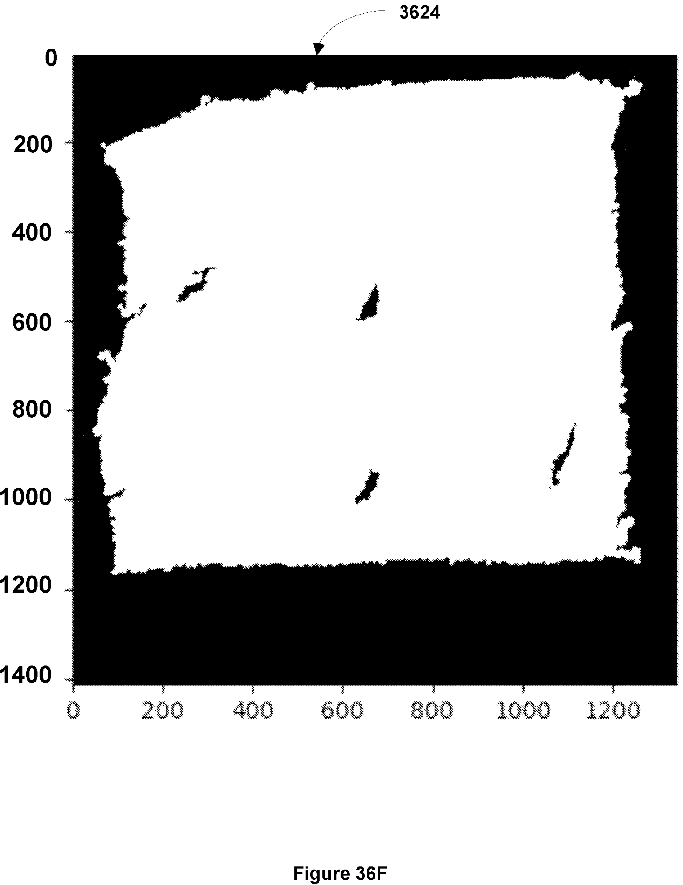

In some embodiments, a global thresholding method is further applied to an image mask comprising the outcome of a local intensity gradient filter represented as an array (*e.g.,* a matrix) of grey-scale pixel values. In some such embodiments, the local intensity gradient array is binarized into two classes using Otsu's method, such that each pixel in the plurality of pixels is converted to a white or a black pixel (*e.g.,* having pixel value of 1 or 0, respectively), representing foreground or background, respectively. FIG. 36F illustrates an example 3624 of the characterization of pixels into the first and second class using Otsu's method applied to a local intensity gradient filter from an obtained image, such that binarization is applied to regions of high and low granularity rather than regions of high and low pixel intensity. This provides an alternative method for classifying foreground and background regions over global thresholding methods.

In some embodiments, binarized local intensity gradients can be further processed by removing small holes and objects, as described previously. In some embodiments, small holes and objects are not removed from binarized local intensity gradient arrays. In some embodiments, a local intensity gradient filter is applied to a thresholded image generated using Otsu's method. In some embodiments, a plurality of heuristic classifiers is applied sequentially to an obtained image such that a second heuristic classifier is applied to a mask resulting from a first heuristic classifier, and a third heuristic classifier is applied to a mask resulting from the second heuristic classifier. In some alternative embodiments, a plurality of heuristic classifiers is applied to an obtained image such that each respective heuristic classifier is independently applied to the obtained image and the independent results are combined. In some embodiments, a plurality of heuristic classifiers is applied to an obtained image using a combination of sequentially and independently applied heuristic classifiers.

In some embodiments, a second heuristic classifier is a two-dimensional Otsu's method, which, in some instances, provides better image segmentation for images with high background noise. In the two-dimensional Otsu's method, the grey-scale intensity value of a respective pixel is compared with the average intensity of a local neighborhood. Rather than determining a global intensity threshold over the entire image, an average intensity value is calculated for a local neighborhood within a fixed distance radius around the respective pixel, and each pair of intensity values (*e.g.,* a value averaged over the local neighborhood and a value for the respective pixel) are binned into a discrete number of bins. The number of instances of each pair of average intensity values for the local neighborhood and for the respective pixel, divided by the number of pixels in the plurality of pixels, determines a joint probability mass function in a 2-dimensional histogram. In some embodiments, the local neighborhood is defined by a disk comprising a radius of fixed length (*e.g.,* 1, 2, 3, 4, 5, 6, 7, 8, 9, or 10 pixels, between 10 and 50 pixels, between 50 and 100 pixels, between 100 and 200 pixels, or more than 200 pixels).

In some embodiments, the plurality of heuristic classifiers comprises a third heuristic classifier that performs edge detection on the plurality of pixels to form a plurality of edges in the image and morphologically closes the plurality of edges to form a plurality of morphologically closed regions in the image. The third heuristic classifier then assigns pixels in the morphologically closed regions to the first class and pixels outside the morphologically closed regions to the second class, thereby causing the third heuristic classifier to cast a vote for each respective pixel in the plurality of pixels for either the first class or the second class.

In some embodiments, a Canny edge detection algorithm is used to detect edges on a grey-scale image. In some such embodiments, edges are identified using a convolution algorithm that identifies the pixel intensity value **1126** for each respective pixel in a plurality of pixels in an array (*e.g.,* an image or a mask) and compares two or more pixels to an edge detection filter (*e.g.,* a box operator that represents a threshold difference in pixel intensity). An edge is thus defined as a set of pixels with a large difference in pixel intensities. Identification of edges is determined by calculating the first-order or second-order derivatives of neighboring pixel intensity values. In some embodiments, the Canny edge detection algorithm results in a binary image where a particular first assigned color value (*e.g.,* white) is applied to pixels that represent edges whereas pixels that are not part of an edge are assigned a second color value (*e.g.,* black). See, Canny, 1986, "A Computational Approach to Edge Detection," IEEE Trans Pattern Anal Mach Intell. 8(6):679-98. **FIG. 36B** illustrates an image mask **3616** comprising the output of a Canny edge detection algorithm on an obtained image.

In some embodiments, edge detection is performed using an edge detection filter other than a Canny edge detection algorithm, including but not limited to Laplacian, Canny, Sobel, Canny-Deriche, Log Gabor, and/or Marr-Hildreth. In some embodiments, a smoothing filter is applied prior to applying the edge detection filter to suppress background noise.

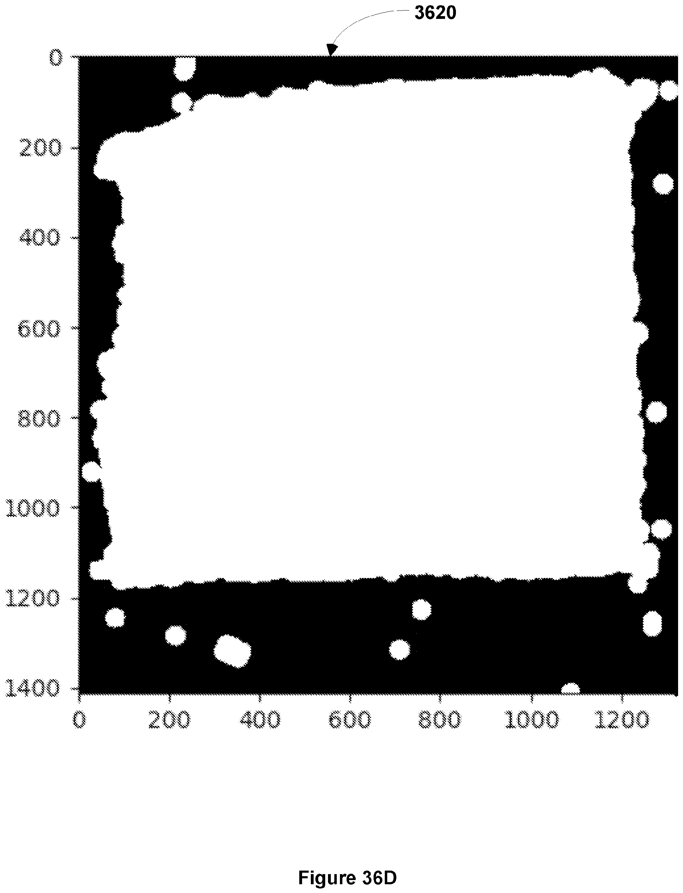

In some embodiments, edges in the plurality of edges are closed to form a plurality of morphologically closed regions. In some embodiments, morphological closing is performed on the plurality of pixels in grey-scale space. In some embodiments, morphological closing comprises a dilation followed by an erosion. In some embodiments, the plurality of pixels in the morphologically closed regions are expressed as an array of 1's and 0's, where pixels assigned to a first class are expressed as 1's (*e.g.,* closed regions) and pixels assigned to a second class are expressed as 0's (*e.g.,* unclosed regions). In some embodiments, the array of 1's and 0's comprise a mask of the image that stores the results of the edge detection and subsequent morphological closing. **FIG. 36D** illustrates an image mask **3620** in which closed regions are formed by morphologically closing a plurality of edges identified using a Canny edge detection algorithm, as pictured in **FIG. 36B.** Closed and unclosed regions comprise a plurality of pixels that are expressed as pixel values 1 and 0, respectively, and are visualized as, for example, white and black pixels, respectively.

In some embodiments, the plurality of heuristic classifiers comprises one or more heuristic classifier described above or any combination thereof. These embodiments are non-limiting and do not preclude substitution of any alternative heuristic classifiers for image manipulation, transformation, binarization, filtration, and segmentation as will be apparent to one skilled in the art.

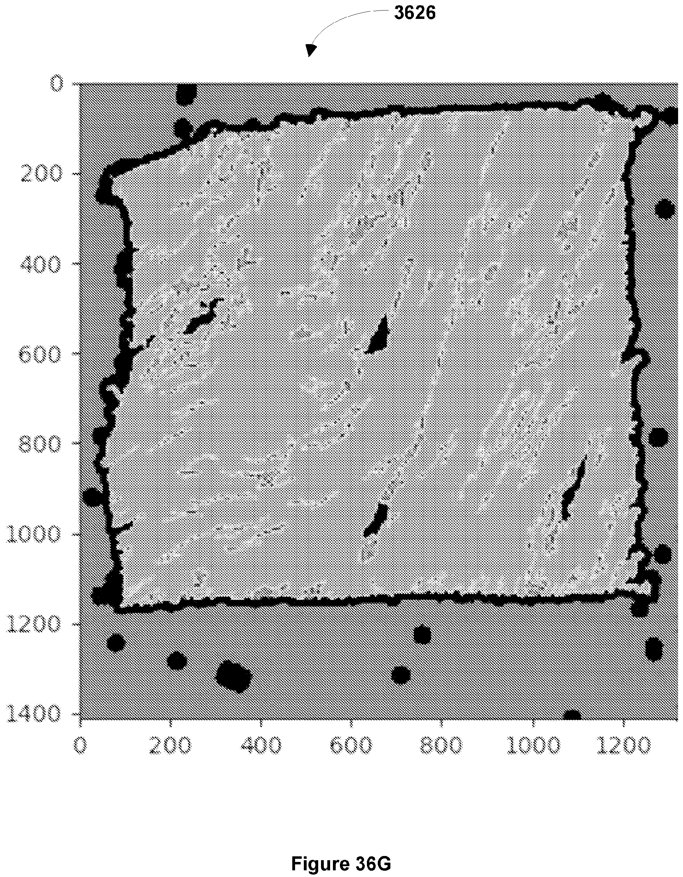

In some embodiments, the plurality of heuristic classifiers consists of a first, second, and third heuristic classier, each respective pixel assigned by each of the heuristic classifiers in the plurality of classifiers to the second class is labelled as obvious second class, and each respective pixel assigned by each of the plurality of heuristic classifiers as the first class is labelled as obvious first class. For example, in some such embodiments, the plurality of heuristic classifiers consists of a first, second and third heuristic classifier, and each respective classifier casts a vote for each respective pixel in the plurality of pixels for either the first class or the second class (*e.g.,* tissue or background, respectively). In some such embodiments, the plurality of votes is aggregated and the aggregate score determines whether the respective pixel is classified as obvious first class, likely first class, likely second class, or obvious second class. In some embodiments, for each respective pixel in a plurality of pixels in grey-scale space, each respective vote for the first class (*e.g.,* foreground and/or tissue) is 1, and each respective vote for the second class (*e.g.,* background) is 0. Thus, for example, an aggregate score of 0 indicates three votes for background, an aggregate score of 1 indicates one vote for tissue and two votes for background, an aggregate score of 2 indicates two votes for tissue and one vote for background, and an aggregate score of 3 indicates three votes for tissue. **FIG.** *36G* illustrates an image mask **3626** representing a sum of a plurality of heuristic classifiers, where each aggregate score is represented as one of a set of four unique classes comprising 0, 1, 2, and 3. In some embodiments, small holes and objects are detected using the image mask of the aggregated scores using a morphological detection algorithm (*e.g.,* in Python).

In some embodiments, a respective pixel in the plurality of pixels is classified as obvious first class, likely first class, likely second class, or obvious second class based on the number and/or type of heuristic classifier votes received. For example, in some embodiments, a respective pixel that receives three votes for background is classified as obvious background, and a respective pixel that receives one vote for tissue in classified as probable background. In some alternative embodiments, a respective pixel that receives one vote for tissue is classified as probable tissue, and a respective pixel that receives two or more votes for tissue is classified as obvious tissue.

In some embodiments, a respective pixel that is classified by at least one heuristic classifier as a hole or object is classified as probable background (*e.g.,* to ensure that that "holes" of non-covered areas surrounded by tissue are initialized with non-"obvious" labels). In some embodiments, a region (a number of pixels in the region) of an obtained image that is classified as obvious tissue based on at least two heuristic classifier votes is reduced in size (*e.g.,* a border of a detected region is resized inward) by a first fixed-length margin. In some embodiments, the first fixed-length margin is 1, 2, 3, 4, 5, 6, 7, 8, 9, 10, or more than 10 pixels. In some embodiments, the first fixed-length margin is a percentage of a length of a side of the obtained image. In some embodiments, the first fixed-length margin is between 0.5% and 10% of the length of the longest side of the obtained image. In some embodiments, a region of an obtained image that is classified as obvious tissue based on at least three heuristic classifier votes is reduced in size by a second fixed-length margin that is smaller than the first fixed-length margin. In some embodiments, the second fixed-length margin has a length that is one-half the length of the first fixed-length margin.

In some embodiments, a respective heuristic classifier is given priority and/or greater weight in the aggregated score. For example, in some embodiments, the first heuristic classifier is global thresholding by Otsu's method. In some such embodiments, a region of an obtained image that is classified as tissue by at least one other heuristic classifier and is not classified as a hole or an object is nevertheless classified as probable background if it is not classified as tissue by the first heuristic classifier (*e.g.,* Otsu's method). In some embodiments, a respective heuristic classifier in the plurality of heuristic classifiers is given priority and/or greater weight in the aggregated score depending on the order in which the respective heuristic classifier is applied (*e.g.,* first, second, or third), or depending on the type of classifier applied (*e.g.,* Otsu's method). In some embodiments, each respective heuristic classifier in the plurality of heuristic classifiers is given equal weight in the aggregated score.

In some embodiments, the aggregated score formed from the plurality of votes from the plurality of heuristic classifiers is a percentage of votes for a first class out of a total number of votes. In some such embodiments, each class in the set of classes comprising obvious first class, likely first class, likely second class, and obvious second class corresponds to a percentage of votes for a first class out of the total number of votes. In some alternative embodiments, each class in the set of classes comprising obvious first class, likely first class, likely second class, and obvious second class corresponds to a number of votes above a threshold number of votes out of the plurality of votes from the plurality of heuristic classifiers. In some embodiments, a specific "truth table" is pre-defined (*e.g.,* via default or user input), giving the respective class assignments for each respective aggregated score.

In some embodiments, a respective pixel that is not assigned a class by any prior method is classified as probable background.

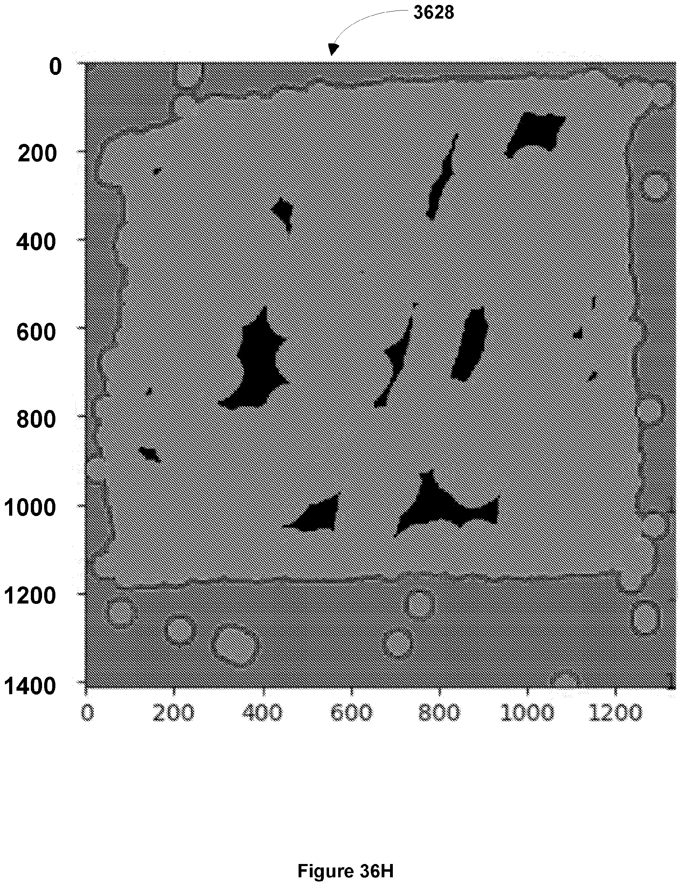

In some embodiments, the classifying of each respective pixel in the plurality of pixels to a class in a set of classes comprising obvious first class, likely first class, likely second class, and obvious second class based on the aggregated score generates a separate array (*e.g.,* image mask), where each pixel in the array comprises a respective separate value or attribute corresponding to the assigned class in the set of classes. **FIG. 36H** illustrates an image mask 36**28** where each pixel is represented by an attribute corresponding to obvious first class, likely first class, likely second class, and obvious second class. Notably, the image masks in **FIG. 36G** and **FIG. 36H** differ in that the image mask **3626 in FIG. 36G** represents a raw aggregate of the plurality of votes from the plurality of heuristic classifiers, whereas the image mask **3628 in FIG. 36H** represents the subsequent classification of each respective pixel based on the aggregated score. As described above, in some embodiments, classification of a respective pixel based on the aggregated score is not dependent solely on the raw sum of the plurality of votes but is, in some instances, dependent on the order and/or importance of a respective heuristic classifier in the plurality of heuristic classifiers. Thus, the image masks depicted in **FIG. 36G** and **FIG. 36H** are similar but not identical, in accordance with some embodiments.

In some embodiments an image mask is generated for quality control purposes (*e.g.,* to provide visual confirmation of classification outcomes to a user or practitioner). In some embodiments, an image mask is generated in grey-scale or in multispectral color (*e.g.,* RGB, 24-bit RGB, and/or float64-bit RGB). In some embodiments, the image mask is re-embedded on the original obtained image for comparison and/or quality control purposes. In some embodiments, an image mask generated at any stage and/or following any number of one or more heuristic classifiers is re-embedded on the original obtained image, and the re-embedding comprises rotating, resizing, transforming, or overlaying a cropped image mask onto the original obtained image.

In some embodiments, the image mask **3628** generated by the classification of each respective pixel in the plurality of pixels to a class in the set of classes, as depicted in the example of **FIG. 36H,** is used as markers for downstream image segmentation (*e.g.,* GrabCut markers). In some embodiments, the image mask used for markers for downstream image segmentation is generated prior to applying the plurality of heuristic classifiers to the obtained image and is iteratively constructed and reconstructed based on the aggregated scores for the plurality of heuristic classifiers after applying each respective heuristic classifier in the plurality of heuristic classifiers. Thus, in some such embodiments, a pixel is in some instances assigned a first classification that is changed to a second classification after the application of subsequent heuristic classifiers.

In some embodiments, the plurality of heuristic classifiers comprises a core tissue detection function that provides initial estimates of the tissue placement, and these estimates are combined into an initialization prediction that is passed to a subsequent segmentation algorithm.

*Image segmentation.* In some embodiments, the method for tissue classification further comprises applying the aggregated score and intensity of each respective pixel in the plurality of pixels to a segmentation algorithm, such as graph cut, to independently assign a probability to each respective pixel in the plurality of pixels of being tissue sample or background.

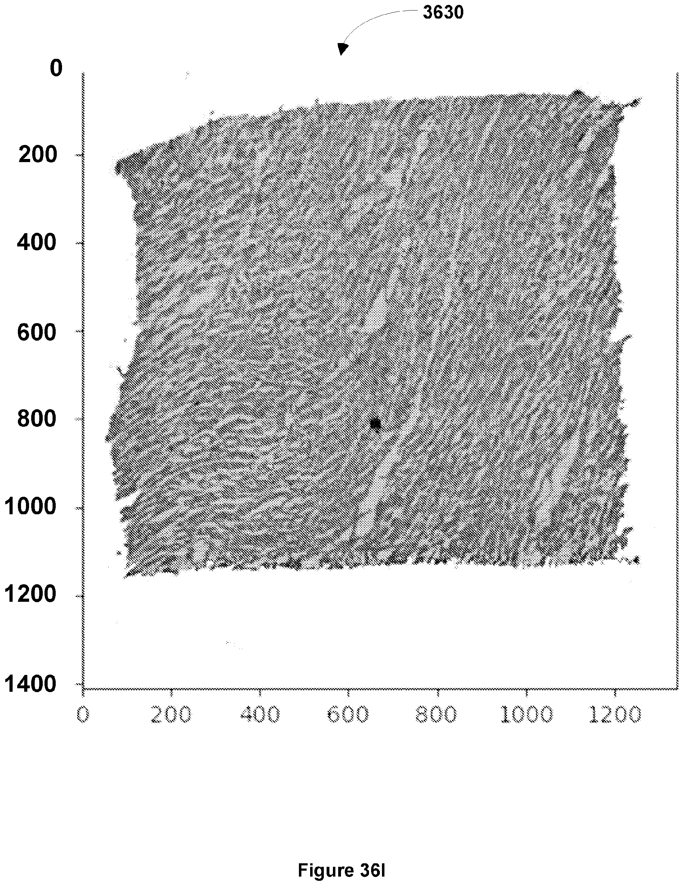

Graph cut performs segmentation of a monochrome image based on an initial trimap $T = \{T_B, T_U, T_F\}$, where $T_B$ indicates background regions, $T_F$ indicates foreground regions, and $T_U$ indicates unknown regions. The image is represented as an array $z = (z_1, ... , z_n, ..., z_N)$ comprising grey-scale pixel values for a respective pixel $n$ in a plurality of $N$ pixels. As in Bayes matting models, the graph cut segmentation algorithm attempts to compute the alpha values for $T_U$ given input regions for $T_B$ and $T_F$, by creating an alpha-matte that reflects the proportion of foreground and background for each respective pixel in a plurality of pixels as an alpha value between 0 and 1, where 0 indicates background and 1 indicates foreground. In some embodiments, an alpha value is computed by transforming a grey-scale pixel value (*e.g.,* for an 8-bit single-channel pixel value between 0 and 255, the pixel value is divided by 255). Graph cut is an optimization-based binarization technique as described above, which uses polynomial-order computations to achieve robust segmentation even when foreground and background pixel intensities are poorly segregated. See, Rother et al., 2004, "'GrabCut' - Interactive Foreground Extraction using Iterated Graph Cuts," ACM Transactions on Graphics. 23(3):309-314, doi:10.1145/1186562.1015720, which is hereby incorporated herein by reference in its entirety. See *also,* Boykov and Jolly, 2001, "Interactive graph cuts for optimal boundary and region segmentation of objects in N-D images," Proc. IEEE Int. Conf. on Computer Vision, CD-ROM, and Greig et al., 1989, "Exact MAP estimation for binary images," J. Roy. Stat. Soc. B. 51, 271-279, for details on graph cut segmentation algorithms; and Chuang et al., 2001, "A Bayesian approach to digital matting," Proc. IEEE Conf. Computer Vision and Pattern Recog., CD-ROM, for details on Bayes matting models and alpha-mattes, each of which is hereby incorporated herein by reference in its entirety. An example of the output is image **3630** of FIG. **36I.**

In some embodiments, the trimap is user specified. In some embodiments, the trimap is initialized using the plurality of heuristic classifiers as an initial tissue detection function. In some such embodiments, the set of classes comprising obvious first class, likely first class, likely second class, and obvious second class are provided to the graph cut segmentation algorithm as a trimap comprising $T_F = \{$obvious first class$\}$ (*e.g.,* obvious foreground), $T_B = \{$obvious second class$\}$ (*e.g.,* obvious background), and $T_U = \{$likely first class, likely second class$\}$ (*e.g.,* concatenation of likely foreground and likely background). In some embodiments, the $T_F = \{$obvious first class, probable first class$\}$ (*e.g.,* obvious foreground and probable foreground), $T_B = \{$obvious second class, probable second class$\}$ (*e.g.,* obvious background and probable background), and $T_U$ is any unclassified pixels in the plurality of pixels in the obtained image. In some embodiments, the set of classes is provided to the graph cut segmentation algorithm using an alternate trimap that is a combination or substitution of the above implementations that will be apparent to one skilled in the art.

In some embodiments, the segmentation algorithm is a GrabCut segmentation algorithm. The GrabCut segmentation algorithm is based on a graph cut segmentation algorithm, but includes an iterative estimation and incomplete labelling function that limits the level of user input required and utilizes a an alpha computation method used for border matting to reduce visible artefacts. Furthermore, GrabCut uses a soft segmentation approach rather than a hard segmentation approach. Unlike graph cut segmentation algorithms, GrabCut uses Gaussian Mixture Models (GMMs) instead of histograms of labelled trimap pixels, where a GMM for a background and a GMM for a foreground are full-covariance Gaussian mixtures with K components. To make the GMM a tractable computation, a unique GMM component is assigned to each pixel in the plurality of pixels from either the background or the foreground model (*e.g.,* 0 or 1). See, Rother et al., 2004, "'GrabCut' - Interactive Foreground Extraction using Iterated Graph Cuts," ACM Transactions on Graphics. 23(3):309-314, doi:10.1145/1186562.1015720, which is hereby incorporated herein by reference in its entirety.

In some embodiments, the GrabCut segmentation algorithm can operate either on a multi-spectral, multi-channel image (*e.g.,* a 3-channel image) or on a single-channel image. In some embodiments, a grey-scale image is provided to the segmentation algorithm. In some embodiments, a grey-scale image is first converted to a multi-spectral, multi-channel image (*e.g.,* RGB, HSV, CMYK) prior to input into the segmentation algorithm. In some embodiments, a multi-spectral, multi-channel color image is applied directly to the segmentation algorithm.

In some embodiments, the GrabCut segmentation algorithm is applied to the image as a convolution method, such that local neighborhoods are first assigned to a classification (*e.g.,* foreground or background) and assignations are then applied to a larger area. In some embodiments, an image comprising a plurality of pixels is provided to the GrabCut algorithm as a color image, using the initialization labels obtained from the plurality of heuristic classifiers, and the binary classification output of the GrabCut algorithm is used for downstream spatial analysis (*e.g.,* on barcoded capture spots). In some embodiments, the plurality of pixels assigned with a greater probability of tissue or background is used to generate a separate construct (*e.g.,* a matrix, array, list or vector) indicating the positions of tissue and the positions of background in the plurality of pixels. For example, **FIG. 36I** illustrates an image mask resulting from the GrabCut algorithm for an obtained image **FIG. 36A** given an input trimap based on GrabCut markers as illustrated in **FIG. 36H.** The GrabCut segmentation algorithm performs binary identification of tissue and background, which is evident from the clear isolation of the tissue section overlay from the background regions.

In some embodiments, the aggregated score and intensity of each respective pixel in the plurality of pixels is applied to a segmentation algorithm other than a graph cut segmentation algorithm or a GrabCut segmentation algorithm, including but not limited to, Magic Wand, Intelligent Scissors, Bayes Matting, Knockout 2, level sets, binarization, background subtraction, watershed method, region growing, clustering, active contour model (*e.g.,* SNAKES), template matching and recognition-based method, Markov random field. In some embodiments, the aggregated score and intensity of each respective pixel in the plurality of pixels is applied to a feature extraction algorithm (*e.g.,* intuition and/or heuristics, gradient analysis, frequency analysis, histogram analysis, linear projection to a trained low-dimensional subspace, structural representation, and/or comparison with another image). In some embodiments, the aggregated score and intensity of each respective pixel in the plurality of pixels is applied to a pattern classification method including but not limited to nearest neighbor classifiers, discriminant function methods (*e.g.,* Bayesian classifier, linear classifier, piecewise linear classifier, quadratic classifier, support vector machine, multilayer perception/neural network, voting), and/or classifier ensemble methods (*e.g.,* boosting, decision tree/random forest). See, Rother et al., 2004, "'GrabCut' - Interactive Foreground Extraction using Iterated Graph Cuts," ACM Transactions on Graphics. 23(3):309-314, doi:10.1145/1186562.1015720, *and,* Uchida, 2013, "Image processing and recognition for biological images," Develop. Growth Differ. 55, 523-549, doi:10.1111/dgd.12054, each of which is hereby incorporated herein by reference in its entirety.

Referring to block **1078 of FIG. 10F,** in some embodiments, the method further comprises overlaying a tissue mask on an image, where the tissue mask causes each respective pixel in the plurality of pixels of the image that has been assigned a greater probability of being tissue to be assigned a first attribute and each respective pixel in the plurality of pixels that has been assigned a greater probability of being background to be assigned a second attribute.

In some embodiments, the assigning of a first or a second attribute to a respective pixel requires a threshold value **1126** for the respective pixel, such that a pixel value above or below the threshold value is assigned a greater probability of being tissue or a greater probability of being background, respectively (*e.g.,* a pixel value between 0 and 1, or a pixel value between 0 and 255). In some embodiments a greater probability of being tissue or a greater probability of being background is assigned based on the aggregated score corresponding to the class in the set of classes that is obvious first class and/or likely first class, or obvious second class and/or likely second class, respectively. In some embodiments, a greater probability of being tissue or a greater probability of being background is determined using an image segmentation algorithm, which applies a binary classification to each respective pixel in a plurality of pixels in an obtained image.

In some such embodiments, the first attribute is a first color and the second attribute is a second color. In some such embodiments, the first color is one of red and blue and the second color is the other of red and blue. In some embodiments, the first color is any one of a group comprising red, orange, yellow, green, blue, violet, white, black, gray, and/or brown, and the second color is any one of the same group that is a different color than the first color. In some embodiments, the first attribute is a first level of brightness or opacity and the second attribute is a second level of brightness or opacity. In some embodiments, the first and second attributes are any contrasting attributes for a visual representation of binary class (*e.g.,* zeros and ones, colors, contrasting shades and/or pixel intensities, symbols (*e.g.,* X's and O's), and/or patterns *(e.g.,* hatch patterns)).

In some embodiments, attributes are assigned based on both class assignment (*e.g.,* tissue or background) and probability (*e.g.,* obvious or likely). For example, in some embodiments, a respective pixel in a plurality of pixels in an obtained image is assigned a first attribute and a second attribute for a first parameter that indicates whether the respective pixel corresponds to a region of the tissue sample or a region of background (*e.g.,* a red color and a blue color), and a first attribute and a second attribute for a second parameter that indicates the probability and/or likelihood of the class assignation (*e.g.,* a level of brightness or opacity). Thus, in some such embodiments, a respective pixel comprises a plurality of attributes (*e.g.,* dark red, light red, light blue, dark blue).

In some embodiments, attributes are assigned based on both class assignment (*e.g.,* tissue or background) and pixel intensity. In some embodiments, respective pixel in a plurality of pixels in an obtained image is assigned two or more attributes for a plurality of parameters.

With reference to **FIG.** 12, in some embodiments, an image **1124** further comprises a representation of a set of capture spots (*e.g.,* **1136-1-1,** ..., **1136-1-4,** ..., **1136-1-13,** ..., **1136-1-M,** where M is a positive integer) in the form of a two-dimensional array of positions on the substrate *904.* Each respective capture spot **1136** in the set of capture spots is (i) at a different position in the two-dimensional array and (ii) associates with one or more analytes from the tissue. Each respective capture spot **1136** in the set of capture spots is characterized by at least one unique spatial barcode in a plurality of spatial barcodes. **FIG. 13** illustrates one such capture spot **1136.** With reference to block **1080** of **FIG. 10F,** in some such embodiments, the method further comprises assigning each respective representation of a capture spot **1136** in the plurality of capture spots the first attribute or the second attribute based upon the assignment of pixels in the vicinity of the respective representation of the capture spot in the composite representation. For instance, referring to **FIG. 12,** capture spots **1136-1,** ..., **1136-4,** ..., **1136-13,** ..., **1136-M** would be assigned to background because they fall outside the region sectioned tissue **1204** is on.

In some embodiments, the assignment of a first or second attribute to a respective representation of a capture spot **1136** in the plurality of capture spots is represented as a tissue position construct (*e.g.,* a matrix, array, list or vector) indicating the positions of tissue and background respective to the plurality of pixels and/or respective to the plurality of capture spots, thus indicating the subset of pixels corresponding to the subset of capture spots that is overlayed with the tissue section. In some embodiments, the assignment of a first or second attribute to a respective representation of a capture spot is performed using an algorithm, function and/or a script (*e.g.,* Python). In some such embodiments the assignment is performed using the analysis module **1120.** In some embodiments, the algorithm returns a tissue position construct (*e.g.,* a matrix, array, list or vector) comprising spatial coordinates as integers in row and column form, and barcode sequences for barcoded capture spots as values. In some embodiments, a tissue position construct is generated based on a plurality of parameters for an obtained image, including but not limited to a list of tissue positions, a list of barcoded capture spots, a list of the coordinates of the centers of each respective barcoded capture spot, one or more scaling factors for the obtained image (*e.g.,* 0.0 - 1.0), one or more image masks generated by the heuristic classifiers and/or image segmentation algorithm, the diameter of a respective capture spot (*e.g.,* in pixels), a data frame with row and column coordinates for the subset of capture spots corresponding to tissue, and/or a matrix comprising barcode sequences. In some such embodiments, the function for generating the tissue position construct determines which capture spots overlap the tissue section based on the spot positions and the tissue mask, where the overlap is determined as the fraction of capture spot pixels that overlap the mask. In some such embodiments, the calculation uses the radius of the capture spots and the scaling factor of the obtained image to estimate the overlap. In some embodiments, the function for generating the tissue position construct further returns an output including but not limited to a list of barcode sequences overlapping the tissue section, a set of scaled capture spot coordinates overlapping tissue, and/or a set of scaled capture spot coordinates corresponding to background.

In some embodiments, the plurality of capture spots **1136** are located directly below the tissue image, while in some alternative embodiments, the plurality of capture spots **1136** are provided on a substrate that is different from the substrate **904** on which the tissue section is imaged. In some embodiments, the tissue section is overlayed directly onto the capture spots on a substrate, either prior to or after the imaging, and the association of the capture spots with the one or more analytes from the tissue occurs through direct contact of the tissue with the capture spots. In some embodiments, the tissue section is not overlayed directly onto the capture spots and the association of the capture spots with the one or more analytes from the tissue occurs through transfer of analytes from the tissue to the capture spots using a porous membrane or transfer membrane.

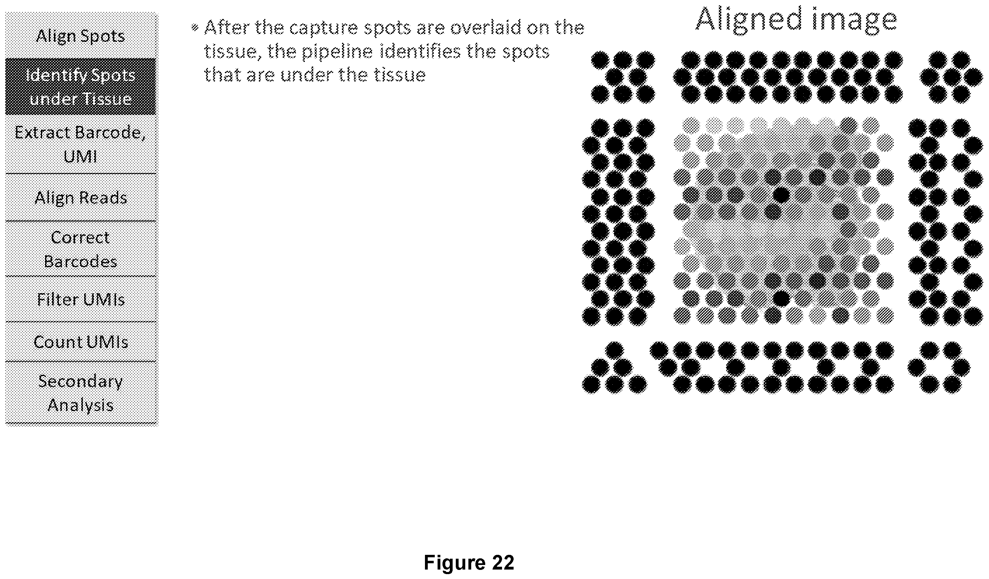

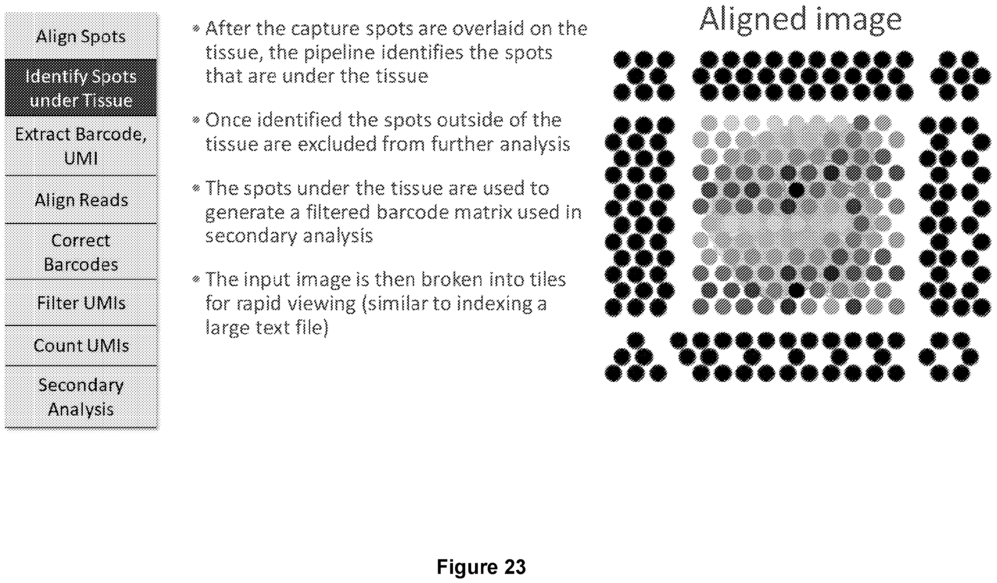

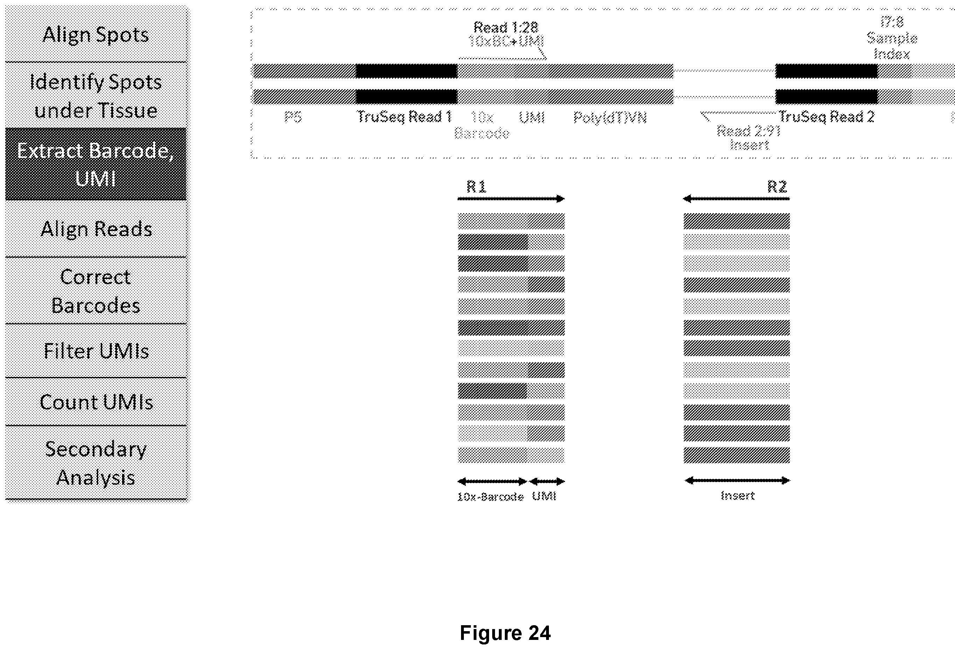

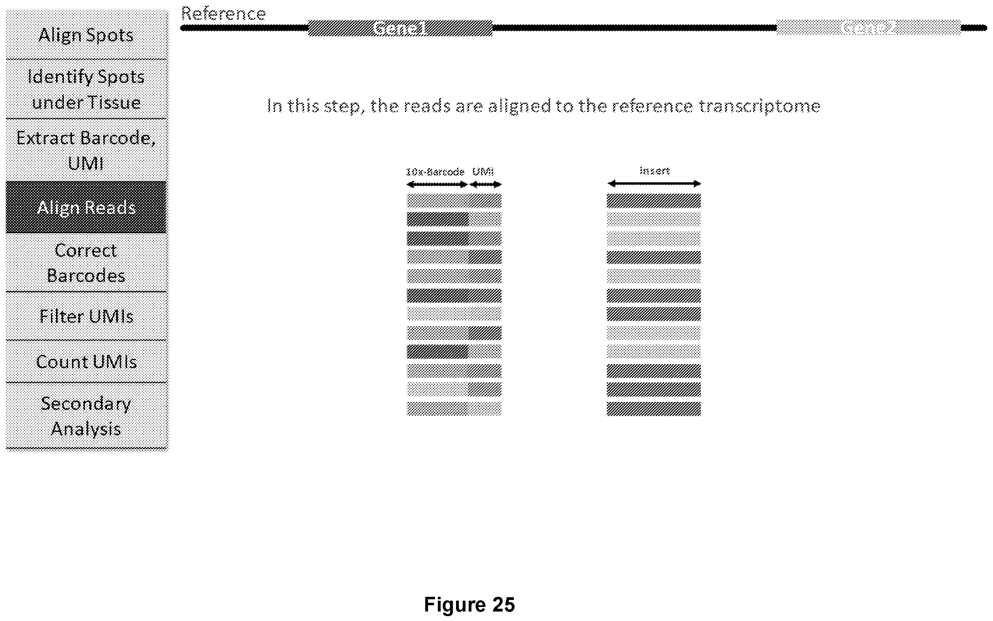

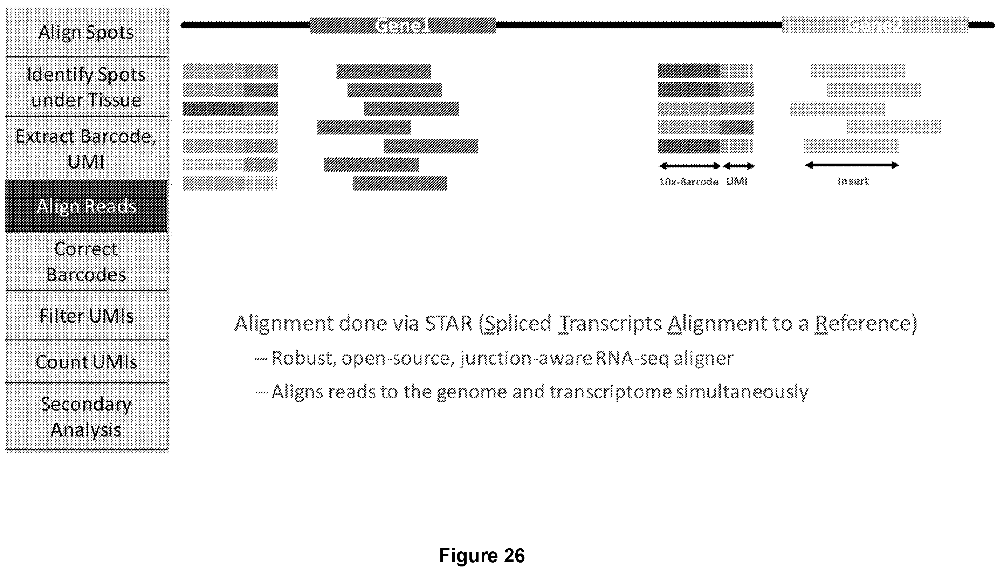

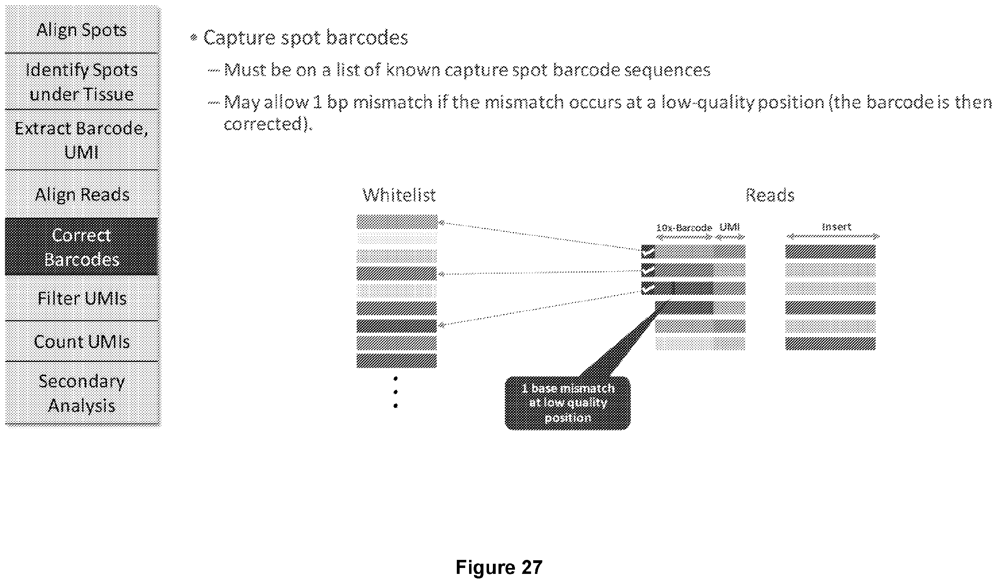

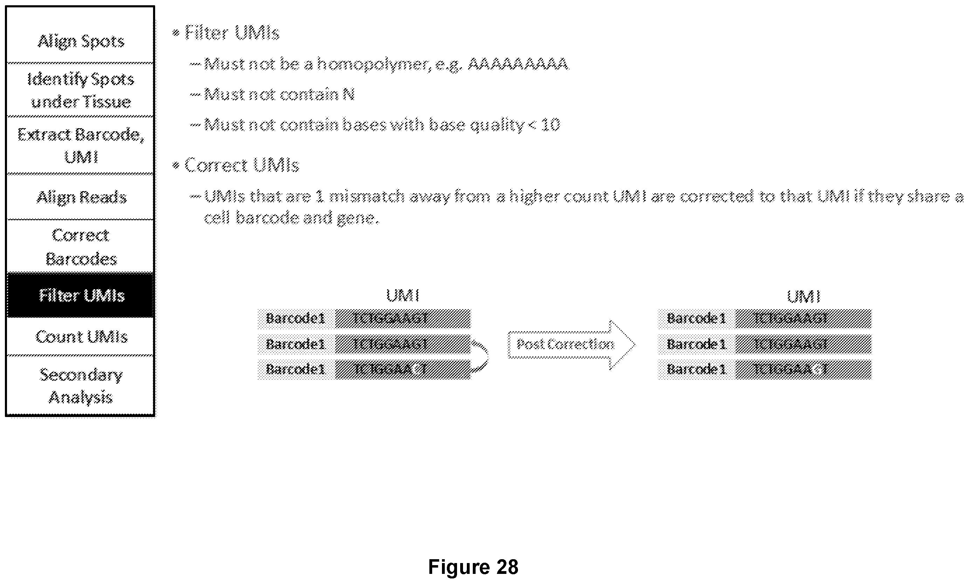

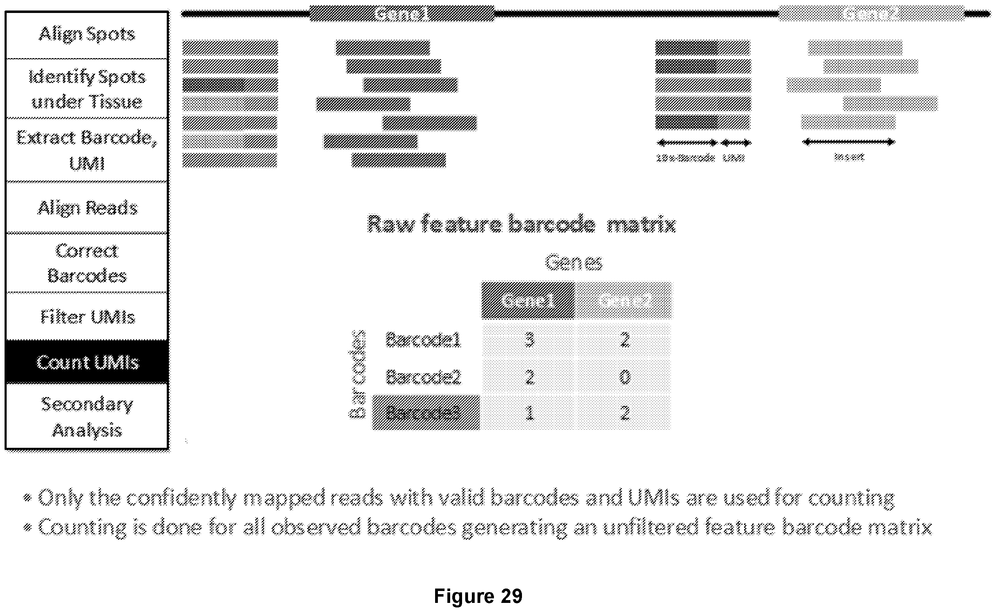

With further reference to block **1066** of **FIG. 10E,** in some embodiments the composite representation is used to perform spatial nucleic acid analysis. This is illustrated in **FIGS. 22 - FIG. 34.** In **FIG. 22,** after the capture spots are overlaid on the image, the spots that are under the tissue sample of the tissue can be identified and the nucleic acid sequencing data of each such capture spot can be analyzed using, for example, the techniques disclosed in the present disclosure as well as those detailed in United States Patent Application No. 16/992,569, entitled "Systems and Methods for Using the Spatial Distribution of Haplotypes to Determine a Biological Condition," filed August 13, 2020; United States Provisional Patent Application No. 62/909,071, entitled "Systems and Methods for Visualizing a Pattern in a Dataset," filed October 1, 2019; and United States Provisional Patent Application No. 62/839,346, entitled "Spatial Transcriptomics of Biological Analytes in Tissue Samples," filed April 26, 2019, each of which is hereby incorporated by reference. Such analysis is further illustrated in **FIG. 23**, which specifies that the capture spots **1136** that are under tissue are used to generate a filtered barcode matrix that is used for secondary analysis that is further illustrated in **FIGS. 24-35.** In particular, **FIG. 24** illustrates how the spatial barcodes **1150** and UMIs are extracted from each sequence read **1136** (*e.g.,* using Read 1) that has been obtained, as further explained in United States Provisional Application No. 62/839,346, entitled "Spatial Transcriptomics of Biological Analytes in Tissue Samples," filed April 26, 2019, which is hereby incorporated by reference and is described above in conjunction with blocks **1026** through **1030**. FIG. **25** illustrates how the sequence reads **1138** are aligned to the reference transcriptome (*e.g.*, using the Read 2 insert read). **FIG. 26** illustrates how sequence reads **1138** don't all map to exactly the same place, even if they share a barcode and UMI, due to the random fragmentation that happens during the workflow steps. **FIG. 27** illustrates how the spatial barcodes in the sequence reads in the capture spots must be in a list of known capture spot spatial barcodes in some embodiments. For instance, if the Chromium Single Cell 3' v3 chemistry gel beads (10X, Pleasanton, California) are used to perform sequencing of analytes from capture spots in accordance with United States Provisional Application No. 62/839,346, entitled "Spatial Transcriptomics of Biological Analytes in Tissue Samples," filed April 26, 2019, each spatial barcode 1150 must be in the set of 3.6 million distinct cell barcodes in the Chromium Single Cell 3' v3 chemistry gel beads. As detailed in **FIG. 27,** in some embodiments a single mismatch in the barcode is permitted. In other embodiments, no mismatch in the spatial barcode **1150** is permitted and sequence reads that have a spatial barcode **1150** that is not in the set of spatial barcode of the sequencing kit used (*e.g.*, the Chromium Single Cell 3' v3 chemistry gel beads) are discarded. **FIG. 28** illustrates how unique molecule identifiers (UMIs) are used to assess and filter out sequence reads **1138** as well in some embodiments. In some embodiments, each capture spot has a large number of capture probes, but each capture probe within a capture spot has a unique UMI (*e.g.*, multiple capture probes within a capture spot share the same UMI). In some embodiments, the capture probes are any combination of capture probes disclosed in U.S. Provisional Patent Application No. 62/979,889, "Capturing Targeted Genetic Targets Using a Hybridization/Capture Approach," filed February 21, 2020, attorney docket number 104371-5028-PR02, which is hereby incorporated by reference. Referring to **FIG. 29,** in some embodiments, only confidently mapped sequence reads **1138** with valid spatial barcodes **1150** and UMIs are used. In some embodiments the UMI of sequence reads are corrected to more abundant UMIs that are one mismatch away in sequence. In some embodiments, sequence reads that are duplicates of the same RNA molecule are recorded and only the unique UMIs are counted as unique RNA molecules.

In such embodiments, these UMI counts form the raw feature barcode matrix. In typical embodiments, a discrete attribute value dataset **1122** will contain a single feature-barcode matrix even if the dataset includes a plurality of images. Further, a set of barcodes is associated with the dataset **1122**. Each capture spot in an image **1124** will contain a unique barcode from the set of barcodes.

In discrete attribute value datasets **1122** that have multiple spatial projections, that is, represent multiple samples such as various slides of a particular tissue and therefore have a corresponding set of images 1124 for each such sample, the feature-barcode matrix originally determined for the one or more images 1124 of each spatial projection is combined into the single feature-barcode matrix of the discrete attribute value dataset **1122**. In some embodiments, in order to combine these matrices, the analyte measurements **1138** of individual spatial projections are adjusted for differences in sequencing depth between spatial projections (*e.g.*, between slides of a biological sample) and, optionally, "batch effect" correction is performed in order to remove signal due to technical differences, such as changes in chemistry (*e.g.* combining 10X, Pleasanton, California CHROMIUM v2 data with 10X CHROMIUM v3 data) across the discrete attribute value data of individual spatial projections (*e.g.*, individual slides). Thus, in the case where capture data **1134-1** represents a first spatial projection and capture data **1134-2** represents a second spatial projection (*e.g.*, because they were acquired from different tissue slides), the analyte measurements of capture data **1134-1** (corresponding to image **1124-1)** and **1134-2** (corresponding to image **1124-2)** are corrected. In the case where capture data **1134-1, 1134-2,** and **1134-3** represents a first spatial projection (*e.g.*, three channels of the same biological sample such as slide 1 of a biological sample) and capture data **1134-4, 1134-5,** and **1134-6** represents a second spatial projection (*e.g.*, three channels of the same biological sample such as slide 2 of a biological sample), the analyte measurements of capture data **1134-1, 1134-2,** and **1134-3** (corresponding to images **1124-1, 1124-2,** and **1124-3)** and **1134-4, 1134-5,** and **1134-6** (corresponding to images **1124-4, 1124-5,** and **1125-6)** are corrected with respect to each other. In some embodiments, this is accomplished using techniques disclosed in Hafemeister and Satija, "Normalization and variance stabilization of single-cell RNA-seq data using regularized negative binomial regression," bioRxiv 576827 (2019). doi:10.1101/576827, which is hereby incorporated by reference.

In some embodiments, images **1124** will be of the same tissue sample but representing different re-emission wavelengths. In some embodiments, images **1124** will be of the same tissue sample but one or more of the images will be brightfield images (with or without staining, such as immunohistochemistry staining) and one or more of the images will be the result of fluorescence imaging as discussed above. In some embodiments, images **1124** will be of the same tissue sample and each such image will be a brightfield image (with or without staining, such as immunohistochemistry staining). In some embodiments, images **1124** will be of the same tissue sample and each such image will be a result of fluorescence imaging (with or without staining, such as immunohistochemistry staining).

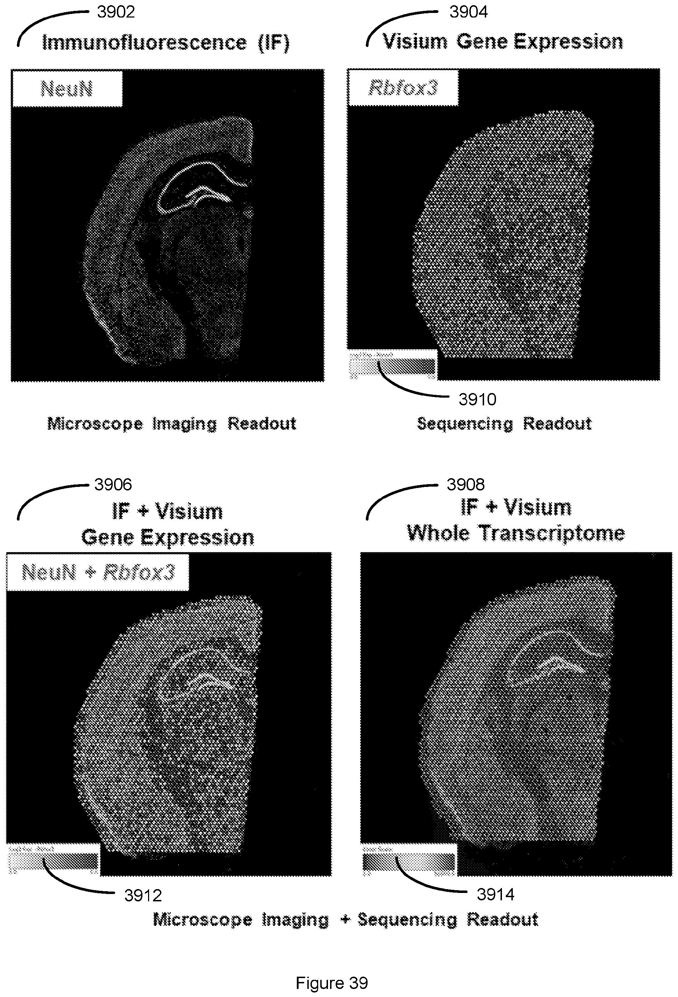

**FIG. 39** illustrates an embodiment in which a biological sample has an image **3902** that has been collected by immunofluorescence. Moreover, the sequence reads of the biological sample have been spatially resolved using the methods disclosed herein. More specifically, a plurality of spatial barcodes has been used to localize respective sequence reads in a plurality of sequence reads obtained from the biological sample (using the methods disclosed herein) to corresponding capture spots in a set of capture spots (through their spatial barcodes), thereby dividing the plurality of sequence reads into a plurality of subsets of sequence reads, each respective subset of sequence reads corresponding to a different capture spot (through their spatial barcodes) in the plurality of capture spots. As such, panel **3904** shows a representation of a portion (that portion that maps to the gene *Rbfox3*) of each subset of sequence reads at each respective position within image **3902** that maps to a respective capture spot corresponding to the respective position. Panel **3906** of **FIG. 39** shows a composite representation comprising (i) the image **3902** and (ii) a representation of a portion (that portion that maps to the gene *Rbfox3*) of each subset of sequence reads at each respective position within image **3902** that maps to a respective capture spot corresponding to the respective position. Finally, panel **3908** of **FIG. 39** shows a composite representation comprising (i) the image **3902** and (ii) a whole transcriptome representation of each subset of sequence reads at each respective position within image **3902** that maps to a respective capture spot corresponding to the respective position. In panels **3904, 3906,** and **3908,** each representation of sequence reads in each subset represents a number of unique UMI, on a capture spot by capture spot basis, in the subsets of sequence reads on a color scale basis as outlined by respective scales **3910, 3912,** and **3914.** While panel **3908** shows mRNA-based UMI abundance on a source image, the present disclosure can also be used to illustrate the spatial quantification of other analytes such as proteins, either superimposed on images of their source tissue or arranged in two-dimensional space using dimension reduction algorithms such as t-SNE or UMAP, including cell surface features (*e.g.*, using the labelling agents described herein), mRNA and intracellular proteins (*e.g.*, transcription factors), mRNA and cell methylation status, mRNA and accessible chromatin (*e.g.*, ATAC-seq, DNase-seq, and/or MNase-seq), mRNA and metabolites (*e.g.*, using the labelling agents described herein), a barcoded labelling agent (*e.g.*, the oligonucleotide tagged antibodies described herein) and a V(D)J sequence of an immune cell receptor (*e.g.*, T-cell receptor), mRNA and a perturbation agent (*e.g.*, a CRISPR crRNA/sgRNA, TALEN, zinc finger nuclease, and/or antisense oligonucleotide as described herein). For general disclosure on how ATAC is spatially quantified using, for example clustering and/or t-SNE (where such cluster and/or t-SNE plots can be displayed in linked windows), see, United States Publication No. US-2020105373-A1 entitled "Systems and Methods for Cellular Analysis Using Nucleic Acid Sequencing" which is hereby incorporated by reference. For general disclosure on how V(D)J sequences are spatially quantified using, for example clustering and/or t-SNE (where such cluster and/or t-SNE plots can be displayed in linked windows), *see*, United States Patent Application No. 15/984,324, entitled "Systems and Methods for Clonotype Screening," filed May 19, 2018, which is hereby incorporated by reference.

In discrete attribute value datasets **1122** that have multiple images **1124** and thus multiple corresponding data constructs **1134,** spatially corresponding capture spots **1136** (probe spots) for the images will have the same barcode. Thus, the upper left capture spot for each image of a discrete attribute value dataset **1122** will have the same barcode and this barcode will be different than all the other probes spots for the images. To discriminate between these spatially corresponding capture spots across images, in some embodiments the barcodes will contain a suffix or a prefix, which will indicate from which image **1124** (that is, which data construct **1134)** the capture spot (and subsequent measurements **1138)** originated. Because the same barcodes are used in every image, this identifies which image each sequence read originated in. For instance, the barcode -- ATAAA-1 from a respective capture spot in the data construct 1134 for image **1124-1** will be different from ATAAA-2 in the spatially corresponding capture spot in the data construct 1134 for image **1124-2.**

In some embodiments, graph-based, k-Means, t-SNE and UMAP projections are derived from the single feature-barcode matrix that has been integrated across all the images **1124** of all the spatial projections of the discrete attribute set **1122.** Thus, in embodiments in which the discrete attribute value dataset includes multiple spatial projections, the mathematical projections will include the measurements **1138** for all capture spots **1136** (probe spots) across multiple spatial projections; a single t-SNE and UMAP plot per locus (gene, antibody capture, specific genetic loci on a reference genome) will be created per dataset **1122.** Thus, spots from similar tissue types or subtypes across multiple tissue slices should cluster together in the abstract t-SNE/UMAP/PCA space, but may span multiple spatial projections.

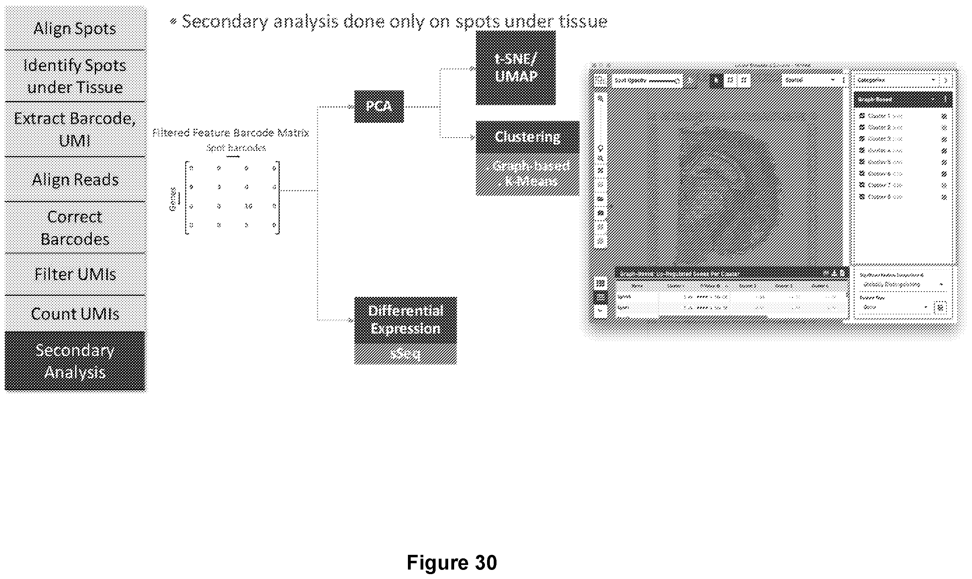

**FIG. 30** further illustrates how the composite representation of block **1066** is analyzed. In some embodiments, the raw feature barcode matrix is subjected to a dimension reduction algorithm such as principal components analysis to reduce G genes to top 10 metagenes. Then, t-SNE is run in the PCA space to generate a two-dimensional projection. Further, graph-based (Louvain) and k-means clustering (k=2...10) in PCA-space is used to identify clusters of cells. In some embodiments an sSeq (negative-binomial test) algorithm is used to find genes that most uniquely define each cluster. See, *for example,* United States Provisional Application No. 62/909,071, entitled "Systems and Methods for Visualizing a Pattern in a Dataset," filed October 1, 2019, which is hereby incorporated by reference.

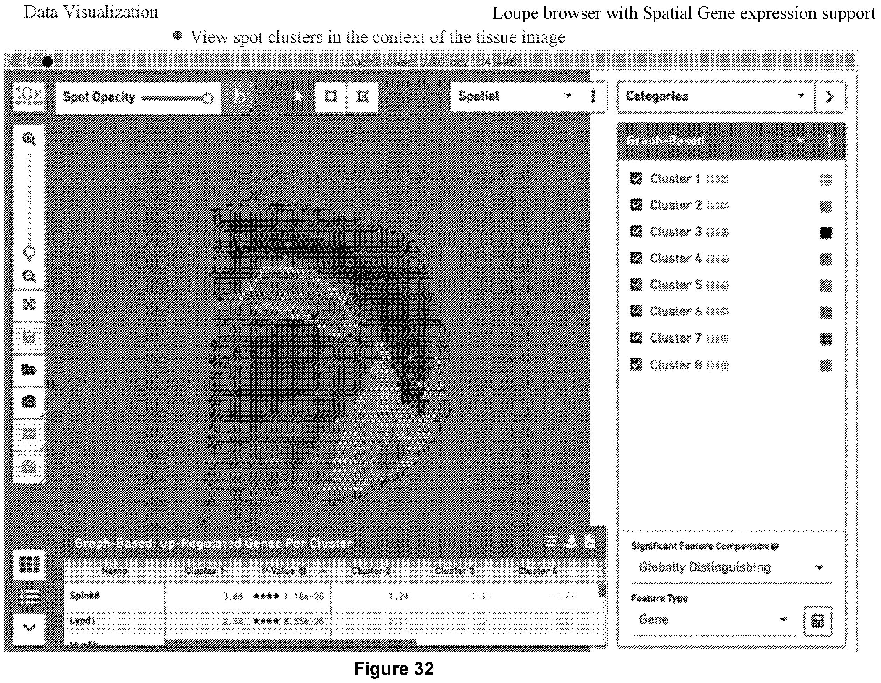

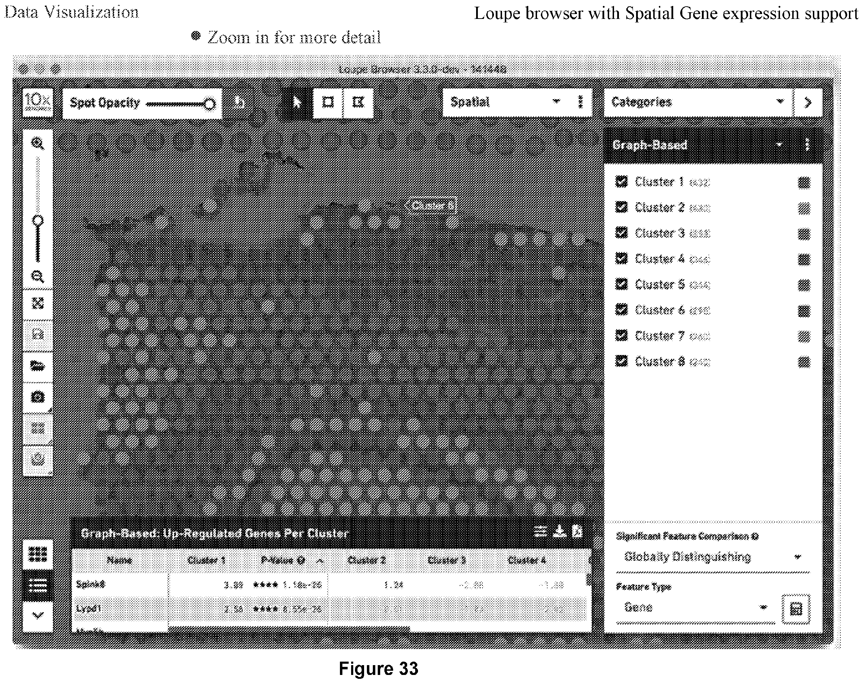

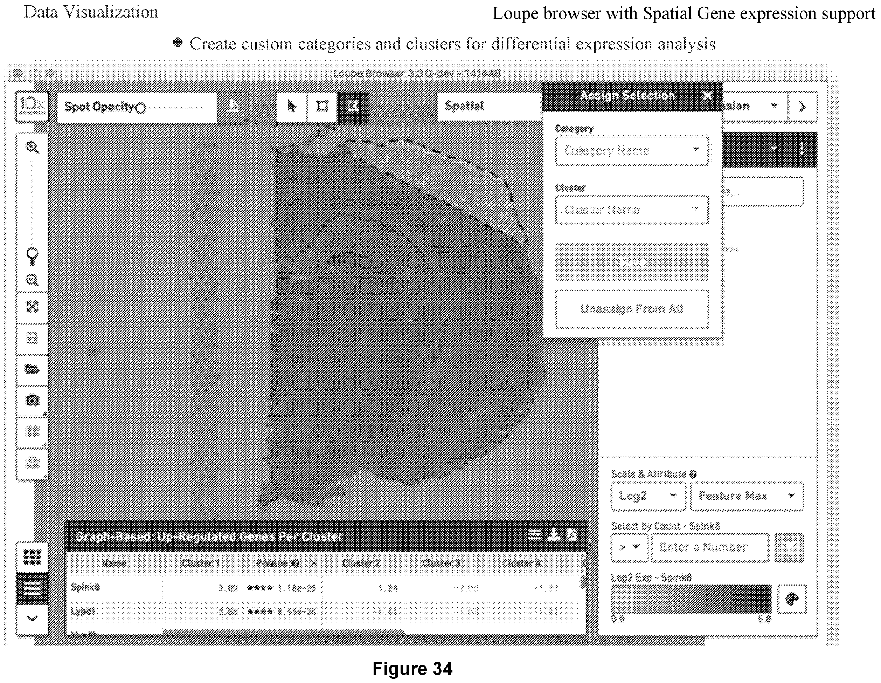

**FIG. 31** illustrates how the acquisition of the image **1124** (*e.g.*, block **1024 of FIG. 10B)** runs parallel, and in conjunction to, the above-described spatial sequencing (*e.g.*, blocks **1026 - 1030 of FIG. 10B). FIG. 32** illustrates the end result of this parallel analysis, with the display of the composite representation of the image **1124** and the nucleic acid sequencing data associated with each capture spot **1136,** in accordance with some embodiments of the present disclosure. **FIG. 33** illustrates how the composite representation can be zoomed in to see further detail as disclosed in United States Provisional Application No. 62/909,071, entitled "Systems and Methods for Visualizing a Pattern in a Dataset," filed October 1, 2019, which is hereby incorporated by reference. **FIG. 34** illustrates how custom categories and clusters for differential expression analysis can be performed as part of the analysis of the composite representation in accordance with some embodiments of the present disclosure.

In some embodiments, for each respective locus in a plurality of loci, a procedure is performed that that comprises i) performing an alignment of each respective sequence read in the plurality of sequence reads that maps to the respective locus thereby determining a haplotype identity for the respective sequence read from among a corresponding set of haplotypes for the respective locus, and ii) categorizing each respective sequence read in the plurality of sequence reads that maps to the respective locus by the spatial barcode of the respective sequence read and by the haplotype identity, thereby determining the spatial distribution of each haplotype in each corresponding set of haplotypes in the biological sample, where the spatial distribution includes, for each capture spot in the set of capture spots on the substrate, an abundance of each haplotype in the set of haplotypes for the respective locus. In some embodiments, the method further comprises using the spatial distribution to characterize a biological condition of the subject. In some embodiments, a respective locus in the plurality of loci is biallelic and the corresponding set of haplotypes for the respective locus consists of a first allele and a second allele. In some embodiments, the respective locus includes a heterozygous single nucleotide polymorphism (SNP), a heterozygous insert, or a heterozygous deletion. In some embodiments, the plurality of loci comprises between two and 100 loci, more than 10 loci, more than 100 loci, or more than 500 loci. In some embodiments, the plurality of loci from a lookup table, file or data structure. In some embodiments, the alignment algorithm is a local alignment that aligns the respective sequence read to a reference sequence using a scoring system that (i) penalizes a mismatch between a nucleotide in the respective sequence read and a corresponding nucleotide in the reference sequence in accordance with a substitution matrix and (ii) penalizes a gap introduced into an alignment of the sequence read and the reference sequence. In some embodiments, the local alignment is a Smith-Waterman alignment. In some embodiments, the reference sequence is all or portion of a reference genome. In some embodiments, the method further comprises removing from the plurality of sequence reads one or more sequence reads that do not overlay any loci in the plurality of loci. In some embodiments, the plurality of sequence reads are RNA-sequence reads and the removing comprises removing one or more sequences reads in the plurality of sequence reads that overlap a splice site in the reference sequence. In some embodiments, the plurality of loci include one or more loci on a first chromosome and one or more loci on a second chromosome other than the first chromosome. See, for example, United States Provisional Patent Application No. 62/886,223 entitled "Systems and Methods for Using the Spatial Distribution of Haplotypes to Determine a Biological Condition," filed August 13, 2019, which is hereby incorporated by reference.

**Example 1.**

The following example provides reaction schemes for the preparation of sequence reads for spatial analysis. **FIG. 37** also provides a reaction scheme for the preparation of sequence reads for spatial analysis.

In some non-limiting examples of the workflows described herein, the biological sample can be immersed in 100% chilled methanol and incubated for 30 minutes at -20 °C. After 20 minutes, the sample can be removed and rinsed in ultrapure water. After rinsing the sample, fresh eosin solution is prepared, and the sample can be covered in isopropanol. After incubating the sample in isopropanol for 1 minute, the reagent can be removed by holding the slide at an angle, where the bottom edge of the slide can be in contact with a laboratory wipe and air dried. The sample can be uniformly covered in hematoxylin solution and incubated for 7 minutes at room temperature. After incubating the sample in hematoxylin for 7 minutes, the reagent can be removed by holding the slide at an angle, where the bottom edge of the slide can be in contact with a laboratory wipe. The slide containing the sample can be immersed in water and the excess liquid can be removed. After that, the sample can be covered with blueing buffer and can be incubated for 2 minutes at room temperature. The slide containing the sample can again be immersed in water, and uniformly covered with eosin solution and incubated for 1 minute at room temperature. The slide can be air-dried for no more than 30 minutes and incubated for 5 minutes at 37°C. The sample can be imaged using a brightfield imaging setting.

Further, the biological sample can be processed by the following exemplary steps for sample permeabilization and cDNA generation. The sample can be exposed to a permeabilization enzyme and incubated at 37°C for the pre-determined permeabilization time (which is tissue type specific). The permeabilization enzyme can be removed and the sample prepared for analyte capture by adding 0.1X SSC buffer. The sample can then subjected to a pre-equilibration thermocycling protocol (e.g., lid temperature and pre-equilibrate at 53 °C, reverse transcription at 53 °C for 45 minutes, and then hold at 4 °C) and the SSC buffer can be removed. A Master Mix, containing nuclease-free water, a reverse transcriptase reagent, a template switch oligo, a reducing agent, and a reverse transcriptase enzyme can be added to the biological sample and substrate, and the sample with the Master Mix can be subjected to a thermocycling protocol (e. g., perform reverse transcription at 53 °C for 45 minutes and hold at 4 °C). Second strand synthesis can be performed on the substrate by subjecting the substrate to a thermocycling protocol (e.g., pre-equilibrate at 65 °C, second strand synthesis at 65 °C for 15 minutes, then hold at 4 °C). The Master Mix reagents can be removed from the sample and 0.8M KOH can be applied and incubated for 5 minutes at room temperature. The KOH can be removed and elution buffer can be added and removed from the sample. A Second Strand Mix, including a second strand reagent, a second strand primer, and a second strand enzyme, can be added to the sample and the sample can be sealed and incubated. At the end of the incubation, the reagents can be removed and elution buffer can be added and removed from the sample, and 0.8 M KOH can be added again to the sample and the sample can be incubated for 10 minutes at room temperature. Tris-HCl can be added and the reagents can be mixed. The sample can be transferred to a new tube, vortexed, and placed on ice.

Further the biological sample can be processed by the following exemplary steps for cDNA amplification and quality control. A qPCR Mix, including nuclease-free water, qPCR Master Mix, and cDNA primers, can be prepared and pipetted into wells in a qPCR plate. A small amount of sample can be added to the plated qPCR Mix, and thermocycled according to a predetermined thermocycling protocol (e.g., step 1: 98 °C for 3 minutes, step 2: 98 °C for 5 seconds, step 3: 63 °C for 30 seconds, step 4: record amplification signal, step 5: repeating 98 °C for 5 seconds, 63 °C for 30 seconds for a total of 25 cycles). After completing the thermocycling, a cDNA amplification mix, including amplification mix and cDNA primers, can be prepared and combined with the remaining sample and mixed. The sample can then be incubated and thermocycled (e.g., lid temperature at 105 °C for ~45-60 minutes; step 1: 98 °C for 3 minutes, step 2: 98 °C for 15 seconds, step 3: 63 °C for 20 seconds, step 4: 72 °C for one minute, step 5: [the number of cycles determined by qPCR Cq Values], step 6: 72 °C for 1 minute, and step 7: hold at 4 °C). The sample can then be stored at 4 °C for up to 72 hours or at -20 °C for up to 1 week, or resuspended in 0.6X SPRIselect Reagent and pipetted to ensure proper mixing. The sample can then be incubated at 5 minutes at room temperature, and cleared by placing the sample on a magnet (e.g., the magnet is in the high position). The supernatant can be removed and 80% ethanol can be added to the pellet, and incubated for 30 seconds. The ethanol can be removed and the pellet can be washed again. The sample can then be centrifuged and placed on a magnet (e.g., the magnet is on the low position). Any remaining ethanol can be removed and the sample can be air dried for up to 2 minutes. The magnet can be removed and elution buffer can be added to the sample, mixed, and incubated for 2 minutes at room temperature. The sample can then be placed on the magnet (e.g., on low position) until the solution clears. The sample can be transferred to a new tube strip and stored at 4 °C for up to 72 hours or at -20 °C for up to 4 weeks. A portion of the sample can be run on an Agilent Bioanalyzer High Sensitivity chip, where a region can be selected and the cDNA concentration can be measured to calculate the total cDNA yield. Alternatively, the quantification can be determined by Agilent Bioanalyzer or Agilent TapeStation.

Further, the biological sample can be processed by the following exemplary steps for spatial gene expression library construction. A Fragmentation Mix, including a fragmentation buffer and fragmentation enzyme, can be prepared on ice. Elution buffer and fragmentation mix can be added to each sample, mixed, and centrifuged. The sample mix can then be placed in a thermocycler and cycled according to a predetermined protocol (e.g., lid temperature at 65 °C for ~ 35 minutes, pre-cool block down to 4 °C before fragmentation at 32 °C for 5 minutes, End-repair and A-tailing at 65 °C for 30 minutes, and holding at 4 °C). The 0.6X SPRIselect Reagent can be added to the sample and incubated at 5 minutes at room temperature. The sample can be placed on a magnet (e.g., in the high position) until the solution clears, and the supernatant can be transferred to a new tube strip. 0.8X SPRIselect Reagent can be added to the sample, mixed, and incubated for 5 minutes at room temperature. The sample can be placed on a magnet (e.g., in the high position) until the solution clears. The supernatant can be removed and 80% ethanol can be added to the pellet, the pellet can be incubated for 30 seconds, and the ethanol can be removed. The ethanol wash can be repeated and the sample placed on a magnet (e.g., in the low position) until the solution clears. The remaining ethanol can be removed and elution buffer can be added to the sample, mixed, and incubated for 2 minutes at room temperature. The sample can be placed on a magnet (e.g., in the high position) until the solution clears, and a portion of the sample can be moved to a new tube strip. An Adaptor Ligation Mix, including ligation buffer, DNA ligase, and adaptor oligos, can be prepared and centrifuged. The Adaptor Ligation Mix can be added to the sample, pipette-mixed, and centrifuged briefly. The sample can then be thermocycled according to a predetermined protocol (e.g., lid temperature at 30 °C for ~15 minutes, step 1: 20 °C for 15 minutes, step 2: 4 °C hold). The sample can be vortexed to resuspend SPRIselect Reagent, additional 0.8X SPRIselect Reagent can be added to the sample and incubated for 5 minutes at room temperature, and placed on a magnet (e.g., in the high position) until the solution clears. The supernatant can be removed and the pellet can be washed with 80% ethanol, incubated for 30 seconds, and the ethanol can be removed. The ethanol wash can be repeated, and the sample can be centrifuged briefly before placing the sample on a magnet (e.g., in the low position). Any remaining ethanol can be removed and the sample can be air dried for a maximum of 2 minutes. The magnet can be removed, and elution buffer can be added to the sample, and the sample can be pipette-mixed, incubated for 2 minutes at room temperature, and placed on a magnet (e.g., in the low position) until the solution clears. A portion of the sample can be transferred to a new tube strip. Amplification mix, can be prepared and combined with the sample. An individual Dual Index TT Set A can be added to the sample, pipette-mixed and subjected to a pre-determined thermocycling protocol (e.g., lid temperature at 105 °C for ~25-40 minutes, step 1: 98 °C for 45 seconds, step 2: 98 °C for 20 seconds, step 3: 54 °C for 30 seconds; step 4: 72 °C for 20 seconds, step 5: reverting to step 2 for a predetermined number of cycles, step 6: 72 °C for 1 minute, and 4 °C on hold). Vortex to resuspend the SPRIselect Reagent, additional 0.6X SPRIselect Reagent can be added to each sample, mixed, and incubated for 5 minutes at room temperature. The sample can be placed on a magnet (e.g., in the high position) until the solution clears, and the supernatant can be transferred to a new tube strip. The 0.8X SPRIselect Reagent can be added to each sample, pipette-mixed, and incubated for 5 minutes at room temperature. The sample can then be placed on a magnet (e.g., in the high position) until the solution clears. The supernatant can be removed, and the pellet can be washed with 80% ethanol, incubated for 30 seconds, and then the ethanol can be removed. The ethanol wash can be repeated, the sample centrifuged, and placed on a magnet (*e.g.*, in the low position) to remove any remaining ethanol. The sample can be removed from the magnet and Elution Buffer can be added to the sample, pipette-mixed, and incubated for 2 minutes at room temperature. The sample can be placed on a magnet (*e.g.*, in the low position) until the solution clears and a portion of the sample can be transferred to a new tube strip. The sample can be stored at 4 °C for up to 72 hours, or at -20 °C for long-term storage. The average fragment size can be determined using a Bioanalyzer trace or an Agilent TapeStation.

The library can be sequenced using available sequencing platforms, including, MiSeq, NextSeq 500/550, HiSeq 2500, HiSeq 3000/4000, NovaSeq, and iSeq.

In non-limiting examples of any of the workflows described herein, a nucleic acid molecule is produced that includes a contiguous nucleotide sequence comprising: (a) a first primer sequence (e.g., Read 1); (b) a spatial barcode; (c) a unique molecular sequence (UMI); (d) a capture domain; (e) a sequence complementary to a sequence present in a nucleic acid from a biological sample; (f) a second primer sequence (*e.g.*, Read 2) that is substantially complementary to a sequence of a template switching oligonucleotide (TSO). In some embodiments of these nucleic acid molecules, the nucleic acid molecule is a single-stranded nucleic acid molecule. In some embodiments of these nucleic acid molecules, the nucleic acid molecule is a double-stranded nucleic acid molecule. In some embodiments of these nucleic acid molecules, (a) through (f) are positioned in a 5' to 3' direction in the contiguous nucleotide sequence. In some embodiments of any of these nucleic acid molecules, the nucleic acid molecule is attached to a substrate (e.g., a slide). In some embodiments of any of these nucleic acid molecules, the 5' end of the contiguous nucleic acid sequence is attached to the substrate (e.g., a slide). In some embodiments of any of these nucleic acid molecules, the contiguous nucleotide sequence is a chimeric RNA and DNA sequence. In some embodiments of any of these nucleic acid molecules, the contiguous nucleotide sequence is a DNA sequence.

In non-limiting examples of any of the workflows described herein, a nucleic acid molecule is produced that includes a contiguous nucleotide sequence comprising: (a) a sequence complementary to a first primer sequence (e.g., a sequence complementary to Read 1); (b) a sequence complementary to a spatial barcode; (c) a sequence complementary to a unique molecular sequence; (d) a sequence complementary to a capture domain; (e) a sequence present in a nucleic acid from a biological sample; and (f) a sequence of a template switching oligonucleotide (TSO). In some embodiments of any of these nucleic acid molecules, the nucleic acid molecule is single-stranded. In some embodiments of any of these nucleic acid molecules, the nucleic acid molecule is double-stranded. In some embodiments of any of these nucleic acid molecules, the contiguous nucleotide sequence is a DNA sequence. In some embodiments of any of these nucleic acid molecules, (a) through (f) are positioned in a 3' to 5' direction in the contiguous nucleotide sequence.

In non-limiting examples of any of the workflows described herein, a nucleic acid molecule is produced that includes a contiguous nucleotide sequence comprising: (a) a first primer sequence (e.g., Read 1); (b) a spatial barcode; (c) a unique molecular sequence (UMI); (d) a capture domain; (e) a sequence complementary to a sequence present in a nucleic acid from a biological sample; and (f) a second primer sequence (Read 2). In some embodiments of any of these nucleic acid molecules, the nucleic acid molecule is a single-stranded nucleic acid molecule. In some embodiments of any of these nucleic acid molecules, the nucleic acid molecule is a double-stranded nucleic acid molecule. In some embodiments of any of these nucleic acid molecules, (a) through (f) are positioned in a 5' to 3' direction in the contiguous nucleotide sequence. In some embodiments of any of these nucleic acid molecules, the contiguous nucleotide sequence is a DNA sequence. In some embodiments of any of these nucleic acid molecules, the contiguous nucleotide sequence further comprises 3' to (f): (g) a sequence complementary to a first adaptor sequence; and (h) a sequence complementary to a third primer sequence. In some embodiments of any of the nucleic acid molecules, the first adaptor sequence is an i7 sample index sequence. In some embodiments of any of these nucleic acid molecules, the third primer sequence is a P7 primer sequence. See, Illumina, Indexed Sequencing Overview Guides, February 2018, Document 15057455v04; and Illumina Adapter Sequences, May 2019, Document #1000000002694v11, each of which is hereby incorporated by reference, for information on P5, P7, i7, i5, TruSeq Read 2, indexed sequencing, and other reagents described herein. In some embodiments of any of these nucleic acid molecules, (h) is 3' positioned relative to (g) in the contiguous nucleotide sequence. In some embodiments of any of these nucleic acid molecules, the contiguous nucleotide sequence further comprises 5' to (a): (i) a second adaptor sequence; and (ii) a fourth primer sequence. In some embodiments of any of these nucleic acid molecules, the second adaptor sequence is an i5 sample index sequence. In some embodiments of any of these nucleic acid molecules, the fourth primer sequence is a P5 primer sequence. In some embodiments of any of these nucleic acid molecules, (ii) is 5' positioned relative to (i) in the contiguous nucleotide sequence.

In non-limiting examples of any of the workflows described herein, a nucleic acid molecule is produced that includes a contiguous nucleotide sequence comprising: (a) a sequence complementary to a first primer sequence; (b) a sequence complementary to a spatial barcode; (c) a sequence complementary to a unique molecular sequence; (d) a sequence complementary to a capture domain; (e) a sequence present in a nucleic acid from a biological sample; and (f) a sequence complementary to a second primer sequence. In some embodiments of these nucleic acid molecules, a sequence complementary to a first primer sequence is a sequence complementary to Read 1. In some embodiments of these nucleic acid molecules, a sequence complementary to a second primer sequence is a sequence complementary to Read 2. In some embodiments of any of these nucleic acid molecules, the nucleic acid molecule is a single-stranded nucleic acid molecule. In some embodiments of any of these nucleic acid molecules, the nucleic acid molecule is a double-stranded nucleic acid molecule. In some embodiments of any of these nucleic acid molecules, (a) through (f) are positioned in a 3' to 5' direction in the contiguous nucleotide sequence. In some embodiments of any of these nucleic acid molecules, the contiguous nucleotide sequence is a DNA sequence. In some embodiments of any of these nucleic acid molecules, the contiguous nucleotide sequence further comprises 5' to (f): (g) a first adaptor sequence; and (h) a third primer sequence. In some embodiments of any of these nucleic acid molecules, the first adaptor sequence is an i7 sample index sequence. In some embodiments of any of these nucleic acid molecules, the third primer sequence is a P7 primer sequence. In some embodiments of any of these nucleic acid molecules, (h) is 5' positioned relative to (g) in the contiguous nucleotide sequence. In some embodiments of any of these nucleic acid molecules, the contiguous nucleotide sequence further comprises 3' to (a): (i) a sequence complementary to a second adaptor sequence; and (ii) a sequence complementary to a fourth primer sequence. In some embodiments of any of these nucleic acid molecules, the second adaptor sequence is an i5 sample index sequence. In some embodiments of any of these nucleic acid molecules, the fourth primer sequence is a P5 primer sequence. In some embodiments of any of these nucleic acid molecules, (ii) is 3' positioned relative to (i) in the contiguous nucleotide sequence.

## Example 2

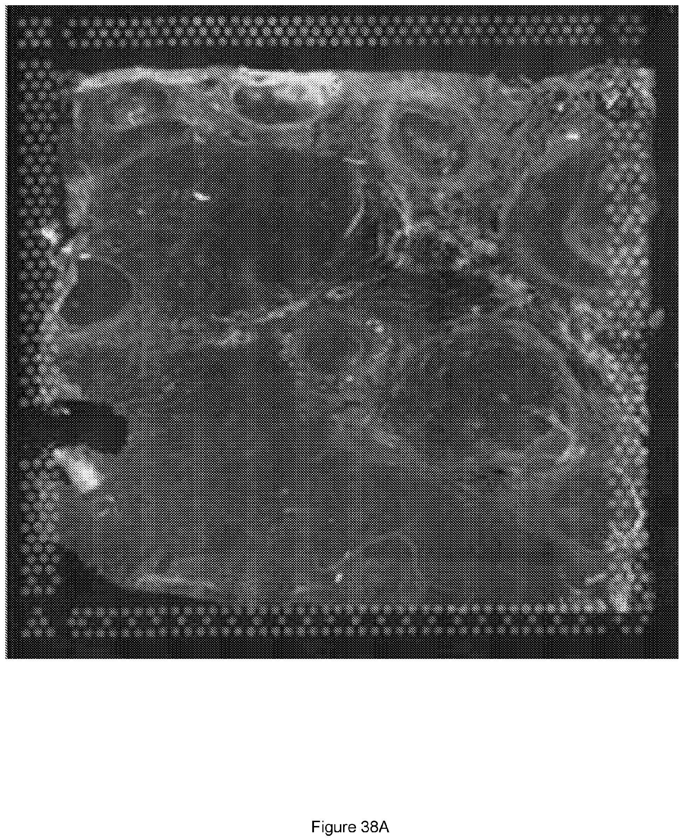

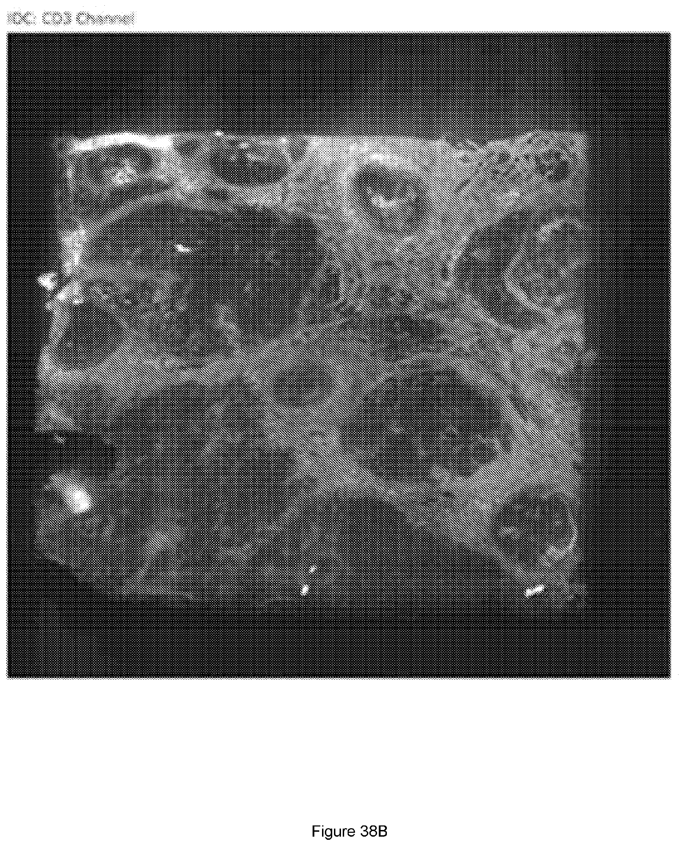

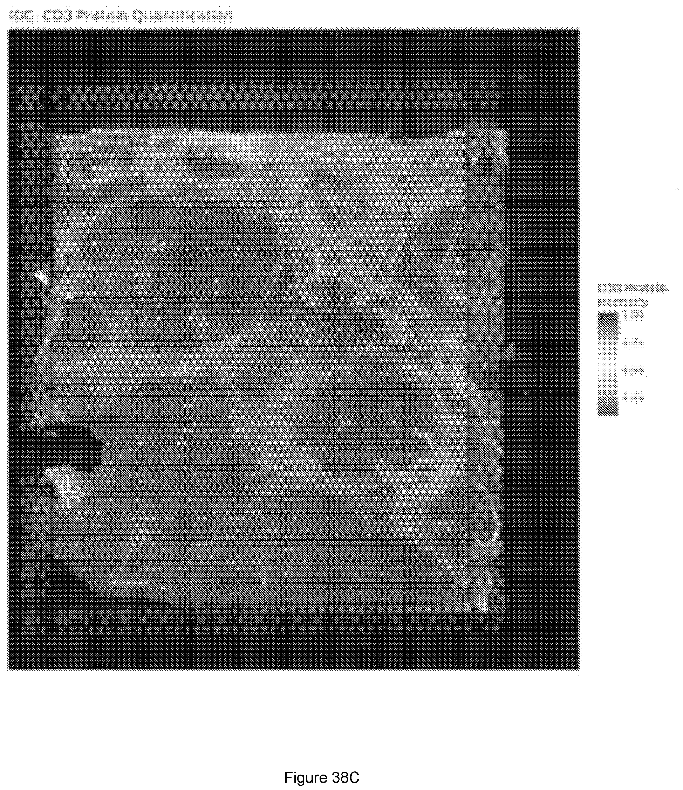

**FIG. 38A** illustrates the case in which all of the images **1124** of a spatial projection in a discrete attribute value dataset 1122 are fluorescence images and are all displayed, whereas Figure 38B shows the case where only one of the fluorescence images (CD3 channel) of this spatial projection is displayed. In some embodiments, relative brightness in fluorescence images has a semi-quantitative relationship to some aspect of the sample under study. For instance, if the fluorescence arises in an immunohistochemistry fluorescent imaging experiment, then brighter areas have greater binding of some antibody to a protein. For example, **FIG. 38C** shows CD3 protein quantification using the image of **FIG. 38B.**

## Example 3 - *Methods for using a spatially-tagged analyte capture agent in a biological sample*

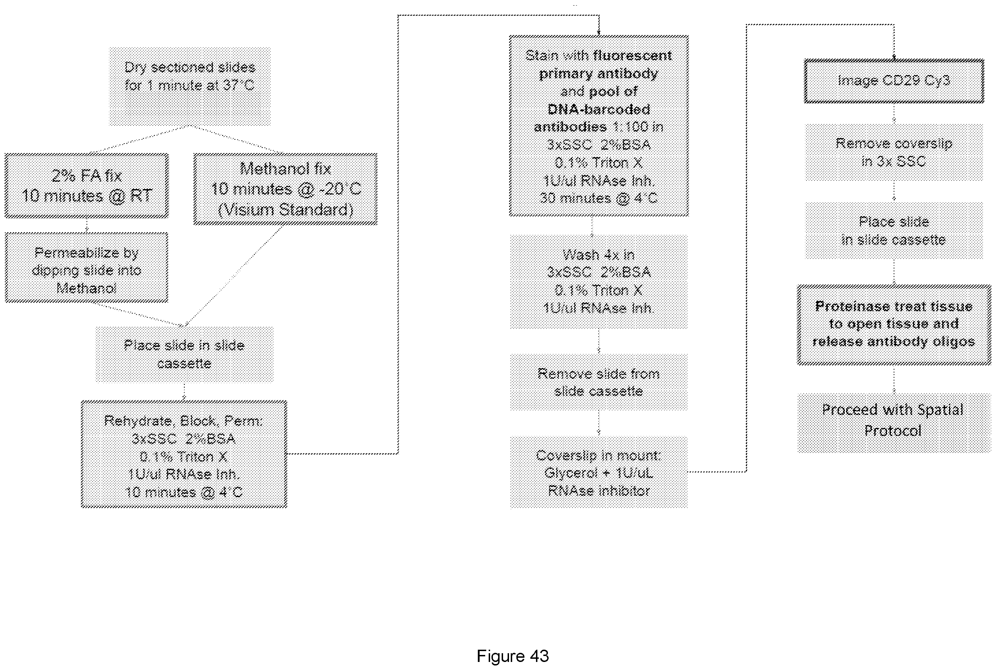

In a non-limiting example, DNA-barcoded antibodies are used to detect proteins in a biological sample. For example, a method of detecting proteins within a tissue sample using DNA-barcoded antibodies can include: (a) providing a capture probe array, where the capture probes include a spatial barcode and a capture domain; (b) contacting the substrate with a tissue sample (*e.g.*, mouse spleen tissue) and drying the sectioned slides for 1 minute at 37°C; (c) fixing the tissue sample with either 2% formaldehyde at room temperature or with methanol at -20°C for 10 minutes; (d) rehydrating, blocking and permeabilizing the tissue sample with 3X SSC, 2% BSA, 0.1% Triton X, and 1U/$\mu$l RNAse inhibitor for 10 minutes at 4°C; (e) staining the tissue sample with fluorescent primary antibodies and a pool of DNA-barcoded antibodies in 3X SSC, 2% BSA, 0.1% Triton X, and 1U/$\mu$l RNAse inhibitor for 30 minutes at 4°C; (f) imaging the tissue sample to spatially detect target proteins (*e.g.*, CD29, CD3) within the tissue using fluorescently-labelled and DNA-barcoded antibodies; (g) treating the tissue sample with a protease to permeabilize the tissue and release the antibody oligonucleotides; and (h) performing spatial transcriptomic analysis to identify the location of the target protein within the tissue sample. The steps of this method are depicted in **FIG. 43.**

The DNA-barcoded antibodies can include an analyte binding moiety (*e.g.*, antibody) and a capture agent barcode domain. The antibodies interact with the target protein of the biological sample, and the capture agent barcode domains interact with the capture probes on the substrate. The fluorescence level from the primary antibodies interacting with the proteins of the biological sample is imaged in step (f), and the spatially-tagged analyte capture agents associated with the capture probes are used to identify the location of the target protein within the biological sample. In some embodiments, non-specific antibody staining can be reduced by introducing a blocking probe to the analyte capture agent(s), prior to applying the analyte capture agents to a tissue sample.

In some embodiments, detecting and identifying the location of a target protein can be performed individually for each analyte of interest. In some embodiments, multiple proteins can be detected and spatially profiled concurrently within the same tissue sample. In some embodiments, multiplexing (*e.g.*, concurrently detecting multiple markers) allows for examination of the spatial arrangement of analytes of interest (*e.g.*, proteins, DNA, RNA) as well as analyte interaction and co-localization thereby facilitating simultaneous analysis of multiple tissue markers.

## Example 4 - *Methods for using spatially-tagged analyte capture agents to detect multiple target proteins by introducing antibodies linked to capture agents and molecular identifiers in a biological sample*

In a non-limiting example, multiple pluralities of DNA-barcoded antibodies can be used to concurrently detect multiple target proteins (*e.g.*, multiplexing) within a biological sample. For example, a method of detecting multiple target proteins within a biological sample can include using two or more pluralities of analyte capture agents **4002** that bind to two or more pluralities of analytes. Each analyte species is associated with a spatially-tagged analyte capture agent **4002** plurality, where each spatially-tagged analyte capture agent plurality possesses a barcode unique to the analyte. Multiple analytes **4006** can be detected and analyzed at the same time by determining the analyte binding moiety barcode that can be determined together with, or separately from the spatial transcriptome analysis using a sequencing technology, as described elsewhere herein. In other embodiments, antibody-barcodes can be determined by fluorescent *in situ* hybridization or *in situ* sequencing approaches, as described elsewhere herein.

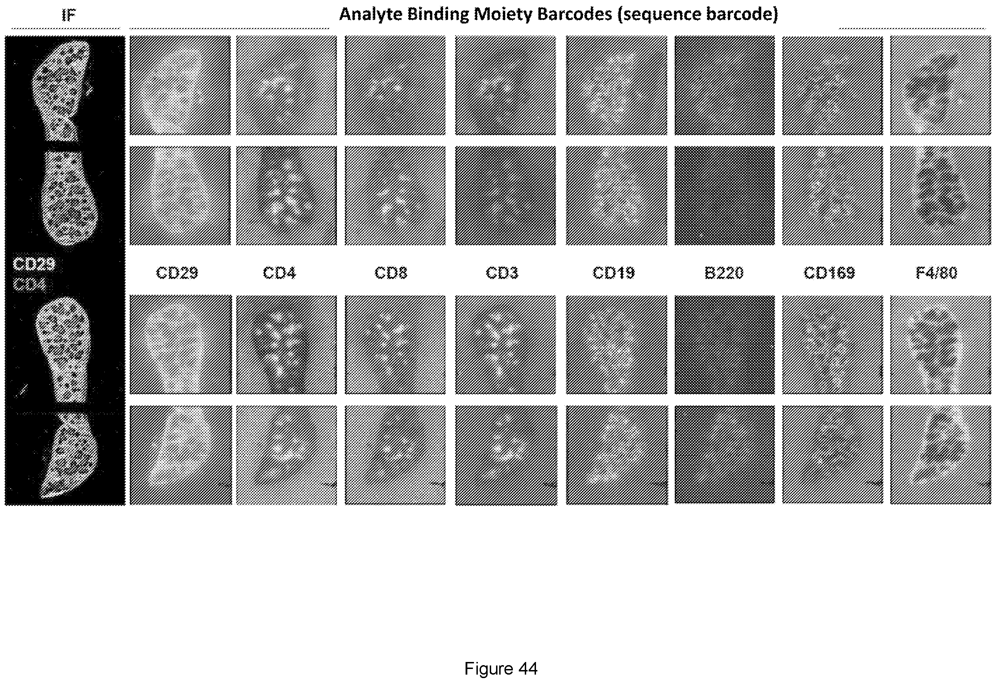

For example, **FIG. 44** shows exemplary multiplexed DNA-barcoded and fluorescent antibody staining and sequencing results using the method depicted above, where the left immunofluorescent image shows tissue sections of mouse spleen with fluorescent and DNA-barcoded antibodies bound to CD29 and CD4. CD29 (Integrin beta 1) is a cell surface marker expressed in many stromal cells and can be seen in the red pulp portion of the spleen, while CD4 is a cell surface marker for T cell subsets and can be seen in the pockets of white pulp of the spleen. The images on the right show the location of the antibody barcodes recognizing target proteins, CD29, CD3, CD4, CD8, CD19, B220, F4/80, and CD169 within the tissue sample using the multiple spatially-tagged analyte capture agents **4002**. Each spatially-tagged analyte capture agent plurality possess an analyte binding moiety barcode unique to that plurality. **FIG. 44** shows that CD3, CD4, and CD8, all cell surface markers for T cells, are seen to be located in the pockets of white pulp of the spleen. CD19, a cell surface marker for B cells, CD29, a cell surface marker for stromal cells, and F4/80 and CD169, both markers for macrophage cells, can be seen within the red pulp and white pulp respectively of the spleen tissue. The data of **FIG. 44** indicates that protein detection using DNA-barcoded antibodies can be used for concurrently identifying spatial locations of multiple (e.g., two or more, three or more) target proteins within a tissue sample.

### Example 5 - *Exemplary spatial proteomic and genomic analysis*

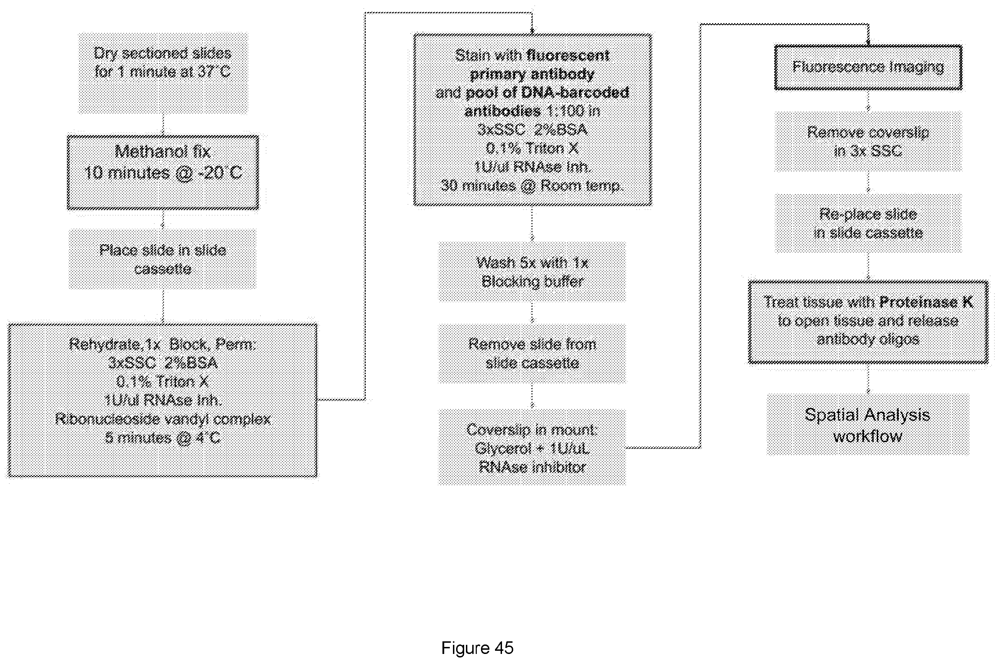

An exemplary protocol for spatial proteogenomic analysis is shown in **FIG. 45.** To prepare a sample for spatial proteogenomic analysis, a fresh-frozen tissue section mounted on a spatial analysis slide (*e.g.*, on an array including a plurality of capture probes, a capture probe of the plurality of capture probes including (i) a spatial barcode, (ii) a unique molecular identifier, and (iii) a capture domain, where the capture domain interacts specifically with an analyte capture agent) was dried for 1 minute at 37 °C. The tissue section was fixed with methanol for 10 minutes at -20 °C. The slide was then placed in a slide holder.

The slide was rehydrated with a 1X blocking and permeabilization solution containing 3X SSC (saline sodium citrate), 2% (w/v) BSA (bovine serum albumin), 0.1% (v/v) Triton X-100, 1 U/$\mu$L Protector RNAse inhibitor (Roche), and 20 mM ribonucleotide vandyl complex for 5 minutes at 4°C.

The blocked slide was stained with a fluorescent primary antibody and a pool of analyte capture agents (e.g., an antibody conjugated to an capture agent barcode domain) 1:100 in 3X SSC, 2% BSA, 0.1% Triton X-100, 1 U/$\mu$L for 30 minutes at room temperature. The stained slide was then washed five times with blocking buffer, followed by removal of the slide from the slide holder.

The stained slide was prepared for fluorescence imaging by mounting a coverslip using glycerol and 1 U/$\mu$L RNAse inhibitor. Fluorescence imaging was then performed. The coverslip was removed using 3X SSC, and the slide was placed again in the slide holder.

The tissue was treated with Proteinase K, and a spatial analysis workflow as described herein was performed to analyze the spatial location of the analyte capture probes and nucleic acids released from the tissue.

### Example 6

Understanding the cellular composition and gene expression of the mammalian central nervous system (CNS) can be helpful for gaining insights into normal, developing, and diseased neuronal tissues. While single cell RNA-seq (scRNA-seq) makes it possible to obtain high-resolution gene expression measurements, the technique requires cells to be dissociated from the CNS, thereby losing anatomical and organizational information. By combining histological techniques and the massive throughput of RNA-seq, this limitation has been addressed. Unbiased capture of native mRNA was achieved using ~5000 different molecularly barcoded, spatially encoded capture probes onto a slide over which tissue was placed, imaged, and permeabilized. RNAseq data was then mapped back to image coordinates placing gene expression into context within the tissue image.

Both immunofluorescent staining and oligo-conjugated antibodies (TOTALSEQ™ from BioLegend) were used to spatially resolve cell-specific proteomic markers along with gene expression in the same tissue. This technique is demonstrated in this Example using serial sections of fresh frozen human cerebrum, cerebellum, and spinal cord. By aggregating proteomic and transcriptomic data from serial sections, the resolution of cell-type identification was improved. This "multi-omics" approach can provide a powerful complement to traditional histopathology, enabling a greater understanding of cellular heterogeneity and organization within the mammalian CNS. This new, more detailed view of the human CNS anatomy as it varies across different regions, can provide essential insight into the cell type-specific nature of neurobiology and neurodegenerative diseases.

This Example demonstrates the ability to examine anatomical and transcriptome profiles from the same tissue section at a much higher resolution and sensitivity, at a shorter time than before (see, *e.g.,* Science. 2016 Jul 1,353(6294):78-82, incorporated herein by reference in its entirety). Also demonstrated in this Example is spatial clustering that correlates with the neuroanatomy across multiple human CNS regions, and that the addition of immunostaining and protein detection using analyte capture agents allows for the simultaneous examination of protein and gene expression from the same tissue.

*Spatial gene expression complemented by protein expression*

Combining immunostaining with spatial transcriptomic analysis showed good agreement between the two techniques. See the disclosure of Figure 58 in Example 17 of PCT/US2020/049048, filed September 2, 2020, which is hereby incorporated by reference.

*Use of conjugated antibody-oligos for spatial proteogenomic analysis*

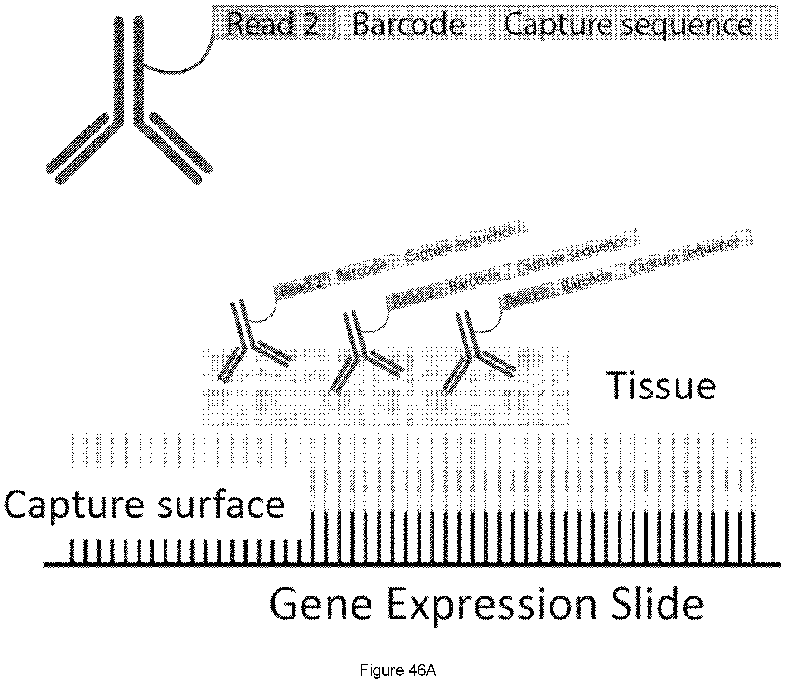

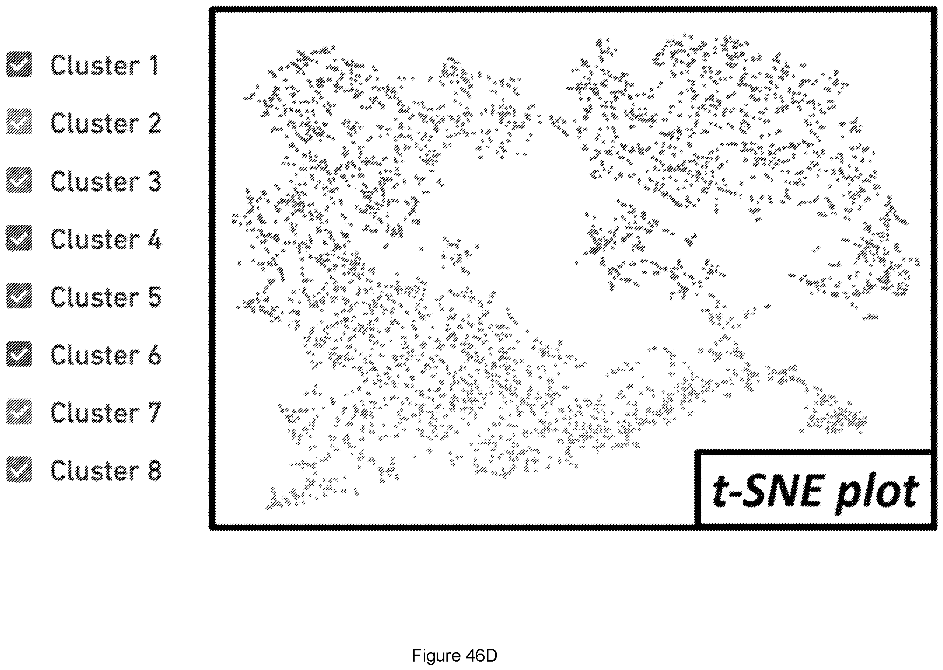

Analyte capture agents (in this case, antibodies coupled to an oligonucleotide containing an analyte capture sequence, analyte binding moiety barcode, and a PCR handle compatible with NGS assays) (TOTALSEQ™-A oligo-conjugated antibodies (BioLegend, San Diego)) were used to analyze human cerebellar tissue (BiolVT-Asterand). These analyte capture agents are designed to work with any sequencing platform that relies on poly-dT oligonucleotides mimicking natural mRNA, thus allowing capture by spatial analysis slides (a basic schematic is shown in **FIG. 46A).** Immunostained samples generated robust spatial clustering, highlighting the laminar organization of the cerebellum, as shown in **FIGS. 46B-C. FIG. 46B** shows a merged fluorescent image of DAPI staining of a section of human cerebellum, and **FIG. 46C** shows a spatial transcriptomic analysis of the same section, overlaid on **FIG. 46B). FIG. 46D** shows a t-SNE projection of the sequencing data illustrating cell-type clustering of the cerebellum. **FIGs. 46E-I** show spatial gene expression (top) and protein staining (bottom) of astrocyte marker glutamine synthase produced by hybridoma (clone O91F4) **(FIG. 46E);** oligodendrocyte markers myelin CNPase (produced by hybridoma clone SMI91) **(FIG. 46F)** and myelin basic protein (produced by hybridoma clone P82H9) **(FIG. 56G);** stem cell marker SOX2 (produced by hybridoma clone 14A6A34) **(FIG. 46H);** and neuronal marker SNAP-25 (produced by hybridoma clone SMI81) **(FIG. 46I),** each overlaid on **FIG. 46B.** Protein staining was carried out using the protocol in Example 5. Scale bar = 1 mm. See also the mutli-omic examination of human spinal cord data of Example 17 and Fig. 60 of PCT/US2020/049048, filed September 2, 2020, which is hereby incorporated by reference.

***Example 7***

**FIG. 47** is an exemplary spatial workflow for the detection of protein analytes in a biological sample. Blocking hybridization of the analyte capture sequence and the capture domain was tested with analyte capture sequences blocked with blocking probes of different lengths (*e.g.*, 9, 14, 16 or 22 nucleotides long) and different compositions (*e.g.*, inosine) and capture domains of different lengths (*e.g.*, 14, 16, or 22 nucleotides long). The various blocking schemes tested are shown in in Table 2 below. The melting temperature (Tm) is based on 19.5 mM salt (Na$^+$) in 0.1X SSC buffer and 20 μM of the blocking probe. The Tm for a uracil containing blocking probe, inosine blocking probe, and abasic blocking probe is based on the longest fragment after cleavage.

Table 2. Blocking Probe Schemes

| Blocker Name | Blocker Sequence | Tm in 3X SSC | Tm in 0.1X SSC |
|---|---|---|---|
| x9 blocker (3') | TTGCTAGGA | 47 | 27.1 |
| x9 blocker 5' (for x14/16) | TAGGACCGG | 53.2 | 35.5 |
| x9 slide | CGGTCCTAG | 50.1 | 32.7 |
| x14 blocker with U | GCCGGUCCUAGCAA | 72.2 | 18.9* |
| x16 abasic | TTGCTAG /idSp/ /idSp/ /idSp/CGGCCT | 40 | 25.6 |
| x16 insoine | TTGCTAIGACCIGCCT | 77.5 | 0.5* |

(continued)

| Blocker Name | Blocker Sequence | Tm in 3X SSC | Tm in 0.1X SSC |
|---|---|---|---|
| x22 abasic | `TTG CTA GGA /idSp/ /idSp/ /idSp/ /idSp/ /idSp/ CTTA AAG C` | 47 | 20.5 |
| x22 inosine | TTGCTAIGACCIICCTTAAIGC | 81.9 | 0* |
| x22 blocker with U | GCTTUAAGGUCGGUCCUAGCAA | 76.8 | 0* |

Mouse spleen samples were fixed in 100% methanol for 30 minutes at -20°C. The TotalSeq antibodies (BioLegend) were incubated with the various blocking probes for 30 minutes to hybridize to the analyte capture sequence. The biological sample was stained and contacted with the analyte capture agents including the blocked analyte capture sequences in 3X SSC for 30 minutes at 4°C. After staining, the biological sample was rinsed in five times in 0.1X SSC at 37°C. Blocking probes removed via an enzyme were incubated in an enzyme blocker removal mix for the 30 minutes. For example, USER cleaves uracil, endonuclease V cleaves inosine, and endonuclease IV cleaves abasic sites. Blocking probes were released from the analyte capture sequences prior to the biological sample being permeabilized with Proteinase K and 1% SDS, thus allowing the analyte capture sequence to hybridize to the capture domain. Following capture of the analyte capture sequence by the capture domain, reverse transcription and second strand synthesis were performed followed by library construction and sequencing. More details are provided in United States Provisional Patent Application Number 63/110,749 entitled "Compositions and Methods for Binding an Analyte to a Capture Probe," which is hereby incorporated by reference.

## REFERENCES CITED AND ALTERNATIVE EMBODIMENTS

All publications, patents, patent applications, and information available on the internet and mentioned in this specification are herein incorporated by reference to the same extent as if each individual publication, patent, patent application, or item of information was specifically and individually indicated to be incorporated by reference. To the extent publications, patents, patent applications, and items of information incorporated by reference contradict the disclosure contained in the specification, the specification is intended to supersede and/or take precedence over any such contradictory material.

The present invention can be implemented as a computer program product that comprises a computer program mechanism embedded in a nontransitory computer readable storage medium. For instance, the computer program product could contain the program modules shown in FIGS. 11A and 11B, and/or described in FIGS. 10A, 10B, 10C, 10D, 10E, and 10F. These program modules can be stored on a CD-ROM, DVD, magnetic disk storage product, USB key, or any other non-transitory computer readable data or program storage product.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Many modifications and variations of this invention can be made without departing from its spirit and scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims, along with the full scope of equivalents to which such claims are entitled.

The following items are disclosed:

1. A method of spatial analysis of analytes comprising:

A) placing a sample on a substrate, wherein the substrate comprises a plurality of fiducial markers and a set of capture spots, wherein the set of capture spots comprises at least 1000 capture spots;
B) obtaining one or more images of the sample on the substrate, wherein each respective image of the one or more images comprises a corresponding plurality of pixels in the form of a corresponding array of pixel values, wherein the corresponding array of pixel values comprises at least 100,000 pixel values;
C) obtaining a plurality of sequence reads, in electronic form, from the set of capture spots after the A) placing, wherein:

each respective capture probe plurality in a set of capture probe pluralities is (i) at a different capture spot in the set of capture spots and (ii) directly or indirectly associates with one or more analytes from the sample, each respective capture probe plurality in the set of capture probe pluralities is characterized by at least one unique spatial barcode in a plurality of spatial barcodes, the plurality of sequence reads comprises sequence reads corresponding to all or portions of the one or more analytes, the plurality of sequence reads comprises at least 10,000 sequence reads, and each respective sequence read in the plurality of sequence reads includes a spatial barcode of the corresponding capture probe plurality in the set of capture probe pluralities or a complement thereof;

D) using all or a subset of the plurality of spatial barcodes to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots; and

E) using the plurality of fiducial markers to provide a composite representation comprising (i) the one or more images aligned to the set of capture spots on the substrate and (ii) a representation of all or a portion of each subset of sequence reads at each respective position within the one or more images that maps to a respective capture spot corresponding to the respective position of the one or more analytes in the sample by aligning a first image in the one or more images to the set of capture spots by a procedure that comprises:

analyzing the corresponding array of pixel values of the first image to identify a plurality of derived fiducial markers of the first image;

using a substrate identifier uniquely associated with the substrate to select a first template in a plurality of templates, wherein each template in the plurality of templates comprises reference positions for a corresponding plurality of reference fiducial markers and a corresponding coordinate system;

aligning the plurality of derived fiducial markers of the first image with the corresponding plurality of reference fiducial markers of the first template using an alignment algorithm to obtain a transformation between the plurality of derived fiducial markers of the first image and the corresponding plurality of reference fiducial markers of the first template; and

using the transformation and the coordinate system of the first template to locate a corresponding position in the first image of each capture spot in the set of capture spots

by running a plurality of heuristic classifiers on the plurality of pixels, wherein, for each pixel in the plurality of pixels, each respective heuristic classifier in the plurality of heuristic classifiers casts a vote for the respective pixel between a first class and the second class, thereby forming a corresponding aggregated score for each respective pixel in the plurality of pixels, and applying the aggregated score and intensity of each respective pixel in the plurality of pixels to a segmentation algorithm to independently assign a probability to each respective pixel in the plurality of pixels of being sample or background.

2. The method of item 1, wherein the composite representation provides a relative abundance of nucleic acid fragments mapping to each analyte in a plurality of analytes at each capture spot in the plurality of capture spots.

3. The method of item1, wherein each corresponding aggregated score is a class in a set of classes comprising obvious first class, likely first class, likely second class, and obvious second class.

4. The method of item1, wherein the method further comprises, for each respective locus in a plurality of loci, performing a procedure that comprises:

i) performing an alignment of each respective sequence read in the plurality of sequence reads that maps to the respective locus thereby determining a haplotype identity for the respective sequence read from among a corresponding set of haplotypes for the respective locus, and

ii) categorizing each respective sequence read in the plurality of sequence reads that maps to the respective locus by the spatial barcode of the respective sequence read and by the haplotype identity,

thereby determining a spatial distribution of each haplotype in each corresponding set of haplotypes in the sample, wherein the spatial distribution includes, for each capture spot in the set of capture spots on the substrate, an abundance of each haplotype in the set of haplotypes for the respective locus.

5. The method of item4, the method further comprises using the spatial distribution to characterize a biological condition in a subject.

6. The method of item2, the method further comprising:
overlaying a mask on the first image, wherein the mask causes each respective pixel in the plurality of pixels of the first image that has been assigned a greater probability of being sample to be assigned a first attribute and each respective pixel in the plurality of pixels that has been assigned a greater probability of being background to be assigned a second attribute.

7. The method of item6, wherein the first attribute is a first color and the second attribute is a second color.

8. The method of item7, wherein the first color is one of red and blue and the second color is the other of red and blue.

9. The method of item6, wherein the first attribute is a first level of brightness or opacity and the second attribute is a second level of brightness or opacity.

10. The method of item6, the method further comprising:
assigning each respective representation, of a capture spot in the plurality of capture spots in the composite representation, the first attribute or the second attribute based upon the independent assignment of pixels in the vicinity of the respective representation of the capture spot in the composite representation.

11. The method of item1, wherein a capture spot in the set of capture spots comprises a capture domain.

12. The method of item1, wherein a capture spot in the set of capture spots comprises a cleavage domain.

13. The method of item1, wherein each capture spot in the set of capture spots is attached directly or is attached indirectly to the substrate.

14. The method of item1, wherein the one or more analytes comprises five or more analytes, ten or more analytes, fifty or more analytes, one hundred or more analytes, five hundred or more analytes, 1000 or more analytes, 2000 or more analytes, or between 2000 and 100,000 analytes.

15. The method of item1, wherein the unique spatial barcode encodes a unique predetermined value selected from the set $\{1, ..., 1024\}$, $\{1, ..., 4096\}$, $\{1, ..., 16384\}$, $\{1, ..., 65536\}$, $\{1, ..., 262144\}$, $\{1, ..., 1048576\}$, $\{1, ..., 4194304\}$, $\{1, ..., 16777216\}$, $\{1, ..., 67108864\}$, or $\{1, ..., 1 \times 10^{12}\}$.

16. The method of any one of items1-15, wherein a respective capture probe plurality in the set of capture probe pluralities includes 1000 or more capture probes, 2000 or more capture probes, 10,000 or more capture probes, 100,000 or more capture probes, $1 \times 10^6$ or more capture probes, $2 \times 10^6$ or more capture probes, or $5 \times 10^6$ or more capture probes.

17. The method of item16, wherein each capture probe in the respective capture probe plurality includes a poly-T sequence and the unique spatial barcode that characterizes the different capture spot.

18. The method of item16, wherein each capture probe in the respective capture probe plurality includes the same spatial barcode from the plurality of spatial barcodes.

19. The method of item16, wherein each capture probe in the respective capture probe plurality includes a different spatial barcode from the plurality of spatial barcodes.

20. The method of item1, wherein the sample is a sectioned tissue sample having a depth of 100 microns or less.

21. The method of item20, wherein the one or more images comprises a plurality of images and a first image in the plurality of images is obtained using a first section of the sample and a second image in the plurality of images is obtained using a second section of the sample.

22. The method of item1, wherein

the one or more analytes is a plurality of analytes,
a respective capture probe plurality in the set of capture probe pluralities includes a plurality of capture probes,
each capture probe in the plurality of capture probes including a capture domain that is characterized by a capture
domain type in a plurality of capture domain types, and
each respective capture domain type in the plurality of capture domain types is configured to bind to a different
analyte in the plurality of analytes.

23. The method of item22, wherein the plurality of capture domain types comprises between 2 and 15,000 capture
domain types and the respective capture probe plurality includes at least five, at least 10, at least 100, or at least 1000
capture probes for each capture domain type in the plurality of capture domain types.

24. The method of item1, wherein

the one or more analytes is a plurality of analytes,
a respective capture spot in the set of capture spots includes a plurality of capture probes, each capture probe in
the plurality of capture probes including a capture domain that is characterized by a single capture domain type
configured to bind to each analyte in the plurality of analytes in an unbiased manner.

25. The method of item1, wherein each capture spot in at least 30 percent, at least forty percent, at least fifty percent, at
least sixty percent, at least seventy percent, at least eighty percent, or at least ninety percent of the capture spots in the
set of capture spots is contained within a corresponding 100 micron by 100 micron square on the substrate.

26. The method of item1, wherein a distance between a center of each respective capture spot to a neighboring
capture spot in the set of capture spots on the substrate is between 50 microns and 300 microns.

27. The method of item1, wherein at least 30 percent, at least forty percent, at least fifty percent, at least sixty percent,
at least seventy percent, at least eighty percent, or at least ninety percent of the capture spots in the set of capture
spots has a diameter of 80 microns or less.

28. The method of item1, wherein at least 30 percent, at least forty percent, at least fifty percent, at least sixty percent,
at least seventy percent, at least eighty percent, or at least ninety percent of the capture spots in the set of capture
spots has a diameter of between 25 microns and 65 microns.

29. The method of item1, wherein a distance between a center of each respective capture spot to a neighboring
capture spot in the set of capture spots on the substrate is between 40 microns and 100 microns.

30. The method of item2, wherein the plurality of heuristic classifier comprises a first heuristic classifier that identifies a
single intensity threshold that divides the plurality of pixels into the first class and the second class, thereby causing the
first heuristic classifier to cast a vote for each respective pixel in the plurality of pixels for either the first class or the
second class, and wherein the single intensity threshold represents a minimization of intra-class intensity variance
between the first and second class or a maximization of inter-class variance between the first class and the second
class.

31. The method of item30, wherein the plurality of heuristic classifier comprises a second heuristic classifier that
identifies local neighborhoods of pixels with the same class identified using the first heuristic classifier and applies a
smoothed measure of maximum difference in intensity between pixels in the local neighborhood thereby causing the
second heuristic classifier to cast a vote for each respective pixel in the plurality of pixels for either the first class or the
second class.

32. The method of item31, wherein the plurality of heuristic classifier comprises a third heuristic classifier that performs
edge detection on the plurality of pixels to form a plurality of edges in the image, morphologically closes the plurality of
edges to form a plurality of morphologically closed regions in the image and assigns pixels in the morphologically
closed regions to the first class and pixels outside the morphologically closed regions to the second class, thereby
causing the third heuristic classifier to cast a vote for each respective pixel in the plurality of pixels for either the first
class or the second class.

33. The method of item32, wherein:

each respective pixel assigned by each of the heuristic classifiers in the plurality of classifiers to the second class is labelled as obvious second class, and
each respective pixel assigned by each of the plurality of heuristic classifiers as the first class is labelled as obvious first class.

34. The method of item33, wherein the graph cut segmentation algorithm is a GrabCut segmentation algorithm.

35. The method of item12, wherein the cleavage domain comprises a sequence recognized and cleaved by a uracil-DNA glycosylase and/or an endonuclease VIII.

36. The method of item1, wherein a capture probe plurality in the set of capture probe pluralities does not comprise a cleavage domain and each capture probe in the capture probe plurality is not cleaved from the substrate.

37. The method of item1, wherein the one or more analytes comprises DNA or RNA.

38. The method of item1, wherein the one or more analytes comprises a protein.

39. The method of item1, wherein each respective capture probe plurality in the set of capture probe pluralities is attached directly or attached indirectly to the substrate.

40. The method of item1, wherein the C) obtaining comprises *in-situ* sequencing of the set of capture spots on the substrate.

41. The method of item1, wherein the C) obtaining comprises high-throughput sequencing.

42. The method of item4, wherein a respective locus in the plurality of loci is biallelic and the corresponding set of haplotypes for the respective locus consists of a first allele and a second allele.

43. The method of item42, wherein the respective locus includes a heterozygous single nucleotide polymorphism (SNP), a heterozygous insert, or a heterozygous deletion.

44. The method of any one of items1-43, wherein the plurality of sequence reads comprises 50,000 or more sequence reads, 100,000 or more sequence reads, or $1 \times 10^6$ or more sequence reads.

45. The method of item4, wherein the plurality of loci comprises between two and 100 loci, more than 10 loci, more than 100 loci, or more than 500 loci.

46. The method of item1, wherein the unique spatial barcode in the respective sequence read is localized to a contiguous set of nucleotides within the respective sequence read.

47. The method of item46, wherein the contiguous set of nucleotides is an N-mer, wherein N is an integer selected from the set {4, ... , 20}.

48. The method of item4, the method further comprising retrieving the plurality of loci from a lookup table, file or data structure.

49. The method of item4, wherein the alignment algorithm is a local alignment that aligns the respective sequence read to a reference sequence using a scoring system that (i) penalizes a mismatch between a nucleotide in the respective sequence read and a corresponding nucleotide in the reference sequence in accordance with a substitution matrix and (ii) penalizes a gap introduced into an alignment of the sequence read and the reference sequence.

50. The method of item49, wherein the local alignment is a Smith-Waterman alignment.

51. The method of item49, wherein the reference sequence is all or portion of a reference genome.

52. The method of item4, the method further comprising removing from the plurality of sequence reads one or more sequence reads that do not overlay any loci in the plurality of loci.

53. The method of item52, wherein the plurality of sequence reads are RNA-sequence reads and wherein the removing comprises removing one or more sequences reads in the plurality of sequence reads that overlap a splice site in the reference sequence.

54. The method of item4, wherein the plurality of loci include one or more loci on a first chromosome and one or more loci on a second chromosome other than the first chromosome.

55. The method of any one of items1-54, wherein the plurality of sequence reads include 3'-end or 5'-end paired sequence reads.

56. The method of item1, wherein

the one or more analytes is a plurality of analytes,
a respective capture probe plurality in the set of capture probe pluralities includes a plurality of probes, each capture probe in the plurality of capture probes including a capture domain that is characterized by a single capture domain type configured to bind to each analyte in the plurality of analytes in an unbiased manner.

57. The method of item5, wherein the biological condition is absence or presence of a disease.

58. The method of item5, wherein the biological condition is a type of a cancer.

59. The method of item5, wherein the biological condition is a stage of a disease.

60. The method of item5, wherein the biological condition is a stage of a cancer.

61. The method of any one of items1-60, wherein the one or more images includes a brightfield image or a fluorescence image of the sample.

62. The method of any one of items1-60, wherein the one or more images is a plurality of images.

63. The method of item62, wherein:

a first image in the plurality of images is a brightfield image of the biological sample, and
a second image in the plurality of images is a fluorescence image of the biological sample.

64. The method of item63, wherein the fluorescence image is an immunofluorescence image.

65. The method of any one of items1-64, wherein the one or more images is a plurality of images and the plurality of images comprises two or more fluorescence images.

66. The method of any one of items1-65, wherein the representation of all or a portion of each subset of sequence reads at a respective position within the one or more images communicates a number of unique molecules that map to a particular analyte or combination of analytes in the sample represented by the subset of sequence reads that, in turn, map to the respective capture spot.

67. The method of item66, wherein the number of unique molecules that map to a particular analyte or combination of analytes in the sample represented by the subset of sequence reads that, in turn, map to the respective capture spot is communicated on a color scale or an intensity scale.

68. The method of any one of items1-67, wherein a respective capture probe plurality in the set of capture probe pluralities directly associates with an analyte from the sample.

69. The method of any one of items1-67, wherein a respective capture probe plurality in the set of capture probe pluralities indirectly associates with an analyte from the sample through an analyte capture agent.

70. A computer system comprising:

one or more processors;
memory; and
one or more programs, wherein the one or more programs are stored in the memory and configured to be executed by the one or more processors, the one or more programs for spatial analysis of analytes, the one or more programs including instructions for:

A) placing a sample on a substrate, wherein the substrate comprises a plurality of fiducial markers and a set of capture spots, wherein the set of capture spots comprises at least 1000 capture spots;
B) obtaining one or more images of the sample on the substrate, wherein each respective image of the one or more images comprises a corresponding plurality of pixels in the form of a corresponding array of pixel values, wherein the corresponding array of pixel values comprises at least 100,000 pixel values;
C) obtaining a plurality of sequence reads, in electronic form, from the set of capture spots after the A) placing, wherein:

each respective capture probe plurality in a set of capture probe pluralities is (i) at a different capture spot in the set of capture spots and (ii) directly or indirectly associates with one or more analytes from the sample,
each respective capture probe plurality in the set of capture probe pluralities is characterized by at least one unique spatial barcode in a plurality of spatial barcodes,
the plurality of sequence reads comprises sequence reads corresponding to all or portions of the one or more analytes,
the plurality of sequence reads comprises at least 10,000 sequence reads, and
each respective sequence read in the plurality of sequence reads includes a spatial barcode of the corresponding capture probe plurality in the set of capture probe pluralities or a complement thereof;

D) using all or a subset of the plurality of spatial barcodes to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots; and
E) using the plurality of fiducial markers to provide a composite representation comprising (i) the one or more images aligned to the set of capture spots on the substrate and (ii) a representation of all or a portion of each subset of sequence reads at each respective position within the one or more images that maps to a respective capture spot corresponding to the respective position of the one or more analytes in the sample by aligning a first image in the one or more images to the set of capture spots by a procedure that comprises:

analyzing the corresponding array of pixel values of the first image to identify a plurality of derived fiducial markers of the first image;
using a substrate identifier uniquely associated with the substrate to select a first template in a plurality of templates, wherein each template in the plurality of templates comprises reference positions for a corresponding plurality of reference fiducial markers and a corresponding coordinate system;
aligning the plurality of derived fiducial markers of the first image with the corresponding plurality of reference fiducial markers of the first template using an alignment algorithm to obtain a transformation between the plurality of derived fiducial markers of the first image and the corresponding plurality of reference fiducial markers of the first template; and
using the transformation and the coordinate system of the first template to locate a corresponding position in the first image of each capture spot in the set of capture spots by running a plurality of heuristic classifiers on the plurality of pixels, wherein, for each pixel in the plurality of pixels, each respective heuristic classifier in the plurality of heuristic classifiers casts a vote for the respective pixel between a first class and the second class, thereby forming a corresponding aggregated score for each respective pixel in the plurality of pixels, and applying the aggregated score and intensity of each respective pixel in the plurality of pixels to a segmentation algorithm to independently assign a probability to each respective pixel in the plurality of pixels of being sample or background.

71. A computer readable storage medium storing one or more programs, the one or more programs comprising instructions, which when executed by an electronic device with one or more processors and a memory cause the electronic device to perform spatial analysis of analytes by a method comprising:

A) placing a sample on a substrate, wherein the substrate comprises a plurality of fiducial markers and a set of capture spots, wherein the set of capture spots comprises at least 1000 capture spots;

B) obtaining one or more images of the sample on the substrate, wherein each respective image of the one or more images comprises a corresponding plurality of pixels in the form of a corresponding array of pixel values, wherein the corresponding array of pixel values comprises at least 100,000 pixel values;

C) obtaining a plurality of sequence reads, in electronic form, from the set of capture spots after the A) placing, wherein:

each respective capture probe plurality in a set of capture probe pluralities is (i) at a different capture spot in the set of capture spots and (ii) directly or indirectly associates with one or more analytes from the sample, each respective capture probe plurality in the set of capture probe pluralities is characterized by at least one unique spatial barcode in a plurality of spatial barcodes,

the plurality of sequence reads comprises sequence reads corresponding to all or portions of the one or more analytes,

the plurality of sequence reads comprises at least 10,000 sequence reads, and

each respective sequence read in the plurality of sequence reads includes a spatial barcode of the corresponding capture probe plurality in the set of capture probe pluralities or a complement thereof;

D) using all or a subset of the plurality of spatial barcodes to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots; and

E) using the plurality of fiducial markers to provide a composite representation comprising (i) the one or more images aligned to the set of capture spots on the substrate and (ii) a representation of all or a portion of each subset of sequence reads at each respective position within the one or more images that maps to a respective capture spot corresponding to the respective position of the one or more analytes in the sample by aligning a first image in the one or more images to the set of capture spots by a procedure that comprises:

analyzing the corresponding array of pixel values of the first image to identify a plurality of derived fiducial markers of the first image;

using a substrate identifier uniquely associated with the substrate to select a first template in a plurality of templates, wherein each template in the plurality of templates comprises reference positions for a corresponding plurality of reference fiducial markers and a corresponding coordinate system;

aligning the plurality of derived fiducial markers of the first image with the corresponding plurality of reference fiducial markers of the first template using an alignment algorithm to obtain a transformation between the plurality of derived fiducial markers of the first image and the corresponding plurality of reference fiducial markers of the first template; and

using the transformation and the coordinate system of the first template to locate a corresponding position in the first image of each capture spot in the set of capture spots

by running a plurality of heuristic classifiers on the plurality of pixels, wherein, for each pixel in the plurality of pixels, each respective heuristic classifier in the plurality of heuristic classifiers casts a vote for the respective pixel between a first class and the second class, thereby forming a corresponding aggregated score for each respective pixel in the plurality of pixels, and applying the aggregated score and intensity of each respective pixel in the plurality of pixels to a segmentation algorithm to independently assign a probability to each respective pixel in the plurality of pixels of being sample or background.

## Claims

1. A method of spatial analysis of analytes comprising:

A) placing a sample on a substrate, wherein the substrate comprises a plurality of fiducial markers and a set of capture spots, wherein the set of capture spots comprises at least 1000 capture spots;

B) obtaining one or more images of the sample on the substrate, wherein each respective image of the one or more images comprises a corresponding plurality of pixels in the form of an array of pixel values, wherein the array of pixel values comprises at least 100,000 pixel values;

C) obtaining a plurality of sequence reads, in electronic form, from the set of capture spots after the A) placing, wherein:

each respective capture probe plurality in a set of capture probe pluralities is (i) at a different capture spot in the set of capture spots and (ii) directly or indirectly associates with one or more analytes from the sample, each respective capture probe plurality in the set of capture probe pluralities is **characterized by** at least one unique spatial barcode in a plurality of spatial barcodes, the plurality of sequence reads comprises sequence reads corresponding to all or portions of the one or more analytes, and the plurality of sequence reads comprises at least 10,000 sequence reads, and each respective sequence read in the plurality of sequence reads includes a spatial barcode of the corresponding capture probe plurality in the set of capture probe pluralities or a complement thereof;

D) using all or a subset of the plurality of spatial barcodes to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the plurality of capture spots; and
E) using the plurality of fiducial markers to provide a composite representation comprising (i) the one or more images aligned to the set of capture spots on the substrate and (ii) a representation of all or a portion of each subset of sequence reads at each respective position within the one or more images that maps to a respective capture spot corresponding to the respective position of the one or more analytes in the sample, wherein the method further comprises, for each respective locus in a plurality of loci, performing a procedure that comprises:

i) performing an alignment of each respective sequence read in the plurality of sequence reads that maps to the respective locus thereby determining a haplotype identity for the respective sequence read from among a corresponding set of haplotypes for the respective locus, and
ii) categorizing each respective sequence read in the plurality of sequence reads that maps to the respective locus by the spatial barcode of the respective sequence read and by the haplotype identity,

thereby determining a spatial distribution of each haplotype in each corresponding set of haplotypes in the sample, wherein the spatial distribution includes, for each capture spot in the set of capture spots on the substrate, an abundance of each haplotype in the set of haplotypes for the respective locus.

2. The method of claim 1, the method further comprises using the spatial distribution to characterize a biological condition in a subject.

3. The method of claim 1, wherein a capture spot in the set of capture spots comprises a capture domain or a cleavage domain.

4. The method of claim 1, wherein the one or more analytes comprises fifty or more analytes.

5. The method of any one of claims 1-4, wherein a respective capture probe plurality in the set of capture probe pluralities includes 1000 or more capture probes.

6. The method of claim 1, wherein the sample is a sectioned tissue sample having a depth of 100 microns or less.

7. The method of claim 6, wherein the one or more images comprises a plurality of images and a first image in the plurality of images is obtained using a first section of the sample and a second image in the plurality of images is obtained using a second section of the sample.

8. The method of claim 1, wherein

the one or more analytes is a plurality of analytes,
a respective capture spot in the set of capture spots includes a plurality of capture probes, each capture probe in the plurality of capture probes including a capture domain that is **characterized by** a single capture domain type configured to bind to each analyte in the plurality of analytes in an unbiased manner.

9. The method of claim 1, wherein each capture spot in at least 30 percent, at least forty percent of the capture spots in the set of capture spots is contained within a corresponding 100 micron by 100 micron square on the substrate.

10. The method of claim 1, wherein at least 30 percent of the capture spots in the set of capture spots has a diameter of 80 microns or less or, a diameter of between 25 microns and 65 microns.

11. The method of claim 1, wherein the one or more analytes comprises DNA, RNA or a protein.

12. The method of claim 1, wherein a respective locus in the plurality of loci is biallelic and the corresponding set of haplotypes for the respective locus consists of a first allele and a second allele.

13. The method of claim 12, wherein the respective locus includes a heterozygous single nucleotide polymorphism (SNP), a heterozygous insert, or a heterozygous deletion.

14. A computer system comprising:

> one or more processors;
> memory; and
> one or more programs, wherein the one or more programs are stored in the memory and configured to be executed by the one or more processors, the one or more programs for spatial analysis of analytes, the one or more programs including instructions for:
>
> > A) obtaining one or more images, in electronic form, of a sample on a substrate, wherein the substrate comprises a plurality of fiducial markers and a set of capture spots, wherein the set of capture spots comprises at least 1000 capture spots, wherein each respective image of the one or more images comprises a corresponding plurality of pixels in the form of an array of pixel values, and wherein the array of pixel values comprises at least 100,000 pixel values;
> > B) obtaining a plurality of sequence reads, in electronic form, from the set of capture spots after the A) obtaining, wherein:
> >
> > > each respective capture probe plurality in a set of capture probe pluralities is (i) at a different capture spot in the set of capture spots and (ii) directly or indirectly associates with one or more analytes from the sample,
> > > each respective capture probe plurality in the set of capture probe pluralities is **characterized by** at least one unique spatial barcode in a plurality of spatial barcodes,
> > > the plurality of sequence reads comprises sequence reads corresponding to all or portions of the one or more analytes from the sample, and
> > > each respective sequence read in the plurality of sequence reads includes a spatial barcode of the corresponding capture probe plurality in the set of capture probe pluralities or a complement thereof;
> >
> > C) using all or a subset of the plurality of spatial barcodes to localize respective sequence reads in each plurality of sequence reads to corresponding capture spots in the set of capture spots, thereby dividing the plurality of sequence reads into a plurality of subsets of sequence reads, each respective subset of sequence reads corresponding to a different capture spot in the corresponding plurality of capture spots; and
> > D) using the plurality of fiducial markers to provide a composite representation comprising (i) the one or more images aligned to the set of capture spots on the substrate and (ii) a representation of all or a portion of each subset of sequence reads at each respective position within the one or more images that maps to the capture spot corresponding to the respective position of the one or more analytes in the sample and, for each respective locus in a plurality of loci, performing a procedure that comprises:
> >
> > > i) performing an alignment of each respective sequence read in the plurality of sequence reads that maps to the respective locus thereby determining a haplotype identity for the respective sequence read from among a corresponding set of haplotypes for the respective locus, and
> > > ii) categorizing each respective sequence read in the plurality of sequence reads that maps to the respective locus by the spatial barcode of the respective sequence read and by the haplotype identity,
>
> thereby determining a spatial distribution of each haplotype in each corresponding set of haplotypes in the sample, wherein the spatial distribution includes, for each capture spot in the set of capture spots on the substrate, an abundance of each haplotype in the set of haplotypes for the respective locus.

15. A computer readable storage medium storing one or more programs, the one or more programs comprising instructions, which when executed by an electronic device with one or more processors and a memory cause the electronic device to perform spatial analysis of analytes by a method comprising:

A) obtaining one or more images, in electronic form, of a sample on a substrate, wherein the substrate includes a plurality of fiducial markers and a set of capture spots, wherein the set of capture spots comprises at least 1000 capture spots, wherein each respective image of the one or more images comprises a corresponding plurality of pixels in the form of an array of pixel values, and wherein the array of pixel values comprises at least 100,000 pixel values;

B) obtaining, for each image in the one or more images, a plurality of sequence reads, in electronic form, from the set of capture spots after the A) obtaining, wherein:

each respective capture probe plurality in a set of capture probe pluralities is (i) at a different capture spot in the set of capture spots and (ii) directly or indirectly associates with one or more analytes from the sample, each respective capture probe plurality in the set of capture probe pluralities is **characterized by** at least one unique spatial barcode in a plurality of spatial barcodes,

the plurality of sequence reads comprises sequence reads corresponding to all or portions of the one or more analytes, and

each respective sequence read in the plurality of sequence reads includes a spatial barcode of the corresponding capture probe plurality in the set of capture probe pluralities or a complement thereof;

C) using all or a subset of the plurality of spatial barcodes to localize respective sequence reads in the plurality of sequence reads to corresponding capture spots in the set of capture spots, thereby dividing the plurality of sequence reads into a plurality of subsets of sequence reads, each respective subset of sequence reads corresponding to a different capture spot in the corresponding plurality of capture spots; and

D) using the plurality of fiducial markers to provide a composite representation comprising (i) the one or more images aligned to the set of capture spots on the substrate and (ii) a representation of all or a portion of each subset of sequence reads at each respective position within the one or more images that maps to the capture spot corresponding to the respective position of the one or more analytes in the sample and, for each respective locus in a plurality of loci, performing a procedure that comprises:

i) performing an alignment of each respective sequence read in the plurality of sequence reads that maps to the respective locus thereby determining a haplotype identity for the respective sequence read from among a corresponding set of haplotypes for the respective locus, and

ii) categorizing each respective sequence read in the plurality of sequence reads that maps to the respective locus by the spatial barcode of the respective sequence read and by the haplotype identity,

thereby determining a spatial distribution of each haplotype in each corresponding set of haplotypes in the sample, wherein the spatial distribution includes, for each capture spot in the set of capture spots on the substrate, an abundance of each haplotype in the set of haplotypes for the respective locus.

Contact sample with array of spatially-barcoded capture probes
(101)

Capture analytes using capture probes
(102)

Analyze capture probes to obtain spatially-resolved analyte information
(103)

**Figure 1**

Contact sample with array of spatially-barcoded capture probes
(201)

Optionally cleave capture probes from array and capture
analyte on/in sample
(202)

Analyze capture probes to obtain spatially-resolved information
about an analyte
(203)

**Figure 2**

Prepare sample on array
(301)

Image tissue sample and/or array
(302)

Capture analytes
(303)

Optionally remove tissue
(304)

Optionally cleave library
(305)

Image array/sample (305B)

Prepare library
(306)

Next-generation sequencing
(307)

Spatial overlay
(308)

**Figure 3A**

Figure 3B

Figure 4

Sample preparation
(501)

Permeabilization
(502)

First strand cDNA synthesis
(503)

Denature & second strand extension
(504)

Denature second strand, neutralize, transfer
(505)

Library preparation
(506)

Optional sample index PCR
(507)

Optional library QC
(508)

**Figure 5**

**Figure 6**

**Figure 7**

Figure 8

EP 4 538 971 A2

**Figure 9**

1000

Systems and method for spatial analysis of analytes are provided. ⌐1002

Place a sample on a substrate. The substrate includes a plurality of fiducial markers and a set of capture spots. ⌐1004

⌐1006
A capture spot in the set of capture spots comprises a capture domain.

⌐1008
A capture spot in the set of capture spots comprises a cleavage domain.

The cleavage domain comprises a sequence recognized and cleaved by a uracil-DNA glycosylase and/or an endonuclease VIII. ⌐1010

Each capture spot in the set of capture spots is attached directly or indirectly to the substrate. ⌐1012

The sample is a sectioned tissue sample having a depth of 100 microns or less. ⌐1014

Each respective capture spot in the set of capture spots is contained within a 100 micron by 100 micron square on the substrate. ⌐1016

A distance between a center of each respective capture spot to a neighboring capture spot in the set of capture spots on the substrate is between 50 microns and 300 microns. ⌐1018

Each capture spot in the set of capture spots has a diameter of 80 microns or less. ⌐1020

A distance between a center of each respective capture spot to a neighboring capture spot in the set of capture spots on the substrate is between 50 microns and 80 microns. ⌐1022

(B)

**Figure 10A**

B

Obtain one or more images 1124 of the sample on the substrate. The such image comprises a plurality of pixels in the form of an array of pixel values. ⌐1024

For each respective image in the one or more images, obtain a corresponding plurality of sequence reads, in electronic form, from the set of capture spots (e.g., by in-situ sequencing of the set of capture spots on the substrate, high-throughput sequencing etc.). ⌐1026

The correpsonding plurality of sequence reads comprises 10,000 or more sequence reads, 50,000 or more sequence reads, 100,000 or more sequence reads, or $1 \times 10^6$ or more sequence reads. ⌐1028

The corresponding plurality of sequence reads include 3'-end or 5'-end paired sequence reads. ⌐1030

Each respective capture probe plurality in a set of capture probe pluralities is (i) at a different capture spot in the set of capture spots and (ii) directly or indirectly associates with one or more analytes from the sample. ⌐1032

The one or more analytes (e.g., DNA, RNA) comprises 5 or more analytes, 10 or more analytes, 50 or more analytes, 100 or more analytes, 500 or more analytes, 1000 or more analytes, 2000 or more analytes, or between 2000 and 10,000 analytes. ⌐1034

The one or more analytes is a plurality of analytes. A respective capture probe plurality in the set of capture probe pluralities includes a plurality of capture probes. Each capture probe in the plurality of capture probes includes a capture domain that is characterized by a capture domain type in a plurality of capture domain types. Each respective capture domain type in the plurality of capture domain types is configured to bind to a different analyte in the plurality of analytes. ⌐1036

The plurality of capture domain types comprises between 5 and 15,000 capture domain types and the respective capture probe plurality includes at least five, at least 10, at least 100, or at least 1000 probes for each capture domain type in the plurality of capture domain types. ⌐1038

C

**Figure 10B**

C

1032
(cont.)
1040

Each respective capture probe plurality in the set of capture probe pluralities includes 1000 or more, 2000 or more, 10,000 or more, 100,000 or more, $1 \times 10^6$ or more, $2 \times 10^6$ or more, or $5 \times 10^6$ or more capture probes.

Each capture probe in the capture probe plurality includes a poly-A or poly-T sequence and a corresponding spatial barcode that characterizes the different capture spot.

1042

Each capture probe in the capture probe plurality includes the same spatial barcode from the plurality of spatial barcodes.

1044

Each capture probe in the capture probe plurality includes a different spatial barcode from the plurality of spatial barcodes.

1046

The one or more analytes is a plurality of analytes. A respective capture spot in the set of capture spots includes a plurality of capture probes, each including a capture domain that is characterized by a single capture domain type configured to bind to each analyte in the plurality of analytes in an unbiased manner.

1048

A capture probe plurality in the set of capture probe pluralities does not comprise a cleavage domain and each capture probe in the capture probe plurality is not cleaved from the substrate.

1050

Each respective capture probe plurality in the set of capture probe pluralities is attached directly or attached indirectly to the substrate.

1052

The one or more analytes is a plurality of analytes. A respective capture probe plurality in the set of capture probe pluralities includes a plurality of probes. Each capture probe in the plurality of capture probes includes a capture domain that is characterized by a single capture domain type configured to bind to each analyte in the plurality of analytes in an unbiased manner.

1054

D

**Figure 10C**

(D)

Each respective capture probe plurality in the set of capture probe pluralities is characterized by at least one different corresponding spatial barcode in a plurality of spatial barcodes. The plurality of sequence reads comprises sequence reads corresponding to all or portions of the one or more analytes. Each respective sequence read in the plurality of sequence reads includes a spatial barcode of the corresponding capture probe plurality in the set of capture probe pluralities. — 1056

The corresponding spatial barcode in the respective sequence read is localized to a contiguous set of oligonucleotides within the respective sequence read. — 1058

The contiguous set of oligonucleotides is an N-mer, where N is an integer selected from the set {4, ..., 20}. — 1060

The corresponding spatial barcode encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., $1 \times 10^{12}$}. — 1062

Use the plurality of spatial barcodes to localize respective sequence reads in the corresponding plurality of sequence reads to corresponding capture spots in the set of capture spots, thereby dividing the corresponding plurality of sequence reads into a plurality of subsets of sequence reads. Each respective subset of sequence reads corresponds to a different capture spot in the plurality of capture spots. — 1064

Use the plurality of fiducial markers to provide a composite representation comprising (i) the one or more images aligned to the set of capture spots on the substrate and (ii) a representation of each subset of sequence reads at a respective position within the image that maps to the corresponding capture spot on the substrate. — 1066

(E)

**Figure 10D**

(E)

1066
(cont.)
1068

The composite representation provides a relative abundance of nucleic acid fragments mapping to each gene in a plurality of genes at each capture spot in the plurality of capture spots.

A first image in the one or more images is aligned to the set of capture spots on the substrate by a procedure that comprises analyzing the array of pixel values to identify a plurality of derived fiducial spots of the first image, using a substrate identifier uniquely associated with the substrate to select a first template in a plurality of templates. Each template in the plurality of templates comprises reference positions for a corresponding plurality of reference fiducial spots and a corresponding coordinate system. The plurality of derived fiducial spots of the first image is aligned with the corresponding plurality of reference fiducial spots of the first template using an alignment algorithm to obtain a transformation between the plurality of derived fiducial spots of the first image and the corresponding plurality of reference fiducial spots of the first template. The transformation and the coordinate system of the first template is used to locate a corresponding position in the first image of each capture spot in the set of capture spots.

1070

The using the transformation and the coordinate system of the first template to locate and measure the one or more optical properties of each capture spot comprises assigning each respective pixel in the plurality of pixels to a first class or a second class, where the first class indicates the sample on the substrate and the second class indicates background, by a procedure that comprises: (i) using the plurality of fiducial markers to define a bounding box within the first image, (ii) removing respective pixels falling outside the bounding box from the plurality of pixels, (iii) running, after the removing (ii), a plurality of heuristic classifiers on the plurality of pixels in grey-scale space, where, for each respective pixel in the plurality of pixels, each respective heuristic classifier in the plurality of heuristic classifiers casts a vote for the respective pixel between the first class and the second class, thereby forming a corresponding aggregated score for each respective pixel in the plurality of pixels, and (iv) applying the aggregated score and intensity of each respective pixel in the plurality of pixels to a graph cut segmentation algorithm to independently assign a probability to each respective pixel in the plurality of pixels of being tissue or background.

1072

(F)

**Figure 10E**

(E)

Each corresponding aggregated score is a class in a set of classes comprising obvious first class, likely first class, likely second class, and obvious second class.

The plurality of heuristic classifiers comprises a first heuristic classifier that identifies a single intensity threshold that divides the plurality of pixels into the first class and the second class, thereby causing the first heuristic classifier to cast a vote for each respective pixel in the plurality of pixels for either the first class or the second class, and wherein the single intensity threshold represents a minimization of intra-class intensity variance between the first and second class or a maximization of inter-class variance between the first class and the second class.

Overlay a tissue mask on the first image. The tissue mask causes each respective pixel in the plurality of pixels of the image that has been assigned a greater probability of being tissue to be assigned a first attribute and each respective pixel in the plurality of pixels that has been assigned a greater probability of being background to be assigned a second attribute (e.g., the first attribute is a first color (e.g., one or red and blue) and the second attribute is a second color (e.g., the other of red and blue); the first attribute is a first level of brightness or opacity and the second attribute is a second level of brightness or opacity).

Assign each respective representation, of a capture spot in the plurality of capture spots in the composite representation, the first attribute or the second attribute based upon the assignment of pixels in the vicinity of the respective representation of the capture spot in the composite representation.

1066
(cont.)
1070
(cont.)
1072
(cont.)
1074

1076

1078

1080

**Figure 10F**

Memory
1112

| | |
|---|---|
| Operating system | 1116 |
| Network communication module | 1118 |
| Analysis module | 1120 |
| Discrete attribute value dataset | 1122 |
|   Substrate (*e.g.*, chip) Image 1 | 1124-1 |
|     Pixel value 1 | 1126-1-1 |
|     ⋮ | |
|     Pixel value N | 1126-1-N |
|   ⋮ | |
|   Substrate (*e.g.*, chip) Image Q | 1124-Q |
|   Substrate identifier | 1128 |
|   Substrate derived fiducial spot 1 | 1130-1 |
|     Coordinates of der. fiducial spot 1 | 1132-1 |
|     ⋮ | |
|   Substrate derived fiducial spot L | 1130-L |
|     Coordinates of der. fiducial spot L | 1132-L |
|   Data construct for set of capture spots of image 1 | 1134-1 |
|     Capture spot 1 | 1136-1-1 |
|       Analyte measurement 1-1 | 1138-1-1-1 |
|       ⋮ | |
|       Analyte measurement 1-M | 1138-1-1-M |
|     ⋮ | |
|     Capture spot Q | 1136-1-Q |
|       Analyte measurement Q-1 | 1138-1-Q-1 |
|       ⋮ | |
|       Analyte measurement Q-T | 1138-1-Q-T |
|   ⋮ | |
|   Data construct for set of capture spots of image Q | 1134-Q |
| Template repository | 1140 |

1100

CPU(s) —1102

1114

Network interface —1104

1106

User interface
1108 — Display
Input
1110

**Figure 11A**

Template repository — 1140

  Template 1 — 1142-1

    Template 1 coordinate system — 1144-1

    Substrate identifier — 1128-1

    Reference fiducial spot 1 — 1146-1-1

      Coordinates of fiducial spot 1 — 1148-1-1

      ⋮

    Reference fiducial spot K — 1146-1-K

      Coordinates of fiducial spot K — 1148-1-K

  ⋮

  Template Q — 1142-Q

    Template Q coordinate system — 1144-Q

    Substrate identifier — 1128-Q

    Reference fiducial spot 1 — 1146-Q-1

      Coordinates of fiducial spot 1 — 1148-Q-1

      ⋮

    Reference fiducial spot P — 1146-Q-P

      Coordinates of fiducial spot P — 1148-Q-P

Sequence read 1-1 — 1138-1-1

  Spatial barcode 1 — 1150-1-1-1

  Analyte encoding portion — 1152-1-1-1

  ⋮

Sequence read 1-2 — 1138-1-2

  ⋮

Sequence read 1-M — 1138-1-M

**Figure 11B**

Figure 12

1136

1301

1302

1303

1304

1305

1306

**Figure 13**

1124

1204

**Figure 14**

**Serial Number**

**Visium Spatial Gene Expression Slide**
PN: 2000233
SN: A99A99-999
Exp: 9999-99-99
Store at Ambient

**Capture Areas**

A1

B1

C1

D1

- The slide serial number is used to identify the slide design

- The slide design file is a text file that contains the location of the fiducial frame and the capture spots on the slide

  - These fiducial marks match the bright field image and are used for placing the functional spots on the tissue for visualization

Figure 15

1148

Figure 16

Figure 17

**1142**

- Align Spots
- Identify Spots under Tissue
- Extract Barcode, UMI
- Align Reads
- Correct Barcodes
- Filter UMIs
- Count UMIs
- Secondary Analysis

- Bright field image contains the location of the tissue in relation to the fiducial frame

- The slide design file (template) contains the location of the capture spots in relation to the fiducial frame

```
ATF       1
20        5
"Type=GenePix ArrayList V1.0"
"BlockCount=16"
"BlockType=2"
"ArrayRevision=A"
"Block1= 4288, 29652, 105, 100, 75, 30, 260"
"Block2= 4825, 30073, 70, 128, 50, 39, 174"
"Block3= 13288, 29652, 105, 100, 75, 30, 260"
"Block4= 13825, 30073, 70, 128, 50, 39, 174"
"Block5= 4288, 38652, 105, 100, 75, 30, 260"
"Block6= 4825, 39073, 70, 128, 50, 39, 174"
"Block7= 13288, 38652, 105, 100, 75, 30, 260"
"Block8= 13825, 39073, 70, 128, 50, 39, 174"
"Block9= 4288, 47652, 105, 100, 75, 30, 260"
"Block10= 4825, 48073, 70, 128, 50, 39, 174"
"Block11= 13288, 47652, 105, 100, 75, 30, 260"
"Block12= 13825, 48073, 70, 128, 50, 39, 174"
"Block13= 4288, 56652, 105, 100, 75, 30, 260"
"Block14= 4825, 57073, 70, 128, 50, 39, 174"
"Block15= 13288, 56652, 105, 100, 75, 30, 260"
"Block16= 13825, 57073, 70, 128, 50, 39, 174"
```

| Block | Column | Row | ID | Name |
|---|---|---|---|---|
| 1 | 1 | 1 | F | FRAME |
| 1 | 2 | 1 | F | FRAME |
| 1 | 3 | 1 | F | FRAME |
| 1 | 4 | 1 | F | FRAME |
| 1 | 5 | 1 | F | FRAME |
| 1 | 6 | 1 | EMPTY | EMPTY |

EP 4 538 971 A2

**Figure 18**

**Align Spots**

Identify Spots under Tissue

Extract Barcode, UMI

Align Reads

Correct Barcodes

Filter UMIs

Count UMIs

Secondary Analysis

- Bright field image contains the location of the tissue in relation to the fiducial frame

- The slide design file contains the location of the capture spots in relation to the fiducial frame

**Template 1142**

1148

1136

1148

**Figure 19**

Figure 20

**Align Spots**

Identify Spots under Tissue

Extract Barcode, UMI

Align Reads

Correct Barcodes

Filter UMIs

Count UMIs

Secondary Analysis

- Bright field image contains the location of the tissue in relation to the fiducial frame

- The slide design file contains the location of the active spots in relation to the fiducial frame

- Using the fiducial frame that is common to both inputs the pipeline can overlay the exact location of the active spots on the tissue

  - I.E Space Ranger uses the slide design to draw the active spots on the tissue

**Template 1142 mapped onto Image 1124**

1136

**Figure 21**

Align Spots

**Identify Spots under Tissue**

Extract Barcode, UMI

Align Reads

Correct Barcodes

Filter UMIs

Count UMIs

Secondary Analysis

* After the capture spots are overlaid on the tissue, the pipeline identifies the spots that are under the tissue

Aligned image

EP 4 538 971 A2

**Figure 22**

Align Spots

**Identify Spots under Tissue**

Extract Barcode, UMI

Align Reads

Correct Barcodes

Filter UMIs

Count UMIs

Secondary Analysis

- After the capture spots are overlaid on the tissue, the pipeline identifies the spots that are under the tissue

- Once identified the spots outside of the tissue are excluded from further analysis

- The spots under the tissue are used to generate a filtered barcode matrix used in secondary analysis

- The input image is then broken into tiles for rapid viewing (similar to indexing a large text file)

Aligned image

Figure 23

Figure 24

Figure 25

EP 4 538 971 A2

Figure 26

| Align Spots |
| Identify Spots under Tissue |
| Extract Barcode, UMI |
| Align Reads |
| **Correct Barcodes** |
| Filter UMIs |
| Count UMIs |
| Secondary Analysis |

* Capture spot barcodes
  — Must be on a list of known capture spot barcode sequences
  — May allow 1 bp mismatch if the mismatch occurs at a low-quality position (the barcode is then corrected).

**Figure 27**

| |
|---|
| Align Spots |
| Identify Spots under Tissue |
| Extract Barcode, UMI |
| Align Reads |
| Correct Barcodes |
| **Filter UMIs** |
| Count UMIs |
| Secondary Analysis |

- Filter UMIs

  – Must not be a homopolymer, e.g. AAAAAAAAA

  – Must not contain N

  – Must not contain bases with base quality < 10

- Correct UMIs

  – UMIs that are 1 mismatch away from a higher count UMI are corrected to that UMI if they share a cell barcode and gene.

**Figure 28**

Figure 29

**Figure 30**

**Figure 31**

Data Visualization

Loupe browser with Spatial Gene expression support

- View spot clusters in the context of the tissue image

Figure 32

Data Visualization

Loupe browser with Spatial Gene expression support

● Zoom in for more detail

Figure 33

## Data Visualization

## Loupe browser with Spatial Gene expression support

- Create custom categories and clusters for differential expression analysis

Figure 34

# Data Visualization

Loupe browser with Spatial Gene expression support

- View expressed genes in the context of the tissue image

**Figure 35**

Figure 36A

Figure 36B

3618

Figure 36C

Figure 36D

Figure 36E

Figure 36F

Figure 36G

Figure 36H

Figure 36I

## On Substrate

Figure 37

Figure 38A

Figure 38B

Figure 38C

3902

**Immunofluorescence (IF)**

NeuN

Microscope Imaging Readout

3904

**Visium Gene Expression**

*Rbfox3*

3910

Sequencing Readout

3906

**IF + Visium
Gene Expression**

NeuN + *Rbfox3*

3912

3908

**IF + Visium
Whole Transcriptome**

3914

Microscope Imaging + Sequencing Readout

Figure 39

Figure 40

Figure 41A

4004

4118    4008    4114

(SEQ ID NO.: 2)

Read 2    Barcode    AAAAAAAAAAAAAAAAAAAAAAAAAAA

4120

4120

Figure 41B

4004

4140

4118    4008    4114

(SEQ ID NO.: 2)

Read 2    Barcode    AAAAAAAAAAAAAAAAAAAAAAAAAAAA

UUUUUUUUUUUUUUUUUUUUUUUUUUUU    TTTTT
T    TT
T    T
T    T
TT    TT
TTTTT

(SEQ ID NO.: 3)

(SEQ ID NO.: 4)

4150

4120

4120

Figure 41C

Figure 41D

Figure 41E

Figure 42

Figure 43

Figure 44

Figure 45

Figure 46A

Figure 46B

Figure 46C

Cluster 1
Cluster 2
Cluster 3
Cluster 4
Cluster 5
Cluster 6
Cluster 7
Cluster 8

Figure 46D

Figure 46G

Figure 46F

Figure 46E

Figure 46I

Figure 46H

EP 4 538 971 A2

```
┌─────────────────────────────┐        ┌─────────────────────────────┐
│  Heat slide for 1 minute    │        │  Coverslip & take           │
│  at 37C (Visium standard)   │        │  fluorescent image          │
└─────────────────────────────┘        └─────────────────────────────┘
              │                                       │
              ▼                                       ▼
┌─────────────────────────────┐        ┌─────────────────────────────┐
│  Fix with 100% methanol     │        │  Remove coverslip, transfer │
│  for 30 minutes @ -20C      │        │  to cassette, and incubate  │
│                             │        │  in wash solution           │
└─────────────────────────────┘        └─────────────────────────────┘
              │                                       │
              ▼                                       ▼
┌─────────────────────────────┐        ┌─────────────────────────────┐
│  Incubate TotalSeq          │        │  For Enzymatic blockers:    │
│  antibodies with blockers   │        │  Incubate in enzyme blocker │
│  for 30 minutes to          │        │  removal mix for 30min      │
│  hybridize                  │        │                             │
└─────────────────────────────┘        └─────────────────────────────┘
              │                                       │
              ▼                                       ▼
┌─────────────────────────────┐        ┌─────────────────────────────┐
│  Stain tissue with blocked  │        │  Perm with ProK (1% SDS)    │
│  antibodies in 3X SSC for   │        │  and RT with TSO            │
│  30 minutes at 4C           │        │                             │
└─────────────────────────────┘        └─────────────────────────────┘
              │                                       │
              ▼                                       ▼
┌─────────────────────────────┐        ┌─────────────────────────────┐
│  Rinse tissue at 37C 5X     │        │  Perform SS synthesis with  │
│  with low salt wash buffer  │───────▶│  mixture of TSO and smRNA   │
│  (0.1X)                     │        │  Rd2 (for Ab tags)          │
└─────────────────────────────┘        └─────────────────────────────┘
```

Figure 47

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63041825 A
- US 62980073 A
- US 62938336 A
- WO 2011127099 A
- WO 2014210233 A
- WO 2014210225 A
- WO 2016162309 A
- WO 2018091676 A
- WO 2012140224 A
- WO 2014060483 A
- US 10002316 B
- US 9727810 B
- US 20170016053
- WO 2018045186 A
- WO 2016007839 A
- WO 2018045181 A
- WO 2014163886 A
- US 20180245142
- WO 2017144338 A
- WO 2018107054 A
- WO 2017222453 A
- WO 2019068880 A
- WO 2011094669 A
- US 7709198 B
- US 8604182 B
- US 8951726 B
- US 9783841 B
- US 10041949 B
- WO 2016057552 A
- WO 2017147483 A
- WO 2018022809 A
- WO 2016166128 A
- WO 2017027367 A
- WO 2017027368 A
- WO 2018136856 A
- WO 2019075091 A
- US 10059990 B
- WO 2018057999 A
- WO 2015161173 A
- US 99256920
- US 2017057269 W
- US 82574017 A
- US 20180156784 A
- WO 202020176788 A1
- US 202020176788 A1
- WO 2012168003 A
- US 7785869 B
- US 20180312822 A
- US 62946885 A
- US 7282328 B
- US 6867028 B
- US 6265552 B
- US 2020059472 W
- US 6391937 B
- US 9512422 B
- US 9889422 B
- US 20170253918 A
- US 20180052081 A
- US 6266459 B
- US 6355431 B
- US 6770441 B
- US 6859570 B
- US 6210891 B
- US 6258568 B
- US 6274320 B
- US 20090026082
- US 20090127589 A
- US 20100137143 A
- US 20100282617 A
- WO 00063437 A
- WO 2017019456 A
- WO 2005065814 A
- US 20080280773 A
- US 5919626 A
- US 6737236 B
- US 7259258 B
- US 7375234 B
- US 7427678 B
- US 5610287 A
- US 5807522 A
- US 5837860 A
- US 5472881 A
- US 20080280773
- US 20110059865 A
- US 99256920 A
- US 201314111482 A
- US 9593365 B2
- US 2019203275 A
- US 20180245142 A
- US 992569
- US 20110059865 A1
- US 62839346 A
- US 9012022 B
- US 99256919
- US 10138509 B
- US 10179932 B
- US 20190032121

- US 20070166705 A
- US 7566537 B
- US 7057026 B
- US 20060240439 A
- US 20060281109 A
- WO 05065814 A
- US 20050100900 A
- WO 06064199 A
- WO 07010251 A
- US 8951781 B2
- US 9193996 B
- US 9453258 B2
- US 7001792 B

- US 5599675 A
- US 5750341 A
- US 6969488 B
- US 6172218 B
- US 6306597 B
- US 8460865 B
- US 62909071 A
- US 62979889 A
- US 2020105373 A1
- US 98432418
- US 62886223 A
- US 2020049048 W
- US 63110749 A

## Non-patent literature cited in the description

- **SATIJA et al.** Spatial reconstruction of single-cell gene expression data. *Nature Biotechnology*, 2015, vol. 33, 495-502
- **ACHIM et al.** High-throughput spatial mapping of single-cell RNA-seq data to tissue of origin. *Nature Biotechnology*, 2015, vol. 33, 503-509
- **UCHIDA**. Image processing and recognition for biological images. *Develop. Growth Differ.*, 2013, vol. 55, 523-549
- **RODRIQUES et al.** *Science*, 2019, vol. 363 (6434), 1463-1467
- **LEE et al.** *Nat. Protoc.*, 2015, vol. 10 (3), 442-458
- **TREJO et al.** *PLoS ONE*, 2019, vol. 14 (2), e0212031
- **CHEN et al.** *Science*, 2015, vol. 348 (6233), 6090
- **GAO et al.** *BMC Biol.*, 2017, vol. 15, 50
- **GUPTA et al.** *Nature Biotechnol.*, 2018, vol. 36, 1197-1202
- **BOLOGNESI et al.** *J. Histochem. Cytochem.*, 2017, vol. 65 (8), 431-444
- **LIN et al.** *Nat Commun.*, 2015, vol. 6, 8390
- **PIRICI et al.** *J. Histochem. Cytochem.*, 2009, vol. 57, 567-75
- **GLASS et al.** *J. Histochem. Cytochem.*, 2009, vol. 57, 899-905
- **CHEN et al.** *Science*, 2015, vol. 347 (6221), 543-548
- **CHEN et al.** *Nat. Methods*, 2016, vol. 13, 679-684
- **ASANO et al.** *Current Protocols*, 2018, vol. 80 (1)
- **GAO et al.** *BMC Biology*, 2017, vol. 15, 50
- **WASSIE et al.** Expansion microscopy: principles and uses in biological research. *Nature Methods*, 2018, vol. 16 (1), 33-41
- **JAMUR et al.** *Method Mol. Biol.*, 2010, vol. 588, 63-66

- **LU et al.** *Lab Chip.*, January 2005, vol. 5 (1), 23-9
- **NIKLAS et al.** *Anal Biochem*, 2011, vol. 416 (2), 218-27
- **COX ; EMILI**. *Nat Protoc.*, 2006, vol. 1 (4), 1872-8
- **CHIANG et al.** *J Biochem. Biophys. Methods.*, 2000, vol. 20 (1-2), 53-68
- **YAMAUCHI ; HERR et al.** *Microsyst. Nanoeng.*, 2017, vol. 3, 16079
- **HAN et al.** *Microsyst Nanoeng.*, 2019, vol. 5, 30
- **GOULD et al.** *Oncotarget*, 2018, vol. 9 (21), 15606-15615
- **OREN ; SHAI**. *Biochemistry*, 1997, vol. 36 (7), 1826-35
- **ALGAYER et al.** *Molecules*, 2019, vol. 24 (11), E2079
- **HIPP et al.** *Leukemia*, 2017, vol. 10, 2278
- **ADICONIS**. Comparative analysis of RNA sequencing methods for degraded and low-input samples. *Nature*, 2013, vol. 10, 623-632
- **ARCHER et al.** Selective and flexible depletion of problematic sequences from RNA-seq libraries at the cDNA stage. *BMC Genomics*, 2014, vol. 15, 401
- **VANDERNOOT**. cDNA normalization by hydroxyapatite chromatography to enrich transcriptome diversity in RNA-seq applications. *Biotechniques*, 2012, vol. 53 (6), 373-80
- **GILL ; GHAEMI**. Nucleic acid isothermal amplification technologies: a review. *Nucleosides, Nucleotides, & Nucleic Acids*, 2008, vol. 27 (3), 224-43
- **VINCENT**. Helicase-dependent isothermal DNA amplification. *EMBO Rep.*, 2004, 795-800
- **ANDRESEN**. Helicase-dependent amplification: use in OnChip amplification and potential for point-of-care diagnostics. *Expert Rev Mol Diagn.*, 2009, vol. 9, 645-650

- **PIEPENBURG et al.** DNA Detection Using Recombinant Proteins. *PLoS Biol.*, 2006, vol. 4 (7), e204
- **LI**. Review: a comprehensive summary of a decade development of the recombinase polymerase amplification. *Analyst*, 2019, vol. 144, 31-67
- **GROKHOVSKY, S.L.** Specificity of DNA cleavage by ultrasound. *Molecular Biology*, 2006, vol. 40 (2), 276-283
- **MACOSKO et al.** *Cell*, 2015, vol. 161, 1202-1214
- **FANG et al.** *Nucleic Acids Res.*, 2003, vol. 31 (2), 708-715
- **LIU et al.** *Acc. Chem. Res.*, 2014, vol. 47 (1), 45-55
- **GOVAN et al.** *Nucleic Acids Research*, 2013, vol. 41 (22), 10518-10528
- **YERSHOV et al.** *Proc. Natl. Acad. Sci. USA*, 1996, vol. 93, 4913-4918
- **KOCH et al.** *Bioconjugate Chem.*, 2000, vol. 11, 474-483
- **HOLMSTRØM et al.** *Analytical Biochemistry*, 1993, vol. 209, 278-283
- **GILLES et al.** *Nature Biotechnology*, 1999, vol. 17, 365-370
- **SHALON et al.** *Genome Research*, 1996, 639-645
- **ROGERS et al.** *Analytical Biochemistry*, 1999, vol. 266, 23-30
- **STIMPSON et al.** *Proc. Natl. Acad. Sci. USA*, 1995, vol. 92, 6379-6383
- **BEATTIE et al.** *Clin. Chem.*, 1995, vol. 45, 700-706
- **LAMTURE et al.** *Nucleic Acids Research*, 1994, vol. 22, 2121-2125
- **BEIER et al.** *Nucleic Acids Research*, 1999, vol. 27, 1970-1977
- **JOOS et al.** *Analytical Biochemistry*, 1997, vol. 247, 96-101
- **NIKIFOROV et al.** *Analytical Biochemistry*, 1995, vol. 227, 201-209
- **TIMOFEEV et al.** *Nucleic Acids Research*, 1996, vol. 24, 3142-3148
- **CHRISEY et al.** *Nucleic Acids Research*, 1996, vol. 24, 3031-3039
- **GUO et al.** *Nucleic Acids Research*, 1994, vol. 22, 5456-5465
- **RUNNING** ; **URDEA**. *BioTechniques*, 1990, vol. 8, 276-279
- **FAHY et al.** *Nucleic Acids Research*, 1993, vol. 21, 1819-1826
- **ROGERS et al.** *Gene Therapy*, 1997, vol. 4, 1387-1392
- **MILLER et al.** Basic concepts of microarrays and potential applications in clinical microbiology. *Clinical Microbiology Reviews*, 2009, vol. 22 (4), 611-633
- **CARTER et al.** *Applied Optics*, 2007, vol. 46, 421-427
- Light Microscopy Method and Protocols. Methods in Molecular Biology. Humana Press, 2018
- Adaptive Optics for Biological Imaging. CRC Press, 2013
- Confocal and Two-Photon Microscopy: Foundations, Applications and Advances. Wiley Liss, 2002
- Handbook of Biological Confocal Microscopy. Springer Science+Business Media, LLC, 2002
- **DAY** ; **DAVIDSON**. The Fluorescent Protein Revolution (In Cellular and Clinical Imaging). CRC Press, 2014
- Quantitative Imaging in Cell Biology. Methods in Cell Biology. 2014, vol. 123
- Advanced Fluorescence Reporters in Chemistry and Biology II: Molecular Constructions. Polymers and Nanoparticles. Springer Series on Fluorescence, 2010
- Fluorescence Spectroscopy and Microscopy: Methods and Protocols (Methods in Molecular Biology). 2014
- **RONAGHI et al.** *Anal. Biochem.*, 1996, vol. 242 (1), 84-9
- **RONAGHI**. *Genome Res.*, 2001, vol. 11 (1), 3-11
- **RONAGHI et al.** *Science*, 1998, vol. 281 (5375), 363
- **MITRA et al.** *Anal. Biochem.*, 2003, vol. 320, 55-65
- **LEE et al.** *Science*, 2014, vol. 343 (6177), 1360-1363
- **WANG et al.** *Science*, 2018, vol. 361 (6499), 5691
- **MOFFITT**. *Methods in Enzymology*, 2016, vol. 572
- **DEAMER et al.** *Trends Biotechnol.*, vol. 18 (14), 7-151
- **DEAMER et al.** *Acc. Chem. Res.*, 2002, vol. 35, 817-825
- **LI et al.** *Nat. Mater.*, 2003, vol. 2, 611-615
- **SONI et al.** *Clin. Chem.*, 2007, vol. 53, 1996-2001
- **HEALY et al.** *Nanomed.*, 2007, vol. 2, 459-481
- **COCKROFT et al.** *J. Am. Chem. Soc.*, 2008, vol. 130, 818-820
- **SHENDURE et al.** *Science*, 2005, vol. 309, 1728-1732
- **GUNDERSON et al.** *Genome Research*, 2004, vol. 14, 870-877
- **LEVENE et al.** *Science*, 2003, vol. 299, 682-686
- **LUNDQUIST et al.** *Opt. Lett.*, 2008, vol. 33, 1026-1028
- **KORLACH et al.** *Proc. Natl. Acad. Sci. USA*, 2008, vol. 105, 1176-1181

- **SEZGIN** ; **SANKUR**. Survey over image thresholding techniques and quantitative performance evaluation. *Journal of Electronic Imaging*, 2004, vol. 13 (1), 146-165
- **ZHANG**. Optimal multi-level Thresholding based on Maximum Tsallis Entropy via an Artificial Bee Colony Approach. *Entropy*, 2011, vol. 13 (4), 841-859
- **DUDA** ; **HART**. Pattern Classification and Scene Analysis. John Wiley & Sons, Inc., 1973
- **DUDA et al.** Pattern Classification. John Wiley & Sons, Inc.
- **KAUFMAN** ; **ROUSSEEUW**. Finding Groups in Data: An Introduction to Cluster Analysis. Wiley, 1990
- **EVERITT**. Cluster analysis. Wiley, 1993
- **BACKER**. Computer-Assisted Reasoning. *Cluster Analysis*, 1995
- **PETRICK et al.** Proceedings of the 7th International Conference, Smolenice. *Measurement*, 2009, 352-355
- **ANDREW**. Another efficient algorithm for convex hulls in two dimensions. *Information Processing Letters*, 1979, vol. 9 (5), 216-219
- **BROWN**. Voronoi diagrams from convex hulls. *Information Processing Letters*, 1979, vol. 9 (5), 223-228
- Emerging Technology in Modeling and Graphics: Processing of IEM Graph. 2018
- Emerging Technology in Modeling and Graphics: Processing of IEM Graph. Springer Singapore, 2018
- **ZHAI**. Making GenePix Array List (GAL) Files. *GenePix Application Note, Molecular Devices*, 2001, 1-9
- **MYRONENKO et al.** Non-rigid point set registration: Coherent Point Drift. *NIPS*, 1009-1016
- **MYRONENKO** ; **SONG**. Point Set Registration: Coherent Point Drift. *arXiv:0905.2635v1*, 15 May 2009
- **CHETVERIKOV et al.** the Trimmed Iterative Closest Point Algorithm. *Object recognition supported by user interaction for service robots*, 2002
- **CHETVERIKOV et al.** Robust Euclidean alignment of 3D point sets; the trimmed iterative closest point algorithm. *Image and Vision Computing*, 2005, vol. 23 (3), 299-309
- **CHUI** ; **RANGARAJANB**. A new point matching algorithm for non-rigid registration. *Computer Vision and Image Understanding*, 2003, vol. 89 (2-3), 114-141
- **YANG**. The thin plate spline robust point matching (TPS-RPM) algorithm: A revisit. *Pattern Recognition Letters*, 2011, vol. 32 (7), 910-918
- **CANNY**. A Computational Approach to Edge Detection. *IEEE Trans Pattern Anal Mach Intell.*, 1986, vol. 8 (6), 679-98
- **ROTHER et al.** GrabCut' - Interactive Foreground Extraction using Iterated Graph Cuts. *ACM Transactions on Graphics.*, 2004, vol. 23 (3), 309-314
- **BOYKOV** ; **JOLLY**. Interactive graph cuts for optimal boundary and region segmentation of objects in N-D images. *Proc. IEEE Int. Conf. on Computer Vision, CD-ROM*, 2001
- **GREIG et al.** Exact MAP estimation for binary images. *J. Roy. Stat. Soc. B.*, 1989, vol. 51, 271-279
- **CHUANG et al.** A Bayesian approach to digital matting. *Proc. IEEE Conf. Computer Vision and Pattern Recog.*, 2001
- *Science*, 01 July 2016, vol. 353 (6294), 78-82